(19) Europäisches Patentamt
European Patent Office
Office européen des brevets

(11) EP 4 183 395 A1

(12) EUROPEAN PATENT APPLICATION
published in accordance with Art. 153(4) EPC

(43) Date of publication:
**24.05.2023 Bulletin 2023/21**

(21) Application number: **21841448.0**

(22) Date of filing: **14.07.2021**

(51) International Patent Classification (IPC):
***A61K 31/519*** *(2006.01)* ***A61P 35/00*** *(2006.01)*
***A61P 43/00*** *(2006.01)* ***C12N 15/113*** *(2010.01)*

(52) Cooperative Patent Classification (CPC):
**A61K 31/519; A61P 35/00; A61P 43/00; C12N 15/113**

(86) International application number:
**PCT/JP2021/026462**

(87) International publication number:
**WO 2022/014640 (20.01.2022 Gazette 2022/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.07.2020 JP 2020121733**

(71) Applicant: **Taiho Pharmaceutical Co., Ltd.**
**Chiyoda-ku, Tokyo 101-8444 (JP)**

(72) Inventors:
- **OGUCHI Kei**
  **Tsukuba-shi, Ibaraki 300-2611 (JP)**
- **SHIKATA Yuki**
  **Tsukuba-shi, Ibaraki 300-2611 (JP)**
- **IWASAKI Junya**
  **Tsukuba-shi, Ibaraki 300-2611 (JP)**

(74) Representative: **Blodig, Wolfgang**
**Wächtershäuser & Hartz**
**Patentanwaltspartnerschaft mbB**
**Weinstrasse 8**
**80333 München (DE)**

(54) **PYRIMIDINE COMPOUND-CONTAINING COMBINATION TO BE USED IN TUMOR TREATMENT**

(57) Provided is a tumor treatment method using a pyrimidine compound or a salt thereof. An antitumor agent that contains a pyrimidine compound represented by general formula (I) or a salt thereof, said antitumor agent being to be administered in combination with another antitumor agent. (In the formula, $R_1$ represents a C1-C4 alkyl group optionally having a C1-C4 alkoxy group as a substituent, or a C3-C4 cycloalkyl group; $R_2$ represents a hydrogen atom, a halogen atom, a C1-C6 alkyl group optionally having, as a substituent(s), one to five C1-C4 alkoxy groups or one to five fluorine atoms, or a C1-C6 alkoxy group; $R_3$ represents a hydrogen atom, or a C1-C4 alkyl group optionally having, as a substituent(s), one to five fluorine atoms; $R_4$ represents a hydrogen atom or a C1-C4 alkyl group; and $R_5$ represents a phenyl group optionally having one to three substituents selected from fluorine atoms and chlorine atoms.)

EP 4 183 395 A1

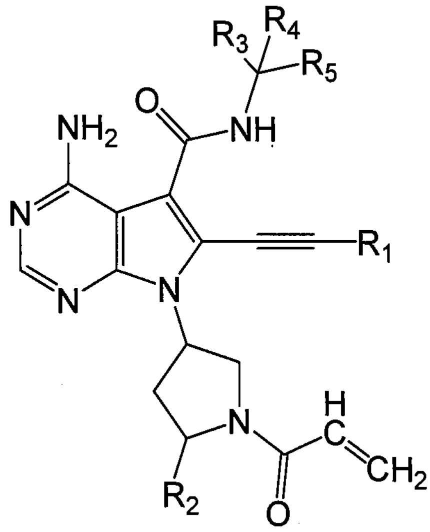

(I)

## Description

Technical Field

The present invention relates to a combination of a pyrimidine compound or a salt thereof and other antitumor agent for use in the treatment of tumor, and an antitumor effect potentiator for other antitumor agent.

Background Art

HER2 (which is also referred to as "ErbB2") is receptor tyrosine kinase belonging to the ErbB family.

HER2 is considered to be a proto-oncogene. It has been reported that HER2 gene amplification, overexpression, mutation and the like occur in various types of cancers. From non-clinical and clinical research data, it is considered that an activation of HER2 and downstream signals plays an important role in the survival and/or proliferation, etc. of cancer cells associated with the genetic abnormality, overexpression and the like of HER2 (Non Patent Literature 1).

Accordingly, an inhibitor capable of regulating the kinase activity of HER2 is assumed to inhibit HER2 and downstream signals in cancer cells having HER2 gene amplification, overexpression or mutation, so as to exhibit antitumor effects on the cancer cells. Therefore, such an inhibitor is considered to be useful for the treatment, life-prolonging, or QOL improvement of cancer patients.

It has been reported that brain metastasis occurs in approximately 25 to 40% of lung cancer cases, in approximately 15 to 30% of breast cancer cases, and in certain percentages of other multiple cancer cases (Non Patent Literatures 2 and 3). As a matter of fact, it has been reported that brain metastasis occurs in approximately 20 to 30% of HER2-positive breast cancer cases (Non Patent Literature 4).

Compounds having HER2 inhibitory activity, such as Lapatinib and Neratinib, have been approved as therapeutic agents against HER2-positive breast cancer. However, it has been reported that since all of these therapeutic agents are substrates of p-gp or Bcrp, the brain penetration properties of these agents are limited in non-clinical tests (Non Patent Literature 5). In fact, in clinical tests using Lapatinib or Neratinib, sufficient effects of these agents could not be obtained against brain metastatic cancer (Non Patent Literatures 6, 7, 8, and 9).

From the viewpoint of the control of pathological conditions including brain metastasis nidus, it has been desired to develop a HER2 inhibitor having inhibitory activity against HER2 and also having brain penetration properties.

One of HER2 mutations, HER2ex20ins mutation has been reported to be an activating mutation in lung cancer, etc. (Non Patent Literature 10), and regarding such HER2ex20ins mutation, multiple clinical trials have been carried out. However, under the current circumstances, a therapeutic method therefor has not yet been established. Therefore, it has been desired to develop a HER2 inhibitor having inhibitory activity against HER2ex20ins mutation.

There are many reports on antitumor effects brought about by combinations of HER2 inhibitors and other antitumor agents. For example, combined use of trastuzumab with paclitaxel (Non Patent Literature 11), paclitaxel/platinum compound (Non Patent Literature 12), or the like has been reported. Also, combined use of lapatinib with capecitabine (Non Patent Literature 13), 5-FU (Non Patent Literature 14), S-1 (Non Patent Literature 15), or the like has been reported.

EGFR (which is also referred to as "ErbB1") is a receptor tyrosine kinase belonging to the ErbB family and has been reported to contribute to cell growth or apoptosis inhibition by binding to epidermal growth factor (which is also referred to as "EGF"), mainly in epithelial tissues, among normal tissues (Non Patent Literature 16).

EGFR is considered to be a proto-oncogene. It has been reported that EGFR gene amplification, overexpression, mutation and the like occur in various types of cancers. From non-clinical and clinical research data, it is considered that an activation of EGFR and downstream signals plays an important role in the survival and/or proliferation, etc. of cancer cells associated with the genetic abnormality, overexpression and the like of EGFR. For example, a mutation in exon 19 region of EGFR, which deletes amino acids at positions 746 to 750 (which is also referred to as "exon 19 deletion mutation"), and a mutation in exon 21 region, which substitutes an amino acid leucine at position 858 with arginine (which is also referred to as "L858R mutation") are considered to contribute to the survival and/or proliferation of cancer cells by autophosphorylating EGFR in an EGF-independent manner and thereby inducing kinase activity (Non Patent Literature 17). It has been reported that these mutations are present in approximately 30 to 50% of non-small cell lung cancer cases in East Asia and approximately 10% of non-small cell lung cancer cases in Europe and the United States (Non Patent Literature 18).

Accordingly, an inhibitor capable of regulating the kinase activity of EGFR is assumed to inhibit EGFR and downstream signals in cancer cells having EGFR gene amplification, overexpression and/or mutation, so as to exhibit antitumor effects on the cancer cells. Therefore, such an inhibitor is considered to be useful for the treatment, life-prolonging, or QOL improvement of cancer patients.

Hence, a plurality of EGFR inhibitors have heretofore been researched and developed as anticancer agents and are used in the treatment of EGFR mutation-positive tumor. For example, drugs such as afatinib, gefitinib, and

erlotinib have been approved as therapeutic agents for EGFR mutation-positive lung cancer having exon 19 deletion mutation or L858R mutation. Also, osimertinib has been approved as a therapeutic agent for EGFR mutation-positive lung cancer having exon 19 deletion mutation or L858R mutation as well as a mutation in exon 20 region, which substitutes an amino acid threonine at position 790 with methionine (which is also referred to as "T790M mutation").

A mutation in exon 20 region, which insets one or more amino acids (which is also referred to as "exon 20 insertion mutation") is also considered as an activating mutation in lung cancer and the like (Non Patent Literature 19). It has been reported that cancer having such a mutation tends to be low sensitive to a plurality of existing EGFR inhibitors. For example, as for the clinical effect of afatinib on EGFR mutation-positive lung cancer, it has been reported that its effect on exon 20 insertion mutation tends to be much lower than that on exon 19 deletion mutation or L858R mutation (Non Patent Literature 20). Regarding lung cancer having exon 20 insertion mutation of EGFR, multiple clinical trials have been carried out. However, under the current circumstances, a therapeutic method therefor has not yet been established. Therefore, it has been desired to develop an EGFR inhibitor having inhibitory activity against exon 20 insertion mutation.

As most of EGFR mutations have been reported on lung cancer, it has been reported that brain metastasis occurs in approximately 25 to 40% of the cases.

From the viewpoint of the control of pathological conditions including brain metastasis nidus, it has been desired to develop an EGFR inhibitor having inhibitory activity against EGFR mutations and also having brain penetration properties.

Antitumor effects brought about by combinations of EGFR inhibitors and other antitumor agents have also been reported. For example, a combination of an EGFR inhibitor erlotinib with CDDP has been found to have a combinatorial effect (Non Patent Literature 21). A combination of erlotinib and RAD001 has also been reported to have a combinatorial effect (Non Patent Literature 22).

Citation List

Patent Literature

Patent Literature 1: International Publication No. WO 2017/146116
Patent Literature 2: International Publication No. WO 2017/038838

Non Patent Literature

Non Patent Literature 1: Cancer Treatment Reviews, 40, pp. 770-780 (2014)
Non Patent Literature 2: Current Oncology, 25, pp. S103-S114 (2018)
Non Patent Literature 3: Breast Cancer Research, 18(1), 8, pp. 1-9 (2016)
Non Patent Literature 4: Journal of Clinical Oncology, 28, pp. 3271-3277 (2010)
Non Patent Literature 5: Journal of Medicinal Chemistry, 59, pp. 10030-10066 (2016)
Non Patent Literature 6: Journal of Medicinal Chemistry, 26, pp. 2999-3005 (2008)
Non Patent Literature 7: Journal of Clinical Oncology, 26, pp. 1993-1999 (2008)
Non Patent Literature 8: Journal of Clinical Oncology, 28, pp. 1301-1307 (2010)
Non Patent Literature 9: Journal of Clinical Oncology, 34, pp. 945-952 (2016)
Non Patent Literature 10: Proc Natl Acad Sci USA., 106, pp. 474-479 (2009)
Non Patent Literature 11: N. Engl. J. Med., vol. 344, pp. 783-792 (2001)
Non Patent Literature 12: J. Natl. Cancer Inst., 96, pp. 739-749 (2004)
Non Patent Literature 13: Biologics., vol. 2, pp. 61-65 (2008)
Non Patent Literature 14: Oncol. Rep., vol. 27, pp. 1639-1645 (2012)
Non Patent Literature 15: Mol. Cancer Ther., vol. 9, pp. 1198-1207 (2010)
Non Patent Literature 16: Nat. Rev. Cancer., vol. 6, pp. 803-812 (2006)
Non Patent Literature 17: Nature Medicine., vol. 19, pp. 1389-1400 (2013)
Non Patent Literature 18: Nat. Rev. Cancer., vol. 7, pp. 169-181 (2007)
Non Patent Literature 19: Signal Transduct Target Ther., vol. 4: 5, pp. 1-10 (2019)
Non Patent Literature 20: Lancet Oncol. vol. 16, pp. 830-838 (2015)
Non Patent Literature 21: Neoplasia. vol. 17, pp. 190-200 (2015)
Non Patent Literature 22: Clin Cancer Res., vol. 13, pp. S4628-4631 (2007)

Summary of Invention

An object of the present invention is to provide a method for treating tumor using a pyrimidine compound or a salt thereof and other antitumor agent.

Specifically, one aspect of the present invention provides the following [1] to [17]:

[1] An antitumor agent for combined administration with other antitumor agent, the antitumor agent comprising a pyrimidine compound represented by the following formula (I), or a salt thereof:

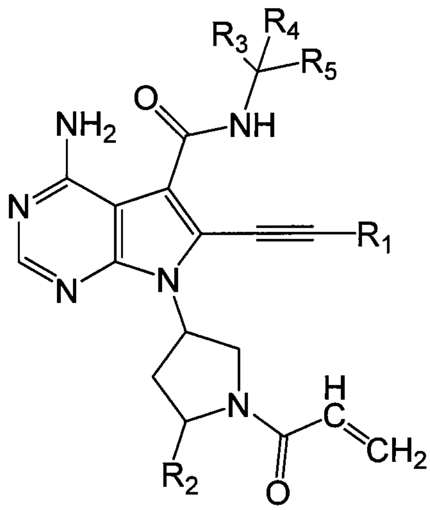

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

[2] The antitumor agent according to [1], wherein the pyrimidine compound is a compound represented by the following formula (II):

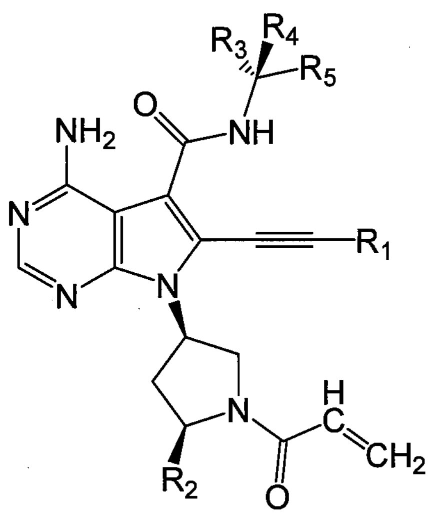

(II)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from the group consisting of fluorine atoms and chlorine atoms.

[3] The antitumor agent according to [1] or [2], wherein the pyrimidine compound is represented by the formula (I) or (II) wherein

$R_1$ is a methyl group, an ethyl group, an isopropyl group, a tert-butyl group, a 1-methyl-1-methoxyethyl group, or a cyclopropyl group;
$R_2$ is a methyl group, an ethyl group, a methoxymethyl group, or an ethoxymethyl group;
$R_3$ is a methyl group, an ethyl group, or a trifluoromethyl group;
$R_4$ is a hydrogen atom or a methyl group; and
$R_5$ is a phenyl group, a 2-fluorophenyl group, a 3-chlorophenyl group, a 2,3-difluorophenyl group, a 2,4-difluorophenyl group, or a 3,5-difluorophenyl group.

[4] The antitumor agent according to any of [1] to [3], wherein the pyrimidine compound is represented by the formula (I) or (II) wherein

$R_1$ is a methyl group, a tert-butyl group, or a cyclopropyl group;
$R_2$ is a methyl group, an ethyl group, or a methoxymethyl group;
$R_3$ is a methyl group;
$R_4$ is a hydrogen atom; and
$R_5$ is a phenyl group.

[5] The antitumor agent according to any of [1] to [4], wherein the pyrimidine compound is a compound selected from the following (1) to (3):

(1) 7-((3R,SS)-1-acryloyl-5-methylpyrrolidin-3-yl)-4-amino-N-((R)-1-phenylethyl)-6-(prop-1-yn-1-yl)-7H-pyrrolo[2,3-d]pyrimidine-5-carboxamide,
(2) 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide, and
(3) 7-((3R,5S)-1-acryloyl-5-methylpyrrolidin-3-yl)-4-amino-6-(3,3-dimethylbut-1-yn-1-yl)-N-((R)-1-phenylethyl)-7H-pyrrolo[2,3-d]pyrimidine-5-carboxamide.

[6] The antitumor agent according to any of [1] to [5], wherein the other antitumor agent is at least one agent selected from antimetabolites, molecular targeting drugs, platinum drugs and alkaloid drugs.
[7] The antitumor agent according to any of [1] to [6], wherein the other antitumor agent is at least one agent selected from 5-fluorouracil (5-FU), trifluridine, gemcitabine, capecitabine, trastuzumab, pertuzumab, trastuzumab emtansine, AZD8055, everolimus, dactolisib, buparlisib, taselisib, palbociclib, fulvestrant, cisplatin and paclitaxel.
[8] The antitumor agent according to any of [1] to [7] for the treatment of tumor.
[9] The antitumor agent according to any of [1] to [8], wherein the pyrimidine compound or the salt thereof is administered to a tumor patient given the other antitumor agent or a tumor patient to be given the other antitumor agent.
[10] The antitumor agent according to any of [1] to [9] for oral administration.
[11] A pharmaceutical combination of a pyrimidine compound or a salt thereof and other antitumor agent, wherein

the pyrimidine compound is a compound represented by the following formula (I):

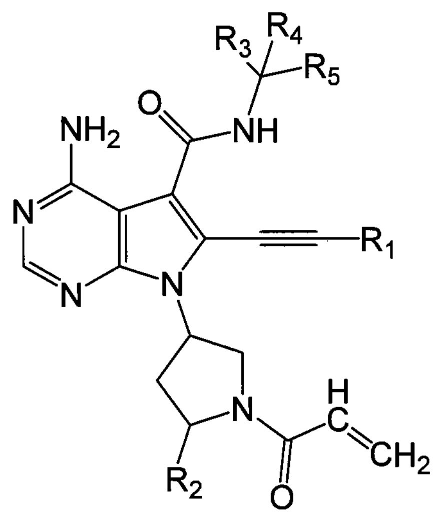

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

[12] The pharmaceutical combination according to [11] for use in the treatment of tumor, wherein the pyrimidine compound or the salt thereof and the other antitumor agent are administered concurrently, sequentially, or at an interval.
[12a] The pharmaceutical combination according to [11] or [12], wherein the pyrimidine compound is a compound according to any of [2] to [5].
[12b] The pharmaceutical combination according to [11] or [12], wherein the other antitumor agent is one described in [6] or [7].
[12c] The pharmaceutical combination according to [11], [12], [12a] or [12b], wherein the pyrimidine compound or the salt thereof is orally administered.
[13] A kit preparation comprising an antitumor agent according to any of [1] to [10] or a pharmaceutical combination according to [11] or [12], and an instruction stating that the pyrimidine compound or the salt thereof and the other antitumor agent are combined-administered.
[14] Use of a pyrimidine compound or a salt thereof for the production of a medicament that is used in combination with other antitumor agent in the treatment of tumor, wherein

the pyrimidine compound is a compound represented by the following formula (I):

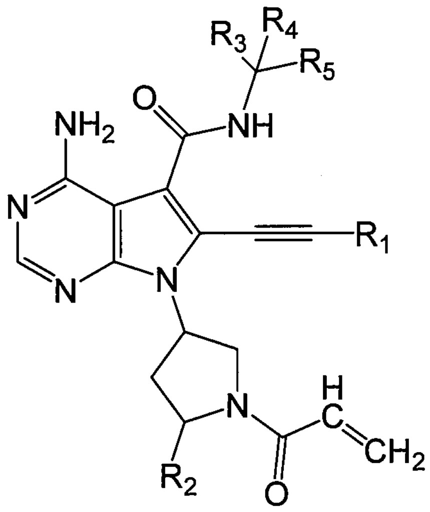

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

[14a] The use of a pyrimidine compound or a salt thereof according to [14], wherein the pyrimidine compound is a compound according to any of [2] to [5].
[14b] The use of a pyrimidine compound or a salt thereof according to [14] or [14a], wherein the other antitumor agent is one described in [6] or [7].
[14c] The use of a pyrimidine compound or a salt thereof according to [14], [14a] or [14b], wherein the pyrimidine compound or the salt thereof is orally administered.
[15] An antitumor effect potentiator for potentiating the antitumor effect of other antitumor agent, the antitumor effect potentiator comprising a pyrimidine compound represented by the following formula (I), or a salt thereof:

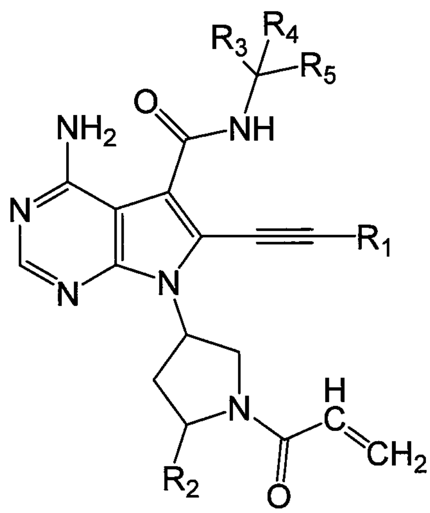

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

[16] A method for treating tumor, comprising administering an effective amount of an antitumor agent according to any of [1] to [10] and other antitumor agent or a pharmaceutical combination according to [11] or [12] to a patient in need thereof.
[17] A method for treating tumor by combined use with other antitumor agent, comprising administering an effective amount of a pyrimidine compound or a salt thereof to a patient in need thereof, wherein the pyrimidine compound is a compound represented by the following formula (I):

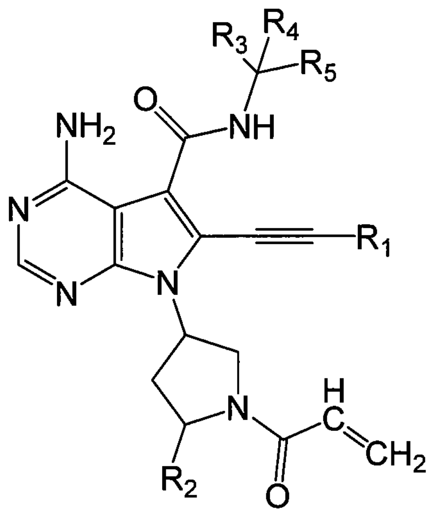

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

[18] A pyrimidine compound or a salt thereof for use in a method for treating tumor by combined use with other antitumor agent, wherein the pyrimidine compound is a compound represented by the following formula (I):

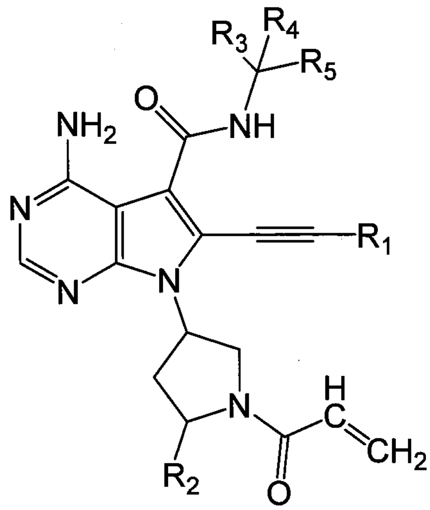

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

[19] Use of a pyrimidine compound or a salt thereof for treating tumor by combined use with other antitumor agent, wherein the pyrimidine compound is a compound represented by the following formula (I):

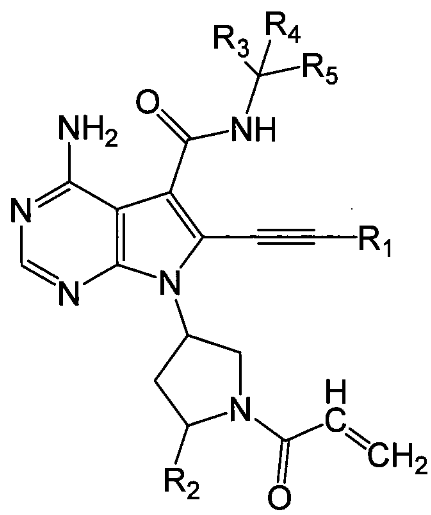

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

[20] The use according to [14] or [19], the antitumor effect potentiator according to [15], the method according to [17], or the pyrimidine compound of the salt thereof according to [18], wherein the pyrimidine compound is a compound according to any of [2] to [5].

[21] The use according to [14], [19], or [20], the antitumor effect potentiator according to [15] or [20], the method according to [17] or [20], or the pyrimidine compound of the salt thereof according to [18] or [20], wherein the other antitumor agent is one described in [6] or [7].

[22] The use according to [14], [19], [20] or [21], the antitumor effect potentiator according to [15], [20] or [21], the method according to [17] , [20] or [21], or the pyrimidine compound of the salt thereof according to [18] , [20] or [21], wherein the pyrimidine compound or the salt thereof is orally administered.

The present invention also relates to the following aspects:
A pharmaceutical composition for the treatment of tumor, comprising a pyrimidine compound represented by the formula (I) or (II) or a salt thereof and other antitumor agent.

A pyrimidine compound represented by the formula (I) or (II) or a salt thereof for use in the treatment of tumor involving combined use with other antitumor agent.

Use of a pyrimidine compound represented by the formula (I) or (II) or a salt thereof for the treatment of tumor involving combined use with other antitumor agent.

Other antitumor agent for use in the treatment of tumor involving combined use with a pyrimidine compound represented by the formula (I) or (II) or a salt thereof.

Use of other antitumor agent for the treatment of tumor involving combined use with a pyrimidine compound represented by the formula (I) or (II) or a salt thereof.

Use of other antitumor agent for the production of a medicament that is used in combination with a pyrimidine compound or a salt thereof in the treatment of tumor.

Use of a combination of a pyrimidine compound or a salt thereof and other antitumor agent for the production of a medicament in the treatment of tumor.

A pyrimidine compound represented by the formula (I) or (II) or a salt thereof for potentiating the antitumor effect of other antitumor agent.

Use of a pyrimidine compound represented by the formula (I) or (II) or a salt thereof for potentiating the antitumor effect of other antitumor agent.

Other antitumor agent for potentiating the antitumor effect of a pyrimidine compound represented by the formula (I) or (II) or a salt thereof.

Use of other antitumor agent for potentiating the antitumor effect of a pyrimidine compound represented by the formula (I) or (II) or a salt thereof.

Use of a pyrimidine compound represented by the formula (I) or (II) or a salt thereof for the production of an antitumor effect potentiator of other antitumor agent.

Use of other antitumor agent for the production of an antitumor effect potentiator of a pyrimidine compound represented by the formula (I) or (II) or a salt thereof.

A method for treating tumor, comprising the step of administering a therapeutically effective amount of a pyrimidine compound represented by the formula (I) or (II) or a salt thereof and other antitumor agent in combination to a patient.

A method for treating tumor, comprising the step of administering a therapeutically effective amount of a pyrimidine compound represented by the formula (I) or (II) or a salt thereof to a tumor patient given other antitumor agent.

A method for treating tumor, comprising the step of administering a therapeutically effective amount of a pyrimidine compound represented by the formula (I) or (II) or a salt thereof to a tumor patient to be given (scheduled to be given) other antitumor agent.

A method for treating tumor or a method for potentiating an antitumor effect, comprising the step of administering a therapeutically effective amount of other antitumor agent to a tumor patient given a pyrimidine compound represented by the formula (I) or (II) or a salt thereof.

A method for treating tumor or a method for potentiating an antitumor effect, comprising the step of administering a therapeutically effective amount of other antitumor agent to a tumor patient to be given (scheduled to be given) a pyrimidine compound represented by the formula (I) or (II) or a salt thereof.

A method for potentiating an antitumor effect, comprising the step of administering a therapeutically effective amount of a pyrimidine compound represented by the formula (I) or (II) or a salt thereof to a tumor patient given other antitumor agent.

A product comprising a pyrimidine compound represented by the formula (I) or (II) or a salt thereof and other antitumor agent as a combination product for concurrent, sequential, or staggered use in treating tumor.

A combination of a pyrimidine compound represented by the formula (I) or (II) or a salt thereof and other antitumor agent for concurrent, sequential, or staggered use in the treatment of tumor.

In one embodiment of the aspects mentioned above, the tumor is malignant tumor having HER2 overexpression, HER2 gene amplification, or a HER2 mutation, and the other antitumor agent is at least one agent selected from antimetabolites, Her2 inhibitors, PI3K/AKT/mTOR inhibitors, CDK4/6 inhibitors, estrogen receptor antagonists, platinum drugs, and vegetable alkaloid drugs.

In one embodiment of the aspects mentioned above, the tumor is malignant tumor having EGFR overexpression, EGFR gene amplification, or an EGFR mutation, and the other antitumor agent is at least one agent selected from antimetabolites, PI3K/AKT/mTOR inhibitors, CDK4/6 inhibitors, estrogen receptor antagonists, platinum drugs, and vegetable alkaloid drugs.

In one embodiment of the aspects mentioned above, the tumor is HER2-positive tumor, and the other antitumor agent is at least one agent selected from antimetabolites, Her2 inhibitors, PI3K/AKT/mTOR inhibitors, CDK4/6 inhibitors, estrogen receptor antagonists, platinum drugs, and vegetable alkaloid drugs.

In one embodiment of the aspects mentioned above, the tumor is EGFR-positive tumor, and the other antitumor agent is at least one agent selected from antimetabolites, PI3K/AKT/mTOR inhibitors, CDK4/6 inhibitors, estrogen receptor antagonists, platinum drugs, and vegetable alkaloid drugs.

The present invention provides a novel method for treating tumor using a pyrimidine compound or a salt thereof and other antitumor agent.

The antitumor agent of the present invention is capable of performing the treatment of tumor that exerts a high antitumor effect (particularly, a cytoreductive effect and a tumor growth delaying effect (life extending effect)) while preventing the development of side effects.

## Brief Description of Drawings

[Figure 1] Figure 1 shows the antitumor effects of the compound of Example 2 against models involving direct brain transplantation of the Luciferase gene-introduced HER2 expressing cell line (NCI-N87-luc).

[Figure 2] Figure 2 shows the antitumor effects of the compound of Example 11 against models involving direct brain transplantation of the Luciferase gene-introduced HER2 expressing cell line (NCI-N87-luc).

[Figure 3] Figure 3 shows the antitumor effects of the compound of Example 12 against models involving direct brain transplantation of the Luciferase gene-introduced HER2 expressing cell line (NCI-N87-luc).

[Figure 4] Figure 4 shows the body weight reduction percentage of models involving direct brain transplantation of the Luciferase gene-introduced HER2 expressing cell line (NCI-N87-luc) caused by the compound of Example 2.

[Figure 5] Figure 5 shows the body weight reduction percentage of models involving direct brain transplantation of the Luciferase gene-introduced HER2 expressing cell line (NCI-N87-luc) caused by the compound of Example 11.

[Figure 6] Figure 6 shows the body weight reduction percentage of models involving direct brain transplantation of the Luciferase gene-introduced HER2 expressing cell line (NCI-N87-luc) caused by the compound of Example 12.

[Figure 7] Figure 7 shows the antitumor effects of compounds of Example 2, 11 and 12 against models involving subcutaneous transplantation of the H1975-EGFRinsSVD cell line.

[Figure 8] Figure 8 shows the body weight change percentage of mice when the compounds of Examples 2, 11 and 12 were administered to the models involving subcutaneous transplantation of the H1975-EGFRinsSVD cell line.

[Figure 9] Figure 9 shows the antitumor effects of the compound of Example 11 against models involving direct brain transplantation of the Luciferase gene-introduced exon 20 insertion mutant EGFR expressing cell line (H1975-EGFRinsSVD-Luc).

[Figure 10] Figure 10 shows the survival rate of mice when the compound of Example 11 was administered to the models involving direct brain transplantation of the Luciferase gene-introduced exon 20 insertion mutant EGFR expressing cell line (H1975-EGFRinsSVD-Luc).

[Figure 11] Figure 11 shows the therapeutic effect of a combination of a compound of Example 11 and trastuzumab on human stomach cancer-derived tumor cells.

[Figure 12] Figure 12 shows the therapeutic effect of a combination of the compound of Example 11, trastuzumab (T-mab), and pertuzumab (P-mab) on human stomach cancer-derived tumor cells.

[Figure 13] Figure 13 shows the therapeutic effect of a combination of the compound of Example 11 and trastuzumab emtansine on human stomach cancer-derived tumor cells.

[Figure 14] Figure 14 shows the therapeutic effect of a combination of the compound of Example 11 and capecitabine on human stomach cancer-derived tumor cells.

[Figure 15] Figure 15 shows the therapeutic effect of a combination of the compound of Example 11 and capecitabine on human stomach cancer-derived tumor cells.

Description of Embodiments

One embodiment of the present invention relates to a combination of a pyrimidine compound represented by the formula (I) having pyrimidine as a basic skeleton, or a salt thereof and another compound having an antitumor effect (other antitumor agent). Combined use of the pyrimidine compound or the salt thereof and the other antitumor agent is capable of potentiating an antitumor effect as compared with use of each alone and is capable of preventing the enhancement of toxicity and the development of side effects. Thus, the combination is capable of performing the treatment of tumor that exerts an excellent antitumor effect while preventing the development of side effects.

In the present invention, a compound that brings about an excellent synergistic effect when used in combination with other antitumor agent is a pyrimidine compound represented by the following formula (I), or a salt thereof:

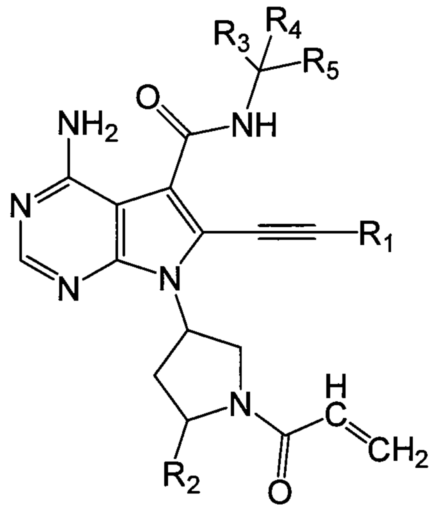

(I)

wherein $R_1$ to $R_5$ are as defined above.

In one preferred embodiment of the present invention, a compound that brings about an excellent synergistic effect when used in combination with other antitumor agent is a compound represented by the following formula (II), or a salt thereof:

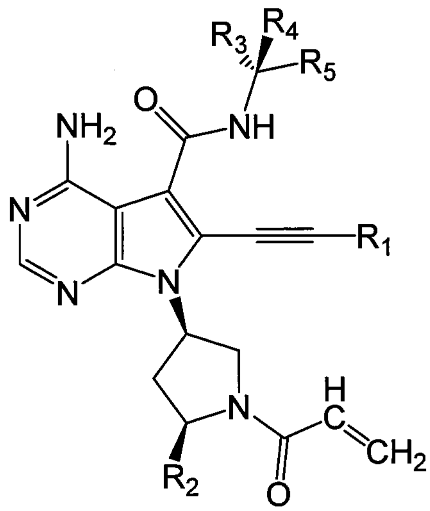

(II)

wherein $R_1$ to $R_5$ are as defined above.

The compound represented by the above formula (I) or formula (II) of the present invention is a compound having pyrrolo[2,3-d]pyrimidine as a basic structure, and this is a novel compound described in none of the aforementioned prior art publications, etc. Hereinafter, the compound represented by the above formula (I) or formula (II) mentioned above is also referred to as "the pyrimidine compound of the present invention or a salt thereof".

In the present description, specific examples of the "halogen atom" may include a chlorine atom, a bromine

atom, a fluorine atom, and an iodine atom. Among these, a chlorine atom and a fluorine atom are preferable, and a fluorine atom is more preferable.

In the present description, the "alkyl group" means a linear or branched saturated hydrocarbon group. Specific examples of the alkyl group may include a methyl group, an ethyl group, an n-propyl group, an isopropyl group, an n-butyl group, an isobutyl group, a sec-butyl group, a tert-butyl group, a pentyl group, and a hexyl group. Among these, a linear or branched alkyl group containing 1 to 4 carbon atoms is preferable, and a methyl group and a tert-butyl group are more preferable.

In the present description, the "haloalkyl group" means a linear or branched saturated hydrocarbon group, in which one to all hydrogen atoms are substituted with the above-described halogen atoms. Specific examples of the haloalkyl group may include a monofluoromethyl group, a difluoromethyl group, a trifluoromethyl group, a 1-fluoroethyl group, a 2-fluoroethyl group, a 1,1-difluoroethyl group, a 1,2-difluoroethyl group, a 2,2-difluoroethyl group, a 2,2,2-trifluoroethyl group, a monochloromethyl group, a dichloromethyl group, a trichloromethyl group, a 1-chloroethyl group, a 2-chloroethyl group, and a 1,1-dichloroethyl group. Among these, a linear or branched saturated hydrocarbon group containing 1 to 6 carbon atoms, in which 1 to 3 hydrogen atoms are substituted with the above-described halogen atoms, is preferable, and a monofluoromethyl group is more preferable.

In the present description, the "cycloalkyl group" means a monocyclic or polycyclic saturated hydrocarbon group containing 3 to 7 carbon atoms. Specific examples of the cycloalkyl group may include a cyclopropyl group, a cyclobutyl group, a cyclopentyl group, a cyclohexyl group, and a cycloheptyl group. Among these, a cyclopropyl group and a cyclobutyl group are preferable.

In the present description, the "aromatic hydrocarbon group" means a cyclic substituent consisting of carbon and hydrogen, having an unsaturated bond, in which 4e + 2 (wherein e represents an integer of 1 or greater) electrons are contained in the cyclic $\pi$ electron system.

In the present description, the "C6-C14 aromatic hydrocarbon group" means a monocyclic or polycyclic aromatic hydrocarbon group containing 6 to 14 carbon atoms. Specific examples of the C6-C14 aromatic hydrocarbon group may include a phenyl group, a naphthyl group, a tetrahydronaphthyl group, and an anthracenyl group. Among these, a phenyl group is preferable.

In the present description, the "aralkyl group" means the above-described alkyl group substituted with the above-described aromatic hydrocarbon group. Specific examples of the aralkyl group may include C7-C16 aralkyl groups such as a benzyl group, a phenylethyl group, a phenylpropyl group, a naphthylmethyl group, and a naphthylethyl group. Among these, a benzyl group is preferable.

In the present description, the "unsaturated hydrocarbon group" means a linear or branched hydrocarbon group containing 2 to 6 carbon atoms, which comprises at least one carbon-carbon double bond or triple bond. Specific examples of the unsaturated hydrocarbon group may include a vinyl group, an allyl group, a methylvinyl group, a propenyl group, a butenyl group, a pentenyl group, a hexenyl group, an ethynyl group, and a 2-propynyl group. Among these, a vinyl group, an allyl group, and a 1-propenyl group are preferable.

In the present description, the "alkenyl group" means a linear or branched hydrocarbon group containing 2 to 6 carbon atoms, which comprises at least one carbon-carbon double bond. Specific examples of the alkenyl group may include C2-C6 alkenyl groups, such as a vinyl group, an allyl group, a 2-methyl-2-propenyl group, an isopropenyl group, a 1-, 2- or 3-butenyl group, a 2-, 3- or 4-pentenyl group, a 2-methyl-2-butenyl group, a 3-methyl-2-butenyl group, and a 5-hexenyl group. Among these, a vinyl group, an allyl group, a 1-propenyl group, and a 2-methyl-2-propenyl group are preferable.

In the present description, the "alkynyl group" means a linear or branched unsaturated hydrocarbon group having at least one triple bond (for example, 1 or 2, and preferably 1 triple bond). Specific examples of the alkynyl group may include C2-C6 alkynyl groups such as an ethynyl group, a 1- or 2-propynyl group, a 1-, 2- or 3-butynyl group, and a 1-methyl-2-propynyl group. Among these, an ethynyl group and a 2-propynyl group are preferable.

In the present description, the "C3-C10 cyclic unsaturated hydrocarbon group" means a monocyclic or polycyclic hydrocarbon group containing 3 to 10 carbon atoms, which comprises at least one carbon-carbon double bond. Specific examples of the C3-C10 cyclic unsaturated hydrocarbon group may include a cyclopropenyl group, a cyclobutenyl group, a cyclopentenyl group, a cyclohexenyl group, a cycloheptenyl group, a cyclooctenyl group, and a cyclononyl group. Among these, a monocyclic or polycyclic hydrocarbon group containing 3 to 7 carbon atoms, which comprises at least one carbon-carbon double bond, is preferable, and a cyclopropenyl group is more preferable.

In the present description, the "alkoxy group" means an oxy group having the above-described alkyl group. Specific examples of the alkoxy group may include C1-C6 alkoxy groups such as a methoxy group, an ethoxy group, an n-propoxy group, an isopropoxy group, an n-butoxy group, an isobutoxy group, a sec-butoxy group, a tert-butoxy group, a pentyloxy group, an isopentyloxy group, and a hexyloxy group. Among these, a methoxy group and an ethoxy group are preferable, and a methoxy group is more preferable.

In the present description, the "haloalkoxy group" may include the above-described alkoxy group having at least one halogen atom (preferably 1 to 13, and more preferably 1 to 3 halogen atoms). Specific examples of the

haloalkoxy group may include C1-C6 haloalkoxy groups such as a fluoromethoxy group, a difluoromethoxy group, a trifluoromethoxy group, a trichloromethoxy group, a fluoroethoxy group, a 1,1,1-trifluoroethoxy group, a monofluoro-n-propoxy group, a perfluoro-n-propoxy group, and a perfluoro-isopropoxy group.

In the present description, the "cycloalkoxy group" means an oxy group having the above-described cycloalkyl group. Specific examples of the cycloalkoxy group may include C3-C7 cycloalkoxy groups such as a cyclopropoxy group, a cyclobutoxy group, a cyclopentyloxy group, a cyclohexyloxy group, and a cycloheptyloxy group. Among these, a cyclobutoxy group, a cyclopentyloxy group, and a cyclohexyloxy group are preferable.

In the present description, the "aralkyloxy group" means an oxy group having the above-described aralkyl group. Specific examples of the aralkyloxy group may include C7-C20 aralkyloxy groups such as a benzyloxy group, a phenethyloxy group, a naphthylmethyloxy group, and a fluorenylmethyloxy group. Among these, a benzyloxy group is preferable.

In the present description, the "alkylthio group" means a thioxy group having the above-described alkyl group. Specific examples of the alkylthio group may include C1-C6 alkylthio groups such as a methylthio group, an ethylthio group, an n-propylthio group, an isopropylthio group, an n-butylthio group, an isobutylthio group, a tert-butylthio group, an n-pentylthio group, an isopentylthio group, and a hexylthio group. Among these, a methylthio group, an ethylthio group, and an n-propylthio group are preferable.

In the present description, the "alkoxyalkyl group" means the above-described alkyl group having at least one of the above-described alkoxy groups. Specific examples of the alkoxyalkyl group may include C1-C6 alkoxy-C1-C6 alkyl groups such as a methoxymethyl group, an ethoxyethyl group, a methoxyethyl group, and a methoxypropyl group.

In the present description, the "alkylamino group" means an amino group in which 1 or 2 hydrogen atoms are substituted with a linear or branched hydrocarbon group(s) containing 1 to 6 carbon atoms. Specific examples of the alkylamino group may include a methylamino group, an ethylamino group, a dimethylamino group, a diethylamino group, and an ethylmethylamino group. Among these, preferable is an amino group in which 1 or 2 hydrogen atoms are substituted with a linear or branched hydrocarbon group containing 1 to 3 carbon atoms.

In the present description, the "monoalkylamino group" means an amino group in which one hydrogen atom is substituted with a linear or branched hydrocarbon group. Specific examples of the monoalkylamino group may include a methylamino group, an ethylamino group, an n-propylamino group, an isopropylamino group, an n-butylamino group, an isobutylamino group, a sec-butylamino group, a tert-butylamino group, a pentylamino group, and a hexylamino group. Among these, preferable is an amino group in which one hydrogen atom is substituted with a linear or branched hydrocarbon group containing 1 to 3 carbon atoms.

In the present description, the "dialkylamino group" means an amino group in which two hydrogen atoms are substituted with linear or branched hydrocarbon groups containing 1 to 6 carbon atoms. Specific examples of the dialkylamino group may include a dimethylamino group, a diethylamino group, and an ethylmethylamino group. Among these, an amino group in which two hydrogen atoms are substituted with linear or branched hydrocarbon groups containing 1 to 3 carbon atoms is preferable, and a dimethylamino group is more preferable.

In the present description, the "acyl group" means a formyl group in which a hydrogen atom is substituted with a linear or branched hydrocarbon group. Specific examples of the acyl group may include an acetyl group, an n-propanoyl group, an isopropanoyl group, an n-butyloyl group, and a tert-butyloyl group. Among these, a formyl group in which a hydrogen atom is substituted with a linear or branched hydrocarbon group containing 1 to 3 carbon atoms is preferable, and an acetyl group is more preferable.

In the present description, the "acyloxy group" means an oxy group having the above-described acyl group. Specific examples of the acyloxy group may include an alkylcarbonyloxy group and an arylcarbonyloxy group. Among these, an oxy group in which a hydrogen atom of formyl group is substituted with a linear or branched hydrocarbon group containing 1 to 3 carbon atoms is preferable, and an alkylcarbonyloxy group is more preferable.

In the present description, the "alkoxycarbonyl group" means a carbonyl group having the above-described alkoxy group. Specific examples of the alkoxycarbonyl group may include (C1-C6alkoxy)carbonyl groups such as a methoxycarbonyl group, an ethoxycarbonyl group, a propoxycarbonyl group, an isopropoxycarbonyl group, a butoxycarbonyl group, an isobutoxycarbonyl group, a tert-butoxycarbonyl group, a pentyloxycarbonyl group, an isopentyloxycarbonyl group, and a hexyloxycarbonyl group. Among these, a tert-butoxycarbonyl group is preferable.

In the present description, the "aralkyloxycarbonyl group" means a carbonyl group having the above-described aralkyloxy. Specific examples of the aralkyloxycarbonyl group may include (C6-C20 aralkyl)oxycarbonyl groups such as a benzyloxycarbonyl group, a phenethyloxycarbonyl group, a naphthylmethyloxycarbonyl group, and a fluorenylmethyloxycarbonyl group. Among these, a benzyloxycarbonyl group is preferable.

In the present description, the "saturated heterocyclic group" means a monocyclic or polycyclic saturated heterocyclic group having at least one heteroatom (preferably 1 to 5, and more preferably 1 to 3 heteroatoms) selected from nitrogen atoms, oxygen atoms, and sulfur atoms. Specific examples of the saturated heterocyclic group may include an aziridinyl group, an azetidinyl group, an imidazolidinyl group, a morpholino group, a pyrrolidinyl group, a piperidinyl group, a piperazinyl group, a tetrahydrofuranyl group, a tetrahydropyranyl group, a tetrahydrothiophenyl group, a thiazolidinyl group, and an oxazolidinyl group. Among these, an azetidinyl group, a pyrrolidinyl group, and a piperidinyl group are preferable, and an azetidinyl group and a pyrrolidinyl group are more preferable.

In the present description, the "unsaturated heterocyclic group" means a monocyclic or polycyclic completely unsaturated or partially unsaturated heterocyclic group having at least one heteroatom (preferably 1 to 5, and more preferably 1 to 3 heteroatoms) selected from nitrogen atoms, oxygen atoms, and sulfur atoms. Specific examples of the unsaturated heterocyclic group may include an imidazolyl group, a thienyl group, a pyrrolyl group, an oxazolyl group, an isoxazolyl group, a thiazolyl group, an isothiazolyl group, a thiadiazolyl group, an oxadiazolyl group, a pyrazolyl group, a triazolyl group, a tetrazolyl group, a pyridyl group, a pyrazyl group, a pyrimidinyl group, a pyridazinyl group, an indolyl group, an isoindolyl group, an indazolyl group, a triazolopyridyl group, a benzimidazolyl group, a benzoxazolyl group, a benzothiazolyl group, a benzothienyl group, a furanyl group, a benzofuranyl group, a purinyl group, a quinolyl group, an isoquinolyl group, a quinazolinyl group, a quinoxalyl group, a methylenedioxyphenyl group, an ethylenedioxyphenyl group, and a dihydrobenzofuranyl group. Among these, an imidazolyl group, a pyrazolyl group, a thiazolyl group, an isoxazolyl group, and a furanyl group are preferable; an imidazolyl group, a pyrazolyl group, and a thiazolyl group are more preferable; and an imidazolyl group is most preferable.

In the present description, the "saturated heterocyclic oxy group" means an oxy group having the above-described saturated heterocyclic group. Specific examples of the saturated heterocyclic oxy group may include a morpholinyloxy group, a 1-pyrrolidinyloxy group, a piperidinooxy group, a piperazinyloxy group, a 4-methyl-1-piperazinyloxy group, a tetrahydrofuranyloxy group, a tetrahydropyranyloxy group, a tetrahydrothiophenyloxy group, a thiazolidinyloxy group, and an oxazolidinyloxy group. Among these, a 1-pyrrolidinyloxy group, a piperidinooxy group, and a piperazinyloxy group are preferable.

In the compound represented by the formula (I) or the formula (II) of the present invention, $R_1$ is a C1-C4 alkyl group optionally having a C1-C4 alkoxy group as a substituent, or a C3-C4 cycloalkyl group.

The "C1-C4 alkoxy group" in the "C1-C4 alkyl group optionally having a C1-C4 alkoxy group as a substituent" represented by $R_1$ is preferably a methoxy group or an ethoxy group, and most preferably a methoxy group. Herein, the number of substituents is preferably 1 to 3, and most preferably 1. When the C1-C4 alkyl group has two or more substituents, the substituents may be identical to or different from each other.

The "C1-C4 alkyl group" in the "C1-C4 alkyl group optionally having a C1-C4 alkoxy group as a substituent" represented by $R_1$ is preferably a methyl group, an ethyl group, an n-propyl group, an isopropyl group, or a tert-butyl group, more preferably a methyl group, an ethyl group, an isopropyl group, or a tert-butyl group, and most preferably a methyl group or a tert-butyl group.

The "C1-C4 alkyl group optionally having a C1-C4 alkoxy group as a substituent" represented by $R_1$ is preferably a C1-C4 alkyl group having 1 to 3 methoxy groups as substituents, more preferably a methyl group, an ethyl group, an isopropyl group, a tert-butyl group, or a 1-methyl-1-methoxyethyl group, and most preferably a methyl group or a tert-butyl group.

The "C3-C4 cycloalkyl group" represented by $R_1$ is preferably a cyclopropyl group or a cyclobutyl group, and most preferably a cyclopropyl group.

$R_1$ is preferably a C1-C4 alkyl group optionally having 1 to 3 C1-C4 alkoxy groups as substituents, or a C3-C4 cycloalkyl group.

$R_1$ is more preferably a C1-C4 alkyl group optionally having 1 to 3 methoxy groups as substituents, or a C3-C4 cycloalkyl group.

$R_1$ is further preferably a methyl group, an ethyl group, an isopropyl group, a tert-butyl group, a 1-methyl-1-methoxyethyl group, or a cyclopropyl group.

$R_1$ is most preferably a methyl group, a tert-butyl group, or a cyclopropyl group.

In the compound represented by the formula (I) or the formula (II) of the present invention, $R_2$ is a hydrogen atom, a halogen atom, a C1-C6 alkyl group optionally having 1 to 5 C1-C4 alkoxy groups or fluorine atoms each as a substituent(s), or a C1-C6 alkoxy group.

The "halogen atom" represented by $R_2$ is preferably a fluorine atom or a chlorine atom.

The "C1-C4 alkoxy group" in the "C1-C6 alkyl group optionally having 1 to 5 C1-C4 alkoxy groups or fluorine atoms each as a substituent(s)" represented by $R_2$ is preferably a methoxy group or an ethoxy group, and most preferably a methoxy group.

The "C1-C6 alkyl group optionally having 1 to 5 C1-C4 alkoxy groups or fluorine atoms each as a substituent(s)" represented by $R_2$ is preferably a methyl group, an ethyl group, an n-propyl group, an isopropyl group, or a tert-butyl group, and most preferably a methyl group.

The "C1-C6 alkyl group" in the "C1-C6 alkyl group optionally having 1 to 5 C1-C4 alkoxy groups or fluorine atoms each as a substituent(s)" represented by $R_2$ is preferably a C1-C6 alkyl group optionally having 1 to 5 methoxy groups, ethoxy groups, or fluorine atoms as a substituent(s) (specifically, a methyl group, a methoxymethyl group, an ethoxymethyl group, a methoxyethyl group, an ethoxyethyl group, a fluoromethyl group, a difluoromethyl group, a trifluoromethyl group, etc.), more preferably a C1-C6 alkyl group, further preferably a methyl group, an ethyl group, an n-propyl group, an isopropyl group, or a tert-butyl group, and most preferably a methyl group.

The "C1-C6 alkoxy group" represented by $R_2$ is preferably a methoxy group or an ethoxy group, and most preferably a methoxy group.

$R_2$ is preferably a C1-C6 alkyl group optionally having 1 to 5 C1-C4 alkoxy groups or fluorine atoms each as a substituent(s). In one embodiment, $R_2$ is a C1-C6 alkyl group optionally having 1 to 5 methoxy groups, ethoxy groups, or fluorine atoms as a substituent(s). In another embodiment, $R_2$ is a methyl group, an ethyl group, an n-propyl group, an isopropyl group, or a tert-butyl group (preferably a methyl group or an ethyl group, and more preferably a methyl group), each optionally having 1 to 5 methoxy groups, ethoxy groups, or fluorine atoms as a substituent(s).

$R_2$ is more preferably a C1-C6 alkyl group optionally having 1 to 5 C1-C4 alkoxy groups as a substituent(s). In one embodiment, $R_2$ is a C1-C6 alkyl group optionally having 1 to 5 methoxy groups or ethoxy groups as a substituent(s). In another embodiment, $R_2$ is a methyl group, an ethyl group, an n-propyl group, an isopropyl group, or a tert-butyl group (preferably a methyl group or an ethyl group, and more preferably a methyl group) each optionally having 1 to 5 methoxy groups or ethoxy groups as a substituent(s). In a further embodiment, $R_2$ is a methyl group, an ethyl group, a methoxymethyl group, or an ethoxymethyl group.

$R_2$ is even more preferably a C1-C6 alkyl group.

$R_2$ is further preferably a methyl group, an ethyl group, an n-propyl group, an isopropyl group, or a tert-butyl group.

$R_2$ is particularly preferably a methyl group or an ethyl group.

$R_2$ is most preferably a methyl group.

In the compound represented by the formula (I) or the formula (II) of the present invention, $R_3$ is a hydrogen atom, or a C1-C4 alkyl group optionally having 1 to 5 fluorine atoms as a substituent(s).

The "C1-C4 alkyl group" in the "C1-C4 alkyl group optionally having 1 to 5 fluorine atoms as a substituent(s)" represented by $R_3$ is preferably a methyl group, an ethyl group, an n-propyl group, an isopropyl group, or a tert-butyl group, more preferably a methyl group or an ethyl group, and most preferably a methyl group.

The "C1-C4 alkyl group optionally having 1 to 5 fluorine atoms as a substituent(s)" represented by $R_3$ is preferably a methyl group, a fluoromethyl group, a difluoromethyl group, a trifluoromethyl group, or an ethyl group, more preferably a methyl group, a trifluoromethyl group, or an ethyl group, and most preferably a methyl group.

$R_3$ is preferably a C1-C4 alkyl group optionally having 1 to 5 fluorine atoms as a substituent(s).

$R_3$ is more preferably a methyl group, a fluoromethyl group, a difluoromethyl group, a trifluoromethyl group, an ethyl group, a fluoroethyl group, a difluoroethyl group, a trifluoroethyl group, an n-propyl group, an isopropyl group, or a tert-butyl group.

$R_3$ is even more preferably a methyl group, a fluoromethyl group, a difluoromethyl group, a trifluoromethyl group, or an ethyl group.

$R_3$ is further preferably a methyl group, a trifluoromethyl group, or an ethyl group.

$R_3$ is particularly preferably a methyl group or an ethyl group.

$R_3$ is most preferably a methyl group.

In the compound represented by the formula (I) or the formula (II) of the present invention, $R_4$ is a hydrogen atom or a C1-C4 alkyl group.

The "C1-C4 alkyl group" represented by $R_4$ is preferably a methyl group, an ethyl group, an n-propyl group, an isopropyl group, or a tert-butyl group, more preferably a methyl group or an ethyl group, and most preferably a methyl group.

$R_4$ is preferably a hydrogen atom, a methyl group, an ethyl group, an n-propyl group, an isopropyl group, or a tert-butyl group.

$R_4$ is more preferably a hydrogen atom, a methyl group, or an ethyl group.

$R_4$ is further preferably a hydrogen atom or a methyl group.

$R_4$ is most preferably a hydrogen atom.

In the compound represented by the formula (I) or the formula (II) of the present invention, $R_5$ is a phenyl group optionally having 1 to 3 substituents selected from the group consisting of fluorine atoms and chlorine atoms.

$R_5$ is preferably a phenyl group optionally having 1 or 2 substituents selected from the group consisting of fluorine atoms and chlorine atoms.

$R_5$ is more preferably a phenyl group, a 2-fluorophenyl group, a 3-chlorophenyl group, a 2,3-difluorophenyl group, a 2,4-difluorophenyl group, or a 3,5-difluorophenyl group.

$R_5$ is most preferably a phenyl group.

The pyrimidine compound of the present invention is preferably the compound represented by the formula (I) or the formula (II), or a salt thereof, wherein, in the formula (I) or the formula (II),

$R_1$ is a C1-C4 alkyl group optionally having a C1-C4 alkoxy group as a substituent, or a C3-C4 cycloalkyl group,
$R_2$ is a C1-C6 alkyl group optionally having 1 to 5 C1-C4 alkoxy groups as a substituent(s),
$R_3$ is a C1-C4 alkyl group optionally having 1 to 5 fluorine atoms as a substituent(s),

$R_4$ is a hydrogen atom or a C1-C4 alkyl group, and
$R_5$ is a phenyl group optionally having 1 or 2 substituents selected from the group consisting of fluorine atoms and chlorine atoms.

The compound of the present invention is more preferably the compound represented by the formula (I) or the formula (II), or a salt thereof, wherein, in the formula (I) or the formula (II),

$R_1$ is a methyl group, an ethyl group, an n-propyl group, an isopropyl group, a tert-butyl group, a 1-methyl-1-methoxyethyl group, a cyclopropyl group, or a cyclobutyl group,
$R_2$ is a methyl group, an ethyl group, an n-propyl group, a tert-butyl group, a methoxymethyl group or an ethoxymethyl group,
$R_3$ is a methyl group, a fluoromethyl group, a difluoromethyl group, a trifluoromethyl group, an ethyl group, a fluoroethyl group, a difluoroethyl group, a trifluoroethyl group, an n-propyl group, an isopropyl group, or a tert-butyl group,
$R_4$ is a hydrogen atom, a methyl group, an ethyl group, an n-propyl group, an isopropyl group, or a tert-butyl group, and
$R_5$ is a phenyl group, a 2-fluorophenyl group, a 3-fluorophenyl group, a 2,4-difluorophenyl group, a 2,3-difluorophenyl group, a 3,5-difluorophenyl group, a 2-chlorophenyl group, a 3-chlorophenyl group, a 2,4-dichlorophenyl group, or a 3,5-dichlorophenyl group.

The compound of the present invention is even more preferably the compound represented by the formula (II), or a salt thereof, wherein, in the formula (II),

$R_1$ is a methyl group, an ethyl group, an isopropyl group, a tert-butyl group, a 1-methyl-1-methoxyethyl group, or a cyclopropyl group,
$R_2$ is a methyl group, an ethyl group, a methoxymethyl group or an ethoxymethyl group,
$R_3$ is a methyl group, a fluoromethyl group, a difluoromethyl group, a trifluoromethyl group, or an ethyl group,
$R_4$ is a hydrogen atom, a methyl group, or an ethyl group, and
$R_5$ is a phenyl group, a 2-fluorophenyl group, a 3-chlorophenyl group, a 2,3-difluorophenyl group, a 2,4-difluorophenyl group, or a 3,5-difluorophenyl group.

The compound of the present invention is further preferably the compound represented by the formula (II), or a salt thereof, wherein, in the formula (II),

$R_1$ is a methyl group, a tert-butyl group, or a cyclopropyl group,
$R_2$ is a methyl group, an ethyl group, a methoxymethyl group or an ethoxymethyl group,
$R_3$ is a methyl group, a trifluoromethyl group, or an ethyl group,
$R_4$ is a hydrogen atom or a methyl group, and
$R_5$ is a phenyl group.

The compound of the present invention is further preferably the compound represented by the formula (II), or a salt thereof, wherein, in the formula (II),

$R_1$ is a methyl group, a tert-butyl group, or a cyclopropyl group,
$R_2$ is a methyl group, an ethyl group, or a methoxymethyl group,
$R_3$ is a methyl group,
$R_4$ is a hydrogen atom, and
$R_5$ is a phenyl group.

The compound of the present invention is particularly preferably the compound represented by the formula (II), or a salt thereof, wherein, in the formula (II),

$R_1$ is a methyl group, a tert-butyl group, or a cyclopropyl group,
$R_2$ is a methyl group,
$R_3$ is a methyl group,
$R_4$ is a hydrogen atom, and
$R_5$ is a phenyl group.

Specific examples of the compound represented by the formula (I) or the formula (II) may include compounds produced in the following Examples, but are not limited thereto. One embodiment of the present invention relates to a

compound selected from the following (1) to (19), or a salt thereof. One embodiment of the present invention relates to a compound selected from the following (1) to (15), or a salt thereof.

(1) 7-((3R,SS)-1-acryloyl-5-methylpyrrolidin-3-yl)-4-amino-N-((R)-1-phenylethyl)-6-(prop-1-yn-1-yl)-7H-pyrrolo[2,3-d]pyrimidine-5-carboxamide,
(2) 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide,
(3) 7-((3R,SS)-1-acryloyl-5-methylpyrrolidin-3-yl)-4-amino-6-(3,3-dimethylbut-1-yn-1-yl)-N-((R)-1-phenylethyl)-7H-pyrrolo [2,3-d]pyrimidine-5-carboxamide,
(4) 7-(R)-((3R,5S)-1-acryloyl-5-methylpyrrolidin-3-yl)-4-amino-N-((R)-1-(3,5-difluorophenyl)ethyl)-6-(prop-1 -yn-1 -yl)-7H-pyrrolo [2,3 -d]pyrimidine-5-carboxamide,
(5) 7-((3R,5S)-1-acryloyl-5-methylpyrrolidin-3-yl)-4-amino-N-(2-phenylpropan-2-yl)-6-(prop-1-yn-1-yl)-7H-pyrrolo[2,3-d]pyrimidine-5-carboxamide,
(6) 7-((3R,SS)-1-acryloyl-5-methylpyrrolidin-3-yl)-4-amino-N-((R)-1-phenylpropyl)-6-(prop-1-yn-1-yl)-7H-pyrrolo[2,3-d]pyrimidine-5-carboxamide,
(7) 7-((3R,SS)-1-acryloyl-5-methylpyrrolidin-3-yl)-4-amino-N-(2-(2-fluorophenyl)propan-2-yl)-6-(prop-1 -yn-1 -yl)-7H-pyrrolo [2,3 -d]pyrimidine-5-carboxamide,
(8) 7-((3R,5S)-1-acryloyl-5-methylpyrrolidin-3-yl)-4-amino-N-((R)-1-(3-chlorophenyl)ethyl)-6-(prop-1-yn-1-yl)-7H-pyrrolo[2,3-d]pyrimidine-5-carboxamide,
(9) 7-((3R,5S)-1 -acryloyl-5-methylpyrrolidin-3-yl)-4-amino-N-((R)-1 -(2,4-difluorophenyl)ethyl)-6-(prop-1-yn-1-yl)-7H-pyrrolo[2,3-d]pyrimidine-5-carboxamide,
(10) 7-((3R,5S)-1-acryloyl-5-methylpyrrolidin-3-yl)-4-amino-6-(prop-1-yn-1-yl)-N-((S)-2,2,2-trifluoro-1-phenylethyl)-7H-pyrrolo[2,3-d]pyrimidine-5-carboxamide,
(11) 7-((3R,5 S)-1 -acryloyl-5-methylpyrrolidin-3 -yl)-4-amino-6-(cyclopropylethynyl)-N-(2-phenylpropan-2-yl)-7H-pyrrolo [2,3-d]pyrimidine-5-carboxamide,
(12) 7-((3R,SS)-1-acryloyl-5-methylpyrrolidin-3-yl)-4-amino-6-(cyclopropylethynyl)-N-((R)-1 -(2,3 -difluorophenyl)ethyl)-7H-pyrrolo [2,3 -d]pynmidine-5 -carboxamide,
(13) 7-((3R,5S)-1-acryloyl-5-methylpyrrolidin-3-yl)-4-amino-6-(3-methoxy-3-methylbut-1 -yn-1 -yl)-N-((R)-1 -phenylethyl)-7H-pyrrolo [2,3 -d]pyrimidine-5-carboxamide,
(14) 7-((3R,5S)-1-acryloyl-5-methylpyrrolidin-3-yl)-4-amino-6-(but-1-yn-1-yl)-N-((R)-1-phenylethyl)-7H-pyrrolo[2,3-d]pyrimidine-5-carboxamide,
(15) 7-((3R,5S)-1-acryloyl-5-methylpyrrolidin-3-yl)-4-amino-N-(2-(2-fluorophenyl)propan-2-yl)-6-(3-methylbut-1-yn-1-yl)-7H-pyrrolo[2,3-d]pyrimidine-5-carboxamide,
(16) 7-((3R,5S)-1-acryloyl-5-ethylpyrrolidin-3-yl)-4-amino-N-((R)-1-phenylethyl)-6-(prop-1-yn-1-yl)-7H-pyrrolo[2,3-d]pyrimidine-5-carboxamide,
(17) 7-((3R,5S)-1 -acryloyl-5-ethylpyrrolidin-3-yl)-4-amino-6-(cyclopropylethynyl)-N-((R)-1-phenylethyl)-7H-pyrrolo[2,3-d]pyrimidine-5-carboxamide,
(18) 7-((3R,5R)-1-acryloyl-5-(methoxymethyl)pyrrolidin-3-yl)-4-amino-6-(cyclopropylethynyl)-N-((R)-1-phenylethyl)-7H-pyrrolo[2,3-d]pyrimidine-5-carboxamide, and
(19) 7-((3R,5R)-1-acryloyl-5-(ethoxymethyl)pyrrolidin-3-yl)-4-amino-6-(cyclopropylethynyl)-N-((R)-1-phenylethyl)-7H-pyrrolo[2,3-d]pyrimidine-5-carboxamide.

A preferred example of the compound represented by the formula (I) or the formula (II) may be a compound selected from the following (1) to (3), or a salt thereof.

(1) 7-((3R,5S)-1-acryloyl-5-methylpyrrolidin-3-yl)-4-amino-N-((R)-1-phenylethyl)-6-(prop-1 -yn-1 -yl)-7H-pyrrolo [2,3 -d]pyrimidine-5 -carboxamide,
(2) 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide, and
(3) 7-((3R,5S)-1-acryloyl-5-methylpyrrolidin-3-yl)-4-amino-6-(3,3-dimethylbut-1-yn-1-yl)-N-((R)-1-phenylethyl)-7H-pyrrolo[2,3-d]pyrimidine-5-carboxamide.

The most preferred pyrimidine compound of the present invention is 7-((3R,5S)-1-acryloyl-5-methylpyrrolidin-3-yl)-4-amino-6-(cyclopropylethynyl)-N-((R)-1-phenylethyl)-7H-pyrrolo [2,3-d]pyrimidine-5-carboxamide.

< Method for producing compound represented by formula (I) >

The compound according to the present invention can be produced by, for example, the following production

method or the methods described in the Examples. However, the method for producing the compound according to the present invention is not limited to these examples.

The compounds (I) and (II) of the present invention can be produced by applying, for example, the following production method.

< Production Method >

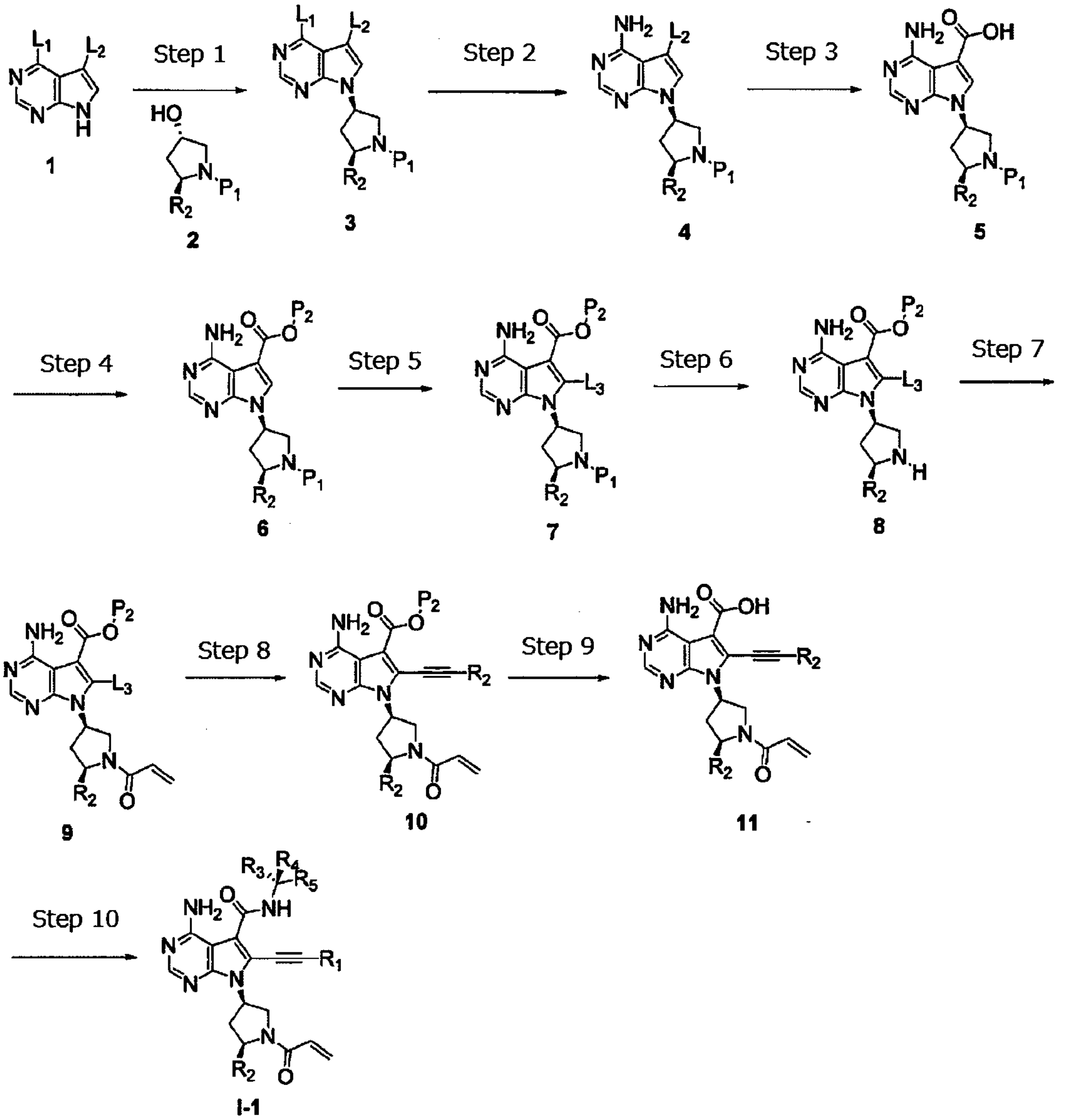


1, 2, 3, 4, 5, 6, 7, 8, 9, 10, 11, I-1

In the above process, $L_1$, $L_2$, and $L_3$, which are the same or different, each represent a leaving group; Pi and $P_2$, which are the same or different, each represent a protective group; and other symbols are as defined above.

< Step 1 >

This step is a method of obtaining a compound represented by the formula 3 by performing a Mitsunobu reaction between a compound represented by the formula 1 and a compound represented by the formula 2 that is a commercially available compound or can be produced by a known method. The Mitsunobu reaction is generally carried out in the presence of a Mitsunobu reagent and a phosphine reagent.

The compound represented by the formula 2 (in the formula 2, Pi represents a protective group for an amino group) can be used in an amount of 1 to 10 equivalents, and preferably 1 to 3 equivalents, based on the amount of the

compound represented by the formula 1 (1 mole).

The "protective group for an amino group" is not particularly limited, as long as it has a protective function. Examples of the protective group for an amino group may include: aralkyl groups, such as a benzyl group, a p-methoxybenzyl group, a 3,4-dimethoxybenzyl group, an o-nitrobenzyl group, a p-nitrobenzyl group, a benzhydryl group, a trityl group, and a cumyl group; lower alkanoyl groups, such as, for example, a formyl group, an acetyl group, a propionyl group, a butyryl group, a pivaloyl group, a trifluoroacetyl group, and a trichloroacetyl group; for example, benzoyl groups; arylalkanoyl groups, such as, for example, a phenylacetyl group and a phenoxyacetyl group; lower alkoxycarbonyl groups, such as, for example, a methoxycarbonyl group, an ethoxycarbonyl group, a propyloxycarbonyl group, and a tert-butoxycarbonyl group; aralkyloxycarbonyl groups, such as, for example, a p-nitrobenzyloxycarbonyl group and a phenethyloxycarbonyl group; lower alkylsilyl groups, such as, for example, a trimethylsilyl group and a tert-butyldimethylsilyl group; for example, tetrahydropyranyl groups; for example, trimethylsilylethoxymethyl groups; lower alkylsulfonyl groups, etc., such as, for example, a methylsulfonyl group, an ethylsulfonyl group, and a tert-butylsulfonyl group; lower alkylsulfinyl groups, etc., such as for example, a tert-butylsulfinyl group; arylsulfonyl groups, etc., such as, for example, a benzenesulfonyl group and a toluenesulfonyl group; and imide groups, such as, for example, a phthalimide group. Among these, a trifluoroacetyl group, an acetyl group, a tert-butoxycarbonyl group, a benzyloxycarbonyl group, a trimethylsilylethoxymethyl group, or a cumyl group is particularly preferable.

As a Mitsunobu reagent, diethyl azodicarboxylate, diisopropyl azodicarboxylate or the like is used. Such a Mitsunobu reagent is used in an amount of generally approximately 1 to 100 moles, and preferably approximately 1 to 10 moles, based on the compound represented by the formula 1 (1 mole).

As a phosphine reagent, triphenylphosphine, tributylphosphine, trifurylphosphine or the like is used. Such a phosphine reagent is used in an amount of generally approximately 1 to 100 moles, and preferably approximately 1 to 10 moles, based on the compound represented by the formula 1 (1 mole).

The solvent is not particularly limited, as long as it does not affect the reaction. Examples of the solvent may include hydrocarbons (e.g., benzene, toluene, xylene, etc.), halogenated hydrocarbons (e.g., chloroform, 1,2-dichloroethane, etc.), nitriles (e.g., acetonitrile, etc.), ethers (e.g., dimethoxyethane, tetrahydrofuran, etc.), alcohols (e.g., methanol, ethanol, etc.), aprotic polar solvents (e.g., N,N-dimethylformamide, dimethyl sulfoxide, hexamethylphosphoramide, etc.), water, and mixtures thereof. The reaction time is 0.1 to 100 hours, and preferably 0.5 to 24 hours. Thereafter, the reaction temperature is 0°C to the temperature at which the solvent is boiled, and preferably 0°C to 100° C.

The thus obtained compound represented by the formula 3 can be isolated and purified by known separation and purification means, or can be subjected to the subsequent step without isolation and purification.

< Step 2 >

This step is a method of obtaining a compound represented by the formula 4 by allowing the compound represented by the formula 3 to react with ammonia or a salt thereof.

The ammonia or a salt thereof can be used in an amount of 1 to 1000 equivalents, and preferably 1 to 100 equivalents, based on the amount of the compound represented by the formula 3 (1 mole).

The solvent is not particularly limited, as long as it does not affect the reaction. Examples of the solvent may include hydrocarbons (e.g., benzene, toluene, xylene, etc.), halogenated hydrocarbons (e.g., chloroform, 1,2-dichloroethane, etc.), nitriles (e.g., acetonitrile, etc.), ethers (e.g., dimethoxyethane, tetrahydrofuran, etc.), alcohols (e.g., methanol, ethanol, etc.), aprotic polar solvents (e.g., N,N-dimethylformamide, dimethyl sulfoxide, hexamethylphosphoramide, etc.), water, and mixtures thereof. The reaction time is 0.1 to 100 hours, and preferably 0.5 to 24 hours. Thereafter, the reaction temperature is 0°C to the temperature at which the solvent is boiled, and preferably 0°C to 150° C.

The thus obtained compound represented by the formula 4 can be isolated and purified by known separation and purification means, or can be subjected to the subsequent step without isolation and purification.

< Step 3 >

This step is a method of obtaining a compound represented by the formula 5 by reacting the compound represented by the formula 4 under a carbon monoxide atmosphere, for example, in the presence of a transition metal catalyst, a base and alcohol.

In this step, the pressure of the carbon monoxide is generally from 1 to 20 atmospheres, and preferably 1 to 10 atmospheres.

Examples of the alcohol may include methanol, ethanol, propanol, isopropanol, diethylaminoethanol, isobutanol, 4-(2-hydroxyethyl)morpholine, 3-morpholinopropanol, and diethylaminopropanol.

The alcohol is used in an amount of generally approximately 1 to 100 moles, and preferably approximately 1 to 50 moles, based on the amount of the compound represented by the formula 4 (1 mole).

Examples of the transition metal catalyst used herein may include palladium catalysts (e.g., palladium acetate,

palladium chloride, tetrakistriphenylphosphine palladium, palladium carbon, etc.). A ligand (e.g., triphenylphosphine, tri-tert-butylphosphine, etc.) may be added, as necessary. The amount of the transition metal catalyst used is different depending on the type of the catalyst. The transition metal catalyst is used in an amount of generally approximately 0.0001 to 1 mole, and preferably approximately 0.01 to 0.5 moles, based on the amount of the compound 4 (1 mole). The ligand is used in an amount of generally approximately 0.0001 to 4 moles, and preferably approximately 0.01 to 2 moles, based on the amount of the compound represented by the formula 4 (1 mole).

Examples of the base may include organic amines (e.g., trimethylamine, triethylamine, diisopropylethylamine, N-methylmorpholine, 1,8-diazabicyclo[5,4,0]undec-7-ene, pyridine, N,N-dimethylaniline, etc.), alkaline metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide, etc.), metal hydrides (e.g., potassium hydride, sodium hydride, etc.), alkaline metal alkoxides (e.g., sodium methoxide, sodium ethoxide, sodium-tert-butoxide, potassium-tert-butoxide, etc.), and alkaline metal disilazides (e.g., lithium disilazide, sodium disilazide, potassium disilazide, etc.). Among others, alkaline metal salts such as potassium carbonate, cesium carbonate, sodium phosphate, and potassium phosphate, alkaline metal alkoxides such as sodium-tert-butoxide and potassium-tert-butoxide, organic amines such as triethylamine and diisopropylethylamine, and the like are preferable. The base is used in an amount of generally approximately 0.1 to 50 moles, and preferably approximately 1 to 20 moles, based on the amount of the compound represented by the formula 4 (1 mole).

The solvent is not particularly limited, as long as it does not affect the reaction. Examples of the solvent may include hydrocarbons (e.g., benzene, toluene, xylene, etc.), halogenated hydrocarbons (e.g., chloroform, 1,2-dichloroethane, etc.), nitriles (e.g., acetonitrile, etc.), ethers (e.g., dimethoxyethane, tetrahydrofuran, etc.), alcohols (e.g., methanol, ethanol, etc.), aprotic polar solvents (e.g., N,N-dimethylformamide, dimethyl sulfoxide, hexamethylphosphoramide, N-methylpyrrolidone, etc.), water, and mixtures thereof. The reaction time is 0.1 to 100 hours, and preferably 0.5 to 24 hours. Thereafter, the reaction temperature is 0°C to the temperature at which the solvent is boiled, and preferably 0°C to 150° C.

After completion of this reaction, an ester form corresponding to the used alcohol, or a mixture of the ester form and the compound represented by the formula 5 is subjected to a hydrolysis reaction, so that it can be converted to the compound represented by the formula 5.

As such a base, sodium hydrogen carbonate, sodium carbonate, potassium carbonate, cesium carbonate, sodium hydroxide, potassium hydroxide, lithium hydroxide, or the like is preferably used. The base is used in an amount of generally approximately 0.5 to 100 moles, and preferably approximately 1 to 10 moles, based on the amount of the compound represented by the formula 4 (1 mole).

The solvent is not particularly limited, as long as it does not affect the reaction. For example, water, methanol, ethanol, isopropanol, tetrahydrofuran, 1,4-dioxane, N,N-dimethylformamide and the like can be used alone or in combination. The reaction time is 0.1 to 100 hours, and preferably 0.5 to 24 hours. Thereafter, the reaction temperature is 0°C to the temperature at which the solvent is boiled, and preferably 0°C to 100° C.

The thus obtained compound represented by the formula 5 can be isolated and purified by known separation and purification means, or can be subjected to the subsequent step without isolation and purification.

< Step 4 >

This step is a method of obtaining a compound represented by the formula 6 (wherein $P_2$ represents a protective group for a carboxyl group) by introducing a protective group into the compound represented by the formula 5. Protection can be carried out according to a generally known method, for example, the method described in Protective Groups in Organic Synthesis third edition, T. W. Greene and P. G. M. Wuts, John Wiley & Sons (1999), or a method equivalent thereto.

The "protective group for a carboxyl group" is not particularly limited, as long as it has a protective function. Examples of the protective group for a carboxyl group may include: lower alkyl groups, such as, for example, a methyl group, an ethyl group, a propyl group, an isopropyl group, and a tert-butyl group; halo lower alkyl groups, such as, for example, a 2,2,2-trichloroethyl group; lower alkenyl groups, such as, for example, an allyl group; for example, a trimethylsilylethoxymethyl group; and aralkyl groups, such as, for example, a benzyl group, a p-methoxybenzyl group, a p-nitrobenzyl group, a benzhydryl group, and a trityl group. In particular, a methyl group, an ethyl group, a tert-butyl group, an allyl group, a benzyl group, a p-methoxybenzyl group, or a trimethylsilylethoxymethyl group is preferable.

In the present reaction, a protective group such as, for example, a tert-butyl ester group, a methyl ester group, or an ethyl ester group, is preferably introduced.

The protective group agent used in the present reaction may be, for example, 2-tert-butyl-1,3-diisopropylisourea. Such a protective group agent is used in an amount of generally approximately 1 to 50 moles, and preferably approximately 1 to 10 moles, based on the amount of the compound represented by the formula 5 (1 mole).

The solvent is not particularly limited, as long as it does not affect the reaction. Examples of the solvent may

include hydrocarbons (e.g., benzene, toluene, xylene, etc.), halogenated hydrocarbons (e.g., chloroform, 1,2-dichloroethane, etc.), nitriles (e.g., acetonitrile, etc.), ethers (e.g., dimethoxyethane, tetrahydrofuran, tert-butyl methyl ether, etc.), alcohols (e.g., methanol, ethanol, etc.), aprotic polar solvents (e.g., N,N-dimethylformamide, dimethyl sulfoxide, hexamethylphosphoramide, etc.), water, and mixtures thereof. The reaction time is 0.1 to 100 hours, and preferably 0.5 to 24 hours. Thereafter, the reaction temperature is 0°C to the temperature at which the solvent is boiled, and preferably 0°C to 100° C.

The thus obtained compound represented by the formula 6 can be isolated and purified by known separation and purification means, or can be subjected to the subsequent step without isolation and purification.

< Step 5 >

This step is a method of obtaining a compound represented by the formula 7 (wherein $L_3$ represents a halogen atom) by halogenating the compound represented by the formula 6. Halogenation can be carried out by a method using fluorine, chlorine, bromine, iodine, etc., or by a method using N-chlorosuccinimide, N-bromosuccinimide, N-iodosuccinimide, etc. In the present reaction, the method using N-chlorosuccinimide, N-bromosuccinimide, N-iodosuccinimide, etc. is preferable.

N-chlorosuccinimide, N-bromosuccinimide, N-iodosuccinimide, etc. can be used in an amount of 1 to 10 equivalents, and preferably 1 to 3 equivalents, based on the amount of the compound represented by the formula 6 (1 mole).

The solvent is not particularly limited, as long as it does not affect the reaction. Examples of the solvent may include hydrocarbons (e.g., benzene, toluene, xylene, etc.), halogenated hydrocarbons (e.g., chloroform, 1,2-dichloroethane, etc.), nitriles (e.g., acetonitrile, etc.), ethers (e.g., dimethoxyethane, tetrahydrofuran, etc.), alcohols (e.g., methanol, ethanol, etc.), aprotic polar solvents (e.g., N,N-dimethylformamide, dimethyl sulfoxide, hexamethylphosphoramide, etc.), water, and mixtures thereof. The reaction time is 0.1 to 100 hours, and preferably 0.5 to 24 hours. Thereafter, the reaction temperature is 0°C to the temperature at which the solvent is boiled, and preferably 0°C to 100°.

The thus obtained compound represented by the formula 7 can be isolated and purified by known separation and purification means, or can be subjected to the subsequent step without isolation and purification.

< Step 6 >

This step is a method of obtaining a compound represented by the formula 8 by removing the protective group for an amino group (Pi in the formula 7) from the compound represented by the formula 7 (deprotection). Such deprotection can be carried out according to a generally known method, for example, the method described in Protective Groups in Organic Synthesis third edition, T. W. Greene and P. G. M. Wuts, John Wiley & Sons (1999), or a method equivalent thereto.

The protective group may be, for example, tert-butyloxycarbonyl. When such a tert-butyloxycarbonyl group is used, for example, as a protective group, deprotection is preferably carried out under acidic conditions. Examples of the acid used herein may include hydrochloric acid, acetic acid, trifluoroacetic acid, sulfuric acid, and tosylic acid.

The acid is preferably used in an amount of approximately 1 to 100 equivalents based on the amount of the compound represented by the formula 7 (1 mole).

The solvent used in the reaction is not particularly limited, as long as it does not affect the reaction. Examples of the solvent used herein may include alcohols (e.g., methanol, etc.), hydrocarbons (e.g., benzene, toluene, xylene, etc.), halogenated hydrocarbons (e.g., methylene chloride, chloroform, 1,2-dichloroethane, etc.), nitriles (e.g., acetonitrile, etc.), ethers (e.g., dimethoxyethane, tetrahydrofuran, etc.), aprotic polar solvents (e.g., N,N-dimethylformamide, dimethyl sulfoxide, hexamethylphosphoramide, etc.), and mixtures thereof. The reaction time is 0.1 to 100 hours, and preferably 0.5 to 24 hours. Thereafter, the reaction temperature is 0°C to 100°C, and preferably 0°C to 50°.

The thus obtained compound represented by the formula 8 can be isolated and purified by known separation and purification means, or can be subjected to the subsequent step without isolation and purification.

< Step 7 >

This step is a method of obtaining a compound represented by the formula 9 by performing an amidation reaction between an amino group of the compound represented by the formula 8 and acrylic acid halide or acrylic acid anhydride.

In the case of using acrylic acid halide or acrylic acid anhydride, such acrylic acid halide or acrylic acid anhydride is used in an amount of generally approximately 0.5 to 10 moles, and preferably approximately 1 to 5 moles, based on the amount of the compound represented by the formula 8 (1 mole). It is to be noted that the present acrylic acid halide or acrylic acid anhydride can be obtained as a commercially available product or can be produced according to a known method.

In addition, a base can be added, as necessary. Examples of the base may include organic amines (e.g., trimethylamine, triethylamine, isopropylethylamine, diisopropylethylamine, N-methylmorpholine, 1,8-diazabicyclo[5,4,0]undec-7-ene, pyridine, N,N-dimethylaniline, etc.), alkaline metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide, etc.), metal hydrides (e.g., potassium hydride, sodium hydride, etc.), and alkaline metal alkoxides (e.g., sodium methoxide, sodium ethoxide, sodium-tert-butoxide, potassium-tert-butoxide, etc.). The base is used in an amount of generally approximately 1 to 100 moles, and preferably approximately 1 to 10 moles, based on the amount of the compound represented by the formula 8 (1 mole).

The solvent used in the reaction is not particularly limited, as long as it does not affect the reaction. Examples of the solvent used herein may include alcohols (e.g., methanol, etc.), hydrocarbons (e.g., benzene, toluene, xylene, etc.), halogenated hydrocarbons (e.g., methylene chloride, chloroform, 1,2-dichloroethane, etc.), nitriles (e.g., acetonitrile, etc.), ethers (e.g., dimethoxyethane, tetrahydrofuran, etc.), aprotic polar solvents (e.g., N,N-dimethylformamide, dimethyl sulfoxide, hexamethylphosphoramide,, etc.), and mixtures thereof. The reaction time is 0.1 to 100 hours, and preferably 0.5 to 24 hours. Thereafter, the reaction temperature is 0°C to the temperature at which the solvent is boiled, and preferably 0°C to 100° C.

The thus obtained compound represented by the formula 9 can be isolated and purified by known separation and purification means, or can be subjected to the subsequent step without isolation and purification.

< Step 8 >

This step is a method of obtaining a compound represented by the formula 10 by performing a Sonogashira reaction between the compound represented by the formula 9 and an acetylene derivative that is a commercially available product or can be produced by a known method.

The acetylene derivative can be used in an amount of 1 to 50 equivalents, and preferably 1 to 10 equivalents, based on the amount of the compound represented by the formula 9 (1 mole).

Examples of the transition metal catalyst used herein may include palladium catalysts (e.g., palladium acetate, palladium chloride, tetrakistriphenylphosphinepalladium, dichlorobis(triphenylphosphine)palladium, etc.), and nickel catalysts (e.g., nickel chloride, etc.). As necessary, a ligand (e.g., triphenylphosphine, tri-tert-butylphosphine, etc.) may be added, and a copper catalyst (e.g., copper iodide, copper bromide, or copper chloride) or the like may be used as a cocatalyst. The amount of the transition metal catalyst used is different depending on the type of the catalyst. The transition metal catalyst is used in an amount of generally approximately 0.0001 to 1 mole, and preferably approximately 0.01 to 0.5 moles, based on the amount of the compound represented by the formula 9 (1 mole). The ligand is used in an amount of generally approximately 0.0001 to 4 moles, and preferably approximately 0.01 to 2 moles, based on the amount of the compound represented by the formula 9 (1 mole). The copper catalyst is used in an amount of generally approximately 0.0001 to 4 moles, and preferably approximately 0.010 to 2 moles, based on the amount of the compound represented by the formula 9 (1 mole).

Examples of the base may include organic amines (e.g., trimethylamine, triethylamine, diisopropylethylamine, N-methylmorpholine, 1,8-diazabicyclo[5,4,0]undec-7-ene, pyridine, N,N-dimethylaniline, etc.), alkaline metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide, etc.), metal hydrides (e.g., potassium hydride, sodium hydride, etc.), alkaline metal alkoxides (e.g., sodium methoxide, sodium ethoxide, sodium-tert-butoxide, potassium-tert-butoxide, etc.), and alkaline metal disilazide (e.g., lithium disilazide, sodium disilazide, potassium disilazide, etc.). Among these, preferred examples of the base may include: alkaline metal salts, such as potassium carbonate, cesium carbonate, sodium phosphate, and potassium phosphate; alkaline metal alkoxides, such as sodium-tert-butoxide and potassium-tert-butoxide; and organic amines, such as triethylamine and diisopropylethylamine. The base is used in an amount of generally approximately 0.1 to 10 moles, and preferably approximately 1 to 5 moles, based on the amount of the compound represented by the formula 9 (1 mole).

The solvent is not particularly limited, as long as it does not affect the reaction. Examples of the solvent may include hydrocarbons (e.g., benzene, toluene, xylene, etc.), halogenated hydrocarbons (e.g., chloroform, 1,2-dichloroethane, etc.), nitriles (e.g., acetonitrile, etc.), ethers (e.g., dimethoxyethane, tetrahydrofuran, etc.), alcohols (e.g., methanol, ethanol, etc.), aprotic polar solvents (e.g., N,N-dimethylformamide, dimethyl sulfoxide, hexamethylphosphoramide, etc.), water, and mixtures thereof. The reaction time is 0.1 to 100 hours, and preferably 0.5 to 24 hours. Thereafter, the reaction temperature is 0°C to the temperature at which the solvent is boiled, and preferably 0°C to 150° C.

The thus obtained compound represented by the formula 10 can be isolated and purified by known separation and purification means, or can be subjected to the subsequent step without isolation and purification.

< Step 9 >

This step is a method of obtaining a compound represented by the formula 11 by deprotecting the protective group for a carboxyl group ($P_2$ in the formula 10) of the compound represented by the formula 10. Deprotection can be carried out according to a generally known method, for example, the method described in Protective Groups in Organic Synthesis third edition, T. W. Greene and P. G. M. Wuts, John Wiley & Sons (1981), or a method equivalent thereto.

The protective group may be, for example, tert-butyl ester. When such a tert-butyl ester group is used as a protective group, for example, deprotection is preferably carried out under acidic conditions. Examples of the acid used herein may include hydrochloric acid, acetic acid, trifluoroacetic acid, sulfuric acid, and tosylic acid.

The acid is preferably used in an amount of approximately 1 to 100 equivalents based on the amount of the compound represented by the formula 10 (1 mole).

The solvent used in the reaction is not particularly limited, as long as it does not affect the reaction. Examples of the solvent used herein may include alcohols (e.g., methanol, etc.), hydrocarbons (e.g., benzene, toluene, xylene, etc.), halogenated hydrocarbons (e.g., methylene chloride, chloroform, 1,2-dichloroethane, etc.), nitriles (e.g., acetonitrile, etc.), ethers (e.g., dimethoxyethane, tetrahydrofuran, etc.), aprotic polar solvents (e.g., N,N-dimethylformamide, dimethyl sulfoxide, hexamethylphosphoramide,, etc.), and mixtures thereof. The reaction time is 0.1 to 100 hours, and preferably 0.5 to 24 hours. Thereafter, the reaction temperature is 0°C to 100°C, and preferably 0°C to 50°.

The thus obtained compound represented by the formula 11 can be isolated and purified by known separation and purification means, or can be subjected to the subsequent step without isolation and purification.

< Step 10 >

This step is a method of obtaining a compound represented by the formula (I) by performing an amidation reaction between a carboxyl group of the compound represented by the formula 11 and an amine that is a commercially available product or can be produced by a known method.

Amidation can be carried out according to a conventionally known method. Examples of the amidation method may include a method of performing the reaction in the presence of a condensing agent, and a method comprising activating a carboxylic acid portion according to a conventionally known method to obtain a reactive derivative, and then performing amidation between the derivative and an amine (for both methods, see "Peptide Gosei no Kiso to Jikken (Principle of Peptide Synthesis and Experiments)" (Nobuo IZUMIYA et al., Maruzen Co., Ltd., 1983)).

Examples of the condensing agent may include N,N'-dicyclohexylcarbodiimide (DCC), N,N'-diisopropylcarbodiimide (DIC), 1-ethyl-3-(3-dimethylaminopropyl)carbodiimide hydrochloride (WSC), diphenylphosphoryl azide (DPPA), benzotriazol-1-yl-oxytrisdimethylaminophosphonium hexafluorophosphate (BOP), benzotriazol-1-yl-oxytripyrrolidinophosphonium hexafluorophosphate (PyBOP), 7-azabenzotriazol-1-yloxytrispyrrolidinophosphonium phosphate (PyAOP), bromotrispyrrolidinophosphonium hexafluorophosphate (BroP), chlorotris(pyrrolidin-1-yl)phosphonium hexafluorophosphate (PyCroP), 3-(diethoxyphosphoryloxy)-1,2,3-benzotriazin-4(3H)-one (DEPBT), O-(azabenzotriazol-1-yl)-N,N,N',N'-tetramethyluronium hexafluorophosphate (HATU), and 4-(5,6-dimethoxy-1,3,5-triazin-2-yl)-4-methylmorpholine hydrochloride (DMTMM). Examples of the additive used at that time may include 1-hydroxybenzotriazole (HOBt), 1-hydroxy-7-azabenzotriazole (HOAt), and N-hydroxysuccinimide (HOSu). Such agents are used in an amount of generally approximately 1 to 100 moles, and preferably approximately 1 to 10 moles, based on the amount of the compound represented by the formula 11 (1 mole).

In addition, as necessary, a base can be added. Examples of such a base may include organic amines (e.g., trimethylamine, triethylamine, diisopropylethylamine, N-methylmorpholine, 1,8-diazabicyclo[5,4,0]undec-7-ene, pyridine, N,N-dimethylaniline, etc.), alkaline metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide, etc.), metal hydrides (e.g., potassium hydride, sodium hydride, etc.), and alkaline metal alkoxides (e.g., sodium methoxide, sodium ethoxide, sodium-tert-butoxide, potassium-tert-butoxide, etc.). The base is used in an amount of generally approximately 1 to 100 moles, and preferably approximately 1 to 10 moles, based on the amount of the compound represented by the formula 11 (1 mole).

The solvent used in the reaction is not particularly limited, as long as it does not affect the reaction. Examples of the solvent used herein may include alcohols (e.g., methanol, etc.), hydrocarbons (e.g., benzene, toluene, xylene, etc.), halogenated hydrocarbons (e.g., methylene chloride, chloroform, 1,2-dichloroethane, etc.), nitriles (e.g., acetonitrile, etc.), ethers (e.g., dimethoxyethane, tetrahydrofuran, etc.), aprotic polar solvents (e.g., N,N-dimethylformamide, dimethyl sulfoxide, hexamethylphosphoramide, , etc.), and mixtures thereof. The reaction time is 0.1 to 100 hours, and preferably 0.5 to 24 hours. Thereafter, the reaction temperature is 0°C to the temperature at which the solvent is boiled, and preferably 0°C to 100° C.

The thus obtained compounds (I) and (II) can be isolated and purified according to known separation and purification means, such as, for example, concentration, vacuum concentration, crystallization, solvent extraction, re-precipitation, or chromatography.

In the above-described production method, the steps ranging from the "introduction of a protective group into a carboxyl group of the compound represented by the formula 5" (Step 4) to the "amidation reaction between a carboxyl group of the compound represented by the formula 11 and an amine that is a commercially available product or can be produced by a known method" (Step 10) are successively carried out in this order. However, the order of performing these steps can be changed. Moreover, the "introduction of a protective group into a carboxyl group of the compound represented by the formula 5" (Step 4) and the "removal of the protective group for a carboxy group from the compound represented by the formula 10" (Step 9) can be omitted.

Specifically, individual steps are carried out in the order of the "amidation reaction between a carboxyl group of the compound represented by the formula 11 and an amine that is a commercially available product or can be produced by a known method" (Step 10), the "halogenation of the compound represented by the formula 6" (Step 5), the "removal of the protective group for an amino group from the compound represented by the formula 7" (Step 6), the "amidation reaction between an amino group of the compound represented by the formula 8 and acrylic acid halide or acrylic acid anhydride" (Step 7), and the "Sonogashira reaction between the compound represented by the formula 9 and an acetylene derivative that is a commercially available product or can be produced by a known method, when L3 of the compound represented by the formula 9 has a leaving group such as halogen" (Step 8), so that the concerned compound can be induced to the compounds represented by the formulae (I) and (II). The conditions applied in individual steps are the same as those as described above.

When the pyrimidine compound of the present invention has an isomer, such as an optical isomer, a stereoisomer, a rotational isomer, or a tautomer, all of these isomers or mixtures thereof are included in the pyrimidine compound of the present invention, unless otherwise stated. For example, when the pyrimidine compound of the present invention has an optical isomer, both a racemate, and an optical isomer obtained as a result of racemic resolution are included in the pyrimidine compound of the present invention, unless otherwise stated.

The salt of the pyrimidine compound of the present invention means a pharmaceutically acceptable salt, and it may be, for example, a base-added salt or an acid-added salt.

The pyrimidine compound of the present invention or a salt thereof also includes a prodrug. The "prodrug" means a compound that is converted to the pyrimidine compound of the present invention or a salt thereof as a result of the reaction with an enzyme, stomach acid, etc. under physiological conditions in a living body; namely, a compound that enzymatically causes oxidation, reduction, hydrolysis, etc., so that it is converted to the pyrimidine compound of the present invention or a salt thereof, or a compound that causes hydrolysis, etc. with stomach acid or the like, so that it is converted to the pyrimidine compound of the present invention or a salt thereof. Otherwise, it may also be a compound that is converted to the pyrimidine compound of the present invention or a salt thereof under physiological conditions as described in "Iyakuhin no Kaihatsu (Development of Pharmaceutical Products)," Hirokawa Shoten, 1990, Vol. 7, Bunshi Sekkei (Molecular Designing), pp. 163 to 198.

The pyrimidine compound of the present invention or a salt thereof may be an amorphous material or a crystal. Although the crystal form thereof may be a single crystal or a polymorphic mixture, they are included in the pyrimidine compound of the present invention or a salt thereof. The crystal can be produced by crystallizing the pyrimidine compound of the present invention or a salt thereof, applying a known crystallization method. The pyrimidine compound of the present invention or a salt thereof may be either a solvate (e.g., a hydrate, etc.), or a non-solvate, and both of them are included in the compound of the present invention or a salt thereof. Compounds labeled with radioisotopes (e.g., $^{3}H$, $^{14}C$, $^{35}S$, $^{125}I$, etc.) and the like are also included in the pyrimidine compound of the present invention or a salt thereof.

Examples of the other antitumor agent include, but are not particularly limited to, antimetabolites, molecular targeting drugs, platinum drugs, and vegetable alkaloid drugs. These other antitumor agents can be used singly or in combination of two or more thereof.

Examples of the antimetabolite include, but are not particularly limited to, 5-fluorouracil (5-FU), 5-fluoro-2'-deoxyuridine (FdUrd), tegafur, tegafur-uracil combination drugs (e.g., UFT(R)), tegafur-gimeracil-oteracil combination drugs (e.g., TS-1(R)), trifluridine (FTD), trifluridine-tipiracil hydrochloride combination drugs (e.g., Lonsurf(R)), gemcitabine, capecitabine, and cytarabine. Preferable is 5-Fluorouracil (5-FU), trifluridine (FTD), gemcitabine, or capecitabine. These antimetabolites can be used singly or in combination of two or more thereof.

Examples of the molecular targeting drug include ErbB family (EGFR, Her2, Her3, or Her4) inhibitors, PI3K/AKT/mTOR inhibitors, CDK4/6 inhibitors, and estrogen receptor antagonists. These molecular targeting drugs can be used singly or in combination of two or more thereof.

The ErbB family (EGFR, Her2, Her3, Her4) inhibitor is preferably a Her2 inhibitor, more preferably an anti-Her2 antibody such as trastuzumab, pertuzumab, trastuzumab emtansine, trastuzumab deruxtecan, or trastuzumab duocarmazine, and still more preferably trastuzumab, pertuzumab, or trastuzumab emtansine. These ErbB family inhibitors can be used singly or in combination of two or more thereof. In one embodiment, the ErbB family inhibitor is trastuzumab and pertuzumab.

The PI3K/AKT/mTOR inhibitor is a compound that inhibits the signaling pathway of PI3K/AKT/mTOR. Examples

thereof include AZD5363, AZD8055, everolimus (RAD001), dactolisib (BEZ235), buparlisib (BKM120), taselisib (GDC-0032), MK2206, and trans-3-amino-1-methyl-3-[4-(3-phenyl-SH-imidazo[1,2-c]pyrido[3,4-e][1,3]oxazin-2-yl)phenyl]-cyclobutanol. Preferable is AZD8055, everolimus, dactolisib, buparlisib, taselisib, or MK2206. These PI3K/AKT/mTOR inhibitors can be used singly or in combination of two or more thereof.

Examples of the CDK4/6 inhibitor include palbociclib and abemaciclib. Preferable is palbociclib. These CDK4/6 inhibitors can be used singly or in combination of two or more thereof.

Examples of the estrogen receptor antagonist include fulvestrant, tamoxifen, and mepitiostane. Preferable is fulvestrant. These estrogen receptor antagonists can be used singly or in combination of two or more thereof.

Examples of the platinum drug include oxaliplatin, carboplatin, cisplatin, and nedaplatin. Preferable is cisplatin. These platinum drugs can be used singly or in combination of two or more thereof.

Examples of the vegetable alkaloid drug include microtubule inhibitors such as paclitaxel, docetaxel, vinblastine, vincristine, vindesine, vinorelbine, and eribulin, and topoisomerase inhibitors such as irinotecan, nogitecan, and etoposide. More preferable is a taxane microtubule inhibitor such as paclitaxel or docetaxel, or a topoisomerase I inhibitor such as irinotecan or nogitecan, and still more preferable is paclitaxel or irinotecan. These vegetable alkaloid drugs can be used singly or in combination of two or more thereof.

The other antitumor agent is preferably at least one antimetabolite, molecular targeting drug, platinum drug, or vegetable alkaloid drug, more preferably at least one antimetabolite, ErbB family (EGFR, Her2, Her3, or Her4) inhibitor, PI3K/AKT/mTOR inhibitor, CDK4/6 inhibitor, estrogen receptor antagonist, platinum drug, or vegetable alkaloid drug, still more preferably at least one antimetabolite, Her2 inhibitor, PI3K/AKT/mTOR inhibitor, CDK4/6 inhibitor, estrogen receptor antagonist, platinum drug, or vegetable alkaloid drug, even more preferably at least one antimetabolite, Her2 inhibitor, PI3K/AKT/mTOR inhibitor, CDK4/6 inhibitor, estrogen receptor antagonist, platinum drug, microtubule inhibitor, or topoisomerase I inhibitor, yet more preferably at least one antimetabolite, Her2 inhibitor, PI3K/AKT/mTOR inhibitor, CDK4/6 inhibitor, estrogen receptor antagonist, platinum drug, taxane microtubule inhibitor, or topoisomerase I inhibitor, further preferably at least one agent selected from 5-fluorouracil (5-FU), 5-fluoro-2'-deoxyuridine (FdUrd), tegafur, tegafur-uracil combination drugs (e.g., UFT(R), tegafur-gimeracil-oteracil combination drugs (e.g., TS-1(R)), trifluridine, trifluridine-tipiracil hydrochloride combination drugs (e.g., Lonsurf(R)), gemcitabine, capecitabine, cytarabine, trastuzumab, pertuzumab, trastuzumab emtansine, trastuzumab deruxtecan, trastuzumab duocarmazine, AZD5363, AZD8055, everolimus, dactolisib, buparlisib, taselisib, MK2206, trans-3-amino-1-methyl-3-[4-(3-phenyl-5H-imidazo[1,2-c]pyrido[3,4-e][1,3]oxazin-2-yl)phenyl]-cyclobutanol, palbociclib, abemaciclib, fulvestrant, tamoxifen, mepitiostane, oxaliplatin, carboplatin, cisplatin, nedaplatin, paclitaxel, and docetaxel, still further preferably at least one agent selected from 5-fluorouracil (5-FU), trifluridine, gemcitabine, capecitabine, trastuzumab, pertuzumab, trastuzumab emtansine, AZD8055, everolimus, dactolisib, buparlisib, taselisib, palbociclib, fulvestrant, cisplatin, and paclitaxel, and particularly preferably at least one agent selected from capecitabine, trastuzumab, pertuzumab and trastuzumab emtansine.

The other antitumor agent also includes DDS preparations thereof. For example, "paclitaxel" includes albumin-bound paclitaxel (e.g., Abraxane(R)) and paclitaxel micelle (e.g., NK105), etc. "Cisplatin" includes cisplatin micelle (e.g., NC-6004), etc.

In one embodiment of the present invention, examples of antitumor agents include chemotherapeutic agents (e.g., cytotoxic agents), immunotherapeutic agents, hormonal and anti-hormonal agents, targeted therapy agents, and anti-angiogenesis agents. Many antitumor agents can be classified within one or more of these groups. While certain antitumor agents have been categorized within a specific group(s) or subgroup(s) herein, many of these agents can also be listed within one or more other group(s) or subgroup(s), as would be presently understood in the art. The antitumor agent is not particularly limited, and examples thereof include, but are not limited to, a chemotherapeutic agent, a mitotic inhibitor, a plant alkaloid, an alkylating agent, an antimetabolite, a platinum analog, an enzyme, a topoisomerase inhibitor, a retinoid, an aziridine, an antibiotic, a hormonal agent, an anti-hormonal agent, an anti-estrogen, an anti-androgen, an anti-adrenal, an androgen, a targeted therapy agent, an immunotherapeutic agent, a biological response modifier, a cytokine inhibitor, a tumor vaccine, a monoclonal antibody, an immune checkpoint inhibitor, an anti-PD-1 agent, an anti-PD-L1 agent, a colony-stimulating factor, an immunomodulator, an immunomodulatory imide (IMiD), an anti-CTLA4 agent, an anti-LAGl agent, an anti-OX40 agent, a GITR agonist, a CAR-T cell, a BiTE, a signal transduction inhibitor, a growth factor inhibitor, a tyrosine kinase inhibitor, an EGFR inhibitor, a HER2 inhibitor, a histone deacetylase (HDAC) inhibitor, a proteasome inhibitor, a cell-cycle inhibitor, an anti-angiogenesis agent, a matrix-metalloproteinase (MMP) inhibitor, a hepatocyte growth factor inhibitor, a TOR inhibitor, a KDR inhibitor, a VEGF inhibitor, a HIF-1$\alpha$ inhibitor a HIF-2$\alpha$ inhibitor, a fibroblast growth factor (FGF) inhibitor, a RAF inhibitor, a MEK inhibitor, an ERK inhibitor, a PI3K inhibitor, an AKT inhibitor, an MCL-1 inhibitor, a BCL-2 inhibitor, an SHP2 inhibitor, a BRAF-inhibitor, a RAS inhibitor, a gene expression modulator, an autophagy inhibitor, an apoptosis inducer, an antiproliferative agent, and a glycolysis inhibitor.

Non-limiting examples of chemotherapeutic agents include mitotic inhibitors and plant alkaloids, alkylating agents, anti-metabolites, platinum analogs, enzymes, topoisomerase inhibitors, retinoids, aziridines, and antibiotics.

Non-limiting examples of mitotic inhibitors and plant alkaloids include taxanes such as cabazitaxel, docetaxel,

larotaxel, ortataxel, paclitaxel, and tesetaxel; demecolcine; epothilone; eribulin; etoposide (VP16); etoposide phosphate; navelbine; noscapine; teniposide; thaliblastine; vinblastine; vincristine; vindesine; vinflunine; and vinorelbine.

Non-limiting examples of alkylating agents include nitrogen mustards such as chlorambucil, chlornaphazine, cholophosphamide, cytophosphane, estramustine, ifosfamide, mannomustine, mechlorethamine, mechlorethamine oxide hydrochloride, melphalan, novembichin, phenesterine, prednimustine, tris(2-chloroethyl)amine, trofosfamide, and uracil mustard; alkyl sulfonates such as busulfan, improsulfan, and piposulfan; nitrosoureas such as carmustine, chlorozotocin, fotemustine, lomustine, nimustine, ranimustine, streptozotocin, and TA-07; ethylenimines and methylamelamines such as altretamine, thiotepa, triethylenemelamine, triethylenethiophosphaoramide, trietylenephosphoramide, and trimethylolomelamine; ambamustine; bendamustine; dacarbazine; cyclophosphamide; etoglucid; irofulven; mafosfamide; mitobronitol; mitolactol; pipobroman; procarbazine; temozolomide; treosulfan; and triaziquone.

Non-limiting examples of anti-metabolites include folic acid analogues such as aminopterin, denopterin, edatrexate, methotrexate, pteropterin, raltitrexed, and trimetrexate; purine analogs such as 6-mercaptopurine, 6-thioguanine, fludarabine, forodesine, thiamiprine, and thioguanine; pyrimidine analogs such as 5-fluorouracil (5-FU), tegafur/gimeracil/oteracil potassium, tegafur/uracil, trifluridine, trifluridine/tipiracil hydrochloride, 6-azauridine, ancitabine, azacytidine, capecitabine, carmofur, cytarabine, decitabine, dideoxyuridine, doxifiuridine, doxifluridine, enocitabine, floxuridine, galocitabine, gemcitabine, and sapacitabine; 3-aminopyridine-2-carboxaldehyde thiosemicarbazone; broxuridine; cladribine; cyclophosphamide; cytarabine; emitefur; hydroxyurea; mercaptopurine; nelarabine; pemetrexed; pentostatin; tegafur; and troxacitabine.

Non-limiting examples of platinum analogs include carboplatin, cisplatin, dicycloplatin, heptaplatin, lobaplatin, nedaplatin, oxaliplatin, satraplatin, and triplatin tetranitrate.

Non-limiting examples of enzymes include asparaginase and pegaspargase.

Non-limiting examples of topoisomerase inhibitors include acridine carboxamide, amonafide, amsacrine, belotecan, elliptinium acetate, exatecan, indolocarbazole, irinotecan, lurtotecan, mitoxantrone, razoxane, rubitecan, SN-38, sobuzoxane, and topotecan.

Non-limiting examples of retinoids include alitretinoin, bexarotene, fenretinide, isotretinoin, liarozole, RII retinamide, and tretinoin.

Non-limiting examples of aziridines include benzodopa, carboquone, meturedopa, and uredopa.

Non-limiting examples of antibiotics include intercalating antibiotics; anthracenediones; anthracycline antibiotics such as aclarubicin, amrubicin, daunomycin, daunorubicin, doxorubicin, epirubicin, idarubicin, menogaril, nogalamycin, pirarubicin, and valrubicin; 6-diazo-5-oxo- L-norleucine; aclacinomycins; actinomycin; authramycin; azaserine; bleomycins; cactinomycin; calicheamicin; carabicin; carminomycin; carzinophilin; chromomycins; dactinomycin; detorubicin; esorubicin; esperamicins; geldanamycin; marcellomycin; mitomycins; mitomycin C; mycophenolic acid; olivomycins; novantrone; peplomycin; porfiromycin; potfiromycin; puromycin; quelamycin; rebeccamycin; rodorubicin; streptonigrin; streptozocin; *tanespimycin*; tubercidin; ubenimex; zinostatin; zinostatin stimalamer; and zorubicin.

Non-limiting examples of hormonal and anti-hormonal agents include antiandrogens such as abiraterone, apalutamide, bicalutamide, darolutamide, enzalutamide, flutamide, goserelin, leuprolide, and nilutamide; anti-estrogens such as 4- hydroxy tamoxifen, aromatase inhibiting 4(5)-imidazoles, EM-800, fosfestrol, fulvestrant, keoxifene, LY 117018, onapristone, raloxifene, tamoxifen, toremifene, and trioxifene; anti-adrenals such as aminoglutethimide, dexaminoglutethimide, mitotane, and trilostane; androgens such as calusterone, dromostanolone propionate, epitiostanol, mepitiostane, and testolactone; abarelix; anastrozole; cetrorelix; deslorelin; exemestane; fadrozole; finasteride; formestane; histrelin (RL 0903); human chorionic gonadotropin; lanreotide; LDI 200 (Milkhaus); letrozole; leuprorelin; mifepristone; nafarelin; nafoxidine; osaterone; prednisone; thyrotropin alfa; and triptorelin.

Non-limiting examples of immunotherapeutic agents (i.e., immunotherapy) include biological response modifiers, cytokine inhibitors, tumor vaccines, monoclonal antibodies, immune checkpoint inhibitors, colony-stimulating factors, and immunomodulators.

Non-limiting examples of biological response modifiers, including cytokine inhibitors (cytokines) such as interferons and interleukins, include interferon alfa/interferon alpha such as interferon alfa-2, interferon alfa-2a, interferon alfa-2b, interferon alfa-nl, interferon alfa-n3, interferon alfacon-1, peginterferon alfa-2a, peginterferon alfa-2b, and leukocyte alpha interferon; interferon beta such as interferon beta-1a, and interferon beta-1b; interferon gamma such as natural interferon gamma-1a, and interferon gamma-1b; aldesleukin; interleukin-1 beta; interleukin-2; oprelvekin; sonermin; tasonermin; and virulizin.

Non-limiting examples of tumor vaccines include APC 8015, AVICINE, bladder cancer vaccine, cancer vaccine (Biomira), gastrin 17 immunogen, Maruyama vaccine, melanoma lysate vaccine, melanoma oncolysate vaccine (New York Medical College), melanoma vaccine (New York University), melanoma vaccine (Sloan Kettering Institute), TICE® BCG (Bacillus Calmette-Guerin), and viral melanoma cell lysates vaccine (Royal Newcastle Hospital).

Non-limiting examples of monoclonal antibodies include abagovomab, adecatumumab, aflibercept, alemtuzumab, blinatumomab, brentuximab vedotin, CA 125 MAb (Biomira), cancer MAb (Japan Pharmaceutical Development), daclizumab, daratumumab, denosumab, edrecolomab, gemtuzumab zogamicin, HER- 2 and Fc MAb (Medarex), ibritumomab tiuxetan, idiotypic 105AD7 MAb (CRC Technology), idiotypic CEA MAb (Trilex), ipilimumab, lintuzumab, LYM-1 -iodine 131 MAb (Techni clone), mitumomab, moxetumomab, ofatumumab, polymorphic epithelial mucin-yttrium 90 MAb (Antisoma), ranibizumab, rituximab, veltuzumab, and trastuzumab.

Non-limiting examples of immune checkpoint inhibitors include anti-PD-1 agents or antibodies such as cemiplimab, nivolumab, and pembrolizumab; anti-PD-L1 agents or antibodies such as atezolizumab, avelumab, and durvalumab; anti-CTLA-4 agents or antibodies such as ipilimumab and tremelimumab; anti-LAG1 agents; and anti-OX40 agents.

Non-limiting examples of colony-stimulating factors include darbepoetin alfa, epoetin alfa, epoetin beta, filgrastim, granulocyte macrophage colony stimulating factor, lenograstim, leridistim, mirimostim, molgramostim, nartograstim, pegfilgrastim, and sargramostim.

Non-limiting examples of additional immunotherapeutic agents include BiTEs, CAR-T cells, GITR agonists, imiquimod, immunomodulatory imides (IMiDs), mismatched double stranded RNA (Ampligen), resiquimod, SRL 172, and thymalfasin. Targeted therapy agents include, for example, monoclonal antibodies and small molecule drugs.

Non-limiting examples of targeted therapy agents include signal transduction inhibitors, growth factor inhibitors, tyrosine kinase inhibitors, EGFR inhibitors, HER2 inhibitors, histone deacetylase (HDAC) inhibitors, proteasome inhibitors, cell-cycle inhibitors, angiogenesis inhibitors, matrix-metalloproteinase (MMP) inhibitors, hepatocyte growth factor inhibitors, TOR inhibitors, KDR inhibitors, VEGF inhibitors, fibroblast growth factors (FGF) inhibitors, RAF inhibitors, MEK inhibitors, ERK inhibitors, PI3K inhibitors, AKT inhibitors, MCL-1 inhibitors, BCL-2 inhibitors, SHP2 inhibitors, BRAF-inhibitors, RAS inhibitors, gene expression modulators, autophagy inhibitors, apoptosis inducers, antiproliferative agents, and glycolysis inhibitors.

Non-limiting examples of signal transduction inhibitors include tyrosine kinase inhibitors, multiple-kinase inhibitors, anlotinib, avapritinib, axitinib, dasatinib, dovitinib, imatinib, lenvatinib, lonidamine, nilotinib, nintedanib, pazopanib, pegvisomant, ponatinib, vandetanib, and EGFR and/or HER2 inhibitory agents (i.e., other than fomula (I) or its salt).

Non-limiting examples of EGFR inhibitors include small molecule antagonists of EGFR such as afatinib, brigatinib, erlotinib, gefitinib, lapatinib, neratinib, dacomitinib, vandetanib, and osimertinib antibody-based EGFR inhibitors, including any anti-EGFR antibody or antibody fragment that can partially or completely block EGFR activation by its natural ligand; and specific antisense nucleotide or siRNA;. Antibody-based EGFR inhibitory agents may include, for example, those described in Modjtahedi, H., et al., 1993, Br. J. Cancer 67:247-253; Teramoto, T., et al., 1996, Cancer 77:639-645; Goldstein et al, 1995, Clin. Cancer Res. 1 : 1311-1318; Huang, S. M., et al., 1999, Cancer Res. 15:59(8): 1935-40; and Yang, X., et al., 1999, Cancer Res. 59: 1236-1243; monoclonal antibody Mab E7.6.3 (Yang, 1999 supra); Mab C225 (ATCC Accession No. HB-8508), or an antibody or antibody fragment having the binding specificity thereof; cetuximab; matuzumab; necitumumab; nimotuzumab; panitumumab; and zalutumumab.

Non-limiting examples of HER2 inhibitors include HER2 tyrosine kinase inhibitors such as afatinib, lapatinib, neratinib, and tucatinib; and anti-HER2 antibodies or drug conjugates thereof such as trastuzumab, trastuzumab emtansine (T-DM1), pertuzumab, margetuximab, trastuzumab deruxtecan (DS-8201a), and trastuzumab duocarmazine.

Non-limiting examples of histone deacetylase (HDAC) inhibitors include belinostat, panobinostat, romidepsin, and vorinostat.

Non-limiting examples of proteasome inhibitors include bortezomib, carfilzomib, ixazomib, marizomib (salinosporamide a), and oprozomib.

Non-limiting examples of cell-cycle inhibitors, including CDK inhibitors, include abemaciclib, alvocidib, palbociclib, and ribociclib.

Non-limiting examples of anti-angiogenic agents (or angiogenesis inhibitors) include, but not limited to, matrix-metalloproteinase (MMP) inhibitors; VEGF inhibitors; EGFR inhibitors; TOR inhibitors such as everolimus and temsirolimus; PDGFR kinase inhibitory agents such as crenolanib; HIF-1$\alpha$ inhibitors such as PX 478; HIF-2$\alpha$ inhibitors such as belzutifan and the HIF-2$\alpha$ inhibitors described in WO 2015/035223; fibroblast growth factor (FGF) or FGFR inhibitory agents such as B-FGF and RG 13577; hepatocyte growth factor inhibitors; KDR inhibitors; anti-Ang1 and anti-Ang2 inhibitory agents; Tie2 kinase inhibitory agents; Tek antagonists (US 2003/0162712; US 6,413,932); anti-TWEAK agents (US 6,727,225); ADAM disintegrin domain to antagonize the binding of integrin to its ligands (US 2002/0042368); anti-eph receptor and/or anti-eph antibodies or antigen binding regions (US 5,981,245; 5,728,813; 5,969,110; 6,596,852; 6,232,447; and 6,057,124); and anti-PDGF-BB antagonists as well as antibodies or antigen binding regions specifically binding to PDGF-BB ligands.

Non-limiting examples of matrix-metalloproteinase (MMP) inhibitors include MMP-2 (matrix-metalloproteinase 2) inhibitors, MMP-9 (matrix-metalloproteinase 9) inhibitors, prinomastat, RO 32-3555, and RS 13-0830. Examples of useful matrix metalloproteinase inhibitors are described, for example, in WO 96/33172, WO 96/27583, EP 1004578 , WO 98/07697, WO 98/03516, WO 98/34918, WO 98/34915, WO 98/33768, WO 98/30566, EP 0606046, EP 0931788, WO 90/05719, WO 99/52910, WO 99/52889, WO 99/29667, WO 1999/007675 , EP 1786785, EP 1181017, US 2009/0012085 , US 5,863,949, US 5,861,510, and EP 0780386. Preferred MMP-2 and MMP-9 inhibitors are those that have little or no activity inhibiting MMP-1. More preferred, are those that selectively inhibit MMP-2 and/or MMP-9 relative

to the other matrix-metalloproteinases (i.e., MMP-1, MMP-3, MMP-4, MMP-5, MMP-6, MMP- 7, MMP- 8, MMP-10, MMP-11, MMP-12, and MMP-13).

Non-limiting examples of VEGF and VEGFR inhibitory agents include bevacizumab, cediranib, CEP 7055, CP 547632, KRN 633, orantinib, pazopanib, pegaptanib, pegaptanib octasodium, semaxanib, sorafenib, sunitinib, VEGF antagonist (Borean, Denmark), and VEGF-TRAP™.

Other anti-angiogenic agents may include, but are not limited to, 2-methoxyestradiol, AE 941, alemtuzumab, alpha-D148 Mab (Amgen, US), alphastatin, anecortave acetate, angiocidin, angiogenesis inhibitors (SUGEN, US), angiostatin, anti-Vn Mab (Crucell, Netherlands), atiprimod, axitinib, AZD 9935, BAY RES 2690 (Bayer, Germany), BC 1 (Genoa Institute of Cancer Research, Italy), beloranib, benefin (Lane Labs, US), cabozantinib, CDP 791 (Celltech Group, UK), chondroitinase AC, cilengitide, combretastatinA4 prodrug, CP 564959 (OSI, US), CV247, CYC 381 (Harvard University, US), E 7820, EHT 0101, endostatin, enzastaurin hydrochloride, ER-68203-00 (IVAX, US), fibrinogen-E fragment, Flk-1 (ImClone Systems, US), forms of FLT 1 (VEGFR 1), FR-111142, GCS-100, GW 2286 (GlaxoSmithKline, UK), IL-8, ilomastat, IM-862, irsogladine, KM-2550 (Kyowa Hakko, Japan), lenalidomide, lenvatinib, MAb alpha5beta3 integrin, VEGF (Xenova, UK), marimastat, maspin (Sosei, Japan), metastatin, motuporamine C, M-PGA, ombrabulin, OXI4503, PI 88, platelet factor 4, PPI 2458, ramucirumab, rBPI 21 and BPI-derived antiangiogenic (XOMA, US), regorafenib, SC-236, SD-7784 (Pfizer, US), SDX 103 (University of California at San Diego, US), SG 292 (Telios, US), SU-0879 (Pfizer, US), TAN-1120, TBC-1635, tesevatinib, tetrathiomolybdate, thalidomide, thrombospondin 1 inhibitor, Tie-2 ligands (Regeneron, US), tissue factor pathway inhibitors (EntreMed, US), tumor necrosis factor-alpha inhibitors, tumstatin, TZ 93, urokinase plasminogen activator inhibitors, vadimezan, vandetanib, vasostatin, vatalanib, VE-cadherin-2 antagonists, xanthorrhizol, XL 784 (Exelixis, US), ziv-aflibercept, and ZD 6126.

The antitumor agent(s) that may be combined with formula (I) may also be an active agent that disrupts or inhibits RAS-RAF-ERK or PI3K-AKT-TOR signaling pathways or is a PD-1 and/or PD-L1 antagonist. Examples of which include, but are not limited to, a RAF inhibitor, an EGFR inhibitor, a MEK inhibitor, an ERK inhibitor, a PI3K inhibitor, an AKT inhibitor, a TOR inhibitor, an MCL-1 inhibitor, a BCL-2 inhibitor, a SHP2 inhibitor, a proteasome inhibitor, or an immune therapy, including monoclonal antibodies, immunomodulatory imides (IMiDs), anti-PD-1, anti-PD-L1, anti-CTLA4, anti-LAG1, and anti-OX40 agents, GITR agonists, CAR-T cells, and BiTEs.

Non-limiting examples of RAF inhibitors include dabrafenib, encorafenib, regorafenib, sorafenib, and vemurafenib.

Non-limiting examples of MEK inhibitors include binimetinib, CI-1040, cobimetinib, PD318088, PD325901, PD334581, PD98059, refametinib, selumetinib, and trametinib.

Non-limiting examples of ERK inhibitors include LY3214996, LTT462, MK-8353, SCH772984, ravoxertinib, ulixertinib, and ASTX029.

Non-limiting examples of PI3K inhibitors include 17-hydroxywortmannin analogs (e.g., WO 06/044453); AEZS-136; alpelisib; AS-252424; buparlisib; CAL263; copanlisib; CUDC-907; dactolisib (WO 06/122806); demethoxyviridin; duvelisib; GNE-477; GSK1059615; IC87114; idelalisib; INK1117; LY294002; Palomid 529; paxalisib; perifosine; PI-103; PI-103 hydrochloride; pictilisib (e.g., WO 09/036,082; WO 09/055,730); PIK 90; PWT33597; SF1126; sonolisib; TGI 00-115; TGX-221; XL147; XL-765; wortmannin; taselisib (GDC-0032); and ZSTK474.

Non-limiting examples of AKT inhibitors include Akt-1-1 (inhibits Akt1) (Barnett et al. (2005) Biochem. J., 385 (Pt. 2), 399-408); Akt-1-1,2 (Barnett et al. (2005) Biochem. J. 385 (Pt. 2), 399-408); API-59CJ-Ome (*e.g.,* Jin et al. (2004) Br. J. Cancer 91, 1808-12); 1-H-imidazo[4,5-c]pyridinyl compounds (*e.g.,* WO05/011700); indole-3-carbinol and derivatives thereof (*e.g.,* U.S. Patent No. 6,656,963; Sarkar and Li (2004) J Nutr. 134(12 Suppl), 3493S-3498S); perifosine, (Dasmahapatra et al. (2004) Clin. Cancer Res. 10(15), 5242-52, 2004); phosphatidylinositol ether lipid analogues (*e.g.,* Gills and Dennis (2004) Expert. Opin. Investig. Drugs 13, 787-97); triciribine (Yang et al. (2004) Cancer Res. 64,4394-9); imidazooxazone compounds including trans-3-amino-1-methyl-3-[4-(3-phenyl-5H-imidazo[1,2-c]pyrido[3,4-e] [1,3]oxazin-2-yl)phenyl]-cyclobutanol hydrochloride (WO 2012/137870); afuresertib; capivasertib; 8-[4-(1-aminocyclobutyl)phenyl]-9-phenyl-1,2,4-triazolo[3,4-f][1,6]naphthyridin-3 (2H)-one (MK2206) and pharmaceutically acceptable salts thereof; AZD5363; trans-3-amino-1-methyl-3-(4-(3-phenyl-5H-imidazo[1,2-c]pyrido[3,4-e][1,3]oxazin-2-yl)phenyl)cyclobutanol (TAS-117) and pharmaceutically acceptable salts thereof; and patasertib.

Non-limiting examples of TOR inhibitors include deforolimus; ATP-competitive TORC1/TORC2 inhibitors, including PI-103, PP242, PP30, and Torin 1; TOR inhibitors in FKBP12 enhancer, rapamycins and derivatives thereof, including temsirolimus, everolimus, WO 9409010; rapalogs, e.g. as disclosed in WO 98/02441 and WO 01/14387, e.g. AP23573, AP23464, or AP23841; 40-(2-hydroxyethyl)rapamycin, 40-[3-hydroxy(hydroxymethyl)methylpropanoate]-rapamycin; 40-epi-(tetrazolyl)-rapamycin (also called ABT578); AZD8055; 32-deoxorapamycin; 16-pentynyloxy-32(S)-dihydrorapanycin, and other derivatives disclosed in WO 05/005434; derivatives disclosed in US 5,258,389, WO 94/090101, WO 92/05179, US 5,118,677, US 5,118,678, US 5,100,883, US 5,151,413, US 5,120,842, WO 93/111130, WO 94/02136, WO 94/02485, WO 95/14023, WO 94/02136, WO 95/16691, WO 96/41807, WO 96/41807 and US 5,256,790; and phosphorus-containing rapamycin derivatives (*e.g.,* WO 05/016252).

Non-limiting examples of MCL-1 inhibitors include AMG-176, MIK665, and S63845.

Non-limiting examples of SHP2 inhibitors include JAB-3068, RMC-4630, TNO155, SHP-099, RMC-4550, and SHP2 inhibitors described in WO 2019/167000, WO 2020/022323 and WO2021/033153.

Non-limiting examples of RAS inhibitors include AMG510, MRTX849, LY3499446, JNJ-74699157 (ARS-3248), ARS-1620, ARS-853, RM-007, and RM-008.

Additional non-limiting examples of antitumor agents that may be suitable for use include, but are not limited to, 2-ethylhydrazide, 2',2'-trichlorotriethylamine, ABVD, aceglatone, acemannan, aldophosphamide glycoside, alpharadin, amifostine, aminolevulinic acid, anagrelide, ANCER, ancestim, anti-CD22 immunotoxins, antitumorigenic herbs, apaziquone, arglabin, arsenic trioxide, azathioprine, BAM 002 (Novelos), bcl-2 (Genta), bestrabucil, biricodar, bisantrene, bromocriptine, brostallicin, bryostatin, buthionine sulfoximine, calyculin, cell-cycle nonspecific antineoplastic agents, celmoleukin, clodronate, clotrimazole, cytarabine ocfosfate, DA 3030 (Dong-A), defofamine, denileukin diftitox, dexrazoxane, diaziquone, dichloroacetic acid, dilazep, discodermolide, docosanol, doxercalciferol, edelfosine, eflomithine, EL532 (Elan), elfomithine, elsamitrucin, eniluracil, etanidazole, exisulind, ferruginol, folic acid replenisher such as frolinic acid, gacytosine, gallium nitrate, gimeracil/oteracil/tegafur combination (S-1), glycopine, histamine dihydrochloride, HIT diclofenac, HLA-B7 gene therapy (Vical), human fetal alpha fetoprotein, ibandronate, ibandronic acid, ICE chemotherapy regimen, imexon, iobenguane, IT-101 (CRLX101), laniquidar, LC 9018 (Yakult), leflunomide, lentinan, levamisole + fluorouracil, lovastatin, lucanthone, masoprocol, melarsoprol, metoclopramide, miltefosine, miproxifene, mitoguazone, mitozolomide, mopidamol, motexafin gadolinium, MX6 (Galderma), naloxone + pentazocine, nitracrine, nolatrexed, NSC 631570 octreotide (Ukrain), olaparib, P-30 protein, PAC-1, palifermin, pamidronate, pamidronic acid, pentosan polysulfate sodium, phenamet, picibanil, pixantrone, platinum, podophyllinic acid, porfimer sodium, PSK (Polysaccharide-K), rabbit antithymocyte polyclonal antibody, rasburiembodiment, retinoic acid, rhenium Re 186 etidronate, romurtide, samarium (153 Sm) lexidronam, sizofiran, sodium phenylacetate, sparfosic acid, spirogermanium, strontium-89 chloride, suramin, swainsonine, talaporfin, tariquidar, tazarotene, tegafur-uracil, temoporfin, tenuazonic acid, tetrachlorodecaoxide, thrombopoietin, tin ethyl etiopurpurin, tirapazamine, TLC ELL-12, tositumomab-iodine 131, trifluridine and tipiracil combination, troponin I (Harvard University, US), urethan, valspodar, verteporfin, zoledronic acid, and zosuquidar.

In the present description, the term "HER2" includes the HER2 of a human or a non-human mammal, and it is preferably human HER2. NCBIGene ID of human HER2 is 2064. Furthermore, the term "HER2" includes isoforms.

In the present description, the term "EGFR" includes the EGFR of a human or a non-human mammal, and it is preferably human EGFR. NCBIGene ID of human EGFR is 1956. Furthermore, the term " EGFR " includes isoforms.

The tumor that is the target of the present invention is not particularly limited as long as the antitumor agent exerts an antitumor effect thereon. Preferable is tumor on which a pyrimidine compound represented by the formula (I) or a salt thereof exerts an antitumor effect, and more preferable is malignant tumor associated with HER2 or malignant tumor associated with EGFR. In this context, the "malignant tumor associated with HER2" means malignant tumor, in which a reduction in the incidence, or the remission, alleviation and/or complete recovery of the symptoms thereof is achieved by deleting, suppressing and/or inhibiting the function of HER2. Such malignant tumor is preferably malignant tumor having HER2 overexpression, HER2 gene amplification, or HER2 mutation. In one embodiment, the "malignant tumor associated with HER2" is HER2-positive tumor. The "malignant tumor associated with EGFR" means malignant tumor, in which a reduction in the incidence, or the remission, alleviation and/or complete recovery of the symptoms thereof is achieved by deleting, suppressing and/or inhibiting the function of EGFR. Such malignant tumor is preferably malignant tumor having EGFR overexpression, EGFR gene amplification, or EGFR mutation. In one embodiment, the "malignant tumor associated with EGFR" is EGFR-positive tumor. In one embodiment, the tumor that is the target of the present invention is malignant tumor having HER2 overexpression, HER2 gene amplification, or HER2 mutation, or malignant tumor having EGFR overexpression, EGFR gene amplification, or EGFR mutation. In one embodiment, the tumor that is the target of the present invention is HER2-positive tumor or EGFR-positive tumor.

One embodiment of the present invention provides an antitumor agent for combined administration with other antitumor agent, the antitumor agent comprising the pyrimidine compound of the present invention or a salt thereof. One embodiment of the present invention provides an antitumor agent involving the combined administration of the pyrimidine compound of the present invention or a salt thereof and other antitumor agent. One embodiment of the present invention provides a combination (pharmaceutical combination, combination method, combination product, or therapeutic combination) of the pyrimidine compound of the present invention or a salt thereof and other antitumor agent.

One embodiment of the present invention provides an antitumor agent for combined administration with other antitumor agent, the antitumor agent comprising the pyrimidine compound of the present invention or a salt thereof for the treatment of tumor. One embodiment of the present invention provides a combination (pharmaceutical combination, combination method, combination product, or therapeutic combination) of the pyrimidine compound of the present invention or a salt thereof and other antitumor agent for the treatment of tumor. One embodiment of the present invention provides use of a combination (pharmaceutical combination, combination method, combination product, or therapeutic combination) of the pyrimidine compound of the present invention or a salt thereof and other antitumor agent for the treatment of tumor. One embodiment of the present invention provides an antitumor agent involving the administration of the pyrimidine compound of the present invention or a salt thereof to a tumor patient given other antitumor agent or

a tumor patient to be given (scheduled to be given) other antitumor agent. One embodiment of the present invention provides an antitumor agent involving the administration of other antitumor agent to a tumor patient given the pyrimidine compound of the present invention or a salt thereof or a tumor patient to be given (scheduled to be given) the pyrimidine compound of the present invention or a salt thereof.

In one embodiment of the present invention, the antitumor agent is orally administered.

One embodiment of the present invention provides use of the pyrimidine compound of the present invention or a salt thereof for the production of a medicament that is used in combination with other antitumor agent in the treatment of tumor. One embodiment of the present invention provides use of other antitumor agent for the production of a medicament that is used in combination with the pyrimidine compound of the present invention or a salt thereof in the treatment of tumor. One embodiment of the present invention provides use of a combination of the pyrimidine compound of the present invention or a salt thereof and other antitumor agent for the production of a medicament in the treatment of tumor.

One embodiment of the present invention provides an antitumor effect potentiator for potentiating the antitumor effect of other antitumor agent, the antitumor effect potentiator containing the pyrimidine compound of the present invention or a salt thereof.

One embodiment of the present invention provides the pyrimidine compound of the present invention or a salt thereof, or use of the pyrimidine compound of the present invention or a salt thereof for use in the treatment of tumor involving combined use with other antitumor agent. One embodiment of the present invention provides other antitumor agent, or use of other antitumor agent for use in the treatment of tumor involving combined use with the pyrimidine compound of the present invention or a salt thereof. One embodiment of the present invention provides the pyrimidine compound of the present invention or a salt thereof, or use of the pyrimidine compound of the present invention or a salt thereof for treating a tumor patient given other antitumor agent or a tumor patient to be given (scheduled to be given) other antitumor agent. One embodiment of the present invention provides other antitumor agent, or use of other antitumor agent for treating a tumor patient given the pyrimidine compound of the present invention or a salt thereof or a tumor patient to be given (scheduled to be given) the pyrimidine compound of the present invention or a salt thereof.

One embodiment of the present invention provides a combination antitumor agent involving the combined administration of the pyrimidine compound of the present invention or a salt thereof and other antitumor agent. One embodiment of the present invention provides a combination (pharmaceutical combination, combination method, combination product, or therapeutic combination) of the pyrimidine compound of the present invention or a salt thereof and other antitumor agent, wherein the pyrimidine compound or the salt thereof and the other antitumor agent are administered concurrently, sequentially, or at an interval in the treatment of tumor.

One embodiment of the present invention provides the pyrimidine compound of the present invention or a salt thereof for potentiating the antitumor effect of other antitumor agent. One embodiment of the present invention provides use of the pyrimidine compound of the present invention or a salt thereof for potentiating the antitumor effect of other antitumor agent.

One embodiment of the present invention provides a method for treating tumor, comprising the step of administering a therapeutically effective amount of the pyrimidine compound of the present invention or a salt thereof and other antitumor agent in combination to a patient. One embodiment of the present invention provides a method for treating tumor, comprising the step of administering an effective amount of the pyrimidine compound of the present invention or a salt thereof and other antitumor agent to a patient in need thereof. One embodiment of the present invention provides a method for treating tumor, comprising administering an effective amount of a combination (pharmaceutical combination or combination product) of the pyrimidine compound of the present invention or a salt thereof and other antitumor agent to a patient in need thereof. One embodiment of the present invention provides a method for treating tumor, comprising the step of administering a therapeutically effective amount of the pyrimidine compound of the present invention or a salt thereof to a tumor patient given other antitumor agent or a tumor patient to be given (scheduled to be given) other antitumor agent. One embodiment of the present invention provides a method for treating tumor by combined use with other antitumor agent, comprising administering an effective amount of the pyrimidine compound of the present invention or a salt thereof to a patient in need thereof.

One embodiment of the present invention provides a pharmaceutical composition for the treatment of tumor, comprising the pyrimidine compound of the present invention or a salt thereof and other antitumor agent.

One embodiment of the present invention provides a method for treating tumor, or a method for potentiating an antitumor effect, comprising the step of administering a therapeutically effective amount of other antitumor agent to a tumor patient given the pyrimidine compound of the present invention or a salt thereof or a tumor patient to be given (scheduled to be given) the pyrimidine compound of the present invention or a salt thereof. One embodiment of the present invention provides a method for potentiating an antitumor effect, comprising the step of administering a therapeutically effective amount of the pyrimidine compound of the present invention or a salt thereof to a tumor patient given other antitumor agent or a tumor patient to be given (scheduled to be given) the pyrimidine compound of the present invention or a salt thereof.

One embodiment of the present invention provides an antitumor agent for combined administration with other

antitumor agent, the antitumor agent comprising 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof. One embodiment of the present invention provides an antitumor agent involving the combined administration of 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof and other antitumor agent. One embodiment of the present invention provides a combination (pharmaceutical combination, combination method, combination product, or therapeutic combination) of 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof and other antitumor agent.

One embodiment of the present invention provides an antitumor agent for combined administration with other antitumor agent, the antitumor agent comprising 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof for the treatment of tumor. One embodiment of the present invention provides a combination (pharmaceutical combination, combination method, combination product, or therapeutic combination) of 7-((3R,SS)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof and other antitumor agent for the treatment of tumor. One embodiment of the present invention provides use of a combination (pharmaceutical combination, combination method, combination product, or therapeutic combination) of 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof and other antitumor agent for the treatment of tumor. One embodiment of the present invention provides an antitumor agent involving the administration of 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof to a tumor patient given other antitumor agent or a tumor patient to be given (scheduled to be given) other antitumor agent. One embodiment of the present invention provides an antitumor agent involving the administration of other antitumor agent to a tumor patient given 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof or a tumor patient to be given (scheduled to be given) 7-((3R,SS)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof.

In one embodiment of the present invention, the antitumor agent is orally administered.

One embodiment of the present invention provides use of 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof for the production of a medicament that is used in combination with other antitumor agent in the treatment of tumor. One embodiment of the present invention provides use of other antitumor agent for the production of a medicament that is used in combination with 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof in the treatment of tumor. One embodiment of the present invention provides use of a combination of 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof and other antitumor agent for the production of a medicament in the treatment of tumor.

One embodiment of the present invention provides an antitumor effect potentiator for potentiating the antitumor effect of other antitumor agent, the antitumor effect potentiator containing 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof.

One embodiment of the present invention provides 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof, or use of 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof for use in the treatment of tumor involving combined use with other antitumor agent. One embodiment of the present invention provides other antitumor agent, or use of other antitumor agent for use in the treatment of tumor involving combined use with 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof. One embodiment of the present invention provides 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof, or use of 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof for treating a tumor patient given other antitumor agent or a tumor patient to be given (scheduled to be given) other antitumor agent. One embodiment of the present invention provides other antitumor agent, or use of other antitumor agent for treating a tumor patient given 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof or a tumor patient to be given (scheduled to be given) 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof.

One embodiment of the present invention provides a combination antitumor agent involving the combined administration of 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof and other antitumor agent. One embodiment of the present

invention provides a combination (pharmaceutical combination, combination method, combination product, or therapeutic combination) of 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof and other antitumor agent, wherein the compound or the salt thereof and the other antitumor agent are administered concurrently, sequentially, or at an interval in the treatment of tumor.

One embodiment of the present invention provides 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof for potentiating the antitumor effect of other antitumor agent. One embodiment of the present invention provides use of 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof for potentiating the antitumor effect of other antitumor agent. One embodiment of the present invention provides use of 7-((3R,SS)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof for potentiating the antitumor effect of other antitumor agent.

One embodiment of the present invention provides a method for treating tumor, comprising the step of administering a therapeutically effective amount of 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof and other antitumor agent in combination to a patient. One embodiment of the present invention provides a method for treating tumor, comprising the step of administering an effective amount of 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof and other antitumor agent to a patient in need thereof. One embodiment of the present invention provides a method for treating tumor, comprising administering an effective amount of a combination (pharmaceutical combination or combination product) of 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof and other antitumor agent to a patient in need thereof. One embodiment of the present invention provides a method for treating tumor, comprising the step of administering a therapeutically effective amount of 7-((3R,5S)-1-acryloyl-5-methylpyrrolidin-3 -yl)-4-amino-6-(cyclopropylethynyl)-N-((R)-1 -phenylethyl)-7H-pyrrolo[2,3-d]pyrimidine-5-carboxamide or a salt thereof to a tumor patient given other antitumor agent or a tumor patient to be given (scheduled to be given) other antitumor agent. One embodiment of the present invention provides a method for treating tumor by combined use with other antitumor agent, comprising administering an effective amount of 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof to a patient in need thereof.

One embodiment of the present invention provides a pharmaceutical composition for the treatment of tumor, comprising 7-((3R,5S)-1-acryloyl-5-methylpyrrolidin-3 -yl)-4-amino-6-(cyclopropylethynyl)-N-((R)-1 -phenylethyl)-7H-pyrrolo[2,3-d]pyrimidine-5-carboxamide or a salt thereof and other antitumor agent.

One embodiment of the present invention provides a method for treating tumor, or a method for potentiating an antitumor effect, comprising the step of administering a therapeutically effective amount of other antitumor agent to a tumor patient given 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof or a tumor patient to be given (scheduled to be given). One embodiment of the present invention provides a method for potentiating an antitumor effect, comprising the step of administering a therapeutically effective amount of 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide or a salt thereof to a tumor patient given other antitumor agent or a tumor patient to be given (scheduled to be given) other antitumor agent.

One embodiment of the present invention provides an antitumor agent for use in the treatment of malignant tumor associated with HER2, wherein other antitumor agent that is used in combination with the pyrimidine compound of the present invention or a salt thereof is at least one antimetabolite, Her2 inhibitor, PI3K/AKT/mTOR inhibitor, CDK4/6 inhibitor, estrogen receptor antagonist, platinum drug, or vegetable alkaloid drug.

One embodiment of the present invention provides an antitumor agent for use in the treatment of malignant tumor associated with EGFR, wherein other antitumor agent that is used in combination with the pyrimidine compound of the present invention or a salt thereof is at least one antimetabolite, PI3K/AKT/mTOR inhibitor, CDK4/6 inhibitor, estrogen receptor antagonist, platinum drug, or vegetable alkaloid drug.

The antitumor agent of the present invention may be used in postoperative adjuvant chemotherapy which is performed for preventing recurrence after surgical removal of tumor, or may be used in preoperative adjuvant chemotherapy which is performed for surgically removing tumor.

The wild-type human HER2 gene is shown in, for example, SEQ ID NO: 1. The wild-type HER2 protein consists of the amino acid sequence set forth in, for example, SEQ ID NO: 2. The nucleotide sequence information of the human HER2 gene can be obtained from, for example, Accession No. NM_004448, or the like and the amino acid sequence information of the wild-type HER2 protein can be obtained from, for example, Accession No. NP_004439, or the like.

In several embodiments, the pyrimidine compound of the present invention or a salt thereof exhibits inhibitory activity against mutant HER2 comprising one or more mutations from G309A, S310F, R678Q, L755S, L755_T759del,

D769H, A775_G776insYVMA, V777L, V842I and R896C, using the amino acid sequence set forth in SEQ ID NO: 2 as a reference. In another embodiment, the pyrimidine compound of the present invention or a salt thereof exhibits inhibitory activity against mutant HER2 comprising A775_G776insYVMA, using the amino acid sequence set forth in SEQ ID NO: 2 as a reference.

Further, in several embodiments, with regard to a mutation in a certain HER2 isoform, even when the position of the mutation is different from the position of an amino acid shown in SEQ ID NO: 2 due to deletion or insertion of an amino acid(s), it is understood that the mutation is the same as the mutation at a position corresponding to the position of the amino acid shown in SEQ ID NO: 2. Hence, for example, the glycine at position 309 in the HER2 shown in SEQ ID NO: 2 corresponds to glycine at position 294 in HER2 consisting of the amino acid sequence set forth in SEQ ID NO: 4. For example, the term "G309A" means that the glycine at position 309 in the HER2 shown in SEQ ID NO: 2 is mutated to alanine. Since such "G309" is at a position corresponding to the amino acid at position 294 in HER2 consisting of the amino acid sequence set forth in SEQ ID NO: 4, "G294A" in the HER2 consisting of the amino acid sequence set forth in SEQ ID NO: 4 corresponds to "G309A" in the HER2 shown in SEQ ID NO: 2. Besides, the position of an amino acid in SEQ ID NO: 2 that corresponds to a certain amino acid in a certain HER2 isoform can be confirmed by Multiple Alignment of BLAST.

Sequence Listing

[Table A]

| |
|---|
| SEQ ID NO: 1<br>Accession No.: NM_004448<br>CDS: 262..4029 |

(continued)

SEQ ID NO: 1

```
   1 gcttgctccc aatcacagga gaaggaggag gtggaggagg agggctgctt gaggaagtat
  61 aagaatgaag ttgtgaagct gagattcccc tccattggga ccggagaaac caggggagcc
 121 ccccgggcag ccgcgcgccc cttcccacgg ggccctttac tgcgccgcgc gcccggcccc
 181 cacccctcgc agcaccccgc gccccgcgcc ctcccagccg ggtccagccg gagccatggg
 241 gccggagccg cagtgagcac catggagctg gcggccttgt gccgctgggg gctcctcctc
 301 gccctcttgc cccccggagc cgcgagcacc caagtgtgca ccggcacaga catgaagctg
 361 cggctccctg ccagtcccga gacccacctg gacatgctcc gccacctcta ccagggctgc
 421 caggtggtgc agggaaacct ggaactcacc tacctgccca ccaatgccag cctgtccttc
 481 ctgcaggata tccaggaggt gcagggctac gtgctcatcg ctcacaacca agtgaggcag
 541 gtcccactgc agaggctgcg gattgtgcga ggcacccagc tctttgagga caactatgcc
 601 ctggccgtgc tagacaatgg agacccgctg aacaatacca cccctgtcac aggggcctcc
 661 ccaggaggcc tgcgggagct gcagcttcga agcctcacag agatcttgaa aggaggggtc
 721 ttgatccagc ggaaccccca gctctgctac caggacacga ttttgtggaa ggacatcttc
 781 cacaagaaca accagctggc tctcacactg atagacacca accgctctcg ggcctgccac
 841 ccctgttctc cgatgtgtaa gggctcccgc tgctggggag agagttctga ggattgtcag
 901 agcctgacgc gcactgtctg tgccggtggc tgtgcccgct gcaaggggcc actgcccact
 961 gactgctgcc atgagcagtg tgctgccggc tgcacgggcc ccaagcactc tgactgcctg
1021 gcctgcctcc acttcaacca cagtggcatc tgtgagctgc actgcccagc cctggtcacc
1081 tacaacacag acacgtttga gtccatgccc aatcccgagg gccggtatac attcggcgcc
1141 agctgtgtga ctgcctgtcc ctacaactac ctttctacgg acgtgggatc ctgcaccctc
1201 gtctgccccc tgcacaacca agaggtgaca gcagaggatg gaacacagcg gtgtgagaag
1261 tgcagcaagc cctgtgcccg agtgtgctat ggtctgggca tggagcactt gcgagaggtg
1321 agggcagtta ccagtgccaa tatccaggag tttgctggct gcaagaagat ctttgggagc
1381 ctggcatttc tgccggagag ctttgatggg gacccagcct ccaacactgc cccgctccag
1441 ccagagcagc tccaagtgtt tgagactctg gaagagatca caggttacct atacatctca
1501 gcatggccgg acagcctgcc tgacctcagc gtcttccaga acctgcaagt aatccgggga
1561 cgaattctgc acaatggcgc ctactcgctg accctgcaag ggctgggcat cagctggctg
1621 gggctgcgct cactgaggga actgggcagt ggactggccc tcatccacca taacacccac
1681 ctctgcttcg tgcacacggt gccctgggac cagctctttc ggaacccgca ccaagctctg
1741 ctccacactg ccaaccggcc agaggacgag tgtgtgggcg agggcctggc ctgccaccag
1801 ctgtgcgccc gagggcactg ctggggtcca gggcccaccc agtgtgtcaa ctgcagccag
```

(continued)

SEQ ID NO: 1

```
1861 ttccttcggg gccaggagtg cgtggaggaa tgccgagtac tgcaggggct ccccagggag
1921 tatgtgaatg ccaggcactg tttgccgtgc caccctgagt gtcagcccca gaatggctca
1981 gtgacctgtt ttggaccgga ggctgaccag tgtgtggcct gtgcccacta taaggaccct
2041 cccttctgcg tggcccgctg ccccagcggt gtgaaacctg acctctccta catgcccatc
2101 tggaagtttc cagatgagga gggcgcatgc cagccttgcc ccatcaactg cacccactcc
2161 tgtgtggacc tggatgacaa gggctgcccc gccgagcaga gagccagccc tctgacgtcc
2221 atcatctctg cggtggttgg cattctgctg gtcgtggtct tgggggtggt ctttgggatc
2281 ctcatcaagc gacggcagca gaagatccgg aagtacacga tgcggagact gctgcaggaa
2341 acggagctgg tggagccgct gacacctagc ggagcgatgc ccaaccaggc gcagatgcgg
2401 atcctgaaag agacggagct gaggaaggtg aaggtgcttg gatctggcgc ttttggcaca
2461 gtctacaagg gcatctggat ccctgatggg gagaatgtga aaattccagt ggccatcaaa
2521 gtgttgaggg aaaacacatc ccccaaagcc aacaaagaaa tcttagacga agcatacgtg
2581 atggctggtg tgggctcccc atatgtctcc cgccttctgg gcatctgcct gacatccacg
2641 gtgcagctgg tgacacagct tatgccctat ggctgcctct tagaccatgt ccgggaaaac
2701 cgcggacgcc tgggctccca ggacctgctg aactggtgta tgcagattgc caaggggatg
2761 agctacctgg aggatgtgcg gctcgtacac agggacttgg ccgctcggaa cgtgctggtc
2821 aagagtccca accatgtcaa aattacagac ttcgggctgg ctcggctgct ggacattgac
2881 gagacagagt accatgcaga tgggggcaag gtgcccatca agtggatggc gctggagtcc
2941 attctccgcc ggcggttcac ccaccagagt gatgtgtgga gttatggtgt gactgtgtgg
3001 gagctgatga cttttggggc caaaccttac gatgggatcc cagcccggga gatccctgac
3061 ctgctggaaa agggggagcg gctgccccag cccccccatct gcaccattga tgtctacatg
3121 atcatggtca aatgttggat gattgactct gaatgtcggc caagattccg ggagttggtg
3181 tctgaattct cccgcatggc cagggacccc cagcgctttg tggtcatcca gaatgaggac
3241 ttgggcccag ccagtccctt ggacagcacc ttctaccgct cactgctgga ggacgatgac
3301 atgggggacc tggtggatgc tgaggagtat ctggtacccc agcagggctt cttctgtcca
3361 gaccctgccc cgggcgctgg gggcatggtc caccacaggc accgcagctc atctaccagg
3421 agtggcggtg gggacctgac actagggctg gagccctctg aagaggaggc ccccaggtct
3481 ccactggcac cctccgaagg ggctggctcc gatgtatttg atggtgacct gggaatgggg
3541 gcagccaagg ggctgcaaag cctccccaca catgacccca gccctctaca gcggtacagt
3601 gaggacccca cagtacccct gccctctgag actgatggct acgttgcccc cctgacctgc
3661 agcccccagc ctgaatatgt gaaccagcca gatgttcggc cccagccccc ttcgccccga
3721 gagggccctc tgcctgctgc ccgacctgct ggtgccactc tggaaaggcc caagactctc
3781 tccccaggga agaatggggt cgtcaaagac gtttttgcct ttgggggtgc cgtggagaac
3841 cccgagtact tgacacccca gggaggagct gcccctcagc cccaccctcc tcctgccttc
3901 agcccagcct tcgacaacct ctattactgg gaccaggacc caccagagcg gggggctcca
3961 cccagcacct tcaaagggac acctacggca gagaacccag agtacctggg tctggacgtg
```

(continued)

| SEQ ID NO: 1 |
|---|
| 4021 ccagtgtgaa ccagaaggcc aagtccgcag aagccctgat gtgtcctcag ggagcaggga<br>4081 aggcctgact tctgctggca tcaagaggtg ggagggccct ccgaccactt ccaggggaac<br>4141 ctgccatgcc aggaacctgt cctaaggaac cttccttcct gcttgagttc ccagatggct<br>4201 ggaaggggtc cagcctcgtt ggaagaggaa cagcactggg gagtctttgt ggattctgag<br>4261 gccctgccca atgagactct agggtccagt ggatgccaca gcccagcttg gccctttcct<br>4321 tccagatcct gggtactgaa agccttaggg aagctggcct gagaggggaa gcggccctaa<br>4381 gggagtgtct aagaacaaaa gcgacccatt cagagactgt ccctgaaacc tagtactgcc<br>4441 ccccatgagg aaggaacagc aatggtgtca gtatccaggc tttgtacaga gtgcttttct<br>4501 gtttagtttt tacttttttt gttttgtttt tttaaagatg aaataaagac ccagggggag<br>4561 aatgggtgtt gtatggggag gcaagtgtgg ggggtccttc tccacaccca ctttgtccat<br>4621 ttgcaaatat attttggaaa acagctaaaa aaaaaaaaaa aaaa |
| SEQ ID NO: 2<br>Accession No.: NM_004448<br>MELAALCRWG LLLALLPPGA ASTQVCTGTD MKLRLPASPE THLDMLRHLY QGCQVVQGNL 60<br>ELTYLPTNAS LSFLQDIQEV QGYVLIAHNQ VRQVPLQRLR IVRGTQLFED NYALAVLDNG 120<br>DPLNNTTPVT GASPGGLREL QLRSLTEILK GGVLIQRNPQ LCYQDTILWK DIFHKNNQLA 180<br>LTLIDTNRSR ACHPCSPMCK GSRCWGESSE DCQSLTRTVC AGGCARCKGP LPTDCCHEQC 240<br>AAGCTGPKHS DCLACLHFNH SGICELHCPA LVTYNTDTFE SMPNPEGRYT FGASCVTACP 300<br>YNYLSTDVGS CTLVCPLHNQ EVTAEDGTQR CEKCSKPCAR VCYGLGMEHL REVRAVTSAN 360<br>IQEFAGCKKI FGSLAFLPES FDGDPASNTA PLQPEQLQVF ETLEEITGYL YISAWPDSLP 420<br>DLSVFQNLQV IRGRILHNGA YSLTLQGLGI SWLGLRSLRE LGSGLALIHH NTHLCFVHTV 480<br>PWDQLFRNPH QALLHTANRP EDECVGEGLA CHQLCARGHC WGPGPTQCVN CSQFLRGQEC 540<br>VEECRVLQGL PREYVNARHC LPCHPECQPQ NGSVTCFGPE ADQCVACAHY KDPPFCVARC 600<br>PSGVKPDLSY MPIWKFPDEE GACQPCPINC THSCVDLDDK GCPAEQRASP LTSIISAVVG 660<br>ILLVVVLGVV FGILIKRRQQ KIRKYTMRRL LQETELVEPL TPSGAMPNQA QMRILKETEL 720<br>RKVKVLGSGA FGTVYKGIWI PDGENVKIPV AIKVLRENTS PKANKEILDE AYVMAGVGSP 780<br>YVSRLLGICL TSTVQLVTQL MPYGCLLDHV RENRGRLGSQ DLLNWCMQIA KGMSYLEDVR 840<br>LVHRDLAARN VLVKSPNHVK ITDFGLARLL DIDETEYHAD GGKVPIKWMA LESILRRRFT 900<br>HQSDVWSYGV TVWELMTFGA KPYDGIPARE IPDLLEKGER LPQPPICTID VYMIMVKCWM 960<br>IDSECRPRFR ELVSEFSRMA RDPQRFVVIQ NEDLGPASPL DSTFYRSLLE DDDMGDLVDA 1020<br>EEYLVPQQGF FCPDPAPGAG GMVHHRHRSS STRSGGGDLT LGLEPSEEEA PRSPLAPSEG 1080<br>AGSDVFDGDL GMGAAKGLQS LPTHDPSPLQ RYSEDPTVPL PSETDGYVAP LTCSPQPEYV 1140<br>NQPDVRPQPP SPREGPLPAA RPAGATLERP KTLSPGKNGV VKDVFAFGGA VENPEYLTPQ 1200<br>GGAAPQPHPP PAFSPAFDNL YYWDQDPPER GAPPSTFKGT PTAENPEYLG LDVPV 1255 |
| |
| SEQ ID NO: 3<br>Accession No.: NM_001289936 |

(continued)

SEQ ID NO: 3
CDS: 583..4305

```
   1 aagttcctgt gttctttatt ctactctccg ctgaagtcca cacagtttaa attaaagttc
  61 ccggattttt gtgggcgcct gccccgcccc tcgtcccccct gctgtgtcca tatatcgagg
 121 cgatagggtt aagggaaggc ggacgcctga tgggttaatg agcaaactga agtgttttcc
 181 atgatctttt ttgagtcgca attgaagtac cacctcccga gggtgattgc ttccccatgc
 241 ggggtagaac ctttgctgtc ctgttcacca ctctacctcc agcacagaat ttggcttatg
 301 cctactcaat gtgaagatga tgaggatgaa aacctttgtg atgatccact tccacttaat
 361 gaatggtggc aaagcaaagc tatattcaag accacatgca aagctactcc ctgagcaaag
 421 agtcacagat aaaacggggg caccagtaga atggccagga caaacgcagt gcagcacaga
 481 gactcagacc ctggcagcca tgcctgcgca ggcagtgatg agagtgacat gtactgttgt
 541 ggacatgcac aaaagtgaga tacttcaaag attccagaag atatgccccg gggtcctgg
 601 aagccacaag tgtgcaccgg cacagacatg aagctgcggc tccctgccag tcccgagacc
 661 cacctggaca tgctccgcca cctctaccag ggctgccagg tggtgcaggg aaacctggaa
 721 ctcacctacc tgcccaccaa tgccagcctg tccttcctgc aggatatcca ggaggtgcag
 781 ggctacgtgc tcatcgctca caaccaagtg aggcaggtcc cactgcagag gctgcggatt
 841 gtgcgaggca cccagctctt tgaggacaac tatgccctgg ccgtgctaga caatggagac
 901 ccgctgaaca ataccacccc tgtcacaggg gcctccccag gaggcctgcg ggagctgcag
 961 cttcgaagcc tcacagagat cttgaaagga ggggtcttga tccagcggaa cccccagctc
1021 tgctaccagg acacgatttt gtggaaggac atcttccaca agaacaacca gctggctctc
1081 acactgatag acaccaaccg ctctcgggcc tgccacccct gttctccgat gtgtaagggc
1141 tcccgctgct ggggagagag ttctgaggat tgtcagagcc tgacgcgcac tgtctgtgcc
1201 ggtggctgtg cccgctgcaa ggggccactg cccactgact gctgccatga gcagtgtgct
1261 gccggctgca cgggccccaa gcactctgac tgcctggcct gcctccactt caaccacagt
1321 ggcatctgtg agctgcactg cccagccctg gtcacctaca acacagacac gtttgagtcc
1381 atgcccaatc ccgagggccg gtatacattc ggcgccagct gtgtgactgc ctgtccctac
1441 aactaccttt ctacggacgt gggatcctgc accctcgtct gcccctgca caaccaagag
1501 gtgacagcag aggatggaac acagcggtgt gagaagtgca gcaagccctg tgcccgagtg
1561 tgctatggtc tgggcatgga gcacttgcga gaggtgaggg cagttaccag tgccaatatc
1621 caggagtttg ctggctgcaa gaagatcttt gggagcctgg catttctgcc ggagagcttt
1681 gatggggacc cagcctccaa cactgccccg ctccagccag agcagctcca agtgtttgag
1741 actctggaag agatcacagg ttacctatac atctcagcat ggccggacag cctgcctgac
1801 ctcagcgtct tccagaacct gcaagtaatc cggggacgaa ttctgcacaa tggcgcctac
```

(continued)

SEQ ID NO: 3

1861 tcgctgaccc tgcaagggct gggcatcagc tggctggggc tgcgctcact gagggaactg
1921 ggcagtggac tggccctcat ccaccataac acccacctct gcttcgtgca cacggtgccc
1981 tgggaccagc tctttcggaa cccgcaccaa gctctgctcc acactgccaa ccggccagag
2041 gacgagtgtg tgggcgaggg cctggcctgc caccagctgt gcgcccgagg gcactgctgg
2101 ggtccagggc ccacccagtg tgtcaactgc agccagttcc ttcggggcca ggagtgcgtg
2161 gaggaatgcc gagtactgca ggggctcccc agggagtatg tgaatgccag gcactgtttg
2221 ccgtgccacc ctgagtgtca gccccagaat ggctcagtga cctgttttgg accggaggct
2281 gaccagtgtg tggcctgtgc ccactataag gaccctccct tctgcgtggc ccgctgcccc
2341 agcggtgtga aacctgacct ctcctacatg cccatctgga agtttccaga tgaggagggc
2401 gcatgccagc cttgccccat caactgcacc cactcctgtg tggacctgga tgacaagggc
2461 tgccccgccg agcagagagc cagccctctg acgtccatca tctctgcggt ggttggcatt
2521 ctgctggtcg tggtcttggg ggtggtcttt gggatcctca tcaagcgacg gcagcagaag
2581 atccggaagt acacgatgcg gagactgctg caggaaacgg agctggtgga gccgctgaca
2641 cctagcggag cgatgcccaa ccaggcgcag atgcggatcc tgaaagagac ggagctgagg
2701 aaggtgaagg tgcttggatc tggcgctttt ggcacagtct acaagggcat ctggatccct
2761 gatggggaga atgtgaaaat tccagtggcc atcaaagtgt tgagggaaaa cacatccccc
2821 aaagccaaca aagaaatctt agacgaagca tacgtgatgg ctggtgtggg ctccccatat
2881 gtctcccgcc ttctgggcat ctgcctgaca tccacggtgc agctggtgac acagcttatg
2941 ccctatggct gcctcttaga ccatgtccgg gaaaaccgcg gacgcctggg ctcccaggac
3001 ctgctgaact ggtgtatgca gattgccaag gggatgagct acctggagga tgtgcggctc
3061 gtacacaggg acttggccgc tcggaacgtg ctggtcaaga gtcccaacca tgtcaaaatt
3121 acagacttcg ggctggctcg gctgctggac attgacgaga cagagtacca tgcagatggg
3181 ggcaaggtgc ccatcaagtg gatggcgctg gagtccattc tccgccggcg gttcacccac
3241 cagagtgatg tgtggagtta tggtgtgact gtgtgggagc tgatgacttt tggggccaaa
3301 ccttacgatg ggatcccagc ccgggagatc cctgacctgc tggaaaaggg ggagcggctg
3361 ccccagcccc ccatctgcac cattgatgtc tacatgatca tggtcaaatg ttggatgatt
3421 gactctgaat gtcggccaag attccgggag ttggtgtctg aattctcccg catggccagg
3481 gacccccagc gctttgtggt catccagaat gaggacttgg gcccagccag tcccttggac
3541 agcaccttct accgctcact gctggaggac gatgacatgg gggacctggt ggatgctgag
3601 gagtatctgg taccccagca gggcttcttc tgtccagacc ctgccccggg cgctgggggc
3661 atggtccacc acaggcaccg cagctcatct accaggagtg gcggtgggga cctgacacta
3721 gggctggagc cctctgaaga ggaggccccc aggtctccac tggcaccctc cgaaggggct
3781 ggctccgatg tatttgatgg tgacctggga atgggggcag ccaaggggct gcaaagcctc
3841 cccacacatg accccagccc tctacagcgg tacagtgagg accccacagt acccctgccc
3901 tctgagactg atggctacgt tgcccccctg acctgcagcc cccagcctga atatgtgaac
3961 cagccagatg ttcggcccca gccccttcg ccccgagagg gccctctgcc tgctgcccga

(continued)

SEQ ID NO: 3

4021 cctgctggtg ccactctgga aaggcccaag actctctccc cagggaagaa tggggtcgtc
4081 aaagacgttt ttgcctttgg gggtgccgtg gagaaccccg agtacttgac accccaggga
4141 ggagctgccc ctcagcccca ccctcctcct gccttcagcc cagccttcga caacctctat
4201 tactgggacc aggacccacc agagcggggg gctccaccca gcaccttcaa agggacacct
4261 acggcagaga acccagagta cctgggtctg gacgtgccag tgtgaaccag aaggccaagt
4321 ccgcagaagc cctgatgtgt cctcagggag cagggaaggc ctgacttctg ctggcatcaa
4381 gaggtgggag ggccctccga ccacttccag gggaacctgc catgccagga acctgtccta
4441 aggaaccttc cttcctgctt gagttcccag atggctggaa ggggtccagc ctcgttggaa
4501 gaggaacagc actggggagt ctttgtggat tctgaggccc tgcccaatga gactctaggg
4561 tccagtggat gccacagccc agcttggccc tttccttcca gatcctgggt actgaaagcc
4621 ttagggaagc tggcctgaga ggggaagcgg ccctaaggga gtgtctaaga acaaaagcga
4681 cccattcaga gactgtccct gaaacctagt actgcccccc atgaggaagg aacagcaatg
4741 gtgtcagtat ccaggctttg tacagagtgc ttttctgttt agtttttact ttttttgttt
4801 tgttttttta aagatgaaat aaagacccag ggggagaatg ggtgttgtat ggggaggcaa
4861 gtgtgggggg tccttctcca cacccacttt gtccatttgc aaatatattt tggaaaacag
4921 ctaaaaaaaa aaaaaaaaaa

SEQ ID NO: 4

Accession No.: NM_001289936

MPRGSWKPQV CTGTDMKLRL PASPETHLDM LRHLYQGCQV VQGNLELTYL PTNASLSFLQ 60
DIQEVQGYVL IAHNQVRQVP LQRLRIVRGT QLFEDNYALA VLDNGDPLNN TTPVTGASPG 120
GLRELQLRSL TEILKGGVLI QRNPQLCYQD TILWKDIFHK NNQLALTLID TNRSRACHPC 180
SPMCKGSRCW GESSEDCQSL TRTVCAGGCA RCKGPLPTDC CHEQCAAGCT GPKHSDCLAC 240
LHFNHSGICE LHCPALVTYN TDTFESMPNP EGRYTFGASC VTACPYNYLS TDVGSCTLVC 300
PLHNQEVTAE DGTQRCEKCS KPCARVCYGL GMEHLREVRA VTSANIQEFA GCKKIFGSLA 360
FLPESFDGDP ASNTAPLQPE QLQVFETLEE ITGYLYISAW PDSLPDLSVF QNLQVIRGRI 420
LHNGAYSLTL QGLGISWLGL RSLRELGSGL ALIHHNTHLC FVHTVPWDQL FRNPHQALLH 480
TANRPEDECV GEGLACHQLC ARGHCWGPGP TQCVNCSQFL RGQECVEECR VLQGLPREYV 540
NARHCLPCHP ECQPQNGSVT CFGPEADQCV ACAHYKDPPF CVARCPSGVK PDLSYMPIWK 600
FPDEEGACQP CPINCTHSCV DLDDKGCPAE QRASPLTSII SAVVGILLVV VLGVVFGILI 660
KRRQQKIRKY TMRRLLQETE LVEPLTPSGA MPNQAQMRIL KETELRKVKV LGSGAFGTVY 720
KGIWIPDGEN VKIPVAIKVL RENTSPKANK EILDEAYVMA GVGSPYVSRL LGICLTSTVQ 780
LVTQLMPYGC LLDHVRENRG RLGSQDLLNW CMQIAKGMSY LEDVRLVHRD LAARNVLVKS 840
PNHVKITDFG LARLLDIDET EYHADGGKVP IKWMALESIL RRRFTHQSDV WSYGVTVWEL 900
MTFGAKPYDG IPAREIPDLL EKGERLPQPP ICTIDVYMIM VKCWMIDSEC RPRFRELVSE 960

(continued)

| SEQ ID NO: 4 |
|---|
| FSRMARDPQR FVVIQNEDLG PASPLDSTFY RSLLEDDDMG DLVDAEEYLV PQQGFFCPDP 1020 |
| APGAGGMVHH RHRSSSTRSG GGDLTLGLEP SEEEAPRSPL APSEGAGSDV FDGDLGMGAA 1080 |
| KGLQSLPTHD PSPLQRYSED PTVPLPSETD GYVAPLTCSP QPEYVNQPDV RPQPPSPREG 1140 |
| PLPAARPAGA TLERPKTLSP GKNGVVKDVF AFGGAVENPE YLTPQGGAAP QPHPPPAFSP 1200 |
| AFDNLYYWDQ DPPERGAPPS TFKGTPTAEN PEYLGLDVPV 1240 |

The human wild-type EGFR gene is shown in, for example, SEQ ID NO: 5. The human wild-type EGFR protein consists of the amino acid sequence set forth in, for example, SEQ ID NO: 6. The nucleotide sequence information of the human wild-type EGFR gene can be obtained from, for example, NCBI Reference Sequence: NM 005228, or the like. and the amino acid sequence information of the human wild-type EGFR protein can be obtained from, for example, NCBI Reference Sequence: NP_005219,, or the like.

In several embodiments, the pyrimidine compound of the present invention or a salt thereof exhibits inhibitory activity against mutant EGFR. In the present description, "mutant EGFR" is EGFR having one or more activating mutations or resistance acquiring mutations, such as insertion mutations point mutations, or deletion mutations, in exon 18 region, exon 19 region, exon 20 region, exon 21 region or the like of human wild-type EGFR.

In the present description, "exon 18" corresponds to a region from positions 688 to 728 in the amino acid sequence of the human wild-type EGFR protein (e.g., a protein consisting of the amino acid sequence set forth in SEQ ID NO: 6).

In the present invention, "exon 18 mutation" refers to a point mutation or a deletion mutation in exon 18 region resulting in an amino acid mutation in the human wild-type EGFR protein (e.g., a protein consisting of the amino acid sequence set forth in SEQ ID NO: 6). Examples of the point mutation in exon 18 include point mutation E709X or G719X in exon 18 region, which substitutes glutamic acid at position 709 or glycine at position 719 with any amino acid. Examples of E709X include point mutation E709K in exon 18 region, which substitutes glutamic acid at position 709 with lysine, and point mutation E709A in exon 18 region, which substitutes glutamic acid at position 709 with alanine. Examples of G719X include point mutation G719A in exon 18 region, which substitutes glycine at position 719 with alanine, point mutation G719S in exon 18 region, which substitutes glycine at position 719 with serine, and point mutation G719C in exon 18 region, which substitutes glycine at position 719 with cysteine. The deletion mutation in exon 18 region encompasses not only a mutation in exon 18 region, which deletes some amino acids, but also a mutation therein, which inserts any one or more amino acids in addition to the amino acid deletion. Examples of the deletion mutation in exon 18 include a mutation in exon 18 region, which deletes glutamic acid at position 709 and threonine at position 710 and then inserts aspartic acid (Del E709-T710insD).

In the present description, "exon 19" corresponds to a region from positions 729 to 761 in the amino acid sequence of the human wild-type EGFR protein (e.g., a protein consisting of the amino acid sequence set forth in SEQ ID NO: 6).

In the present description, "exon 19 mutation" refers to a mutation in exon 19 region, which deletes one or more amino acids in the human wild-type EGFR protein (e.g., a protein consisting of the amino acid sequence set forth in SEQ ID NO: 6). The deletion mutation in exon 19 region encompasses not only a mutation in exon 19 region, which deletes some amino acids, but also a mutation therein, which inserts any one or more amino acids in addition to the amino acid deletion. Examples of the exon 19 deletion mutation include a mutation in exon 19 region, which deletes 5 amino acids from glutamic acid at position 746 to alanine at position 750 (Del E746-A750 (or also referred to as d746-750)), a mutation in exon 19 region, which deletes 7 amino acids from leucine at position 747 to proline at position 753 and then inserts serine (Del L747-P753insS), a mutation in exon 19 region, which deletes 5 amino acids from leucine at position 747 to threonine at position 751 (Del L747-T751), and a mutation in exon 19 region, which deletes 4 amino acids from leucine at position 747 to alanine at position 750 and then inserts proline (Del L747-A750insP). In one preferred embodiment of the present invention, the exon 19 deletion mutation is a mutation in exon 19 region, which deletes 5 amino acids from glutamic acid at position 746 to alanine at position 750 (Del E746-A750).

In the present description, "exon 20" corresponds to a region from positions 762 to 823 in the amino acid sequence of the human wild-type EGFR protein (e.g., a protein consisting of the amino acid sequence set forth in SEQ ID NO: 6).

In the present invention, "exon 20 mutation" refers to a point mutation, an insertion mutation, a deletion mutation,

or the like in exon 20 region resulting in an amino acid mutation in the human wild-type EGFR protein (e.g., a protein consisting of the amino acid sequence set forth in SEQ ID NO: 6). Examples of the exon 20 mutation include A763insFQEA, A767insASV, S768dupSVD, V769insASV, D770insNPG, D770insSVD, and D773insNPH (Nature medicine,24,p638-646,2018). In one preferred embodiment of the present invention, the exon 20 mutation is one or more insertion mutations or point mutations selected from V769_D770insASV, D770_N771insNPG, D770_N771insSVD, H773_V774insNPH, and T790M.

In the present description, "exon 21" corresponds to a region from positions 824 to 875 in the amino acid sequence of the human wild-type EGFR protein (e.g., a protein consisting of the amino acid sequence set forth in SEQ ID NO: 6).

In the present invention, "exon 21 mutation" refers to a point mutation in exon 21 region resulting in an amino acid mutation in the human wild-type EGFR protein (e.g., a protein consisting of the amino acid sequence set forth in SEQ ID NO: 6). Examples of the point mutation in exon 21 include point mutations in exon 21 region, which substitute one amino acid and preferably include position mutation L858X or L861X in exon 21 region, which substitutes leucine at position 858 or leucine at position 861 with any amino acid. Examples of L858X include point mutation L858R in exon 21 region, which substitutes leucine at position 858 with arginine. Examples of L861X include point mutation L861Q in exon 21 region, which substitutes leucine at position 861 with glutamine. In one preferred embodiment of the present invention, the point mutation in exon 21 is L858R.

In several embodiments, with regard to a mutation in a certain EGFR isoform, even when position of the mutation is different from position of an amino acid shown in SEQ ID NO: 6 due to deletion or insertion of an amino acid(s), it is understood that the mutation is the same as the mutation at a position corresponding to position of the amino acid shown in SEQ ID NO: 6. Hence, for example, the threonine at position 790 in the EGFR shown in SEQ ID NO: 6 corresponds to threonine at position 745 in EGFR consisting of the amino acid sequence set forth in SEQ ID NO: 8. For example, the term "T790M" means that the threonine at position 790 in the EGFR shown in SEQ ID NO: 6 is mutated to methionine. Since such "T790M" is at a position corresponding to the amino acid at position 745 in EGFR consisting of the amino acid sequence set forth in SEQ ID NO: 8, "T745M" in the EGFR consisting of the amino acid sequence set forth in SEQ ID NO: 8 corresponds to "T790M" in the EGFR shown in SEQ ID NO: 6. For example, the threonine at position 790 in the EGFR shown in SEQ ID NO: 6 corresponds to threonine at position 523 in EGFR consisting of the amino acid sequence set forth in SEQ ID NO: 10. For example, the term "T790M" means that the threonine at position 790 in the EGFR shown in SEQ ID NO: 6 is mutated to methionine. Since such "T790M" is at a position corresponding to the amino acid at position 523 in EGFR consisting of the amino acid sequence set forth in SEQ ID NO: 10, "T523M" in the EGFR consisting of the amino acid sequence set forth in SEQ ID NO: 10 corresponds to "T790M" in the EGFR shown in SEQ ID NO: 6. Besides, the position of an amino acid in SEQ ID NO: 6 that corresponds to a certain amino acid in a certain EGFR isoform can be confirmed by Multiple Alignment of BLAST.

[Table B]

| |
|---|
| EGFR variant 1<br>Nucleotide sequence (SEQ ID NO: 5) |

(continued)

| EGFR variant 1 |
|---|
| ATGCGACCCTCCGGGACGG<br>CCGGGGCAGCGCTCCTGGCGCTGCTGGCTGCGCTCTGCCCGGCGAGTCGGGCTCTGGAGGAAAAGAAAGT<br>TTGCCAAGGCACGAGTAACAAGCTCACGCAGTTGGGCACTTTTGAAGATCATTTTCTCAGCCTCCAGAGG<br>ATGTTCAATAACTGTGAGGTGGTCCTTGGGAATTTGGAAATTACCTATGTGCAGAGGAATTATGATCTTT<br>CCTTCTTAAAGACCATCCAGGAGGTGGCTGGTTATGTCCTCATTGCCCTCAACACAGTGGAGCGAATTCC<br>TTTGGAAAACCTGCAGATCATCAGAGGAAATATGTACTACGAAAATTCCTATGCCTTAGCAGTCTTATCT<br>AACTATGATGCAAATAAAACCGGACTGAAGGAGCTGCCCATGAGAAATTTACAGGAAATCCTGCATGGCG<br>CCGTGCGGTTCAGCAACAACCCTGCCCTGTGCAACGTGGAGAGCATCCAGTGGCGGGACATAGTCAGCAG<br>TGACTTTCTCAGCAACATGTCGATGGACTTCCAGAACCACCTGGGCAGCTGCCAAAAGTGTGATCCAAGC<br>TGTCCCAATGGGAGCTGCTGGGGTGCAGGAGAGGAGAACTGCCAGAAACTGACCAAAATCATCTGTGCCC<br>AGCAGTGCTCCGGGCGCTGCCGTGGCAAGTCCCCCAGTGACTGCTGCCACAACCAGTGTGCTGCAGGCTG<br>CACAGGCCCCCGGGAGAGCGACTGCCTGGTCTGCCGCAAATTCCGAGACGAAGCCACGTGCAAGGACACC<br>TGCCCCCCACTCATGCTCTACAACCCCACCACGTACCAGATGGATGTGAACCCCGAGGGCAAATACAGCT<br>TTGGTGCCACCTGCGTGAAGAAGTGTCCCCGTAATTATGTGGTGACAGATCACGGCTCGTGCGTCCGAGC<br>CTGTGGGGCCGACAGCTATGAGATGGAGGAAGACGGCGTCCGCAAGTGTAAGAAGTGCGAAGGGCCTTGC<br>CGCAAAGTGTGTAACGGAATAGGTATTGGTGAATTTAAAGACTCACTCTCCATAAATGCTACGAATATTA<br>AACACTTCAAAAACTGCACCTCCATCAGTGGCGATCTCCACATCCTGCCGGTGGCATTTAGGGGTGACTC<br>CTTCACACATACTCCTCCTCTGGATCCACAGGAACTGGATATTCTGAAAACCGTAAAGGAAATCACAGGG |

(continued)

| EGFR variant 1 |
|---|
| TTTTTGCTGATTCAGGCTTGGCCTGAAAACAGGACGGACCTCCATGCCTTTGAGAACCTAGAAATCATAC<br>GCGGCAGGACCAAGCAACATGGTCAGTTTTCTCTTGCAGTCGTCAGCCTGAACATAACATCCTTGGGATT<br>ACGCTCCCTCAAGGAGATAAGTGATGGAGATGTGATAATTTCAGGAAACAAAAATTTGTGCTATGCAAAT<br>ACAATAAACTGGAAAAAACTGTTTGGGACCTCCGGTCAGAAAACCAAAATTATAAGCAACAGAGGTGAAA<br>ACAGCTGCAAGGCCACAGGCCAGGTCTGCCATGCCTTGTGCTCCCCCGAGGGCTGCTGGGGCCCGGAGCC<br>CAGGGACTGCGTCTCTTGCCGGAATGTCAGCCGAGGCAGGGAATGCGTGGACAAGTGCAACCTTCTGGAG<br>GGTGAGCCAAGGGAGTTTGTGGAGAACTCTGAGTGCATACAGTGCCACCCAGAGTGCCTGCCTCAGGCCA<br>TGAACATCACCTGCACAGGACGGGGACCAGACAACTGTATCCAGTGTGCCCACTACATTGACGGCCCCCA<br>CTGCGTCAAGACCTGCCCGGCAGGAGTCATGGGAGAAAACAACACCCTGGTCTGGAAGTACGCAGACGCC<br>GGCCATGTGTGCCACCTGTGCCATCCAAACTGCACCTACGGATGCACTGGGCCAGGTCTTGAAGGCTGTC<br>CAACGAATGGGCCTAAGATCCCGTCCATCGCCACTGGGATGGTGGGGGCCCTCCTCTTGCTGCTGGTGGT<br>GGCCCTGGGGATCGGCCTCTTCATGCGAAGGCGCCACATCGTTCGGAAGCGCACGCTGCGGAGGCTGCTG<br>CAGGAGAGGGAGCTTGTGGAGCCTCTTACACCCAGTGGAGAAGCTCCCAACCAAGCTCTCTTGAGGATCT<br>TGAAGGAAACTGAATTCAAAAAGATCAAAGTGCTGGGCTCCGGTGCGTTCGGCACGGTGTATAAGGGACT<br>CTGGATCCCAGAAGGTGAGAAAGTTAAAATTCCCGTCGCTATCAAGGAATTAAGAGAAGCAACATCTCCG<br>AAAGCCAACAAGGAAATCCTCGATGAAGCCTACGTGATGGCCAGCGTGGACAACCCCCACGTGTGCCGCC<br>TGCTGGGCATCTGCCTCACCTCCACCGTGCAGCTCATCACGCAGCTCATGCCCTTCGGCTGCCTCCTGGA<br>CTATGTCCGGGAACACAAAGACAATATTGGCTCCCAGTACCTGCTCAACTGGTGTGTGCAGATCGCAAAG<br>GGCATGAACTACTTGGAGGACCGTCGCTTGGTGCACCGCGACCTGGCAGCCAGGAACGTACTGGTGAAAA<br>CACCGCAGCATGTCAAGATCACAGATTTTGGGCTGGCCAAACTGCTGGGTGCGGAAGAGAAAGAATACCA<br>TGCAGAAGGAGGCAAAGTGCCTATCAAGTGGATGGCATTGGAATCAATTTTACACAGAATCTATACCCAC<br>CAGAGTGATGTCTGGAGCTACGGGGTGACTGTTTGGGAGTTGATGACCTTTGGATCCAAGCCATATGACG<br>GAATCCCTGCCAGCGAGATCTCCTCCATCCTGGAGAAAGGAGAACGCCTCCCTCAGCCACCCATATGTAC<br>CATCGATGTCTACATGATCATGGTCAAGTGCTGGATGATAGACGCAGATAGTCGCCCAAAGTTCCGTGAG<br>TTGATCATCGAATTCTCCAAAATGGCCCGAGACCCCCAGCGCTACCTTGTCATTCAGGGGGATGAAAGAA<br>TGCATTTGCCAAGTCCTACAGACTCCAACTTCTACCGTGCCCTGATGGATGAAGAAGACATGGACGACGT<br>GGTGGATGCCGACGAGTACCTCATCCCACAGCAGGGCTTCTTCAGCAGCCCCTCCACGTCACGGACTCCC<br>CTCCTGAGCTCTCTGAGTGCAACCAGCAACAATTCCACCGTGGCTTGCATTGATAGAAATGGGCTGCAAA<br>GCTGTCCCATCAAGGAAGACAGCTTCTTGCAGCGATACAGCTCAGACCCCACAGGCGCCTTGACTGAGGA<br>CAGCATAGACGACACCTTCCTCCCAGTGCCTGAATACATAAACCAGTCCGTTCCCAAAAGGCCCGCTGGC<br>TCTGTGCAGAATCCTGTCTATCACAATCAGCCTCTGAACCCCGCGCCCAGCAGAGACCCACACTACCAGG<br>ACCCCCACAGCACTGCAGTGGGCAACCCCGAGTATCTCAACACTGTCCAGCCCACCTGTGTCAACAGCAC<br>ATTCGACAGCCCTGCCCACTGGGCCCAGAAAGGCAGCCACCAAATTAGCCTGGACAACCCTGACTACCAG<br>CAGGACTTCTTTCCCAAGGAAGCCAAGCCAAATGGCATCTTTAAGGGCTCCACAGCTGAAAATGCAGAAT<br>ACCTAAGGGTCGCGCCACAAAGCAGTGAATTTATTGGAGCATGA |
| Amino acid sequence (SEQ ID NO: 6) |

(continued)

| |
|---|
| EGFR variant 1<br>MRPSGTAGAALLALLAALCPASRALEEKKVCQGTSNKLTQLGTFEDHFLSLQRMFNNCEVVLGNLEITYV<br>QRNYDLSFLKTIQEVAGYVLIALNTVERIPLENLQIIRGNMYYENSYALAVLSNYDANKTGLKELPMRNL<br>QEILHGAVRFSNNPALCNVESIQWRDIVSSDFLSNMSMDFQNHLGSCQKCDPSCPNGSCWGAGEENCQKL<br>TKIICAQQCSGRCRGKSPSDCCHNQCAAGCTGPRESDCLVCRKFRDEATCKDTCPPLMLYNPTTYQMDVN<br>PEGKYSFGATCVKKCPRNYVVTDHGSCVRACGADSYEMEEDGVRKCKKCEGPCRKVCNGIGIGEFKDSLS<br>INATNIKHFKNCTSISGDLHILPVAFRGDSFTHTPPLDPQELDILKTVKEITGFLLIQAWPENRTDLHAF<br>ENLEIIRGRTKQHGQFSLAVVSLNITSLGLRSLKEISDGDVIISGNKNLCYANTINWKKLFGTSGQKTKI<br>ISNRGENSCKATGQVCHALCSPEGCWGPEPRDCVSCRNVSRGRECVDKCNLLEGEPREFVENSECIQCHP<br>ECLPQAMNITCTGRGPDNCIQCAHYIDGPHCVKTCPAGVMGENNTLVWKYADAGHVCHLCHPNCTYGCTG<br>PGLEGCPTNGPKIPSIATGMVGALLLLLVVALGIGLFMRRRHIVRKRTLRRLLQERELVEPLTPSGEAPN<br>QALLRILKETEFKKIKVLGSGAFGTVYKGLWIPEGEKVKIPVAIKELREATSPKANKEILDEAYVMASVD<br>NPHVCRLLGICLTSTVQLITQLMPFGCLLDYVREHKDNIGSQYLLNWCVQIAKGMNYLEDRRLVHRDLAA<br>RNVLVKTPQHVKITDFGLAKLLGAEEKEYHAEGGKVPIKWMALESILHRIYTHQSDVWSYGVTVWELMTF<br>GSKPYDGIPASEISSILEKGERLPQPPICTIDVYMIMVKCWMIDADSRPKFRELIIEFSKMARDPQRYLV<br>IQGDERMHLPSPTDSNFYRALMDEEDMDDVVDADEYLIPQQGFFSSPSTSRTPLLSSLSATSNNSTVACI<br>DRNGLQSCPIKEDSFLQRYSSDPTGALTEDSIDDTFLPVPEYINQSVPKRPAGSVQNPVYHNQPLNPAPS<br>RDPHYQDPHSTAVGNPEYLNTVQPTCVNSTFDSPAHWAQKGSHQISLDNPDYQQDFFPKEAKPNGIFKGS<br>TAENAEYLRVAPQSSEFIGA |
| EGFR variant 5<br>Nucleotide sequence (SEQ ID NO: 7)<br>ATGCGACCCTCCGGGACGG<br>CCGGGGCAGCGCTCCTGGCGCTGCTGGCTGCGCTCTGCCCGGCGAGTCGGGCTCTGGAGGAAAAGAAAGT<br>TTGCCAAGGCACGAGTAACAAGCTCACGCAGTTGGGCACTTTTGAAGATCATTTTCTCAGCCTCCAGAGG<br>ATGTTCAATAACTGTGAGGTGGTCCTTGGGAATTTGGAAATTACCTATGTGCAGAGGAATTATGATCTTT<br>CCTTCTTAAAGACCATCCAGGAGGTGGCTGGTTATGTCCTCATTGCCCTCAACACAGTGGAGCGAATTCC<br>TTTGGAAAACCTGCAGATCATCAGAGGAAATATGTACTACGAAAATTCCTATGCCTTAGCAGTCTTATCT<br>AACTATGATGCAAATAAAACCGGACTGAAGGAGCTGCCCATGAGAAATTTACAGGGCCAAAAGTGTGATC<br>CAAGCTGTCCCAATGGGAGCTGCTGGGGTGCAGGAGAGGAGAACTGCCAGAAACTGACCAAAATCATCTG<br>TGCCCAGCAGTGCTCCGGGCGCTGCCGTGGCAAGTCCCCCAGTGACTGCTGCCACAACCAGTGTGCTGCA<br>GGCTGCACAGGCCCCCGGGAGAGCGACTGCCTGGTCTGCCGCAAATTCCGAGACGAAGCCACGTGCAAGG<br>ACACCTGCCCCCCACTCATGCTCTACAACCCCACCACGTACCAGATGGATGTGAACCCCGAGGGCAAATA<br>CAGCTTTGGTGCCACCTGCGTGAAGAAGTGTCCCCGTAATTATGTGGTGACAGATCACGGCTCGTGCGTC<br>CGAGCCTGTGGGGCCGACAGCTATGAGATGGAGGAAGACGGCGTCCGCAAGTGTAAGAAGTGCGAAGGGC |

(continued)

| |
|---|
| EGFR variant 5 |
| CTTGCCGCAAAGTGTGTAACGGAATAGGTATTGGTGAATTTAAAGACTCACTCTCCATAAATGCTACGAA<br>TATTAAACACTTCAAAAACTGCACCTCCATCAGTGGCGATCTCCACATCCTGCCGGTGGCATTTAGGGGT<br>GACTCCTTCACACATACTCCTCCTCTGGATCCACAGGAACTGGATATTCTGAAAACCGTAAAGGAAATCA<br>CAGGGTTTTTGCTGATTCAGGCTTGGCCTGAAAACAGGACGGACCTCCATGCCTTTGAGAACCTAGAAAT<br>CATACGCGGCAGGACCAAGCAACATGGTCAGTTTTCTCTTGCAGTCGTCAGCCTGAACATAACATCCTTG<br>GGATTACGCTCCCTCAAGGAGATAAGTGATGGAGATGTGATAATTTCAGGAAACAAAAATTTGTGCTATG<br>CAAATACAATAAACTGGAAAAAACTGTTTGGGACCTCCGGTCAGAAAACCAAAATTATAAGCAACAGAGG<br>TGAAAACAGCTGCAAGGCCACAGGCCAGGTCTGCCATGCCTTGTGCTCCCCCGAGGGCTGCTGGGGCCCG<br>GAGCCCAGGGACTGCGTCTCTTGCCGGAATGTCAGCCGAGGCAGGGAATGCGTGGACAAGTGCAACCTTC<br>TGGAGGGTGAGCCAAGGGAGTTTGTGGAGAACTCTGAGTGCATACAGTGCCACCCAGAGTGCCTGCCTCA<br>GGCCATGAACATCACCTGCACAGGACGGGGACCAGACAACTGTATCCAGTGTGCCCACTACATTGACGGC<br>CCCCACTGCGTCAAGACCTGCCCGGCAGGAGTCATGGGAGAAAACAACACCCTGGTCTGGAAGTACGCAG<br>ACGCCGGCCATGTGTGCCACCTGTGCCATCCAAACTGCACCTACGGATGCACTGGGCCAGGTCTTGAAGG<br>CTGTCCAACGAATGGGCCTAAGATCCCGTCCATCGCCACTGGGATGGTGGGGGCCCTCCTCTTGCTGCTG<br>GTGGTGGCCCTGGGGATCGGCCTCTTCATGCGAAGGCGCCACATCGTTCGGAAGCGCACGCTGCGGAGGC<br>TGCTGCAGGAGAGGGAGCTTGTGGAGCCTCTTACACCCAGTGGAGAAGCTCCCAACCAAGCTCTCTTGAG<br>GATCTTGAAGGAAACTGAATTCAAAAAGATCAAAGTGCTGGGCTCCGGTGCGTTCGGCACGGTGTATAAG<br>GGACTCTGGATCCCAGAAGGTGAGAAAGTTAAAATTCCCGTCGCTATCAAGGAATTAAGAGAAGCAACAT<br>CTCCGAAAGCCAACAAGGAAATCCTCGATGAAGCCTACGTGATGGCCAGCGTGGACAACCCCCACGTGTG<br>CCGCCTGCTGGGCATCTGCCTCACCTCCACCGTGCAGCTCATCACGCAGCTCATGCCCTTCGGCTGCCTC<br>CTGGACTATGTCCGGGAACACAAAGACAATATTGGCTCCCAGTACCTGCTCAACTGGTGTGTGCAGATCG<br>CAAAGGGCATGAACTACTTGGAGGACCGTCGCTTGGTGCACCGCGACCTGGCAGCCAGGAACGTACTGGT<br>GAAAACACCGCAGCATGTCAAGATCACAGATTTTGGGCTGGCCAAACTGCTGGGTGCGGAAGAGAAAGAA<br>TACCATGCAGAAGGAGGCAAAGTGCCTATCAAGTGGATGGCATTGGAATCAATTTTACACAGAATCTATA<br>CCCACCAGAGTGATGTCTGGAGCTACGGGGTGACTGTTTGGGAGTTGATGACCTTTGGATCCAAGCCATA<br>TGACGGAATCCCTGCCAGCGAGATCTCCTCCATCCTGGAGAAAGGAGAACGCCTCCCTCAGCCACCCATA<br>TGTACCATCGATGTCTACATGATCATGGTCAAGTGCTGGATGATAGACGCAGATAGTCGCCCAAAGTTCC<br>GTGAGTTGATCATCGAATTCTCCAAAATGGCCCGAGACCCCCAGCGCTACCTTGTCATTCAGGGGGATGA<br>AAGAATGCATTTGCCAAGTCCTACAGACTCCAACTTCTACCGTGCCCTGATGGATGAAGAAGACATGGAC<br>GACGTGGTGGATGCCGACGAGTACCTCATCCCACAGCAGGGCTTCTTCAGCAGCCCCTCCACGTCACGGA<br>CTCCCCTCCTGAGCTCTCTGAGTGCAACCAGCAACAATTCCACCGTGGCTTGCATTGATAGAAATGGGCT<br>GCAAAGCTGTCCCATCAAGGAAGACAGCTTCTTGCAGCGATACAGCTCAGACCCCACAGGCGCCTTGACT<br>GAGGACAGCATAGACGACACCTTCCTCCCAGTGCCTGGTGAGTGGCTTGTCTGGAAACAGTCCTGCTCCT<br>CAACCTCCTCGACCCACTCAGCAGCAGCCAGTCTCCAGTGTCCAAGCCAGGTGCTCCCTCCAGCATCTCC<br>AGAGGGGGAAACAGTGGCAGATTTGCAGACACAGTGA |
| Amino acid sequence (SEQ ID NO: 8) |

(continued)

| |
|---|
| EGFR variant 5<br>MRPSGTAGAALLALLAALCPASRALEEKKVCQGTSNKLTQLGTFEDHFLSLQRMFNNCEVVLGNLEITYV<br>QRNYDLSFLKTIQEVAGYVLIALNTVERIPLENLQIIRGNMYYENSYALAVLSNYDANKTGLKELPMRNL<br>QGQKCDPSCPNGSCWGAGEENCQKLTKIICAQQCSGRCRGKSPSDCCHNQCAAGCTGPRESDCLVCRKFR<br>DEATCKDTCPPLMLYNPTTYQMDVNPEGKYSFGATCVKKCPRNYVVTDHGSCVRACGADSYEMEEDGVRK<br>CKKCEGPCRKVCNGIGIGEFKDSLSINATNIKHFKNCTSISGDLHILPVAFRGDSFTHTPPLDPQELDIL<br>KTVKEITGFLLIQAWPENRTDLHAFENLEIIRGRTKQHGQFSLAVVSLNITSLGLRSLKEISDGDVIISG<br>NKNLCYANTINWKKLFGTSGQKTKIISNRGENSCKATGQVCHALCSPEGCWGPEPRDCVSCRNVSRGREC<br>VDKCNLLEGEPREFVENSECIQCHPECLPQAMNITCTGRGPDNCIQCAHYIDGPHCVKTCPAGVMGENNT<br>LVWKYADAGHVCHLCHPNCTYGCTGPGLEGCPTNGPKIPSIATGMVGALLLLLVVALGIGLFMRRRHIVR<br>KRTLRRLLQERELVEPLTPSGEAPNQALLRILKETEFKKIKVLGSGAFGTVYKGLWIPEGEKVKIPVAIK<br>ELREATSPKANKEILDEAYVMASVDNPHVCRLLGICLTSTVQLITQLMPFGCLLDYVREHKDNIGSQYLL<br>NWCVQIAKGMNYLEDRRLVHRDLAARNVLVKTPQHVKITDFGLAKLLGAEEKEYHAEGGKVPIKWMALES<br>ILHRIYTHQSDVWSYGVTVWELMTFGSKPYDGIPASEISSILEKGERLPQPPICTIDVYMIMVKCWMIDA<br>DSRPKFRELIIEFSKMARDPQRYLVIQGDERMHLPSPTDSNFYRALMDEEDMDDVVDADEYLIPQQGFFS<br>SPSTSRTPLLSSLSATSNNSTVACIDRNGLQSCPIKEDSFLQRYSSDPTGALTEDSIDDTFLPVPGEWLV<br>WKQSCSSTSSTHSAAASLQCPSQVLPPASPEGETVADLQTQ |
| EGFR vIII<br>Nucleotide sequence (SEQ ID NO: 9)<br>ATGCGACCCTCCGGGACGG<br>CCGGGGCAGCGCTCCTGGCGCTGCTGGCTGCGCTCTGCCCGGCGAGTCGGGCTCTGGAGGAAAAGAAAGG<br>TAATTATGTGGTGACAGATCACGGCTCGTGCGTCCGAGCCTGTGGGGCCGACAGCTATGAGATGGAGGAA<br>GACGGCGTCCGCAAGTGTAAGAAGTGCGAAGGGCCTTGCCGCAAAGTGTGTAACGGAATAGGTATTGGTG<br>AATTTAAAGACTCACTCTCCATAAATGCTACGAATATTAAACACTTCAAAAACTGCACCTCCATCAGTGG<br>CGATCTCCACATCCTGCCGGTGGCATTTAGGGGTGACTCCTTCACACATACTCCTCCTCTGGATCCACAG<br>GAACTGGATATTCTGAAAACCGTAAAGGAAATCACAGGGTTTTTGCTGATTCAGGCTTGGCCTGAAAACA<br>GGACGGACCTCCATGCCTTTGAGAACCTAGAAATCATACGCGGCAGGACCAAGCAACATGGTCAGTTTTC<br>TCTTGCAGTCGTCAGCCTGAACATAACATCCTTGGGATTACGCTCCCTCAAGGAGATAAGTGATGGAGAT<br>GTGATAATTTCAGGAAACAAAAATTTGTGCTATGCAAATACAATAAACTGGAAAAAACTGTTTGGGACCT<br>CCGGTCAGAAAACCAAAATTATAAGCAACAGAGGTGAAAACAGCTGCAAGGCCACAGGCCAGGTCTGCCA<br>TGCCTTGTGCTCCCCCGAGGGCTGCTGGGGCCCGGAGCCCAGGGACTGCGTCTCTTGCCGGAATGTCAGC<br>CGAGGCAGGGAATGCGTGGACAAGTGCAACCTTCTGGAGGGTGAGCCAAGGGAGTTTGTGGAGAACTCTG<br>AGTGCATACAGTGCCACCCAGAGTGCCTGCCTCAGGCCATGAACATCACCTGCACAGGACGGGGACCAGA<br>CAACTGTATCCAGTGTGCCCACTACATTGACGGCCCCCACTGCGTCAAGACCTGCCCGGCAGGAGTCATG |

(continued)

EGFR vIII

GGAGAAAACAACACCCTGGTCTGGAAGTACGCAGACGCCGGCCATGTGTGCCACCTGTGCCATCCAAACT
GCACCTACGGATGCACTGGGCCAGGTCTTGAAGGCTGTCCAACGAATGGGCCTAAGATCCCGTCCATCGC
CACTGGGATGGTGGGGGCCCTCCTCTTGCTGCTGGTGGTGGCCCTGGGGATCGGCCTCTTCATGCGAAGG
CGCCACATCGTTCGGAAGCGCACGCTGCGGAGGCTGCTGCAGGAGAGGGAGCTTGTGGAGCCTCTTACAC
CCAGTGGAGAAGCTCCCAACCAAGCTCTCTTGAGGATCTTGAAGGAAACTGAATTCAAAAAGATCAAAGT
GCTGGGCTCCGGTGCGTTCGGCACGGTGTATAAGGGACTCTGGATCCCAGAAGGTGAGAAAGTTAAAATT
CCCGTCGCTATCAAGGAATTAAGAGAAGCAACATCTCCGAAAGCCAACAAGGAAATCCTCGATGAAGCCT
ACGTGATGGCCAGCGTGGACAACCCCCACGTGTGCCGCCTGCTGGGCATCTGCCTCACCTCCACCGTGCA
GCTCATCACGCAGCTCATGCCCTTCGGCTGCCTCCTGGACTATGTCCGGGAACACAAAGACAATATTGGC
TCCCAGTACCTGCTCAACTGGTGTGTGCAGATCGCAAAGGGCATGAACTACTTGGAGGACCGTCGCTTGG
TGCACCGCGACCTGGCAGCCAGGAACGTACTGGTGAAAACACCGCAGCATGTCAAGATCACAGATTTTGG
GCTGGCCAAACTGCTGGGTGCGGAAGAGAAAGAATACCATGCAGAAGGAGGCAAAGTGCCTATCAAGTGG
ATGGCATTGGAATCAATTTTACACAGAATCTATACCCACCAGAGTGATGTCTGGAGCTACGGGGTGACTG
TTTGGGAGTTGATGACCTTTGGATCCAAGCCATATGACGGAATCCCTGCCAGCGAGATCTCCTCCATCCT
GGAGAAAGGAGAACGCCTCCCTCAGCCACCCATATGTACCATCGATGTCTACATGATCATGGTCAAGTGC
TGGATGATAGACGCAGATAGTCGCCCAAAGTTCCGTGAGTTGATCATCGAATTCTCCAAAATGGCCCGAG
ACCCCCAGCGCTACCTTGTCATTCAGGGGGATGAAAGAATGCATTTGCCAAGTCCTACAGACTCCAACTT
CTACCGTGCCCTGATGGATGAAGAAGACATGGACGACGTGGTGGATGCCGACGAGTACCTCATCCCACAG
CAGGGCTTCTTCAGCAGCCCCTCCACGTCACGGACTCCCCTCCTGAGCTCTCTGAGTGCAACCAGCAACA
ATTCCACCGTGGCTTGCATTGATAGAAATGGGCTGCAAAGCTGTCCCATCAAGGAAGACAGCTTCTTGCA
GCGATACAGCTCAGACCCCACAGGCGCCTTGACTGAGGACAGCATAGACGACACCTTCCTCCCAGTGCCT
GAATACATAAACCAGTCCGTTCCCAAAAGGCCCGCTGGCTCTGTGCAGAATCCTGTCTATCACAATCAGC
CTCTGAACCCCGCGCCCAGCAGAGACCCACACTACCAGGACCCCCACAGCACTGCAGTGGGCAACCCCGA
GTATCTCAACACTGTCCAGCCCACCTGTGTCAACAGCACATTCGACAGCCCTGCCCACTGGGCCCAGAAA
GGCAGCCACCAAATTAGCCTGGACAACCCTGACTACCAGCAGGACTTCTTTCCCAAGGAAGCCAAGCCAA
ATGGCATCTTTAAGGGCTCCACAGCTGAAAATGCAGAATACCTAAGGGTCGCGCCACAAAGCAGTGAATT
TATTGGAGCATGA

Amino acid sequence (SEQ ID NO: 10)

MRPSGTAGAALLALLAALCPASRALEEKKGNYVVTDHGSCVRACGADSYEMEEDGVRKCKKCEGPCRKVC
NGIGIGEFKDSLSINATNIKHFKNCTSISGDLHILPVAFRGDSFTHTPPLDPQELDILKTVKEITGFLLI
QAWPENRTDLHAFENLEIIRGRTKQHGQFSLAVVSLNITSLGLRSLKEISDGDVIISGNKNLCYANTINW
KKLFGTSGQKTKIISNRGENSCKATGQVCHALCSPEGCWGPEPRDCVSCRNVSRGRECVDKCNLLEGEPR
EFVENSECIQCHPECLPQAMNITCTGRGPDNCIQCAHYIDGPHCVKTCPAGVMGENNTLVWKYADAGHVC
HLCHPNCTYGCTGPGLEGCPTNGPKIPSIATGMVGALLLLLVVALGIGLFMRRRHIVRKRTLRRLLQERE
LVEPLTPSGEAPNQALLRILKETEFKKIKVLGSGAFGTVYKGLWIPEGEKVKIPVAIKELREATSPKANK

(continued)

| EGFR vIII |
|---|
| EILDEAYVMASVDNPHVCRLLGICLTSTVQLITQLMPFGCLLDYVREHKDNIGSQYLLNWCVQIAKGMNY LEDRRLVHRDLAARNVLVKTPQHVKITDFGLAKLLGAEEKEYHAEGGKVPIKWMALESILHRIYTHQSDV WSYGVTVWELMTFGSKPYDGIPASEISSILEKGERLPQPPICTIDVYMIMVKCWMIDADSRPKFRELIIE FSKMARDPQRYLVIQGDERMHLPSPTDSNFYRALMDEEDMDDVVDADEYLIPQQGFFSSPSTSRTPLLSS LSATSNNSTVACIDRNGLQSCPIKEDSFLQRYSSDPTGALTEDSIDDTFLPVPEYINQSVPKRPAGSVQN PVYHNQPLNPAPSRDPHYQDPHSTAVGNPEYLNTVQPTCVNSTFDSPAHWAQKGSHQISLDNPDYQQDFF PKEAKPNGIFKGSTAENAEYLRVAPQSSEFIGA |

In the present invention, "HER2-positive tumor" is tumor in which the HER2 protein or the HER2 gene is detected. The HER2 protein and the HER2 gene also include mutant HER2 protein and mutant HER2 gene having a point mutation, an insertion mutation, or a deletion mutation, etc.

Examples of the method for detecting the HER2 protein include usual detection methods commonly used, such as ELISA, Western blotting, and immunostaining using an antibody specifically binding to the HER2 protein. The antibody specifically binding to the HER2 protein may be a commercially available product or may be prepared by a usual method commonly used.

Examples of the method for detecting the HER2 gene include usual detection methods commonly used, such as Northern blotting, Southern blotting, RT-PCR, real-time PCR, digital PCR, DNA microarrays, *in situ* hybridization, and sequence analysis.

In the present invention, "EGFR-positive tumor" is tumor in which the EGFR protein or the EGFR gene is detected. The EGFR protein and the EGFR gene also include mutant EGFR protein and mutant EGFR gene having a point mutation, an insertion mutation, or a deletion mutation, etc.

Examples of the method for detecting the EGFR protein include usual detection methods commonly used, such as ELISA, Western blotting, and immunostaining using an antibody specifically binding to the EGFR protein. The antibody specifically binding to the EGFR protein may be a commercially available product or may be prepared by a usual method commonly used.

Examples of the method for detecting the EGFR gene include usual detection methods commonly used, such as Northern blotting, Southern blotting, RT-PCR, real-time PCR, digital PCR, DNA microarrays, *in situ* hybridization, and sequence analysis. Another example thereof includes a detection method using cobas EGFR mutation detection kit (Roche Diagnostics K.K.), which is a commercially available EGFR gene mutation detection kit.

In the present description, the term "effective amount" used regarding the pyrimidine compound and the other antitumor agent of the present invention means the amount of the pyrimidine compound and the other antitumor agent of the present invention that induces the biological or medical response of a subject, such as, for example, reduction or inhibition of enzyme or protein activity; or ameliorates symptoms, alleviates conditions, and retards or delays the progression of disease; or prevents disease; etc. (therapeutically effective amount).

In the present description, the term "subject" includes mammals and non-mammals. Examples of the mammal may include, but are not limited to, a human, a chimpanzee, an ape, a monkey, a bovine, a horse, sheep, a goat, a swine, a rabbit, a dog, a cat, a rat, a mouse, a Guinea pig, a hedgehog, a kangaroo, a mole, a wild pig, a bear, a tiger, and a lion. Examples of the non-mammal may include, but are not limited to, birds, fish, and reptiles. In one embodiment, the subject is a human, and may be a human who has been diagnosed to need the treatment for the symptoms, conditions or disease disclosed in the present description.

Upon the use of the pyrimidine compound of the present invention or a salt thereof and other antitumor agent as a medicament, a pharmaceutically acceptable carrier is mixed into it, as necessary, and various types of dosage forms can be adopted depending on the preventive or therapeutic purpose. Examples of the dosage form may include all of an oral agent, an injection, a suppository, an ointment, and a patch. Preferably, an oral agent is adopted. These dosage forms can be produced by commonly used production methods that are known to skilled persons. The pyrimidine compound of the present invention or the salt thereof and the other antitumor agent may be administered in the same dosage forms or may be administered in different dosage forms.

The dosing schedule of the pyrimidine compound of the present invention or a salt thereof and other antitumor agent is appropriately selected within a range in which each active ingredient exerts an antitumor effect. These active ingredients are administered concurrently or separately at an interval. For separate administration, either of them may be administered first.

The pyrimidine compound of the present invention or a salt thereof and other antitumor agent may be produced in a plurality of divided dosage forms of the respective active ingredients or may be produced together in one dosage form, on the basis of the dosage form or dosing schedule of each active ingredient. Also, the respective preparations may be produced and sold together in one package suitable for combined use or may be produced and sold in separate packages.

In one embodiment, the pyrimidine compound of the present invention or the salt thereof and the other antitumor agent are in the same preparation. Such a preparation may be, for example, a pharmaceutical composition comprising the pyrimidine compound of the present invention or the salt thereof, the other antitumor agent, and a pharmaceutically acceptable carrier.

In one embodiment, the pyrimidine compound of the present invention or the salt thereof and the other antitumor agent are in individual preparations. Such preparations may be, for example, a combination of a pharmaceutical composition comprising the pyrimidine compound of the present invention or the salt thereof and a pharmaceutically acceptable carrier, and a pharmaceutical composition comprising the other antitumor agent and a pharmaceutically acceptable carrier.

One embodiment of the present invention provides a kit preparation comprising the antitumor agent or the pharmaceutical combination mentioned above and an instruction stating that the pyrimidine compound or the salt thereof and the other antitumor agent are combined-administered.

As pharmaceutically acceptable carriers, various types of organic or inorganic carrier substances, which are commonly used as preparation materials, are used. When the compound of the present invention is processed into a solid preparation, examples of the pharmaceutically acceptable carrier mixed into the compound of the present invention may include an excipient, a binder, a disintegrator, a lubricant, a coating agent, and a coloring agent. When the compound of the present invention is processed into a liquid preparation, examples of the pharmaceutically acceptable carrier mixed into the compound of the present invention may include a solvent, a solubilizer, a suspending agent, a tonicity agent, a buffer, and a soothing agent. In addition, preparation additives such as an antiseptic, an antioxidant, a sweetener, and a stabilizer can also be used, as necessary.

In the case of preparing a solid preparation for oral administration, an excipient, and as necessary, a binder, a disintegrator, a lubricant, a coloring agent, a corrigent, etc. are added to the pyrimidine compound of the present invention, and thereafter, a tablet, a coated tablet, a granule, a powder agent, a capsule, etc. can be produced according to ordinary methods.

In the case of preparing an injection, a pH adjuster, a buffer, a stabilizer, a tonicity agent, a local anesthetic, etc. are added to the pyrimidine compound of the present invention, and thereafter, subcutaneous, intramuscular, and intravenous injections can be produced according to ordinary methods.

The amount of the pyrimidine compound or the salt thereof of the present invention to be mixed into the above-described each dosage unit form depends on the symptoms of a subject to whom the present compound should be applied, the dosage form and the like, and thus, the amount of the compound of the present invention is not constant. In general, it is preferable that the applied dose is set to be 0.05 to 1000 mg per dosage unit form as the pyrimidine compound in the case of an oral agent, it is set to be 0.01 to 500 mg per dosage unit form as the pyrimidine compound in the case of an injection, and it is set to be 1 to 1000 mg per dosage unit form as the pyrimidine compound in the case of a suppository.

The amount of the other antitumor agent to be mixed into the above-described each dosage unit form depends on the symptoms of a subject to whom the other antitumor agent should be applied, the dosage form and the like, and thus, the amount of the other antitumor agent is not constant. In general, it is preferable that the applied dose is set to be 0.05 to 1000 mg per dosage unit form in the case of an oral agent, it is set to be 0.01 to 500 mg per dosage unit form in the case of an injection, and it is set to be 1 to 1000 mg per dosage unit form in the case of a suppository.

The daily dose of a drug having the above-described dosage form is different depending on the symptoms, body weight, age, sex and the like of a subject, and thus, it cannot be generally determined. The pyrimidine compound of the present invention may be administered to an adult (body weight: 50 kg) at a daily dose of generally 0.05 to 5000 mg, and preferably 0.1 to 1000 mg. Also the amount of the other antitumor agent is different depending on the symptoms, body weight, age, sex and the like of a subject, and thus, it cannot be generally determined. For example, the other antitumor agent may be administered to an adult (body weight: 50 kg) at a daily dose of generally 0.05 to 5000 mg, and preferably 0.1 to 1000 mg.

In the present invention, the daily dose of the pyrimidine compound or the salt thereof and the other antitumor agent may be a dose that is determined by clinical trials or the like and brings about the maximum therapeutic effect within a range in which they can be safely used without developing serious side effects. Specific examples thereof include doses that are approved, recommended, and/or advised by public agencies or institutions such as the Pharmaceuticals and Medical Devices Agency (PMDA), the Food and Drug Administration (FDA), and the European Medicines Agency (EMA) and described in package inserts, interview forms, and/or treatment guidelines. Preferable is a dose approved by any public institution of PMDA, FDA and EMA.

The malignant tumor that is the target of the present invention is not particularly limited. Examples of the tumor may include brain tumor, head and neck cancer, digestive cancer (esophageal cancer, stomach cancer, duodenal cancer, liver cancer, biliary tract cancer (gallbladder and/or bile duct cancer, etc.), pancreatic cancer, colorectal cancer (colon cancer, rectal cancer, etc.), etc.), lung cancer (non-small cell lung cancer, small cell lung cancer, mesothelioma, etc.), breast cancer, genital cancer (ovarian cancer, uterine cancer (cervical cancer, endometrial cancer, etc.), etc.), urinary organ cancer (kidney cancer, bladder cancer, prostate cancer, testicular tumor, etc.), hematopoietic tumor (leukemia, malignant lymphoma, multiple myeloma, etc.), bone and/or soft tissue tumor, and skin cancer. Among these, preferable is brain tumor, lung cancer, breast cancer, stomach cancer, colorectal cancer, bladder cancer, biliary tract cancer or uterine cancer, and more preferable is brain tumor, lung cancer, breast cancer, stomach cancer, colorectal cancer, bladder cancer, or biliary tract cancer.

In one embodiment, the tumor that is the target of the present invention is malignant tumor having HER2 overexpression, HER2 gene amplification, or HER2 mutation. Examples of the malignant tumor may include brain tumor, head and neck cancer, digestive cancer (esophageal cancer, stomach cancer, duodenal cancer, liver cancer, biliary tract cancer (gallbladder and/or bile duct cancer, etc.), pancreatic cancer, colorectal cancer (colon cancer, rectal cancer, etc.), etc.), lung cancer (non-small cell lung cancer, small cell lung cancer, mesothelioma, etc.), breast cancer, genital cancer (ovarian cancer, uterine cancer (cervical cancer, endometrial cancer, etc.), etc.), urinary organ cancer (kidney cancer, bladder cancer, prostate cancer, testicular tumor, etc.), hematopoietic tumor (leukemia, malignant lymphoma, multiple myeloma, etc.), bone and/or soft tissue tumor, and skin cancer. Among these, preferable is brain tumor, lung cancer, breast cancer, stomach cancer, colorectal cancer, bladder cancer, biliary tract cancer or uterine cancer, and more preferable is brain tumor, lung cancer, breast cancer, stomach cancer, bladder cancer, or biliary tract cancer.

In one embodiment, the tumor that is the target of the present invention is HER2-positive tumor. Examples of the tumor may include brain tumor, head and neck cancer, digestive cancer (esophageal cancer, stomach cancer, duodenal cancer, liver cancer, biliary tract cancer (gallbladder and/or bile duct cancer, etc.), pancreatic cancer, colorectal cancer (colon cancer, rectal cancer, etc.), etc.), lung cancer (non-small cell lung cancer, small cell lung cancer, mesothelioma, etc.), breast cancer, genital cancer (ovarian cancer, uterine cancer (cervical cancer, endometrial cancer, etc.), etc.), urinary organ cancer (kidney cancer, bladder cancer, prostate cancer, testicular tumor, etc.), hematopoietic tumor (leukemia, malignant lymphoma, multiple myeloma, etc.), bone and/or soft tissue tumor, and skin cancer. Among these, preferable is brain tumor, lung cancer, breast cancer, stomach cancer, colorectal cancer, bladder cancer, biliary tract cancer or uterine cancer, and more preferable is brain tumor, lung cancer, breast cancer, stomach cancer, bladder cancer, or biliary tract cancer.

In one embodiment, the tumor that is the target of the present invention is malignant tumor having EGFR overexpression, EGFR gene amplification, or an EGFR mutation. Examples of the malignant tumor may include brain tumor, head and neck cancer, digestive cancer (esophageal cancer, stomach cancer, duodenal cancer, liver cancer, biliary tract cancer (gallbladder and/or bile duct cancer, etc.), pancreatic cancer, colorectal cancer (colon cancer, rectal cancer, etc.), etc.), lung cancer (non-small cell lung cancer, small cell lung cancer, mesothelioma, etc.), breast cancer, genital cancer (ovarian cancer, uterine cancer (cervical cancer, endometrial cancer, etc.), etc.), urinary organ cancer (kidney cancer, bladder cancer, prostate cancer, testicular tumor, etc.), hematopoietic tumor (leukemia, malignant lymphoma, multiple myeloma, etc.), bone and/or soft tissue tumor, and skin cancer. Among these, preferable is brain tumor, head and neck cancer, lung cancer, breast cancer, stomach cancer, colorectal cancer, bladder cancer, biliary tract cancer or uterine cancer, and more preferable is brain tumor, lung cancer, breast cancer, or colorectal cancer.

In one embodiment, the tumor that is the target of the present invention is EGFR-positive tumor. Examples of the tumor may include brain tumor, head and neck cancer, digestive cancer (esophageal cancer, stomach cancer, duodenal cancer, liver cancer, biliary tract cancer (gallbladder and/or bile duct cancer, etc.), pancreatic cancer, colorectal cancer (colon cancer, rectal cancer, etc.), etc.), lung cancer (non-small cell lung cancer, small cell lung cancer, mesothelioma, etc.), breast cancer, genital cancer (ovarian cancer, uterine cancer (cervical cancer, endometrial cancer, etc.), etc.), urinary organ cancer (kidney cancer, bladder cancer, prostate cancer, testicular tumor, etc.), hematopoietic tumor (leukemia, malignant lymphoma, multiple myeloma, etc.), bone and/or soft tissue tumor, and skin cancer. Among these, preferable is brain tumor, head and neck cancer, lung cancer, breast cancer, stomach cancer, colorectal cancer, bladder cancer, biliary tract cancer or uterine cancer, and more preferable is brain tumor, lung cancer, breast cancer, or colorectal cancer.

In one embodiment, the tumor is a brain tumor. The pyrimidine compound of the present invention may be useful for the treatment of the symptoms of brain that is required to pass through the blood-brain barrier. The pyrimidine compound of one embodiment has favorable permeability through the blood-brain barrier for the delivery thereof into the brain, namely, excellent brain penetration properties. As an indicator of the penetration properties of the compound into the brain, the concentration of the compound in the brain or a Kp value (brain-to-plasma drug concentration ratio) is applied.

The brain tumor to be treated iIn the present invention includes metastatic brain tumor and primary brain tumor.

Examples of the brain tumor may include, but are not particularly limited to, metastatic brain tumor (e.g., brain

metastasis of lung cancer, breast cancer, stomach cancer, colorectal cancer, bladder cancer, biliary tract cancer, uterine cancer, etc. (preferably, lung cancer, breast cancer, or stomach cancer)), piliocytic astrocytoma, diffuse astrocytoma, oligodendroma and/or oligodendroastrocytoma, anaplastic astrocytoma and/or anaplastic oligodendroglioma, anaplastic oligodendroastrocytoma, glioblastoma, ependymoma, anaplastic ependymoma, ganglioglioma, central neurocytoma, medulloblastoma, germinoma, central nervous system malignant lymphoma, meningioma, neurilemmoma, GH secreting pituitary adenoma, PRL-secreting pituitary adenoma, ACTH-secreting pituitary adenoma, nonfunctional pituitary adenoma, craniopharyngioma, chordoma, hemangioblastoma, and epidermoid tumor.

EXAMPLES

Hereinafter, the present invention will be described in detail in the following examples. However, these examples are not intended to limit the scope of the present invention.

In the present description, "room temperature" generally means a temperature that is from approximately 10°C to approximately 35°C. In addition, in the following Examples regarding compounds, "%" indicates weight percent, unless otherwise specified.

Various types of reagents used in the Examples were commercially available products, unless otherwise specified. Silica gel chromatography was carried out using Biotage SNAP Cartridge Ultra, manufactured by Biotage Japan Ltd. Basic silica gel chromatography was carried out using Biotage SNAP Cartridge Isolute Flash-NH2, manufactured by Biotage Japan Ltd.

Preparative thin-layer chromatography was carried out using Kieselgel TM60F254, Art. 5744, manufactured by Merck, or NH2 Silica Gel 60F254 Plate-Wako, manufactured by FUJIFILM Wako Pure Chemical Cooperation.

$^{1}$H-NMR was measured using tetramethylsilane as a reference material, and employing AL400 (400 MHz) manufactured by JEOL, Mercury (400 MHz) manufactured by Varian, or Inova (400 MHz) manufactured by Varian. Moreover, mass spectrum was measured using Micromass ZQ or SQD manufactured by Waters, according to electrospray ionization (ESI) or atmospheric pressure chemical ionization (APCI). Microwave reaction was carried out using Initiator manufactured by Biotage Japan Ltd.

Abbreviations have the following meanings.

s: Singlet
d: Doublet
t: Triplet
q: Quartet
dd: Double doublet
dt: Double triplet
td: Triple doublet
tt: Triple triplet
ddd: Double double doublet
ddt: Double double triplet
dtd: Double triple doublet
tdd: Triple double doublet
m: Multiplet
br: Broad
ATP: Adenosine triphosphate
DMSO-d6: Deuterated dimethyl sulfoxide
$CDCl_3$: Deuterated chloroform
EDTA: Ethylenediaminetetraacetic acid
THF: Tetrahydrofuran
DMF: N,N-dimethylformamide
DMSO: Dimethyl sulfoxide
NMP: N-methyl pyrrolidone
HATU: O-(7-azabenzotriazol-1-yl)-N,N,N',N'-tetramethyluronium hexafluorophosphate
HPMC: Hypromellose
$PdCl_2(PPh_3)_2$: Dichlorobis(triphenylphosphine)palladium(II)
po: Oral administration
iv: Intravenous administration
T-mab: Trastuzumab
P-mab: Pertuzumab

Reference Example 1

Reference Example 1(1)

tert-Butyl (2S,4R)-4-(4-amino-5-iodo-7H-pyrrolo[2,3-d]pyrimidin-7-yl)-2 - methylpyrrolidine-1-carboxylate

tert-Butyl (2S,4S)-4-hydroxy-2-methylpyrrolidine-1-carboxylate (19.0 g) and 4-chloro-5-iodo-7H-pyrrolo[2,3-d]pyrimidine (13.1 g) were dissolved in THF (190 mL), and the obtained solution was then cooled to 0°C. Thereafter, triphenylphosphine (37.2 g) and diisopropyl azodicarboxylate (28.1 mL) were added to the reaction solution, and the temperature of the mixture was then increased to room temperature, followed by stirring for 1 hour. Thereafter, the reaction mixture was concentrated under reduced pressure, and the obtained residue was then purified by silica gel chromatography (hexane : ethyl acetate) to obtain the corresponding coupling body. The obtained compound was used in the subsequent reaction without being further purified.

The obtained coupling body, THF (114 mL) and ammonia water (114 mL) were added into a pressure resistant tube, and the obtained mixture was then stirred at 100°C for 14 hours. Thereafter, the reaction mixture was cooled to room temperature, and was then poured into water (285 mL). The thus obtained mixture was stirred at room temperature for 5 hours. Thereafter, the precipitated solid was collected by filtration, was then washed with water, and was then dried to obtain a product of interest (34.5 g). $^1$HNMR ($CDCl_3$)δ: 8.27(s,1H) 7.15(s,1H) 5.55-5.73(m,2H) 5.12-5.25(m,1H) 3.86-4.18(m,2H) 3.43-3.57(m,1H) 2.59-2.69(m,1H) 1.92-2.03(m,1H) 1.48(s,9H) 1.30-1.40(m,3H)
ESI-MS m/z 444 (MH+)

Reference Example 1(2)

4-Amino-7-((3R,5S)-1-(tert-butoxycarbonyl)-5-methylpyrrolidin-3-yl)-7H-pyrrolo[2,3-d]pyrimidine-5-carboxylic acid

The compound of Reference Example 1(1) (28.0 g), 10% palladium carbon catalyst (720 mg), NMP (84 mL), methanol (26 mL), and triethylamine (17.6 mL) were added into a pressure resistant tube, followed by carbon monoxide substitution, and the obtained mixture was stirred at 100°C for 2 hours. Thereafter, the reaction mixture was cooled to room temperature, a 2 M sodium hydroxide aqueous solution (79 mL) was then added thereto, and the obtained mixture was then stirred at 80°C for 2 hours. Thereafter, the reaction mixture was cooled to room temperature, was then filtrated through Celite, and was then washed with methanol. Subsequently, methanol in the filtrate was concentrated under reduced pressure. Water was further added, and the water layer was then washed with tert-butyl methyl ether. A 1 M potassium hydrogen sulfate aqueous solution was added to the water layer to adjust the pH to approximately 3. The precipitated solid was collected by filtration, was then washed with water, and was then dried to obtain a product of interest (23.4 g).
$^1$HNMR (400MHz, DMSO-d6)δ: 8.14 (s, 1H) 8.08 (s, 1H) 5.16-4.93(m,1H) 4.07-3.79(m,2H) 3.61-3.45(m,1H) 2.53(m,1H) 2.33-2.02(m,1H) 1.42(s,9H) 1.29(d,J = 6.1Hz,3H) ESI-MS m/z 362 (MH+)

Examples

Example 1(1)

tert-Butyl-4-amino-6-bromo-7-((3R,5S)-1-(tert-butoxycarbonyl)-5-methylpyrrolidin-3-yl)-7H-pyrrolo[2,3-d]pyrimidine-5-carboxylate

Under a nitrogen atmosphere, the compound of Reference Example 1(2) (15.0 g) was dissolved in chloroform (150 mL), and 2-tert-butyl-1,3-diisopropylisourea (25 mL) was then added to the above obtained solution. The temperature of the obtained mixture was increased to 60°C, and the mixture was then stirred for 2 hours. Thereafter, 2-tert-butyl-1,3-diisopropylisourea (25 mL) was further added to the reaction mixture, and the thus obtained mixture was then stirred for 2 hours. Thereafter, the reaction mixture was cooled to room temperature, and was then concentrated under reduced pressure. To the obtained residue, tert-butyl methyl ether was added, and the precipitated solid was collected by filtration and was then washed with tert-butyl methyl ether. The filtrate was concentrated under reduced pressure, and tert-butyl methyl ether was then added to the obtained residue. The precipitated solid was collected by filtration, and was then washed with tert-butyl methyl ether. The obtained residue was purified by silica gel chromatography (hexane : ethyl acetate) to obtain a tert-butyl ester form. The obtained compound was used in the subsequent halogenation reaction without being further purified.

The obtained tert-butyl ester form was dissolved in chloroform (140 mL), and N-bromosuccinimide (11.8 g) was then added to the above obtained solution. The obtained mixture was stirred at room temperature for 24 hours. Thereafter,

to the reaction mixture, chloroform and 10% sodium bisulfite aqueous solution were successively added, and the obtained mixture was then extracted with chloroform. The gathered organic layer was washed with saturated saline, was then dried over anhydrous sodium sulfate, and was then concentrated under reduced pressure. The obtained residue was purified by silica gel chromatography (hexane : ethyl acetate) to obtain a product of interest (13.8 g).
$^1$HNMR (CDCl3)δ: 8.02 (s, 1H) 5.74-5.13(m,2H) 4.07-3.64(m,2H) 2.43-2.29(m,1H) 2.07-1.97(m,1H) 1.63(s,9H) 1.48(m,12H)
ESI-MS m/z 496,498 (MH+)

Example 1(2)

tert-Butyl-7-((3R,5S)-1-acryloyl-5-methylpyrrolidin-3-yl)-4-amino-6-bromo-7H-pyrrolo[2,3-d]pyrimidine-5-carboxylate

The compound of Example 1(1) (11.4 g) was dissolved in THF (57 mL), and the obtained solution was then cooled to 0°C. Thereafter, 4 M hydrogen chloride in 1,4-dioxane solution (114 mL) was added to the mixture, and the thus obtained mixture was then stirred at 0°C for 10 hours. Subsequently, to the reaction mixture, a 5 M sodium hydroxide aqueous solution (92 mL), acetonitrile (57 mL), diisopropylethylamine (20 mL), and acryloyl chloride (2.0 mL) were added, and the obtained mixture was then stirred for 30 minutes. Thereafter, the reaction mixture was extracted with ethyl acetate, and the gathered organic layer was washed with saturated saline, was then dried over anhydrous sodium sulfate, and was then concentrated under reduced pressure. The obtained residue was purified by silica gel chromatography (hexane : acetone) to obtain a product of interest (7.72 g).
$^1$HNMR ($CDCl_3$)δ: 8.26-8.16(m,1H) 6.62-6.30(m,2H) 5.81-5.64(m,1H) 5.33-5.14(m,1H) 4.81-3.75(m,3H) 3.07-2.86(m,1H) 2.67-2.33(m,1H) 1.69-1.61(m,9H) 1.60-1.51(m,3H) ESI-MS m/z 450,452 (MH+)

Example 1(3)

tert-Butyl-7-((3R,5S)-1-acryloyl-5-methylpyrrolidin-3-yl)-4-amino-6-(prop-1-yn-1-yl)-7H-pyrrolo[2,3-d]pyrimidine-5-carboxylate

1.0 M Propyne in DMF solution (85.7 mL) was added to the compound of Example 1(2) (7.72 g), acetonitrile (154 mL), triethylamine (7.2 mL), $PdCl_2(PPh_3)_2$ (1.2 g), and copper(I) iodide (330 mg), followed by nitrogen substitution. Thereafter, the mixture was stirred at 70°C for 4 hours. Thereafter, the reaction mixture was cooled to room temperature, and ethyl acetate and a saturated sodium hydrogen carbonate aqueous solution were added to the mixture. Thereafter, the obtained mixture was extracted with ethyl acetate, and the gathered organic layer was washed with water, and then with saturated saline. The resultant was dried over anhydrous sodium sulfate, and was then concentrated under reduced pressure. The obtained residue was purified by silica gel chromatography (hexane : acetone) to obtain a product of interest (4.06 g). $^1$HNMR ($CDCl_3$)δ: 8.29-8.17(m,1H) 6.63-6.30(m,2H) 5.81-5.63(m,1H) 5.42-5.15(m,1H) 4.66-3.81(m,3H) 3.01-2.82(m,1H) 2.65-2.32(m,1H) 2.92-2.13(m,3H) 1.65-1.59(m,9H) 1.57-1.49(m,3H)
ESI-MS m/z 410 (MH+)

Example 1(4)

7-((3R,5 S)-1 -acryloyl-5-methylpyrrolidin-3-yl)-4-amino-6-(prop-1 -yn-1 -yl)-7H-pyrrolo[2,3-d]pyrimidine-carboxylic acid

The compound of Example 1(3) (1.52 g) was dissolved in chloroform (5 mL), and trifluoroacetic acid (5 mL) was then added to the above obtained solution. The mixture was stirred at room temperature for 2 hours, and the reaction mixture was then concentrated under reduced pressure. To the residue, chloroform was added, and the obtained mixture was concentrated under reduced pressure again. The residue was dried under reduced pressure to obtain a product of interest (1.25 g).
ESI-MS m/z 354 (MH+)

Example 1(5)

7-(R)-((3R,5 S)-1-acryloyl-5-methylpyrrolidin-3-yl)-4-amino-N-((R)-1-(3,5-difluorophenyl)ethyl)-6-(prop-1-yn-1-yl)-7H-pyrrolo[2,3-d]pyrimidine-5-carboxamide

To the compound of Example 1(4) (100 mg) in DMF (1.0 mL) solution, (R)-1-(3,5-difluorophenyl)ethan-1-amine (89.0 mg), diisopropylethylamine (0.25 mL), and HATU (215 mg) were added, and the obtained mixture was then stirred at room temperature for 2 hours. Thereafter, to the reaction mixture, a saturated sodium hydrogen carbonate aqueous

solution was added, and the obtained mixture was then extracted with ethyl acetate. The gathered organic layer was washed with saturated saline, was then dried over anhydrous sodium sulfate, and was then concentrated under reduced pressure. The obtained residue was purified by silica gel chromatography (hexane : acetone) to obtain the title compound (60 mg).
$^1$HNMR (DMSO-d6)δ: 8.51(d,*J* = 7.3Hz,1H) 8.16 (s, 1H) 7.25-7.07(m,3H) 6.74-6.47(m,1H) 6.25-6.08(m,1H) 5.78-5.58(m,1H) 5.41-5.21(m,1H) 5.21-5.06(m,1H) 4.45-4.29(m,1H) 4.24-3.91(m,2H) 2.78-2.58(m,1H) 2.52-2.41(m,1H) 2.23(s,3H) 1.48(d,*J* = 7.1Hz,3H) 1.39(d,*J*= 6.1Hz,3H)
ESI-MS m/z 493 (MH+)

Example 2

7-((3R,5S)-1-acryloyl-5-methylpyrrolidin-3-yl)-4-amino-N-((R)-1-phenylethyl)-6-(prop-1-yn-1-yl)-7H-pyrrolo[2,3-d]pyrimidine-5-carboxamide

The title compound was obtained in the same manner as that of Example 1, with the exception that (R)-1-phenylethan-1-amine was used instead of (R)-1-(3,5-difluorophenyl)ethan-1-amine in Example 1(5).
$^1$HNMR (DMSO-d6)δ: 8.35(d,*J* = 7.8Hz,1H) 8.17-8.13(m,1H) 7.48-7.23(m,5H) 6.76-6.46(m,1H) 6.28-6.06(m,1H) 5.81-5.58(m,1H) 5.43-5.02(m,2H) 4.42-4.28(m,1H) 4.21-3.96(m,2H) 2.74-2.59(m,1H) 2.54-2.41(m,1H) 2.17(s,3H) 1.50(d,*J* = 6.8Hz,3H) 1.42-1.33(m,3H)
ESI-MS m/z 457 (MH+)

Example 3

7-((3R,5S)-1-acryloyl-5-methylpyrrolidin-3-yl)-4-amino-N-(2-phenylpropan-2-yl)-6-(prop-1-yn-1-yl)-7H-pyrrolo[2,3-d]pyrimidine-5-carboxamide

The title compound was obtained in the same manner as that of Example 1, with the exception that 2-phenylpropan-2-amine was used instead of (R)-1-(3,5-difluorophenyl)ethan-1-amine in Example 1(5).
$^1$HNMR (DMSO-d6)δ: 8.26(s,1H) 8.16-8.08(m,1H) 7.44(dd,*J* = 8.8,1.2Hz,2H) 7.38-7.28(m,2H) 7.21(tt,*J* = 7.3,1.27Hz,1H) 6.76-6.50(m,1H) 6.25-6.10(m,1H) 5.79-5.62(m,1H) 5.45-5.19(m,1H) 4.45-4.30(m,1H) 4.26-4.01(m,2H) 2.79-2.42(m,2H) 2.29-2.22(m,3H) 1.71(s,6H) 1.43-1.36(m,3H)
ESI-MS m/z 471 (MH+)

Example 4

7-((3R,5S)-1-acryloyl-5-methylpyrrolidin-3-yl)-4-amino-N-((R)-1-phenylpropyl)-6-(prop-1-yn-1-yl)-7H-pyrrolo[2,3-d]pyrimidine-5-carboxamide

The title compound was obtained in the same manner as that of Example 1, with the exception that (R)-1-phenylpropan-1-amine was used instead of (R)-1-(3,5-difluorophenyl)ethan-1-amine in Example 1(5).
$^1$HNMR (DMSO-d6)δ: 8.35(brd,*J* = 8.0Hz,1H) 8.17-8.11(m,1H) 7.46-7.22(m,5H) 6.74-6.50(m,1H) 6.26-6.08(m,1H) 5.79-5.60(m,1H) 5.40-5.21(m,1H) 4.99-4.87(m,1H) 4.43-4.30(m,1H) 4.23-3.94(m,2H) 2.76-2.42(m,2H) 2.21(s,3H) 1.95-1.74(m,2H) 1.44-1.34(m,3H) 0.91(t,*J* = 7.3Hz,3H)
ESI-MS m/z 471 (MH+)

Example 5

7-((3R,5S)-1-acryloyl-5-methylpyrrolidin-3-yl)-4-amino-N-(2-(2-fluorophenyl)propan-2-yl)-6-(prop-1-yn-1-yl)-7H-pyrrolo[2,3-d]pyrimidine-5-carboxamide

The title compound was obtained in the same manner as that of Example 1, with the exception that 2-(2-fluorophenyl)propan-2-amine was used instead of (R)-1-(3,5-difluorophenyl)ethan-1-amine in Example 1(5).
$^1$HNMR ($CDCl_3$)δ: 8.28(s,1H) 8.11(d,J = 4.4Hz,1H) 8.02(s,1H) 7.47-7.42(m,1H) 7.29-7.23(m,1H) 7.15(t, J = 7.7Hz,1H) 7.02(ddd,J = 12.5,8.1,1.1Hz,1H) 6.58-6.35(m,2H) 5.79-5.70(m,1H) 5.30-5.19(m,1H) 4.53(t,J = 10.1Hz,0.7H) 4.38-4.25(m,1.6H) 3.92(t,J = 8.8Hz,0.7H) 2.91-2.78(m,1H) 2.70-2.60(m,0.3H) 2.54-2.43(m,0.7H) 2.28(d,J = 7.0Hz,3H) 1.88(dt,J = 10.0,5.0Hz,6H) 1.53(t,J = 6.2Hz,3H)
ESI-MS m/z 489 (MH+)

Example 6

7-((3R,5S)-1-acryloyl-5-methylpyrrolidin-3-yl)-4-amino-N-((R)-1-(3-chlorophenyl)ethyl)-6-(prop-1-yn-1-yl)-7H-pyrrolo[2,3-d]pyrimidine-5-carboxamide

The title compound was obtained in the same manner as that of Example 1, with the exception that (R)-(+)-1-(3-chlorophenyl)ethylamine hydrochloride was used instead of (R)-1-(3,5-difluorophenyl)ethan-1-amine in Example 1(5).
$^1$HNMR (CDCl3)δ: 8.22(d,J = 5.9Hz,1H) 7.75(d,J = 7.0Hz,1H) 7.38(s,1H) 7.35-7.27(m,3H) 6.58-6.33(m,2H) 5.78-5.66(m,1H) 5.29-5.19(m,2H) 4.56(t,J = 10.3Hz,0.7H) 4.39-4.20(m,1.6H) 3.89(t,J = 8.8Hz,0.7H) 2.94-2.82(m,1H) 2.66-2.58(m,0.3H) 2.46(dt,J = 14.5,6.1Hz,0.7H) 2.18(d,J = 11.0Hz,3H) 1.60(d,J = 7.0Hz,3H) 1.55-1.51(m,3H)
ESI-MS m/z 491,493 (MH+)

Example 7

7-((3R,5S)-1-acryloyl-5-methylpyrrolidin-3-yl)-4-amino-N-((R)-1-(2,4-difluorophenyl)ethyl)-6-(prop-1-yn-1-yl)-7H-pyrrolo[2,3-d]pyrimidine-5-carboxamide

The title compound was obtained in the same manner as that of Example 1, with the exception that (R)-(+)-1-(2,4-difluorophenyl)ethylamine hydrochloride was used instead of (R)-1-(3,5-difluorophenyl)ethan-1-amine in Example 1(5).
$^1$HNMR ($CDCl_3$)δ: 8.20(d,*J* = 5.9Hz,1H) 7.98(d,*J* = 7.7Hz,1H) 7.37-7.31(m,1H) 6.90-6.81(m,2H) 6.58-6.35(m,2H) 5.78-5.65(m,1H) 5.44-5.37(m,1H) 5.30-5.19(m,1H) 4.56(t,*J* = 10.1Hz,0.7H) 4.38-4.23(m,1.6H) 3.88(t,*J* = 8.8Hz,0.7H) 2.94-2.83(m,1H) 2.66-2.57(m,0.3H) 2.51-2.42(m,0.7H) 2.27(d,*J* = 9.2Hz,3H) 1.61(d,*J* = 7.0Hz,3H) 1.56-1.51(m,3H)
ESI-MS m/z 493 (MH+)

Example 8

7-((3R,5S)-1-acryloyl-5-methylpyrrolidin-3-yl)-4-amino-6-(prop-1-yn-1-yl)-N-((S)-2,2,2-trifluoro-1-phenylethyl)-7H-pyrrolo[2,3-d]pyrimidine-5-carboxamide

The title compound was obtained in the same manner as that of Example 1, with the exception that (S)-2,2,2-trifluoro-1-phenylethan-1-amine was used instead of (R)-1-(3,5-difluorophenyl)ethan-1-amine in Example 1(5).
$^1$HNMR ($CDCl_3$)δ: 8.40(d,*J* = 8.8Hz,1H) 8.16(s,1H) 7.44(s,5H) 6.58-6.38(m,2H) 5.92-5.84(m,1H) 5.81-5.69(m,1H) 5.29-5.19(m,1H) 4.55(t,*J* = 10.3Hz,0.7H) 4.41-4.24(m,1.6H) 3.91(t,*J* = 8.6Hz,0.7H) 2.92-2.80(m,1H) 2.70-2.61(m,0.3H) 2.54-2.46(m,0.7H) 2.35(d,*J* = 8.4Hz,3H) 1.54(t,*J* = 7.3Hz,3H)
ESI-MS m/z 511 (MH+)

Example 9

7-((3R,5S)-1-acryloyl-5-methylpyrrolidin-3-yl)-4-amino-6-(cyclopropylethynyl)-N-(2-phenylpropan-2-yl)-7H-pyrrolo[2,3-d]pyrimidine-5-carboxamide

The title compound was obtained in the same manner as that of Example 1, with the exceptions that cyclopropylacetylene was used instead of 1.0 M propyne in DMF solution in Example 1(3), and that 2-phenylpropan-2-amine was used instead of (R)-1-(3,5-difluorophenyl)ethan-1-amine in Example 1(5).
$^1$HNMR ($CDCl_3$)δ: 8.15(s,1H) 8.00(s,1H) 7.44(d,*J* = 7.7Hz,2H) 7.37(t,*J* = 7.7Hz,2H) 7.32-7.27(m, 1H) 6.66-6.30(m,2H) 5.81-5.69(m,1H) 5.38-5.24(m,1H) 4.48(t,*J* = 9.9Hz,0.7H) 4.42-4.29(m,1.6H) 4.22(t,*J* = 10.4Hz,0.7H) 2.77-2.68(m,1H) 2.67-2.60(m,0.3H) 2.59-2.52(m,0.7H) 1.83(s,6H) 1.60-1.52(m,4H) 1.08-1.01(m,2H) 0.92-0. 8 8 (m,2H)
ESI-MS m/z 497 (MH+)

Example 10

7-((3R,5S)-1-acryloyl-5-methylpyrrolidin-3-yl)-4-amino-6-(cyclopropylethynyl)-N-((R)-1-(2,3-difluorophenyl)ethyl)-7H-pyrrolo[2,3-d]pyrimidine-5-carboxamide

The title compound was obtained in the same manner as that of Example 1, with the exceptions that cyclopropylacetylene was used instead of 1.0 M propyne in DMF solution in Example 1(3), and that (R)-(+)-1-(2,3-difluorophenyl)ethylamine was used instead of (R)-1-(3,5-difluorophenyl)ethan-1-amine in Example 1(5).
$^1$HNMR ($CDCl_3$)δ: 8.17(d,*J* = 4.0Hz,1H) 8.04(d,*J* = 8.1Hz,1H) 7.15-7.05(m,3H) 6.58-6.36(m,2H)5.80-5.68(m,1H) 5.49-5.42(m,1H) 5.34-5.24(m,1H) 4.52(t,*J* = 10.1Hz,0.7H) 4.37-4.23(m,1.6H) 3.92(t,*J* = 8.8Hz,0.7H) 2.86-2.76(m,1H) 2.69-2.63(m,0.3H) 2.52-2.46(m,0.7H) 1.73-1.63(m,4H) 1.55(t,*J*= 5.3Hz,3H) 1.14-1.07(m,2H) 1.01-0.92(m,2H) ESI-MS m/z 519 (MH+)

Example 11

Example 11(1)

tert-Butyl(2S,4R)-4-(4-amino-6-bromo-5-(((R)-1-phenylethyl)carbamoyl)-7H-pyrrolo[2,3-d]pyrimidin-7-yl)-2-methylpyrrolidine-1-carboxylate

The compound of Reference Example 1(2) (1.00 g), (R)-(+)-1-phenylethylamine (0.503 g), diisopropylethylamine (1.79 g), and N,N-dimethylformamide (10 mL) were added, and subsequently, HATU (1.58 g) was added. The obtained mixture was stirred at room temperature overnight. Thereafter, to the reaction mixture, ethyl acetate and a saturated sodium hydrogen carbonate aqueous solution were added, and the obtained mixture was then extracted with ethyl acetate. The gathered organic layer was washed with water, and then with saturated saline. The resultant was dried over anhydrous sodium sulfate, and was then concentrated under reduced pressure. The obtained residue was purified by silica gel chromatography (hexane : acetone) to obtain an amide form (1.53 g). The obtained compound was used in the subsequent reaction without being further purified.

To the amide form (1.53 g), chloroform (15 mL) was added, and the obtained mixture was then cooled to 0°C. Thereafter, N-bromosuccinimide (0.88 g) was added to the reaction mixture, and the obtained mixture was then stirred at 0°C for 1 hour. Thereafter, the reaction mixture was concentrated under reduced pressure, and the obtained residue was purified by silica gel chromatography (hexane : ethyl acetate) to obtain a product of interest (1.39 g).
$^1$HNMR ($CDCl_3$)δ: 8.21 (s, 1H) 7.42-7.28(m,5H) 6.97(d,*J*= 7.3Hz,1H) 5.36-5.29(m,1H) 5.20-5.07(m,1H) 4.30(t,*J* = 10.3Hz,1H) 4.04-3.72(m,2H) 3.00-2.86(m,1H) 2.38(dt,*J* = 14.3,6.0Hz,1H) 1.63(d,*J* = 7.0Hz,3H) 1.53-1.43(m,12H)
ESI-MS m/z 543,545 (MH+)

Example 11(2)

7-((3R,5S)-1-acryloyl-5-methylpyrrolidin-3-yl)-4-amino-6-bromo-N-((R)-1-phenylethyl)-7H-pyrrolo[2,3-d]pyrimidine-5-carboxamide

To the compound of Example 11(1) (600 mg), chloroform(3 mL) was added, and the obtained mixture was then cooled to 0°C. Thereafter, trifluoroacetic acid (4.44 g) was added to the reaction mixture, and the thus obtained mixture was then stirred at room temperature for 1 hour. Thereafter, the reaction mixture was concentrated under reduced pressure, and acetonitrile (5 mL) was then added to the residue. The obtained mixture was concentrated under reduced pressure again to obtain an amine form. The obtained compound was used in the subsequent reaction without being further purified.

To the obtained amine form, acetonitrile (3 mL) was added, and the obtained mixture was then cooled to 0°C. Thereafter, acryloyl chloride (99.9 mg) and diisopropylethylamine (713 mg) were added, and the obtained mixture was then stirred at 0°C for 1 hour. Thereafter, the reaction mixture was concentrated under reduced pressure, and the obtained residue was purified by silica gel chromatography (ethyl acetate : methanol) to obtain a product of interest (281 mg).
$^1$HNMR ($CDCl_3$)δ: 8.20(d,*J* = 7.3Hz,1H) 7.42-7.36(m,4H) 7.32-7.28(m,1H) 7.00-6.94(m,1H) 6.57-6.33(m,2H) 5.76-5.66(m,1H) 5.36-5.29(m, 1H) 5.14-5.08(m,1H) 4,71(t,*J*= 9.9Hz,0.7H) 4.42-4.23(m,1.6H) 3.83(t,*J*= 8.6Hz,0.7H) 3.03-2.92(m,1H) 2.60-2.57(m,0.3H) 2.44-2.40(m, 0.7H) 1.64(d,*J*= 6.6Hz,3H) 1.56(dd,*J*= 11:7,6.2Hz,3H)
ESI-MS m/z 497,499 (MH+)

Example 11(3)

7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide

The compound of Example 11(2) (65 mg), dichlorobis(triphenylphosphine)palladium (9.2 mg), copper(I) iodide (5.0 mg), cyclopropylacetylene (13.0 mg), triethylamine (39.7 mg), and N,N-dimethylformamide (1.3 mL) were added, and the inside of the reaction system was then substituted with nitrogen. After that, the mixture was stirred at 70°C for

2.5 hours. Thereafter, to the reaction mixture, ethyl acetate and a saturated ammonium chloride aqueous solution were added, and the obtained mixture was then extracted with ethyl acetate. The gathered organic layer was washed with water, and then with saturated saline. The resultant was dried over anhydrous sodium sulfate, and was then concentrated under reduced pressure. The obtained residue was purified by silica gel chromatography (chloroform : methanol) to obtain a product of interest (50 mg).
$^1$HNMR ($CDCl_3$)δ: 8.22(d,*J* = 5.1Hz,1H) 7.82(d,*J*= 7.3Hz,1H) 7.43-7.35(m,4H) 7.30(t,*J* = 6.8Hz,1H) 6.58-6.34(m,2H) 5.77-5.66(m,1H) 5.35-5.20(m,2H) 4.54(t,*J* = 10.1Hz,0.7H) 4.35-4.25(m,1.6H) 3.88(t,*J* = 8.8Hz,0.7H) 2.90-2.78(m,1H) 2.65-2.56(m,0.3H) 2.49-2.40(m,0.7H) 1.63(d,*J* = 7.0Hz,3H) 1.56-1.45(m,4H) 1.03-0.91(m,2H) 0.84-0.69(m,2H)
ESI-MS m/z 483 (MH+)

Example 12

7-((3R,5S)-1-acryloyl-5-methylpyrrolidin-3-yl)-4-amino-6-(3,3-dimethylbut-1-yn-1-yl)-N-((R)-1-phenylethyl)-7H-pyrrolo[2,3-d]pyrimidine-5-carboxamide

The title compound was obtained in the same manner as that of Example 11, with the exception that 3,3-dimethyl-1-butyne was used instead of cyclopropylacetylene in Example 11(3).
$^1$HNMR ($CDCl_3$)δ: 8.22(d,*J* = 5.9Hz,1H) 7.75(d,*J* = 7.7Hz,1H) 7.38(dt,*J* = 15.5,7.1Hz,4H) 7.31-7.25(m,1H) 6.57-6.34(m,2H) 5.77-5.65(m,1H) 5.44-5.35(m,1H) 5.33-5.15(m,1H) 4.63(t,*J* = 10.1Hz,0.7H) 4.40-4.20(m,1.6H) 3.89(t,*J* = 8.8Hz,0.7H) 2.90-2.76(m,1H) 2.65-2.55(m,0.3H) 2.49-2.40(m,0.7H) 1.85(s,1H) 1.64(d,*J*= 7.0Hz,3H) 1.55(d,J= 5.9Hz,3H) 1.26(s,9H)
ESI-MS m/z 499 (MH+)

Example 13

7-((3R,5S)-1-acryloyl-5-methylpyrrolidin-3-yl)-4-amino-6-(3-methoxy-3-methylbut-1-yn-1-yl)-N-((R)-1-phenylethyl)-7H-pyrrolo[2,3-d]pyrimidine-5-carboxamide

The title compound was obtained in the same manner as that of Example 11, with the exception that 3-methoxy-3-methyl-1-butyne was used instead of cyclopropylacetylene in Example 11(3).
$^1$HNMR ($CDCl_3$)δ: 8.17 (s, 1H) 7.61(d,*J* = 7.7Hz,1H) 7.43-7.35(m,4H) 7.30(d,*J* = 7.0Hz,1H) 6.57-6.33(m,2H) 5.81-5.68(m,1H) 5.43-5.33(m,1H) 5.29-5.12(m,1H) 4.59(t,*J* = 10.1Hz,0.7H) 4.38-4.22(m,1.6H) 3.92(t,*J*= 8.6Hz,0.7H) 3.30(s,3H) 2.86-2.72(m,1H) 2.70-2.60(m,1.3H) 2.52-2.44(m,0.7H) 1.64(d,*J* = 7.0Hz,3H) 1.55(t,*J* = 5.5Hz,3H) 1.46(d,*J*= 2.2Hz,6H)
ESI-MS m/z 515 (MH+)

Example 14

7-((3R,5S)-1-acryloyl-5-methylpyrrolidin-3-yl)-4-amino-6-(but-1-yn-1-yl)-N-((R)-1-phenylethyl)-7H-pyrrolo[2,3-d]pyrimidine-5-carboxamide

The title compound was obtained in the same manner as that of Example 11, with the exception that 1-trimethylsilyl-1-butyne and tetra-n-butylammonium fluoride were used instead of cyclopropylacetylene in Example 11(3).
$^1$HNMR ($CDCl_3$)δ: 8.26-8.25(m,1H) 7.79(d,*J* = 7.3Hz,1H) 7.42-7.36(m,4H) 7.32-7.30(m,1H) 6.57-6.37(m,2H) 5.76-5.66(m,1H) 5.33-5.20(m,2H) 4.57(t,*J* = 10.3Hz,0.7H) 4.36-4.22(m,1.6H) 3.88(t,*J* = 8.8Hz,0.7H) 2.92-2.81(m,1H) 2.65-2.57(m,0.3H) 2.48-2.38(m,2.7H) 1.63(d,*J*= 7.0Hz,3H) 1.54-1.51(m,3H) 1.17-1.12(m,3H)
ESI-MS m/z 471 (MH+)

Example 15

7-((3R,5S)-1-acryloyl-5-methylpyrrolidin-3-yl)-4-amino-N-(2-(2-fluorophenyl)propan-2-yl)-6-(3-methylbut-1-yn-1-yl)-7H-pyrrolo[2,3-d]pyrimidine-5-carboxamide

The title compound was obtained in the same manner as that of Example 11, with the exceptions that 2-(2-fluorophenyl)propan-2-amine was used instead of (R)-(+)-1-phenylethylamine in Example 11(1), and that 3-methyl-1-butyne was used instead of cyclopropylacetylene in Example 11(3).
$^1$HNMR ($CDCl_3$)δ: 7.92 (s, 1H) 7.44(t,*J* = 7.9Hz,1H) 7.30-7.23(m,1H) 7.14(t,*J* = 7.5Hz,1H) 7.02(dd,*J* = 12.6,8.2Hz,1H) 6.58-6.35(m,2H) 5.80-5.69(m,1H) 5.33-5.16(m,1H) 4.58(t,*J* = 9.9Hz,0.7H) 4.38-4.23(m,1.6H) 3.91(t,*J* = 8.4Hz,0.7H)

3.03-2.93(m,1H) 2.89-2.75(m,1H) 2.69-2.60(m,0.3H) 2.53-2.43(m,0.7H) 1.88(s,6H) 1.55(d,*J* = 5.1Hz,3H) 1.36(d,*J*= 6.6Hz,6H)
ESI-MS m/z 517 (MH+)

Example 16

Example 16(1)

tert-Butyl (2R,4S)-4-(benzyloxy)-2-((tosyloxy)methyl)pyrrolidine-1-carboxylate

tert-Butyl (2R,4S)-4-(benzyloxy)-2-(hydroxymethyl)pyrrolidine-1-carboxylate (2.0 g) was dissolved in methylene chloride (20 mL), and the obtained solution was then cooled to 0°C. Thereafter, 1,4-diazabicyclo[2.2.2]octane (2.2 g) and tosylate chloride (1.9 g) were added to the reaction solution, and the temperature of the mixture was then increased to room temperature. The mixture was stirred for 4 hours. Thereafter, a saturated sodium hydrogen carbonate aqueous solution was added to the reaction mixture, and the obtained mixture was then extracted with ethyl acetate. The gathered organic layer was washed with saturated saline, was then dried over anhydrous sodium sulfate, and was then concentrated under reduced pressure. The obtained residue was purified by silica gel chromatography (hexane : ethyl acetate) to obtain a product of interest (4.32 g).
$^1$HNMR ($CDCl_3$)δ: 7.78(d,J = 8.1Hz,2H), 7.42-7.29(m,7H), 4.57-4.41(m,2H), 4.39-3.96(m,4H), 3.61-3.20(m,2H),2.46(s,3H), 2.27-2.02(m,2H), 1.48-1.31(m,9H)
ESI-MS m/z 462 ($MH^+$)

Example 16(2)

tert-Butyl (2S,4S)-4-(benzyloxy)-2-ethylpyrrolidine-1-carboxylate

Under a nitrogen atmosphere, copper iodide (2.04 g) was suspended in diethyl ether (12 mL), and the obtained suspension was then cooled to 0°C. Thereafter, 1.04 M methyl lithium in diethyl ether solution (0.36 mL) was added, and the obtained mixture was then stirred at 0°C for 30 minutes. Subsequently, the compound of Example 16(1) (1.98 g) in methylene chloride (4.0 mL) solution was added to the reaction mixture, and the temperature of the obtained mixture was then increased to room temperature. The mixture was stirred for 1 hour. Thereafter, the reaction mixture was cooled to 0°C, and a saturated ammonium chloride aqueous solution was then added to the reaction mixture. The thus obtained mixture was extracted with ethyl acetate. The gathered organic layer was washed with saturated saline, was then dried over anhydrous sodium sulfate, and was then concentrated under reduced pressure. The obtained residue was purified by silica gel chromatography (hexane : ethyl acetate) to obtain a product of interest (707 mg). $^1$HNMR ($CDCl_3$)δ 7.42-7.25(m,5H), 4.66-4.40(m,2H), 4.17-4.03(m,1H), 4.00-3.26(m,3H), 2.24-2.09(m,1H), 1.96-1.71(m,2H), 1.48(s,9H),1.45- 1.31(m,1H), 0.86(t,J = 7.4Hz,3H)
ESI-MS m/z 306 ($MH^+$)

Example 16(3)

tert-Butyl (2S,4S)-2-ethyl-4-hydroxypyrrolidine-1-carboxylate

The compound of Example 16(2) (1.06 g) and a 10% palladium hydroxide carbon catalyst (160 mg) were suspended in ethanol (11 mL) and THF (11 mL), followed by hydrogen substitution, and the resultant was then stirred at room temperature for 20 hours. Thereafter, the reaction mixture was filtrated through Celite, and was then washed with ethanol, and the filtrate was then concentrated under reduced pressure. The obtained residue was purified by silica gel chromatography (hexane : ethyl acetate) to obtain a product of interest (709 mg).
$^1$HNMR ($CDCl_3$)δ 4.46-4.36(m,1H), 4.02-3.81(m,1H), 3.71-3.35(m,2H), 2.15-1.99(m,1H), 1.95-1.72(m,2H), 1.49(s,9H), 1.46-1.35(m,1H), 0.86(t,J = 7.5Hz,3H) ESI-MS m/z 216 ($MH^+$)

Example 16(4)

tert-Butyl (2S,4R)-4-(4-chloro-5-iodo-7H-pyrrolo[2,3-d]pyrimidin-7-yl)-2-ethylpyrrolidine-1-carboxylate

The compound of Example 16(3) (709 mg) and 4-chloro-5-iodo-7H-pyrrolo[2,3-d]pyrimidine (1.11 g) were dissolved in THF (7.1 mL), and the obtained solution was then cooled to 0°C. Thereafter, triphenylphosphine (1.3 g) and diisopropyl azodicarboxylate (1.00 mL) were added, and the temperature of the obtained mixture was then increased

to room temperature, followed by stirring the mixture for 1 hour. Thereafter, the reaction mixture was concentrated under reduced pressure, and the obtained residue was then purified by silica gel chromatography (hexane : ethyl acetate) to obtain the corresponding coupling body. The obtained compound was used in the subsequent reaction without being further purified. Into a pressure resistant tube, the obtained coupling body, THF (5.4 mL), and ammonia water (5.4 mL) were added, and the obtained mixture was then stirred at 100°C for 14 hours. Thereafter, the reaction mixture was cooled to room temperature, and was then poured into water (12.8 mL), and the mixed solution was then extracted with ethyl acetate. The gathered organic layer was washed with saturated saline, was then dried over anhydrous sodium sulfate, and was then concentrated under reduced pressure. The obtained residue was purified by silica gel chromatography (hexane : acetone) to obtain a product of interest (797 mg).
$^1$HNMR ($CDCl_3$)δ 8.29(s,1H), 7.14(s,1H), 5.67(br s,2H), 5.32-5.09(m,1H), 4.24-4.08(m,1H), 3.95-3.79(m,1H), 3.46(dd,J = 9.3,11.0Hz,1H), 2.70-2.55(m,1H), 2.06-1.95(m,1H), 1.59-1.51(m,2H), 1.49(s,9H), 0.91(t,J = 7.5Hz,3H)
ESI-MS m/z 458 ($MH^+$)

Example 16(5)

tert-Butyl (2S,4R)-4-(4-amino-6-bromo-5-(((R)-1-phenylethyl)carbamoyl)-7H-pyrrolo [2,3-d]pyrimidin-7-yl)-2-ethylpyrrolidine-1-carboxylate

The compound of Example 16(4) (797 mg), dichlorobis(triphenylphosphine)palladium (25 mg), and (R)-(+)-1-phenylethylamine (0.55 mL) were suspended in DMF (8.0 mL), followed by carbon monoxide substitution, and the resultant was then stirred at 80°C for 2 hours. Thereafter, the reaction mixture was cooled to room temperature, water was then added thereto, and the obtained mixture was then extracted with ethyl acetate. The gathered organic layer was washed with saturated saline, was then dried over anhydrous sodium sulfate, and was then concentrated under reduced pressure. The obtained residue was purified by silica gel chromatography (hexane : acetone) to obtain the corresponding amide form. The obtained compound was used in the subsequent reaction without being further purified. The obtained amide form was dissolved in acetonitrile (8.2 mL), and the obtained solution was then cooled to -10°C. Thereafter, N-bromosuccinimide (457 mg) in acetonitrile (8.2 mL) solution was slowly added dropwise to the solution, and the reaction mixture was then stirred for 30 minutes. Thereafter, to the reaction mixture, a sodium sulfite aqueous solution and a sodium hydrogen carbonate aqueous solution were added, and the obtained mixture was then extracted with ethyl acetate. The gathered organic layer was washed with saturated saline, was then dried over anhydrous sodium sulfate, and was then concentrated under reduced pressure. The obtained residue was purified by silica gel chromatography (hexane : acetone) to obtain a product of interest (650 mg).
$^1$HNMR ($CDCl_3$)δ 8.23(s,1H), 7.49-7.29(m,5H), 6.98(d,J = 7.4Hz,1H), 5.41-5.28(m,1H), 5.24-5.04(m,1H), 4.38-4.22(m,1H), 4.07-3.68(m,1H), 3.19-2.83(m,1H), 2.43-2.29(m,1H), 2.25-1.67(m,3H), 1.66(d,J = 6.9Hz,3H), 1.51(s,9H), 0.98(t,J = 7.4Hz,3H) ESI-MS m/z 557,559 ($MH^+$)

Example 16(6)

7-((3R,5S)-1-acryloyl-5-ethylpyrrolidin-3-yl)-4-amino-6-bromo-N-((R)-1-phenylethyl)-7H-pyrrolo[2,3-d]pyrimidine-5-carboxamide

To the compound of Example 16(5) (650 mg), acetonitrile (9.7 mL) was added, and the obtained mixture was then cooled to 0°C. Thereafter, sodium iodide (1.05 g) and trimethylsilyl chloride (0.89 mL) were added, and the obtained mixture was then stirred at 0°C for 1 hour. Thereafter, to the reaction mixture, ethanol (9.7 mL), isopropylethylamine (2.0 mL), and acrylic acid anhydride (0.16 mL) were successively added, and the obtained mixture was then stirred at 0°C for 30 minutes. Thereafter, to the reaction mixture, ammonia water and water were added, and the obtained mixture was then extracted with ethyl acetate. The gathered organic layer was washed with saturated saline, was then dried over anhydrous sodium sulfate, and was then concentrated under reduced pressure. The obtained residue was purified by silica gel chromatography (hexane : acetone) to obtain a product of interest (256 mg).
$^1$HNMR ($CDCl_3$)δ 8.27-8.16(m,1H), 7.47-7.29(m,5H), 6.98(d,J = 7.3Hz,1H), 6.61-6.29(m,2H), 5.84-5.63(m,1H), 5.43-5.26(m,1H), 5.22-5.01(m,1H), 4.80-3.82(m,3H), 3.23-2.92(m,1H), 2.58-2.30(m,1H), 2.22-1.79(m,2H),1.66(d,J = 7.0Hz,3H),1.07-0.96(m,3H)
ESI-MS m/z 511,513 ($MH^+$)

Example 16(7)

7-((3R,5S)-1-acryloyl-5-ethylpyrrolidin-3-yl)-4-amino-N-((R)-1-phenylethyl)-6-(prop-1-yn-1-yl)-7H-pyrrolo[2,3-d]pyrimidine-5-carboxamide

1.0 M Propyne in DMF solution (0.70 mL) was added to the compound of Example 16(6) (120 mg), acetonitrile (1.2 mL), triethylamine (0.10 mL), $PdCl_2(PPh_3)_2$ (8.2 mg), and copper(I) iodide (0.4 mg), followed by nitrogen substitution, and the mixture was then stirred at 60°C for 2 hours. Thereafter, the reaction mixture was cooled to room temperature, and ethyl acetate and a saturated ammonium chloride aqueous solution were added to the mixture. The thus obtained mixture was extracted with ethyl acetate, and the gathered organic layer was washed with water, and then with saturated saline. The resultant was dried over anhydrous sodium sulfate, and was then concentrated under reduced pressure. The obtained residue was purified by silica gel chromatography (ethyl acetate : methanol) to obtain a product of interest (102 mg).
$^1$HNMR ($CDCl_3$)δ: 8.26(s,1H), 7.79(br d,J = 7.0Hz,1H), 7.46-7.30(m,5H), 6.58-6.31(m,2H), 5.80-5.65(m,1H), 5.33-5.15(m,2H), 4.59-3.85(m,3H), 3.03-2.33(m,2H), 2.25-1.70(m,5H), 1.65(d,J = 6.8Hz,6H), 1.09-0.91(m,3H)
ESI-MS m/z 471 ($MH^+$)

Example 17

7-((3R,5 S)-1-acryloyl-5-ethylpyrrolidin-3-yl)-4-amino-6-(cyclopropylethynyl)-N-((R)-1-phenylethyl)-7H-pyrrolo[2,3-d]pyrimidine-5-carboxamide

The title compound was obtained in the same manner as that of Example 16, with the exception that cyclopropylacetylene was used instead of 1.0 M propyne in DMF solution in Example 16(7).
$^1$HNMR ($CDCl_3$)6: 8.31-8.16(m,1H), 7.84(d,J = 7.4Hz,1H), 7.46-7.30(m,5H), 6.64-6.32(m,2H), 5.82-5.67(m,1H), 5.39-5.17(m,2H), 4.67-3.81(m,3H), 3.02-2.80(m,1H), 2.62-1.71(m,3H), 1.65(d,J = 6.9Hz,3H), 1.58-1.47(m,1H), 1.06-0.92(m,5H), 0.85-0.70(m,2H)
ESI-MS m/z 497 ($MH^+$)

Example 18

7-((3R,5R)-1-acryloyl-5-(methoxymethyl)pyrrolidin-3-yl)-4-amino-6-(cyclopropylethynyl)-N-((R)-1-phenylethyl)-7H-pyrrolo[2,3-d]pyrimidine-5-carboxamide

The title compound was obtained in the same manner as that of Example 16, with the exceptions that tert-butyl (2R,4S)-4-hydroxy-2-(methoxymethyl)pyrrolidine-1 - carboxylate was used instead of the compound of Example 16(3) in Example 16(4), and that cyclopropylacetylene was used instead of 1.0 M propyne in DMF solution in Example 16(7).
$^1$HNMR ($CDCl_3$)δ: 8.29-8.22(m,1H), 7.86-7.80(m,1H), 7.36-7.44(m,4H), 7.34-7.28(m,1H), 6.48-6.37(m,2H), 5.78-5.69(m,1H), 5.29-5.15(m,2H), 4.55-4.30(m,2H), 3.96-3.65(m,3H), 3.42(s,3H), 3.18-3.06(m,0.3H), 2.90-2.80(m,0.3H), 2.64-2.58(m,0.3H), 2.47-2.35(m,0.7H),1.64(d,3H,J = 6.9Hz), 1.58-1.47(m,1H), 1.04-0.94(m,2H), 0.87-0.69(m,2H)
ESI-MS m/z 513 ($MH^+$)

Example 19

7-((3R,5R)-1-acryloyl-5-(ethoxymethyl)pyrrolidin-3-yl)-4-amino-6-(cyclopropylethynyl)-N-((R)-1-phenylethyl)-7H-pyrrolo[2,3-d]pyrimidine-5-carboxamide

The title compound was obtained in the same manner as that of Example 16, with the exceptions that tert-butyl (2R,4S)-2-(ethoxymethyl)-4-hydroxypyrrolidine-1 - carboxamide was used instead of the compound of Example 16(3) in Example 16(4), and that cyclopropylacetylene was used instead of 1.0 M propyne in DMF solution in Example 16(7).
$^1$HNMR ($CDCl_3$)δ: 8.28-8.18(m,1H),7.84(br d,J = 7.0Hz,1H), 7.47-7.29(m,5H), 6.82-6.35(m,2H), 5.79-5.68(m,1H), 5.40-5.14(m,2H), 4.63-3.53(m,7H), 3.20-2.79(m,1H), 2.69-2.40(m,1H), 1.67-1.63(m,3H), 1.59-1.47(m,1H),1.22(t,J = 7.0Hz,3H), 1.05-0.92(m,2H), 0.87-0.72(m,2H)
ESI-MS m/z 527 ($MH^+$)

Comparative Example 1

4-Amino-N-(4-(methoxymethyl)phenyl)-7-(1 -methylcyclopropyl)-6-(prop-1 -yn-1 -yl)-7H-pyrrolo[2,3-d]pyrimidine-5-carboxamide

The title compound was obtained by the method described in Example 95 of International Publication No. WO 2017/146116.
ESI-MS m/z 390 (MH+)

Comparative Example 2

1-((3R,5S)-1-acryloyl-5-methylpyrrolidin-3-yl)-4-amino-N-(4-(2-(dimethylamino)-2-oxoethyl)-2,3 -dimethylphenyl)-1H-pyrazolo [3,4-d]pyrimidine-3 -carboxamide

The title compound was obtained by the method described in Example 79 of International Publication No. WO 2017/038838.
ESI-MS m/z 505 (MH+)

Comparative Example 3

7-((3R,5S)-1-acryloyl-5-methylpyrrolidin-3-yl)-4-amino-N-(cyclohexylmethyl)-6-(prop-1-yn-1-yl)-7H-pyrrolo[2,3-d]pyrimidine-5-carboxamide

The title compound was obtained in the same manner as that of Example 1, with the exception that cyclohexylmethanamine was used instead of (R)-1-(3,5-difluorophenyl)ethan-1-amine in Example 1(5).
$^1$HNMR (DMSO-d6)δ:8.68-8.31(m,1H) 8.20-8.10(m,1H) 8.09-7.97(m,1H) 7.59-7.20(m,1H) 6.74-6.49(m,1H) 6.25-6.09(m,1H) 5.78-5.60(m,1H) 5.40-5.20(m,1H) 4.44-4.29(m,1H) 4.23-3.92(m,2H) 3.25-3.12(m,2H) 2.76-2.40(m,2H) 2.25(s,3H) 1.81-1.45(m,5H) 1.43-1.34(m,3H) 1.30-0.90(m,6H)
ESI-MS m/z 449 (MH+)

Comparative Example 4

7-((3R,5S)-1-acryloyl-5-methylpyrrolidin-3-yl)-4-amino-N-(2-methylbenzyl)-6-(prop-1-yn-1-yl)-7H-pyrrolo[2,3-d]pyrimidine-5-carboxamide

The title compound was obtained in the same manner as that of Example 1, with the exception that o-tolylmethanamine was used instead of (R)-1-(3,5-difluorophenyl)ethan-1-amine in Example 1(5).
$^1$HNMR (DMSO-d6)δ:8.37-8.27(m,1H) 8.19-8.09(m,1H) 7.39-7.30(m,1H) 7.26-7.11(m,4H) 6.68-6.48(m,1H) 6.24-6.07(m,1H) 5.80-5.60(m,1H) 5.36-5.17(m,1H) 4.52(d,*J* = 5.7Hz,2H) 4.42-4.28(m,1H) 4.22-3.92(m,2H) 2.73-2.42(m,2H) 2.33(s,3H) 2.02(s,3H) 1.43-1.32(m,3H)
ESI-MS m/z 457 (MH+)

Comparative Example 5

7-((3R,5S)-1-acryloyl-5-methylpyrrolidin-3-yl)-4-amino-N-methyl-N-((R)-1-phenylethyl)-6-(prop-1-yn-1-yl)-7H-pyrrolo[2,3-d]pyrimidine-5-carboxamide

The title compound was obtained in the same manner as that of Example 1, with the exception that (R)-N-methyl-1-phenylethan-1-amine was used instead of (R)-1-(3,5-difluorophenyl)ethan-1-amine in Example 1(5).
$^1$HNMR ($CDCl_3$)δ: 8.23(d,*J* = 5.9Hz,1H) 7.50-7.28(m,4H) 7.09-6.88(m,1H) 6.57-6.34(m,2H) 5.79-5.64(m,1H) 5.22(t,*J* = 9.3Hz,1H) 4.48(t,*J* = 9.7Hz,0.6H) 4.39-4.20(m,1.9H) 3.90(t,*J* = 8.6Hz,0.5H) 2.85(s,4H) 2.66-2.63(m,0.4H) 2.51-2.44(m,0.6H) 2.07(s,2H) 1.66(d,*J* = 4.8Hz,3H) 1.52(d,*J* = 5.9Hz,3H)
ESI-MS m/z 471 (MH+)

Comparative Example 6(1)

tert-Butyl (2S,4R)-4-(4-amino-5-(((R)-1-phenylethyl)carbamoyl)-6-(prop-1-yn-1-yl)-7H -pyrrolo[2,3-d]pyrimidin-7-yl)-2-methylpyrrolidine-1-carboxylate

1.0 M Propyne in DMF solution(2.1 mL) was added to the compound of Example 11(1) (230 mg), acetonitrile (4.6 mL), triethylamine (0.29 mL), $PdCl_2(PPh_3)_2$ (5.9 mg), and copper(I) iodide (1.6 mg), followed by nitrogen substitution, and the obtained mixture was then stirred at 70°C for 1 hour. Thereafter, the reaction mixture was cooled to room temperature, and ethyl acetate and a saturated sodium hydrogen carbonate aqueous solution were then added to the mixture. The thus obtained mixture was extracted with ethyl acetate, and the gathered organic layer was washed with water and then with saturated saline. The resultant was dried over anhydrous sodium sulfate, and was then concentrated under reduced pressure. The obtained residue was purified by silica gel chromatography (hexane : ethyl acetate) to obtain a product of interest (193 mg).
$^1$HNMR ($CDCl_3$)δ: 8.23 (s, 1H) 7.79(d,*J* = 6.8Hz,1H)7.46-7.27(m,5H) 5.40-5.17(m,2H) 4.28-3.64(m,3H) 2.85-2.68(m,1H) 2.46-2.36(m,1H) 2.15-1.97(m,3H) 1.62(d,*J* = 6.8Hz,3H) 1.56-1.32(m,12H)
ESI-MS m/z 503 (MH+)

Comparative Example 6(2)

4-Amino-7-((3R,5 S)-5-methylpyrrolidin-3-yl)-N-((R)-1-phenylethyl)-6-(prop-1 -yn-1-yl)-7H-pyrrolo[2,3-d]pyrimidine-5-carboxamide hydrochloride

To the compound of Comparative Example 6(1) (530 mg), 4 M hydrochloric acid in 1,4-dioxane solution (5 mL) was added, and the obtained mixture was then stirred at room temperature for 2 hours. Thereafter, the reaction mixture was concentrated under reduced pressure to obtain a product of interest (420 mg).
ESI-MS m/z 403 (MH+)

Comparative Example 6(3)

4-Amino-7-((3R,5S)-1-((E)-but-2-enoyl)-5-methylpyrrolidin-3-yl)-N-((R)-1-phenylethyl)-6-(prop-1-yn-1-yl)-7H-pyrrolo[2,3-d]pyrimidine-5-carboxamide

To the compound of Comparative Example 6(2) (18 mg), acetonitrile (0.5 mL) was added, and the obtained mixture was then cooled to 0°C. Thereafter, acryloyl chloride (0.004 mL) and diisopropylethylamine (0.036 mL) were added to the reaction mixture, and the thus obtained mixture was then stirred at 0°C for 1 hour. Thereafter, the reaction mixture was concentrated under reduced pressure, and was then subjected to reverse phase preparative HPLC (water : acetonitrile (0.1% formic acid)) to obtain a product of interest (8.7 mg).
$^1$HNMR ($CDCl_3$)δ: 8.31 (s, 1H) 8.14(d,*J* = 6.2Hz,1H)7.77(d,*J* = 7.0Hz,1H) 7.39-7.36(m,4H) 7.33-7.31(m,1H) 7.03-6.90(m,1H) 6.06(dd,*J*= 14.3Hz,1H) 5.28-5.17(m,2H) 4.48(t,*J* = 10.1Hz,1H) 4.35-4.20(m,2H) 3.88(t,8.8Hz,1H) 2.84-2.76(m,1H) 2.64-2.40(m,1H) 2.05(d,*J* = 10.6Hz,3H) 1.92(d,*J* = 6.6Hz,1H) 1.85(d,*J* = 7.0Hz,2H) 1.62(d,*J* = 7.0Hz,3H) 1.55(dd,*J* = 9.0,5.7Hz,3H)
ESI-MS m/z 471 (MH+)
The compounds synthesized in the above-described Examples and Comparative Examples are shown below.

[Table 1]

| Examp le No. | Structural Formula | Examp le No. | Structural Formula |
|---|---|---|---|
| 1 | 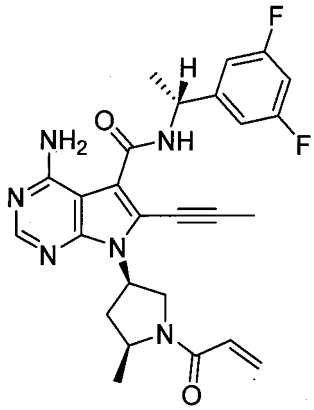 | 2 | 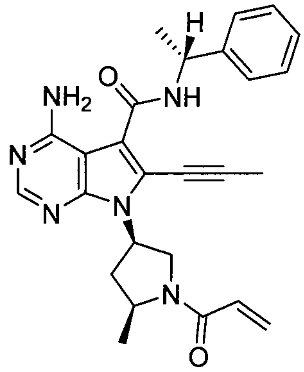 |
| 3 | 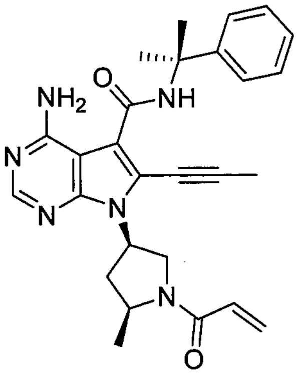 | 4 | 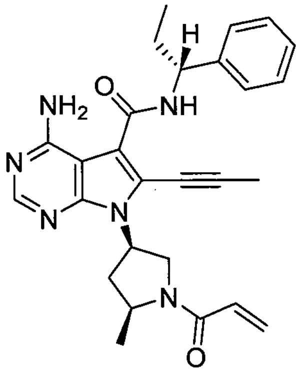 |
| 5 | 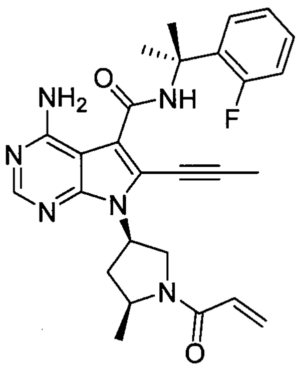 | 6 | 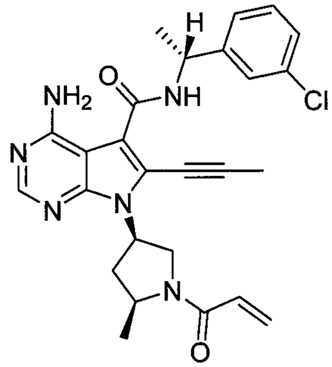 |
| 7 | 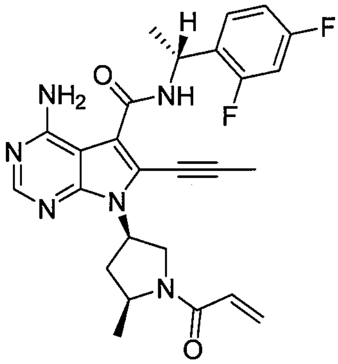 | 8 | 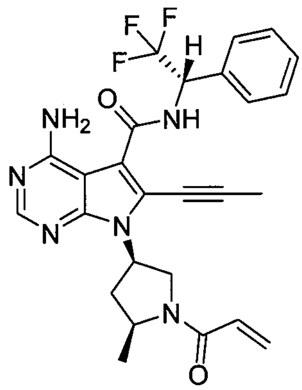 |

(continued)

| Examp le No. | Structural Formula | Examp le No. | Structural Formula |
|---|---|---|---|
| 9 | 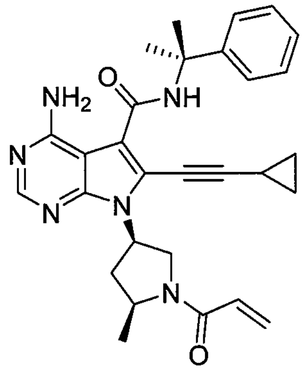 | 10 | 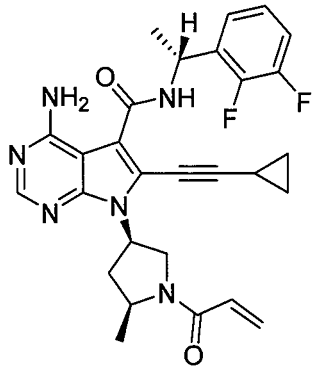 |
| 11 | 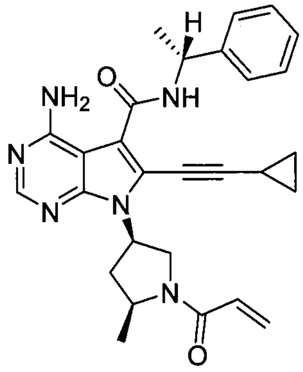 | 12 | 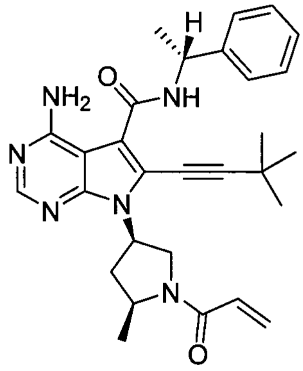 |
| 13 | 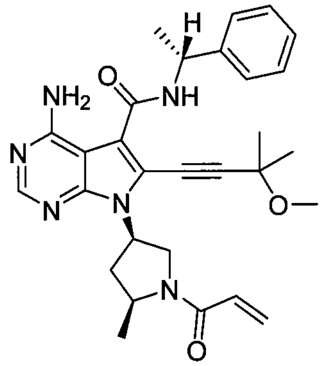 | 14 | 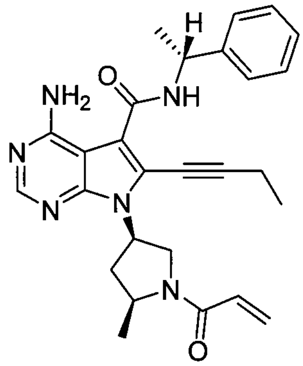 |
| 15 | 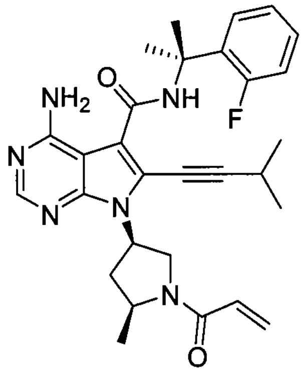 | 16 | 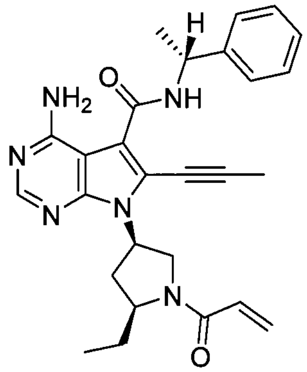 |

(continued)

| Examp le No. | Structural Formula | Examp le No. | Structural Formula |
|---|---|---|---|
| 17 | 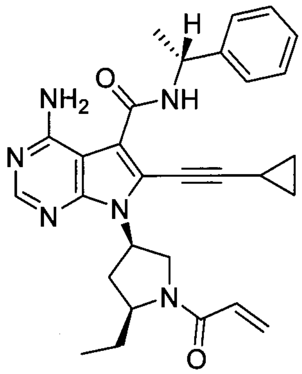 | 18 | 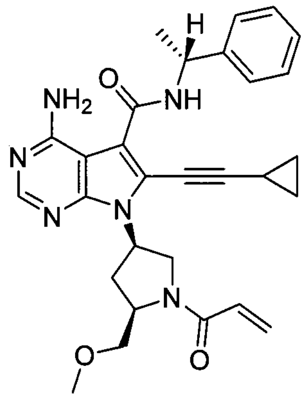 |
| 19 | 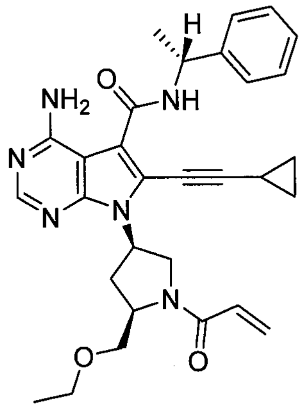 | | |

[Table 2]

| Comp . Ex. No. | Structural Formula | Comp . Ex. No. | Structural Formula |
|---|---|---|---|
| 1 | 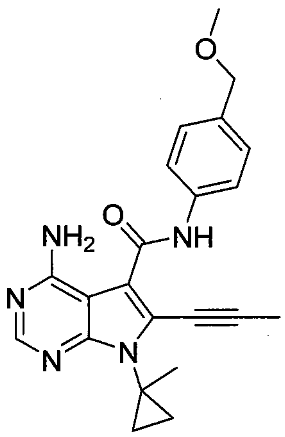 | 2 | 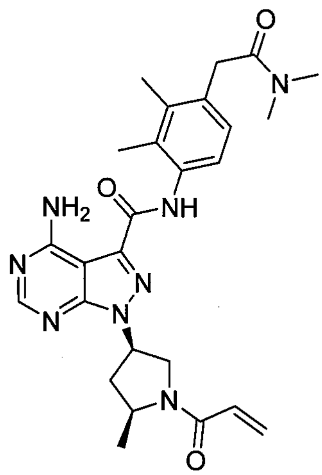 |

(continued)

| Comp . Ex. No. | Structural Formula | Comp . Ex. No. | Structural Formula |
|---|---|---|---|
| 3 | 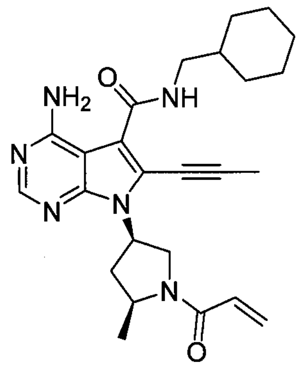 | 4 | 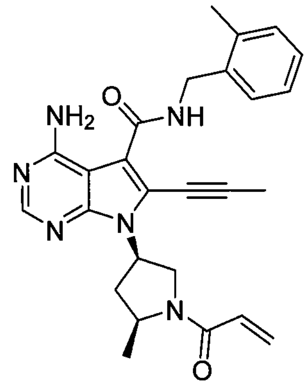 |
| 5 | 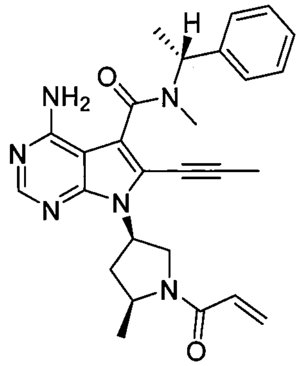 | 6 | 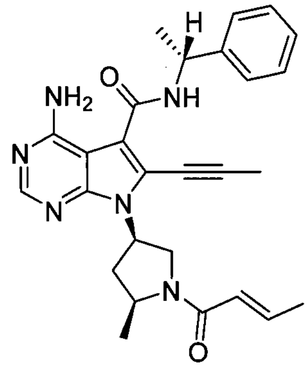 |

Test Example 1 Measurement of inhibitory effect *(in vitro)* on HER2 phosphorylation activity

In order to determine conditions for a method of measuring the *in vitro* inhibitory activity of a compound against HER2 phosphorylation activity, based on the report regarding a HER2 kinase reaction using, as a substrate, a peptide having the same sequence (5-FAM-EEPLYWSFPAKKK-$CONH_2$) as that of ProfilerPro Peptide 22 of PerkinElmer (Xie H et al., PLoS One.2011; 6(7): e21487), ProfilerPro Peptide 22 was used as a substrate. A purified recombinant human HER2 protein used in the present test was purchased from Carna Biosciences, Inc. Upon the measurement of the inhibitory activity of the pyrimidine compound, first, the compound of the present invention was diluted stepwise with dimethyl sulfoxide (DMSO). Subsequently, the HER2 protein, the substrate peptide (final concentration: 1 μM), manganese chloride (final concentration: 10 mM), ATP (final concentration: 5 μM), and the pyrimidine compound of the present invention in DMSO solution (final concentration of DMSO: 5%) were added to a buffer for the kinase reaction (13.5 mM Tris (pH 7.5), 2 mM dithiothreitol, and 0.009% Tween 20), and the obtained mixture was then incubated at 25°C for 30 minutes, so that the kinase reaction was carried out. To the reaction solution, EDTA was added to a final concentration of 30 mM, so as to terminate the reaction. Finally, using LabChip (registered trademark) EZ Reader II (PerkinElmer), an unphosphorylated substrate peptide (S) and a phosphorylated peptide (P) were separated and detected according to microchannel capillary electrophoresis. From the peak heights of S and P, the amount of the phosphorylation reaction was obtained, and the concentration of the compound capable of inhibiting the phosphorylation reaction by 50% was defined as an IC50 value (nM). The results are shown in Table 3.

Test Example 2 Measurement of inhibitory action *(in vitro)* against HER2 exon 20 insertion mutant (HER2ex20insYVMA) phosphorylation activity

In order to determine conditions for a method of measuring the *in vitro* inhibitory activity of a compound against HER2 exon 20 insertion mutant phosphorylation activity, as in the case of HER2, ProfilerPro Peptide 22 was used as a substrate. A purified recombinant human HER2 exon 20 insertion mutant (A775 _G776insYVMA) protein was purchased from SignalChem. Upon the measurement of the inhibitory activity of the compound, first, the pyrimidine compound of the present invention was diluted stepwise with dimethyl sulfoxide (DMSO). Subsequently, the HER2 exon 20 insertion mutant protein and the pyrimidine compound of the present invention in DMSO solution (final concentration of DMSO:

5%) were added into a buffer for the kinase reaction (13.5 mM Tris (pH 7.5), 2 mM dithiothreitol, and 0.009% Tween 20), and the obtained mixture was then pre-incubated at 25°C for 30 minutes. Thereafter, the substrate peptide (final concentration: 1 μM), manganese chloride (final concentration: 25 mM), magnesium chloride (final concentration: 20 mM), and ATP (final concentration: 200 μM) were added into the reaction mixture, and the thus obtained mixture was then incubated at 25°C for 220 minutes, so that the kinase reaction was carried out. To the reaction solution, EDTA was added to a final concentration of 30 mM, so as to terminate the reaction. Finally, using LabChip (registered trademark) EZ Reader II (PerkinElmer), an unphosphorylated substrate peptide (S) and a phosphorylated peptide (P) were separated and detected according to microchannel capillary electrophoresis. From the peak heights of S and P, the amount of the phosphorylation reaction was obtained, and the concentration of the compound capable of inhibiting the phosphorylation reaction by 50% was defined as an IC50 value (nM). The results are shown in Table 3.

[Table 3]

| Example No. | HER2 inhibitory activity IC50 value (nM) | HER2ex20insYVMA inhibitory activity IC50 value (nM) |
|---|---|---|
| 1 | 2.7 | 0.34 |
| 2 | 2.5 | < 0.30 |
| 3 | 5.8 | < 0.30 |
| 4 | 3.9 | 0.37 |
| 5 | 7.7 | 0.38 |
| 6 | 2.8 | < 0.30 |
| 7 | 4.9 | 0.39 |
| 8 | 10 | < 0.30 |
| 9 | 5.6 | 0.32 |
| 10 | 2.2 | < 0.30 |
| 11 | 3.2 | < 0.30 |
| 12 | 3.4 | 0.39 |
| 13 | 5.2 | 0.44 |
| 14 | 2.2 | < 0.30 |
| 15 | 4.6 | 0.42 |
| 16 | 3.3 | 0.44 |
| 17 | 2.9 | 0.54 |
| 18 | 2.3 | < 0.30 |
| 19 | 4.4 | 1.1 |
| Comp. Ex. 1 | > 10000 | > 10000 |
| Comp. Ex. 2 | 19 | 4.4 |
| Comp. Ex. 3 | 630 | 380 |
| Comp. Ex. 4 | 54 | 11 |
| Comp. Ex. 5 | 130 | 14 |
| Comp. Ex. 6 | 390 | > 10000 |

From the above results, it was found that the pyrimidine compound of the present invention has excellent inhibitory activity against phosphorylation of HER2 and against phosphorylation of HER2 exon 20 insertion mutant.

Test Example 3 Measurement of growth inhibitory activity against HER2 expressing cell line

SK-BR-3 cells as a HER2 overexpressing human breast cancer cell line were suspended in a McCoy's 5a

medium (manufactured by Life Technologies) supplemented with 10% fetal bovine serum. The cell suspension was seeded in each well of a 384-well flat-bottom microplate, and was then cultured in a 5% carbon dioxide gas-containing culture vessel at 37°C for 1 day. Thereafter, the compound of the present invention was dissolved in DMSO, and the pyrimidine compound was diluted to 500 times the final concentration in DMSO. The compound in the DMSO solution was diluted with DMSO solution or the medium used in the suspension of the cells, and the obtained solution was then added to each well of the culture plate so that the final concentration of DMSO was 0.2%. The obtained mixture was further cultured in the 5% carbon dioxide gas-containing culture vessel at 37°C for 3 days. After completion of the culture for 3 days in the presence of the compound, the cells were counted using CellTiter-Glo 2.0

(manufactured by Promega), and the growth inhibition percentage was then calculated according to the following equation. The concentration of the compound, in which the growth of the cells can be inhibited by 50%, was defined as IC50 (nM).

$$\text{Growth inhibitory percentage (\%)} = (C-T) / (C) \times 100$$

T: Emission intensity from the well to which the test compound was added
C: Emission intensity from the well to which the test compound was not added

The results are shown in the following Table 4.

Test Example 4 Measurement of growth inhibitory activity against HER2 exon 20 insertion mutant expressing cell line

Growth inhibitory activity against the HER2 exon 20 insertion mutant was measured using Ba/F3 cells that were a mouse B lymphocyte precursor cell line, into which a human HER2 exon 20 insertion mutant gene had been introduced. The Ba/F3 cells were maintained in an RPMI-1640 medium (Thermo Fisher Scientific) supplemented with 10% fetal bovine serum (FBS), 100 U/mL penicillin, 100 μg/mL streptomycin (Thermo Fisher Scientific) and 1 ng/mL mouse interleukin-3 (mIL-3) (CST). Thereafter, a pCDNA3.1-hyg(+) vector, into which a human HER2 exon 20 insertion mutant gene (A775_G776insYVMA (HER2ex20insYVMA)), Internal Ribosome Binding Sequence (IRES), and a Kusabira orange gene had been incorporated, was introduced into the Ba/F3 cells according to an electroporation method using Amaxa (registered trademark) Cell Line Nucleofector (registered trademark) Kit V. The Ba/F3 cells expressing the HER2 exon 20 insertion mutant (Ba/F3-HER2insYVMA), which were selected with hygromycin B (Nacalai Tesque), exhibited mIL-3-independent growth.

Upon evaluation of cell growth inhibitory activity, the Ba/F3-HER2insYVMA cells were suspended in an RPMI-1640 medium supplemented with 10% FBS, 100 U/mL penicillin, and 100 μg/mL streptomycin. The cell suspension was seeded in each well of a 96-well flat-bottom microplate, and was then cultured in a 5% carbon dioxide gas-containing culture vessel at 37°C for 1 day. The pyrimidine compound of the present invention was dissolved in DMSO, and was then diluted with DMSO or the medium used in the suspension of the cells. The obtained solution was then added to each well of the culture plate, so that the final concentration of DMSO became 0.2%. The obtained mixture was further cultured in the 5% carbon dioxide gas-containing culture vessel at 37°C for 3 days. After completion of the culture for 3 days in the presence of the compound, the cells were counted using CellTiter-Glo 2.0 (manufactured by Promega), and the growth inhibition percentage was then calculated according to the following equation. The concentration of the compound, in which the growth of the cells can be inhibited by 50%, was defined as IC50 (nM).

$$\text{Growth inhibitory percentage (\%)} = (C-T) / (C) \times 100$$

T: Emission intensity from the well to which the test compound was added
C: Emission intensity from the well to which the test compound was not added

The results are shown in the following Table 4.

[Table 4]

| Example No. | SK-BR-3 cell growth inhibitory activity IC50 value (nM) | HER2ex20insYVMA cell growth inhibitory activity IC50 value (nM) |
|---|---|---|
| 1 | 9.3 | 17 |
| 2 | 2.8 | 12 |

(continued)

| Example No. | SK-BR-3 cell growth inhibitory activity IC50 value (nM) | HER2ex20insYVMA cell growth inhibitory activity IC50 value (nM) |
|---|---|---|
| 3 | 4.5 | 25 |
| 4 | 5.6 | 20 |
| 5 | 4.0 | 14 |
| 6 | 10 | 28 |
| 7 | 12 | 40 |
| 8 | 14 | 26 |
| 9 | 4.2 | 24 |
| 10 | 13 | 29 |
| 11 | 6.6 | 29 |
| 12 | 17 | 43 |
| 13 | 14 | 23 |
| 14 | 8.1 | 27 |
| 15 | 7.0 | 40 |
| 16 | 1.4 | 9.7 |
| 17 | 4.0 | 20 |
| 18 | 3.4 | 14 |
| 19 | 17 | 50 |
| Comp. Ex. 1 | > 10000 | > 10000 |
| Comp. Ex. 2 | 25 | 1900 |
| Comp. Ex. 3 | 4300 | 3400 |
| Comp. Ex. 4 | 340 | 900 |
| Comp. Ex. 5 | 400 | 1300 |
| Comp. Ex. 6 | 3000 | 4100 |

From the above results, it was found that the pyrimidine compound group of the present invention has excellent cell growth inhibitory activity even against the HER2 expressing cell line (SK-BR-3) and also, against the HER2 exon 20 insertion mutant expressing cell line (Ba/F3-HER2insYVMA).

Test Example 5 Measurement of growth inhibitory activity against HER2 expressing cell line (NCI-N87)

NCI-N87 cells as a HER2 overexpressing human stomach cancer cell line (American Type Culture Collection, Cat No. ATCC (registered trademark) CRL-5822) were suspended in an RPMI1640 medium (FUJIFILM Wako Pure Chemical Cooperation) supplemented with 10% fetal bovine serum. Subsequently, the cell suspension was seeded in each well of a 96-well flat-bottom microplate, and was then cultured in a 5% carbon dioxide gas-containing culture vessel at 37°C for 1 day. Thereafter, the compound of the present invention was dissolved in DMSO, and the compound was diluted to 1000 times the final concentration in DMSO. The compound in the DMSO solution was diluted with the medium used in the suspension of the cells, and the obtained solution was then added to each well of the culture plate, so that the final concentration of DMSO became 0.1%. Regarding a control well, DMSO was diluted with the medium used in the suspension of the cells, and the obtained solution was then added to each well of the culture plate, so that the final concentration of DMSO became 0.1%. After addition of a drug solution, the obtained mixture was further cultured in the 5% carbon dioxide gas-containing culture vessel at 37°C for 3 days. After completion of the culture for 3 days in the presence of the compound, the cells were counted using CellTiter-Glo 2.0 (manufactured by Promega) in accordance with the protocols recommended by Promega. The growth inhibition percentage was calculated according to the following

equation. The concentration of the compound, in which the growth of the cells can be inhibited by 50%, was defined as IC50 (nM).

$$\text{Growth inhibitory percentage (\%)} = (C\text{-}T) / (C) \times 100$$

T: Emission intensity from the well to which the test compound was added
C: Emission intensity from the well to which the test compound was not added

The results are shown in the following Table 5.

[Table 5]

| Example No. | NCI-N87 cell growth inhibitory activity IC50 value (nM) |
|---|---|
| 1 | 10.7 |
| 2 | 3.0 |
| 3 | 4.7 |
| 4 | 5.6 |
| 5 | 7.0 |
| 6 | 8.5 |
| 7 | 11.1 |
| 8 | 9.7 |
| 9 | 6.2 |
| 10 | 10.5 |
| 11 | 9.9 |
| 12 | 15.0 |
| 13 | 11.7 |
| 14 | 9.1 |
| 15 | 11.6 |
| 16 | 0.9 |
| 17 | 2.2 |
| 18 | 2.6 |
| 19 | 7.9 |

From the above results, it was found that the pyrimidine compound of the present invention has excellent cell growth inhibitory activity even against the HER2 overexpressing cell line (NCI-N87).

Test Example 6 Measurement of phosphorylation activity inhibitory effect *(in vitro)* on wild-type and mutant EGFR

The measurement of the *in vitro* inhibitory activity of a compound against wild-type and mutant EGFR was outsourced to Carna Biosciences, Inc. (Kinase Profiling Book, https://www.camabio.com/japanese/product/search.cgi?mode=profiling).

Specifically, first, the pyrimidine compound of the present invention was diluted stepwise with dimethyl sulfoxide (DMSO). Subsequently, the EGFR protein, the substrate peptide (Srctide, final concentration: 1 μM), magnesium chloride (final concentration: 5 mM), manganese chloride (final concentration: 1 mM), ATP (final concentration: near Km of each EGFR), and the pyrimidine compound of the present invention in DMSO solution (final concentration of DMSO: 1%) were added to a buffer for the kinase reaction (20 mM HEPES (pH 7.5), 1 mM dithiothreitol, and 0.01% Triton X-100), and the obtained mixture was then incubated at room temperature for 1 hour, so that the kinase reaction was carried out. To the reaction solution, Termination Buffer was added, so as to terminate the kinase reaction. Finally, using

LabChip(TM) EZ Reader II (PerkinElmer), an unphosphorylated substrate peptide (S) and a phosphorylated peptide (P) were separated and detected according to microchannel capillary electrophoresis. From the peak heights of S and P, the amount of the phosphorylation reaction was obtained, and the concentration of the compound capable of inhibiting the phosphorylation reaction by 50% was defined as an IC50 value (nM). The results are shown in the following table.

As the pyrimidine compound of the present invention, the compound of Example 11 (Example compound 11) was used.

[Table 6]

| | IC50 (nmol/L) |
|---|---|
| kinases | Example compound 11 |
| EGFR | 0.47 |
| EGFR [d746-750] | 0.25 |
| EGFR [d746-750/T790M] | 6.8 |
| EGFR [L858R] | 0.36 |
| EGFR [T790M/L858R] | 6.6 |
| EGFR [D770_N771insNPG] | 0.55 |

From the above results, it was found that the pyrimidine compound of the present invention has excellent inhibitory activity against wild-type and mutant EGFR.

Test Example 7 Measurement of growth inhibitory activity against EGFR overexpressing cell line and exon 20 insertion mutant EGFR expressing cell line

Growth inhibitory activity against an EGFR overexpressing cell line and an exon 20 insertion mutant EGFR expressing cell line was evaluated using MDA-MB-468 cells (ATCC) as an EGFR overexpressing human breast cancer cell line; NCI-H1975 cells (ATCC) as L858R and T790M mutant EGFR-positive human lung cancer cells; and MCF10A _EGFR cells, MCF10A _EGFR/V769_D770insASV cells, MCF10A_EGFR/D770_N771insSVD cells, and MCF10A _EGFR/H773_V774insNPH cells (Fukushima Medical University) which were cells obtained by introducing the EGFR gene (WT, D769_N770insASV mutant, D770_N771insSVD mutant, H773_V774insNPH mutant) into MCF10A cells as human normal mammary gland cells.

The MDA-MB-468 cells were suspended in Leibovitz's L-15 medium containing 10% inactivated fetal bovine serum. The NCI-H1975 cells were suspended in RPMI-1640 medium containing 10% inactivated fetal bovine serum. The MCF10A_EGFR cells, the MCF10A EGFR/V769_D770insASV cells, the MCF10A_EGFR/D770_N771insSVD cells, or the MCF10A _EGFR/H773_V774insNPH cells were suspended in DMEM/Ham's F-12 medium (containing L-glutamine, phenol red, HEPES, and sodium pyruvate) containing 10 μg/mL insulin, 500 ng/mL hydrocortisone, 5 μmol/L forskolin, and 5% inactivated horse serum in terms of their final concentrations. Each cell suspension was seeded in each well of a 96-well flat-bottom plate such that the number of cells per well was 500, and was then cultured in a carbon dioxide gas-free culture vessel for the MDA-MB-468 cells and in a 5% carbon dioxide gas-containing culture vessel for the other cells at 37°C for 1 day. The pyrimidine compound of the present invention was prepared at 1 mM in DMSO and then diluted 1/200 with a medium to prepare a 5 μM solution. Thereafter, the pyrimidine compound of the present invention in the DMSO solution was diluted with the medium used in the suspension of the cells, and the obtained solution was then added to each well so that the final concentration of the highest concentration of the test compound was 1000 nM. The obtained mixture was further cultured in a carbon dioxide gas-free culture vessel for the MDA-MB-468 cells and in the 5% carbon dioxide gas-containing culture vessel for the other cells at 37°C for 3 days.

The cells at the start of the culture (day 0) and after the culture (day 3) were counted using CellTiter-Glo(R) 2.0 Reagent (Promega Corp.) according to the protocol recommended by the manufacturer. The growth inhibition percentage was calculated according to the equation given below to determine the 50% inhibition concentration (GI50 (nM)) of the test compound. The results are shown in Table 7. As the pyrimidine compound of the present invention, the compounds of Examples 2, 11, and 12 (Example compounds 2, 11, and 12) was used.

1) In the case of $T_{day3} \geq C_{day0}$

$$\text{Growth percentage (\%)} = (T_{day3} - C_{day0}) / (C_{day3} - C_{day0}) \times 100$$

T: Emission intensity from the well to which the test compound was added
C: Emission intensity from the well to which the test compound was not added
day0: Day on which the test compound was added
day3: Evaluation day

2) In the case of $T_{day3} < C_{day0}$

$$\text{Growth percentage (\%)} = (T_{day3} - C_{day0}) / (C_{day0}) \times 100$$

T: Emission intensity from the well to which the test compound was added
C: Emission intensity from the well to which the test compound was not added
day0: Day on which the test compound was added
day3: Evaluation day

[Table 7]

| | Cell growth inhibitory activity GI 50 value (nM) | | | | | |
|---|---|---|---|---|---|---|
| | MDA-MB-468 | NCI-H1975 | MCF10A_EGFR cell (EGFR-WT) | MCF10A E GFR/ V769_D770insASV cell | MCF10A_EG FR/ D770_N771insSVD cell | MCF10A_E GFR/ H773_V774insNPH cell |
| Example compound 2 | 81 | 77 | 55 | 6.7 | 8.2 | 16 |
| Example compound 11 | 99 | 87 | 81 | 7.3 | 10 | 18 |
| Example compound 12 | 221 | 153 | 206 | 24 | 27 | 50 |

From the above results, it was found that the pyrimidine compound group of the present invention also has excellent cell growth inhibitory activity against the wild-type EGFR overexpressing line MDA-MB-468 cells, the MCF10A_EGFR cells expressing the introduced wild-type EGFR gene, the L858R and T790M mutant EGFR-positive cells NCI-H1975 cells, and the exon 20 insertion mutant EGFR expressing cell lines (MCF10A_EGFR/V769_D770insASV cells, MCF10A_EGFR/D770_N771insSVD cells, and MCF10A_EGFR/H773_V774insNPH cells).

Test Example 8 Measurement of growth inhibitory activity against exon 20 insertion mutant EGFR expressing cell line

Growth inhibitory activity against exon 20 insertion mutant EGFR was evaluated using H1975-EGFRinsSVD cells obtained by genetically modifying NCI-H1975 cells so as to express D770_N771insSVD mutant EGFR and to knock out endogenous EGFR (T790M/L858R), and LXF 2478 cells (Charles River Laboratories, Inc.) of V769_D770insASV mutant EGFR-positive human lung cancer patient-derived tumor.

The H1975-EGFRinsSVD cells were prepared by introducing PB-CMV-MCS-EF1-RFP+Puro vector encoding D770_N771insSVD (insSVD), together with Super PiggyBacTransposase expression vector, to NCI-H1975 cells by electroporation with Amaxa(R) Cell Line Nucleofector(R) Kit R, then selecting cells using puromycin (Sigma-Aldrich Co. LLC.), then introducing thereto XTN(R) TALENs Site-Specific Nucleases (Transposagen Bio) by electroporation with Amaxa(R) Cell Line Nucleofector(R) Kit R, and selecting, by sequencing, cells in which endogenous EGFR (T790M/L858R) was knocked out.

Upon evaluation of cell growth inhibitory effect, the cells of each line were suspended in RPMI-1640 medium. The cell suspension was seeded in each well of a 96-well flat-bottom plate such that the number of cells per well was 3,000, and was then cultured in a 5% carbon dioxide gas-containing culture vessel at 37°C for 1 day. The pyrimidine compound of the present invention obtained in Examples 1 to 15 (Example compounds 1-15) or compounds obtained in Comparative Examples 1 and 2 (Comparative Example compounds 1-2) were dissolved at 1 mM in DMSO and then added to each well using Tecan D300e digital dispenser (Tecan Trading AG) such that the final concentration of the

highest concentration of the test compound was 1000 nM and the common ratio was 3. The cells were cultured in a 5% carbon dioxide gas-containing culture vessel at 37°C for 3 days. The cells at the start of the culture (day 0) and after the culture (day 3) were counted using CellTiter-Glo(R) 2.0 Reagent (Promega Corp.) according to the protocol recommended by the manufacturer. The growth inhibition percentage was calculated according to the equation given below to determine the 50% inhibition concentration (GI50 (nM)) of the test compound. The results are shown in Table 8.

1) In the case of $T_{day3} \geq C_{day0}$

$$\text{Growth percentage (\%)} = (T_{day3} - C_{day0}) / (C_{day3} - C_{day0}) \times 100$$

T: Emission intensity from the well to which the test compound was added
C: Emission intensity from the well to which the test compound was not added
day0: Day on which the test compound was added
day3: Evaluation day

2) In the case of $T_{day3} < C_{day0}$

$$\text{Growth percentage (\%)} = (T_{day3} - C_{day0}) / (C_{day0}) \times 100$$

T: Emission intensity from the well to which the test compound was added
C: Emission intensity from the well to which the test compound was not added
day0: Day on which the test compound was added
day3: Evaluation day

[Table 8]

| | Cell growth inhibitory activity GI 50 value (nM) | |
|---|---|---|
| | H1975-EGFRinsSVD cell | LXF 2478 cell |
| Example compound 1 | 44 | 29 |
| Example compound 2 | 9.5 | 11 |
| Example compound 3 | 77 | 24 |
| Example compound 4 | 13 | 15 |
| Example compound 5 | 33 | 24 |
| Example compound 6 | 29 | 29 |
| Example compound 7 | 33 | 33 |
| Example compound 8 | 25 | 26 |
| Example compound 9 | 65 | 34 |
| Example compound 10 | 39 | 40 |
| Example compound 11 | 17 | 19 |
| Example compound 12 | 52 | 48 |
| Example compound 13 | 48 | 46 |
| Example compound 14 | 30 | 27 |
| Example compound 15 | 101 | 58 |
| Comparative Example 1 | > 1000 | > 1000 |
| Comparative Example 2 | > 1000 | > 1000 |

From the above results, it was found that the pyrimidine compound group of the present invention also has

excellent cell growth inhibitory activity against the exon 20 insertion mutant EGFR expressing cell lines (H1975-EGFRinsSVD and LXF 2478).

Test Example 9 Evaluation of oral absorbability

The pyrimidine compound of the present invention was suspended or dissolved in 0.5% HPMC aqueous solution and 0.1 N hydrochloric acid, and the obtained suspension or solution was orally administered to BALB/cA mice (CLEA Japan, Inc.) at a dose of 50 mg/kg/day. At 0.5, 1, 2, 4 and 6 hours after completion of the oral administration, blood was collected from the facial vein over time, so as to obtain plasma. The concentration of the compound in the obtained plasma was measured by LC-MS/MS, and the oral absorbability of the present compound was evaluated.

The results are shown in the following Table 9.

Table 9

| Example No. | AUC 0 - 6 hr (μM·hr) | Example No. | AUC 0 - 6 hr (μM·hr) |
|---|---|---|---|
| 1 | 50 | 2 | 15 |
| 3 | 24 | 4 | 12 |
| 5 | 20 | 6 | 17 |
| 7 | 15 | 8 | 15 |
| 9 | 51 | 10 | 50 |
| 11 | 31 | 12 | 36 |
| 13 | 18 | 14 | 27 |
| 15 | 34 | 16 | 15 |
| 17 | 21 | 18 | 15 |
| 19 | 6.1 | Comp. Ex. 2 | 1.5 |

From the above results, it was found that the pyrimidine compound of the present invention was contained in a sufficient concentration in the plasma, so that the present pyrimidine compound exhibited favorable oral absorbability. In contrast, the compound of Comparative Example 2 had oral absorbability that was more than 4 times more attenuated than the compound of the present invention.

Test Example 10 Evaluation of brain penetration properties

The pyrimidine compound of the present invention was suspended or dissolved in 0.5% HPMC aqueous solution and 0.1 N hydrochloric acid, and the obtained suspension or solution was orally administered to BALB/cA mice (CLEA Japan, Inc.) at a dose of 50 mg/kg/day. At 0.5 hours after completion of the oral administration, blood was collected from the facial vein, and whole brain was then excised, so as to obtain plasma and brain samples. Water was added to the obtained brain sample in 3 times the volume of the brain sample, and the resultant was then homogenized using an ultrasonic homogenizer, so as to obtain a brain homogenate. The concentration of the compound in the obtained plasma and brain homogenate was measured by LC-MS/MS, and the brain penetration properties of the present compound were evaluated from the brain/plasma concentration of the compound.

The results are shown in the following Table 10.

[Table 10]

| Example No. | Compound concentration in plasma (μM) | Compound concentration in brain (μM) | Kp value (Compound concentration in brain/plasma) |
|---|---|---|---|
| 1 | 9.1 | 1.4 | 0.15 |
| 2 | 6.6 | 1.8 | 0.27 |
| 3 | 11 | 1.4 | 0.13 |
| 4 | 8.3 | 2.8 | 0.34 |

(continued)

| Example No. | Compound concentration in plasma (μM) | Compound concentration in brain (μM) | Kp value (Compound concentration in brain/plasma) |
|---|---|---|---|
| 5 | 15 | 2.2 | 0.15 |
| 6 | 7.5 | 1.3 | 0.17 |
| 7 | 7.9 | 1.1 | 0.14 |
| 8 | 9.9 | 3.3 | 0.33 |
| 9 | 13 | 2.4 | 0.18 |
| 10 | 13 | 2.4 | 0.18 |
| 11 | 12 | 2.7 | 0.23 |
| 12 | 11 | 3.2 | 0.29 |
| 13 | 13 | 2.8 | 0.22 |
| 14 | 9.9 | 2.1 | 0.21 |
| 15 | 8.1 | 1.2 | 0.15 |
| 16 | 12 | 4.4 | 0.35 |
| 17 | 17 | 6.5 | 0.39 |
| 18 | 7.7 | 1.6 | 0.22 |
| 19 | 4.9 | 0.7 | 0.14 |
| Comp. Ex. 2 | 1.6 | 0.008 | 0.005 |

From the above results, it was found that the pyrimidine compound of the present invention had a high brain/plasma compound concentration (Kp value) and thus, exhibited favorable brain penetration properties. On the other hand, the brain concentration of the compound of Comparative Example 2 was more than 80 times more attenuated than that of the compound of the present invention.

Test Example 11 Antitumor effect confirmation test *(in vivo)* on direct brain transplantation models, into which Luciferase gene-introduced HER2 expressing cell line (NCI-N87-luc) is directly transplanted

In order to confirm the antitumor effects of a test compound on direct brain transplantation models, NCI-N87-Luc, which was obtained by introducing a Luciferase gene into NCI-N87 that was a human stomach cancer tumor cell line purchased from American Type Culture Collection, was used. The NCI-N87-Luc was added into a 10% fetal bovine serum (FBS)-containing RPMI-1640 medium (supplemented with 4.5 g/L glucose, 10 mM HEPES, and 1 mM sodium pyruvate) (FUJIFILM Wako Pure Chemical Corporation), and this cell line was then cultured in a 5% CO2 incubator at 37°C.

The NCI-N87-Luc cells were re-suspended in PBS in a concentration of $6.25 \times 10^7$ cells/mL.

Using a mouse ear bar, a nude mouse with 6 to 7 weeks old (BALB/cAJcl-nu/nu, CLEA Japan, Inc.) was fixed in a brain stereotaxic apparatus, and the skin on the upper brain portion was disinfected with alcohol cotton and was then excised with a surgical knife.

A microdrill was used to drill a hole in the skull, and then, using a needle, a manipulator, and a syringe pump, 4 μL of the cell suspension was transplanted into the brain at a rate of 0.8 μL/min.

As a reference of the amount of brain tumor, approximately 3 weeks after the transplantation, Total Flux (Photon/sec) was measured in all of the survival cases, using IVIS (PerkinElmer, Inc., model: Lumina II). Based on the obtained results, 6 animals were assigned to each group, using the grouping program of MiSTAT (Ver. 2.00).

The test compound was orally administered to the mice once a day, every day, for 21 days from the following day of the grouping (Days 1 - 21).

For judgment of the presence or absence of effects, the value (Log10) obtained by logarithmic transformation of the total flux on the judgment date was used. The test compound was administered to the mice at a dose of 25 mg/kg/day in Example 2 and Example 11, whereas it was administered at a dose of 50 mg/kg/day in Example 12.

A graph was prepared with the value obtained by logarithmic transformation (Log10) of the mean total flux of each group as a vertical axis, and with the number of days (Day) after the transplantation as a horizontal axis. The transition of the total flux over time in the drug administration period was observed.

As test compounds, the compounds of Example 2, Example 11, and Example 12 were used, and as a control, 0.1 N HCl and 0.5% HPMC aqueous solution were used.

The results are shown in the following Figure 1 to Figure 6. The value obtained by logarithmic transformation (Log10) of the total flux on Day 22 in each group was analyzed by a Dunnett's test or a Student-t test. As a result, it was demonstrated that the aforementioned value of the test compound group was statistically significantly lower than the value of the control group (significance level (both sides): 5%) (Figure 1: the compound of Example 2 was used, P = 0.0077; Figure 2: the compound of Example 11 was used, P = 0.0007; and Figure 3: the compound of Example 12 was used, P = 0.0012). For the measurement of the body weight, an animal electronic balance was used. A body weight change percentage (BWCn) from the body weight on the $n^{th}$ day (BWn) was calculated according to the following equation:

$$BWCn\ (\%) = [(\text{body weight on } n^{th} \text{ day}) - (\text{body weight on grouping day})] / [(\text{body weight on grouping day})] \times 100.$$

From the results of this test, it was found that the pyrimidine compound of the present invention has excellent antitumor effects against the HER2 overexpressing cell line (NCI-N87-luc) transplanted into the nude mice. Moreover, a body weight reduction of -20% or more was not observed in all of the mice to which the compound of Example 2 or Example 11 had been administered. Accordingly, it was found that there were no serious side effects (Figures 4-6).

Test Example 12 Antitumor effect confirmation test *(in vivo)* on subcutaneous transplantation models, into which H1975-EGFRinsSVD cell line is transplanted

H1975-EGFRinsSVD cell line was cultured in RPMI-1640 (containing 4.5 g/L glucose, 10 mM HEPES and 1 mM sodium pyruvate) (FUJIFILM Wako Pure Chemical Corporation) medium containing 10% inactivated fetal bovine serum (FBS) in a 5% $CO_2$ incubator at 37°C.

The H1975-EGFRinsSVD cells were resuspended at a concentration of $8 \times 10^7$ cells/mL in PBS. The cell suspension was subcutaneously transplanted at $8 \times 10^6$ cells/0.1 mL to the right chest of each 6 week old nude mouse (BALB/cAJcl-nu/nu, CLEA Japan, Inc.) using a 1 mL syringe for tuberculin and a 25 G injection needle.

When the tumor volumes of nude mice having tumor engraftment became on the order of 100 to 200 $mm^3$, the mice were assigned to groups each involving 6 animals by random stratification such that the mean tumor volume was equal among the groups.

The test compound used was the compounds of Examples 2, 11, and 12, and a 0.5% HPMC aqueous solution was used as a control. The compounds of Examples 2, 11 and 12 were orally administered at doses of 25 mg/kg/day, 25 mg/kg/day and 50 mg/kg/day, respectively.

Each test compound or the control was orally administered every day for 14 days (Days 1 - 14) from the day following the grouping.

To compare the transition of tumor growth over time by the administration of each test compound, a tumor volume (which is also referred to as "TV" below) was measured at a frequency of twice a week over time. For the measurement of the body weight, an animal electronic balance was used. A body weight change percentage is also referred to as "BWC" below. A body weight change percentage on the $n^{th}$ day (BWCn) from the body weight on the $n^{th}$ day (BWn) was calculated according to the equation given below. The transition of mean TV and BWC values of the individuals is shown in Figures 7 and 8.

$$BWCn\ (\%) = ((\text{body weight on } n^{th} \text{ day}) - (\text{body weight on grouping day})] / [(\text{body weight on grouping day}) \times 100$$

When the mean TV value of the compound administration group on the final evaluation day (Day 15) was smaller than that of the control group and exhibited statistically significant difference (Dunnett's type multiple comparison test), this compound was judged as being effective (P < 0.001) and is indicated by the mark * in the drawing. The results are shown in Figure 7.

As a result of conducting analysis by the Dunnett's type multiple comparison test, it was shown that the tumor volume was statistically significantly low (P < 0.001) for all the pyrimidine compounds of the present invention as compared

with the control group. From the results of this test, it was found that the compounds of Examples 2, 11, and 12 have excellent antitumor effects against the exon 20 insertion mutant EGFR expressing cell line (H1975-EGFRinsSVD) transplanted into the nude mouse brain. Moreover, a body weight reduction of 20% or more was not observed in all of the mice to which each compound had been administered.

Test Example 13 Antitumor effect confirmation test *(in vivo)* on direct brain transplantation models, into which luciferase gene-introduced exon 20 insertion mutant EGFR expressing cell line (H1975-EGFRinsSVD-Luc) is directly transplanted

The antitumor effects and life-extending effects of the pyrimidine compounds of the present invention on direct brain transplantation models were evaluated using H1975-EGFRinsSVD-Luc line obtained by introducing luciferase into the human mutant EGFR-introduced cell line H1975-EGFRinsSVD.

The H1975-EGFRinsSVD-Luc cells used were prepared by introducing pJTI(R) FAST DEST vector encoding luciferase, together with pJTI(R) PhiC31 Integrase expression vector, to NCI-H1975-EGFRinsSVD cells by electroporation with Amaxa(R) Cell Line Nucleofector(R) Kit R, followed by selection using hygromycin B (Nacalai Tesque, Inc.).

The H1975-EGFRinsSVD-Luc cell line was cultured in RPMI-1640 (containing 4.5 g/L glucose, 10 mM HEPES and 1 mM sodium pyruvate) (FUJIFILM Wako Pure Chemical Corporation) medium containing 10% inactivated fetal bovine serum (FBS) in a 5% $CO_2$ incubator at 37°C.

The H1975-EGFRinsSVD-Luc cells were resuspended at a concentration of $12.5 \times 10^7$ cells/mL in PBS.

Using a mouse ear bar, a nude mouse with 6 to 7 weeks old (BALB/cAJcl-nu/nu, CLEA Japan, Inc.) was fixed in a brain stereotaxic apparatus, and the skin on the top of the head was disinfected by the application of an Isodine-containing antiseptic solution using a sterile cotton swab and was then excised with a surgical knife.

A microdrill was used to drill a hole in the skull, and then, using a needle, a manipulator, and a syringe pump, 2 μL of the cell suspension was transplanted into the brain at a rate of 0.8 μL/min.

As a reference of the amount of brain tumor, 26 days after the transplantation, Total Flux (Photon/sec) was measured in all of the survival cases, using IVIS (PerkinElmer, Inc., model: Lumina II). Based on the obtained results, 10 animals were assigned to each group by random stratification such that the mean total flux was equal among the groups.

The test compound used was the compound of Example 11, and a 0.5% HPMC aqueous solution was used as a control. The compound of Example 11 was administered at a dose of 12.5 mg/kg/day or 25 mg/kg/day.

The pyrimidine compound of the present invention or the control was orally administered once a day, every day, for 38 days (Days 27 - 64) from the following day of the grouping day.

For judgment of the presence or absence of antitumor effects, the value (Log10) obtained by logarithmic transformation of the total flux on the antitumor effect judgment day (Day 47) after 3-week drug administration from the following day (Day 27) of the grouping day was used.

A graph was prepared with the value obtained by the mean total flux of each group as a vertical axis, and with the number of days (Day) after the transplantation as a horizontal axis. The transition of the total flux over time in the drug administration period was observed.

For judgment of the presence or absence of life-extending effects, the number of survival days on the final life-extending effect evaluation day from after cell transplantation (Days 0 - 65) in the test compound group compared with the control group was analyzed by the Log-Rank test.

The results are shown in Figures 9 and 10 given below. The value obtained by logarithmic transformation (Log10) of the total flux on Day 47 in each group was analyzed by the Dunnett's type multiple comparison test. As a result, it was demonstrated that the aforementioned value of the test compound group was statistically significantly lower than the value of the control group (significance level (both sides): 5%) ($P < 0.001$). From the results of this test, it was found that the the pyrimidine compound of the present invention has antitumor effects against the exon 20 insertion mutant EGFR expressing cell line (H1975-EGFRinsSVD-Luc) transplanted into the nude mouse brain.

In addition, the number of survival days on the final life-extending effect evaluation day from after cell transplantation (Days 0 - 65) in the test compound group compared with the control group was analyzed by the Log-Rank test. As a result, statistically significant life-extending effects were observed, as compared with the control group ($P < 0.05$).

Test Example 14 Antitumor *effect (in vitro)* brought about by combined use of pyrimidine compound and other antitumor agent

The distributor of each reagent, the distributor of a tumor cell line, the medium used, and the number of seeded cells are shown in the following tables.

[Table 11]

| Reagent | Distributor |
|---|---|
| Fetal bovine serum (FBS) | GIBCO or SIGMA-ALDRICH Co. LCC |
| McCoy's 5A medium | Thermo Fisher Scientific, Inc. |
| Dulbecco's Modified Eagle medium (DMEM) | FUJIFILM Wako Pure Chemical Corporation |
| RPMI-1640 medium | FUJIFILM Wako Pure Chemical Corporation |
| AZD8055 | Cayman Chemical Company |
| Everolimus | Cayman Chemical Company |
| Dactolisib | Cayman Chemical Company |
| Buparlisib | Cayman Chemical Company |
| Taselisib | Selleck Chemicals |
| Palbociclib | Cayman Chemical Company |
| Fulvestrant | MedChemExpress |
| MK-2206 | Sun-shine Chemical |
| 5-Fluorouracil | FUJIFILM Wako Pure Chemical Corporation |
| Paclitaxel | FUJIFILM Wako Pure Chemical Corporation |
| Cisplatin | SIGMA-ALDRICH Co. LCC |
| Trifluridine (trifluorothymidine) | Yuki Gosei Kogyo Co., Ltd. |
| Gemcitabine | FUJIFILM Wako Pure Chemical Corporation |
| SN-38 (active metabolite of | Tokyo Chemical Industry Co., Ltd. |
| irinotecan) | |

[Table 12]

| Tumor cell line (origin) | Distributor of cell line | Medium | The number of seeded cells per well |
|---|---|---|---|
| BT-474 (human breast cancer) | ATCC | Dulbecco's Modified Eagle medium containing 10% FBS | 250 |
| SK-BR-3 (human breast cancer) | Sumitomo Dainippon Pharma Co., Ltd. (formerly Dainippon Pharmaceutical Co., Ltd.) | McCoy's 5A medium containing 10% FBS | 125 |
| HCC827 (human lung cancer) | ATCC | RPMI-1640 medium containing 10%FBS | 250 |

HER2-positive human breast cancer-derived SK-BR-3 cells [Sumitomo Dainippon Pharma Co., Ltd. (formerly Dainippon Pharmaceutical Co., Ltd.) were suspended in McCoy's 5A medium containing 10% inactivated fetal bovine serum. Likewise, HER2-positive human breast cancer-derived BT-474 cells [American Type Culture Collection (ATCC)] were suspended in Dulbecco's Modified Eagle medium containing 10% inactivated fetal bovine serum. Exon 19 mutation (del E746-A750)-positive human lung cancer-derived HCC827 cells (ATCC) were suspended in RPMI-1640 medium containing 10% inactivated fetal bovine serum. Each cell suspension was seeded at 25 μL/well in a 384-well flat-bottom culture plate. The plate with the seeded cells was incubated in a 5% carbon dioxide gas-containing culture vessel at 37°C.

On the following day of the seeding, drug solutions of the compound of Example 11 and other antitumor agent mixed in combinations of varying concentrations were added to the cells.

On the following day of the seeding, the pyrimidine compound of the present invention and the other antitumor agent were dissolved in DMSO and then added according to the final concentrations of the maximum concentrations

and the common ratios shown in Table 13. Specifically, 8 concentrations (including 0 nM) of the compound of Example 11 as the pyrimidine compound of the present invention, and 10 concentrations (including 0 nM) of the other antitumor agent were set to 80 in total of all possible combinations and added using Tecan D300e digital dispenser (Tecan Trading AG), and the plate was incubated in a 5% carbon dioxide gas-containing culture vessel at 37°C for 3 days.

Three days later, CellTiter-Glo(TM) 2.0 Reagent (Promega Corporation) was added at 25 μL/well, and chemiluminescence was measured using a plate reader (EnSpire(R) Multimode Plate Reader, PerkinElmer Japan Co., Ltd.).

[Table 13]

| Other antitumor agent | Tumor cell line | Other antitumor agent | | Example 11 | |
|---|---|---|---|---|---|
| | | Maximum concentration (nM) | Common ratio | Maximum concentration (nM) | Common ratio |
| Gemcitabine | BT-474 | 30000 | 3 | 100 | 3 |
| | SK-BR-3 | 30000 | 3 | 100 | 3 |
| | HCC827 | 30000 | 3 | 100 | 3 |
| Paclitaxel | BT-474 | 1000 | 3 | 100 | 3 |
| | SK-BR-3 | 1000 | 3 | 100 | 3 |
| | HCC827 | 100 | 3 | 100 | 3 |
| 5-Fluorouracil | BT-474 | 1000000 | 3 | 100 | 3 |
| | SK-BR-3 | 1000000 | 3 | 100 | 3 |
| Trifluridine | BT-474 | 1000000 | 3 | 100 | 3 |
| | SK-BR-3 | 1000000 | 3 | 100 | 3 |
| | HCC827 | 1000000 | 3 | 100 | 3 |
| Buparlisib | BT-474 | 30000 | 3 | 100 | 3 |
| | SK-BR-3 | 30000 | 3 | 100 | 3 |
| | HCC827 | 30000 | 3 | 100 | 3 |
| Taselisib | BT-474 | 100 | 3 | 100 | 3 |
| | SK-BR-3 | 100 | 3 | 100 | 3 |
| | HCC827 | 100 | 3 | 100 | 3 |
| Dactolisib | BT-474 | 300 | 3 | 100 | 3 |
| | SK-BR-3 | 300 | 3 | 100 | 3 |
| Everolimus | BT-474 | 30000 | 3 | 100 | 3 |
| | SK-BR-3 | 30000 | 3 | 100 | 3 |
| | HCC827 | 30000 | 3 | 100 | 3 |
| AZD8055 | BT-474 | 1000 | 3 | 100 | 3 |
| | SK-BR-3 | 1000 | 3 | 100 | 3 |
| | HCC827 | 1000 | 3 | 100 | 3 |
| MK-2206 | BT-474 | 10000 | 3 | 100 | 3 |
| | SK-BR-3 | 10000 | 3 | 100 | 3 |
| | HCC827 | 10000 | 3 | 100 | 3 |
| Palbociclib | BT-474 | 20000 | 3 | 100 | 3 |
| | SK-BR-3 | 20000 | 3 | 100 | 3 |
| | HCC827 | 20000 | 3 | 100 | 3 |

(continued)

| Other antitumor agent | Tumor cell line | Other antitumor agent | | Example 11 | |
|---|---|---|---|---|---|
| | | Maximum concentration (nM) | Common ratio | Maximum concentration (nM) | Common ratio |
| Fulvestrant | BT-474 | 100000 | 3 | 100 | 3 |
| | SK-BR-3 | 100000 | 3 | 100 | 3 |
| | HCC827 | 100000 | 3 | 100 | 3 |
| Cisplatin | HCC827 | 10000 | 3 | 100 | 3 |
| SN-38 | HCC827 | 100 | 3 | 100 | 3 |

The potentiation of an effect by combined use of the drugs was evaluated in accordance with the Chou-Talalay method (Adv. Enzyme Regul. 22: 27-55, 1984).

A mean from 4 wells of each combination was calculated from the obtained data, and a cell survival rate normalized against a control supplemented with a medium containing a vehicle was calculated. The cell survival rate was subtracted from 1 to calculate a Fa (fraction of affect) value. A combination index (CI) was calculated by applying the Median Effect equation to the experimental data.

It is considered that the combinations of the concentrations of the drugs for calculating CI fall within a concentration range in which the effect of one of the drugs is too strong if the Fa value is close to 1, and fall within a concentration range in which the effect of any of the drugs is too weak if the Fa value is close to 0. Such combinations are not appropriate for discussing a synergistic effect. Accordingly, combinations were extracted which attained $0.2 \le Fa \le 0.8$ from the Fa values calculated with the combinations of the respective concentrations of the compound of Example 11 and the other antitumor agent.

A combinatorial effect was judged as shown in the following table (Pharmacol Rev., 58 (3), 621-81, 2006).

[Table 14]

| Range of CI (upper limit) | Description |
|---|---|
| 0.1 | Very strong synergistic effect |
| 0.3 | Strong synergistic effect |
| 0.7 | Synergistic effect |
| 0.85 | Moderate synergistic effect |
| 0.9 | Slight synergistic effect |
| 1.0 | Almost additive |
| 1.2 | Slight antagonistic effect |
| 1.45 | Moderate antagonistic effect |
| 3.3 | Antagonistic effect |
| 10 | Strong antagonistic effect |
| > 10 | Very strong antagonistic effect |

The results are shown in the tables given below.

In the tables, the "combined use ratio" represents a molar ratio of the other antitumor agent with the compound of Example 11 defined as 1.

Combined use of compound of Example 11 and gemcitabine

[Table 15]

| Cell line: | BT-474 | | | |
|---|---|---|---|---|
| Combined use ratio | Gemcitabine (nM) | Example 11 (nM) | Fa | CI |
| 8100 | 30000 | 3.70 | 0.331 | 0.890 |
| 2700 | 30000 | 11.1 | 0.497 | 0.844 |
| 900 | 3333 | 3.70 | 0.279 | 0.726 |
| 900 | 10000 | 11.1 | 0.476 | 0.784 |
| 300 | 3333 | 11.1 | 0.454 | 0.821 |
| 100 | 1111 | 11.1 | 0.444 | 0.843 |
| 1.2 | 13.7 | 11.1 | 0.446 | 0.828 |
| 0.412 | 4.57 | 11.1 | 0.468 | 0.740 |
| 0.137 | 4.57 | 33.3 | 0.671 | 0.796 |

[Table 16]

| Cell line: | SK-BR-3 | | | |
|---|---|---|---|---|
| Combined use ratio | Gemcitabine (nM) | Example 11 (nM) | Fa | CI |
| 218700 | 30000 | 0.137 | 0.546 | 0.514 |
| 72900 | 10000 | 0.137 | 0.485 | 0.633 |
| 24300 | 3333 | 0.137 | 0.542 | 0.065 |
| 24300 | 10000 | 0.412 | 0.478 | 0.740 |
| 8100 | 1111 | 0.137 | 0.463 | 0.117 |
| 8100 | 3333 | 0.412 | 0.488 | 0.208 |
| 8100 | 30000 | 3.70 | 0.529 | 0.824 |
| 2700 | 370 | 0.137 | 0.473 | 0.035 |
| 2700 | 1111 | 0.412 | 0.438 | 0.211 |
| 2700 | 3333 | 1.23 | 0.456 | 0.444 |
| 2700 | 10000 | 3.70 | 0.513 | 0.459 |
| 2700 | 30000 | 11.1 | 0.555 | 0.674 |
| 900 | 123 | 0.137 | 0.440 | 0.027 |
| 900 | 370 | 0.412 | 0.503 | 0.029 |
| 900 | 1111 | 1.23 | 0.411 | 0.421 |
| 900 | 3333 | 3.70 | 0.431 | 0.865 |
| 900 | 10000 | 11.1 | 0.596 | 0.248 |
| 900 | 30000 | 33.3 | 0.695 | 0.294 |
| 300 | 41.2 | 0.137 | 0.386 | 0.032 |
| 300 | 123 | 0.412 | 0.475 | 0.026 |
| 300 | 370 | 1.23 | 0.442 | 0.117 |
| 300 | 1111 | 3.70 | 0.471 | 0.242 |
| 300 | 3333 | 11.1 | 0.515 | 0.438 |
| 300 | 10000 | 33.3 | 0.641 | 0.434 |

(continued)

| Cell line: | SK-BR-3 | | | |
|---|---|---|---|---|
| Combined use ratio | Gemcitabine (nM) | Example 11 (nM) | Fa | CI |
| 100 | 13.7 | 0.137 | 0.366 | 0.023 |
| 100 | 41.2 | 0.412 | 0.367 | 0.068 |
| 100 | 123 | 1.235 | 0.458 | 0.068 |
| 100 | 370 | 3.704 | 0.484 | 0.159 |
| 100 | 1111 | 11.1 | 0.532 | 0.320 |
| 100 | 3333 | 33.3 | 0.612 | 0.524 |
| 100 | 10000 | 100 | 0.796 | 0.338 |
| 33.3 | 13.7 | 0.412 | 0.314 | 0.091 |
| 33.3 | 41.2 | 1.23 | 0.455 | 0.060 |
| 33.3 | 123 | 3.70 | 0.505 | 0.122 |
| 33.3 | 370 | 11.1 | 0.522 | 0.326 |
| 33.3 | 1111 | 33.3 | 0.666 | 0.345 |
| 33.3 | 3333 | 100 | 0.772 | 0.426 |
| 11.1 | 13.7 | 1.23 | 0.327 | 0.165 |
| 11.1 | 41.2 | 3.70 | 0.445 | 0.182 |
| 11.1 | 123 | 11.1 | 0.479 | 0.429 |
| 11.1 | 370 | 33.3 | 0.650 | 0.388 |
| 11.1 | 1111 | 100.0 | 0.795 | 0.341 |
| 3.70 | 4.57 | 1.23 | 0.232 | 0.422 |
| 3.70 | 13.7 | 3.70 | 0.294 | 0.592 |
| 3.70 | 41.2 | 11.11 | 0.498 | 0.372 |
| 3.70 | 123 | 33.33 | 0.653 | 0.381 |
| 3.70 | 370 | 100.00 | 0.745 | 0.545 |
| 1.23 | 4.57 | 3.70 | 0.245 | 0.886 |
| 1.23 | 13.7 | 11.11 | 0.427 | 0.598 |
| 1.23 | 41.2 | 33.33 | 0.595 | 0.574 |
| 1.23 | 123 | 100.00 | 0.757 | 0.490 |
| 0.412 | 4.57 | 11.11 | 0.377 | 0.849 |
| 0.412 | 13.7 | 33.33 | 0.604 | 0.539 |
| 0.412 | 41.2 | 100.00 | 0.749 | 0.527 |
| 0.137 | 4.57 | 33.33 | 0.564 | 0.712 |
| 0.137 | 13.7 | 100.00 | 0.738 | 0.582 |
| 0.0457 | 4.57 | 100.00 | 0.748 | 0.532 |

[Table 17]

| Cell line: | HCC827 | | | |
|---|---|---|---|---|
| Combined use ratio | Gemcitabine (nM) | Example 11 (nM) | Fa | CI |
| 24300 | 3333 | 0.137 | 0.519 | 0.018 |
| 8100 | 1111 | 0.137 | 0.474 | 0.030 |
| 2700 | 370 | 0.137 | 0.379 | 0.313 |
| 2700 | 1111 | 0.412 | 0.468 | 0.047 |
| 2700 | 3333 | 1.23 | 0.420 | 0.618 |
| 2700 | 10000 | 3.70 | 0.522 | 0.150 |
| 900 | 123 | 0.137 | 0.377 | 0.117 |
| 900 | 370 | 0.412 | 0.371 | 0.441 |
| 900 | 1111 | 1.23 | 0.471 | 0.075 |
| 900 | 3333 | 3.70 | 0.529 | 0.119 |
| 900 | 10000 | 11.1 | 0.551 | 0.310 |
| 900 | 30000 | 33.3 | 0.680 | 0.459 |
| 300 | 123 | 0.412 | 0.353 | 0.323 |
| 300 | 370 | 1.23 | 0.456 | 0.068 |
| 300 | 1111 | 3.70 | 0.536 | 0.109 |
| 300 | 3333 | 11.1 | 0.527 | 0.345 |
| 300 | 10000 | 33.3 | 0.673 | 0.478 |
| 100 | 123 | 1.23 | 0.336 | 0.676 |
| 100 | 370 | 3.70 | 0.526 | 0.113 |
| 100 | 1111 | 11.1 | 0.684 | 0.150 |
| 100 | 3333 | 33.3 | 0.719 | 0.368 |
| 33.3 | 123 | 3.70 | 0.387 | 0.297 |
| 33.3 | 370 | 11.1 | 0.598 | 0.236 |
| 33.3 | 1111 | 33.3 | 0.738 | 0.326 |
| 11.1 | 123 | 11.1 | 0.368 | 0.891 |
| 11.1 | 370 | 33.3 | 0.700 | 0.409 |
| 11.1 | 1111 | 100 | 0.783 | 0.723 |
| 3.70 | 123 | 33.3 | 0.695 | 0.422 |
| 3.70 | 370 | 100 | 0.760 | 0.849 |

Combined use of compound of Example 11 and paclitaxel

[Table 18]

| Cell line: | BT-474 | | | |
|---|---|---|---|---|
| Combined use ratio | Paclitaxcel (nM) | Example 11 (nM) | Fa | CI |
| 810 | 111 | 0.137 | 0.692 | 0.619 |
| 270 | 37.0 | 0.137 | 0.702 | 0.186 |

(continued)

| Cell line: | BT-474 | | | |
|---|---|---|---|---|
| Combined use ratio | Paclitaxcel (nM) | Example 11 (nM) | Fa | CI |
| 270 | 111 | 0.412 | 0.695 | 0.605 |
| 90 | 12.3 | 0.137 | 0.580 | 0.235 |
| 90 | 37.0 | 0.412 | 0.738 | 0.132 |
| 90 | 111 | 1.23 | 0.711 | 0.542 |
| 30 | 12.3 | 0.412 | 0.637 | 0.150 |
| 30 | 37.0 | 1.23 | 0.703 | 0.235 |
| 30 | 111 | 3.70 | 0.685 | 0.845 |
| 10 | 12.3 | 1.23 | 0.652 | 0.176 |
| 10 | 37.0 | 3.70 | 0.713 | 0.316 |
| 3.33 | 4.12 | 1.23 | 0.419 | 0.563 |
| 3.33 | 12.3 | 3.70 | 0.694 | 0.238 |
| 1.11 | 4.12 | 3.70 | 0.599 | 0.331 |
| 1.11 | 12.3 | 11.1 | 0.780 | 0.344 |
| 0.370 | 1.37 | 3.70 | 0.449 | 0.604 |
| 0.370 | 4.12 | 11.1 | 0.752 | 0.392 |
| 0.123 | 0.152 | 1.23 | 0.240 | 0.574 |
| 0.123 | 0.457 | 3.70 | 0.469 | 0.495 |
| 0.123 | 1.37 | 11.1 | 0.662 | 0.616 |
| 0.0412 | 0.152 | 3.70 | 0.458 | 0.502 |
| 0.0412 | 0.457 | 11.1 | 0.669 | 0.587 |
| 0.0137 | 0.152 | 11.1 | 0.678 | 0.561 |

[Table 19]

| Cell line: | SK-BR-3 | | | |
|---|---|---|---|---|
| Combined use ratio | Paclitaxcel (nM) | Example 11 (nM) | Fa | CI |
| 30.0 | 4.12 | 0.137 | 0.452 | 0.671 |
| 10.0 | 4.12 | 0.412 | 0.497 | 0.525 |
| 10.0 | 12.3 | 1.23 | 0.778 | 0.208 |
| 10.0 | 37.0 | 3.70 | 0.769 | 0.677 |
| 3.33 | 4.12 | 1.23 | 0.442 | 0.847 |
| 3.33 | 12.3 | 3.70 | 0.761 | 0.312 |
| 1.11 | 4.12 | 3.70 | 0.526 | 0.713 |
| 1.11 | 12.3 | 11.1 | 0.787 | 0.427 |
| 0.370 | 4.12 | 11.1 | 0.681 | 0.605 |
| 0.123 | 1.37 | 11.1 | 0.567 | 0.888 |
| 0.041 | 0.457 | 11.1 | 0.630 | 0.619 |

(continued)

| Cell line: | SK-BR-3 | | | |
|---|---|---|---|---|
| Combined use ratio | Paclitaxcel (nM) | Example 11 (nM) | Fa | CI |
| 0.0137 | 0.152 | 11.1 | 0.619 | 0.635 |
| 0.0137 | 0.457 | 33.3 | 0.791 | 0.776 |

[Table 20]

| Cell line: | HCC827 | | | |
|---|---|---|---|---|
| Combined use ratio | Paclitaxcel (nM) | Example 11 (nM) | Fa | CI |
| 27 | 11.1 | 0.412 | 0.785 | 0.620 |
| 9 | 11.1 | 1.23 | 0.787 | 0.614 |
| 3 | 0.412 | 0.137 | 0.288 | 0.675 |
| 3 | 11.1 | 3.70 | 0.779 | 0.664 |
| 0.333 | 0.0457 | 0.137 | 0.242 | 0.118 |
| 0.333 | 3.70 | 11.1 | 0.707 | 0.420 |
| 0.0370 | 1.23 | 33.3 | 0.699 | 0.231 |
| 0.0123 | 0.41 | 33.3 | 0.681 | 0.159 |
| 0.0123 | 1.23 | 100 | 0.792 | 0.183 |
| 0.00412 | 0.137 | 33.3 | 0.581 | 0.261 |
| 0.00412 | 0.412 | 100 | 0.743 | 0.224 |
| 0.00137 | 0.0152 | 11.1 | 0.277 | 0.737 |
| 0.00137 | 0.0457 | 33.3 | 0.502 | 0.413 |
| 0.00137 | 0.137 | 100 | 0.748 | 0.192 |
| 0.000457 | 0.0152 | 33.3 | 0.486 | 0.450 |
| 0.000457 | 0.0457 | 100 | 0.675 | 0.345 |
| 0.000152 | 0.0152 | 100 | 0.702 | 0.274 |

Combined use of compound of Example 11 and 5-fluorouracil (5-FU)

[Table 21]

| Cell line: | BT-474 | | | |
|---|---|---|---|---|
| Combined use ratio | 5-FU (nM) | Example 11 (nM) | Fa | CI |
| 7290000 | 1000000 | 0.137 | 0.650 | 0.602 |
| 2430000 | 333333 | 0.137 | 0.497 | 0.760 |
| 2430000 | 1000000 | 0.412 | 0.626 | 0.756 |
| 810000 | 111111 | 0.137 | 0.373 | 0.744 |
| 810000 | 1000000 | 1.23 | 0.635 | 0.725 |
| 270000 | 37037 | 0.137 | 0.259 | 0.771 |
| 270000 | 333333 | 1.23 | 0.501 | 0.800 |

(continued)

| Cell line: | BT-474 | | | |
|---|---|---|---|---|
| Combined use ratio | 5-FU (nM) | Example 11 (nM) | Fa | CI |
| 270000 | 1000000 | 3.70 | 0.651 | 0.703 |
| 90000 | 333333 | 3.70 | 0.510 | 0.874 |
| 90000 | 1000000 | 11.1 | 0.727 | 0.505 |
| 30000 | 111111 | 3.70 | 0.428 | 0.733 |
| 30000 | 333333 | 11.1 | 0.629 | 0.600 |
| 30000 | 1000000 | 33.3 | 0.794 | 0.585 |
| 10000 | 37037 | 3.70 | 0.350 | 0.693 |
| 10000 | 111111 | 11.1 | 0.574 | 0.584 |
| 10000 | 333333 | 33.3 | 0.730 | 0.750 |
| 3333 | 37037 | 11.1 | 0.525 | 0.625 |
| 3333 | 111111 | 33.3 | 0.699 | 0.816 |
| 1111 | 12346 | 11.1 | 0.544 | 0.533 |
| 1111 | 37037 | 33.3 | 0.681 | 0.862 |
| 370 | 1372 | 3.70 | 0.254 | 0.664 |
| 370 | 4115 | 11.1 | 0.501 | 0.625 |
| 370 | 12346 | 33.3 | 0.671 | 0.891 |
| 123 | 1372 | 11.1 | 0.508 | 0.601 |
| 123 | 4115 | 33.3 | 0.676 | 0.868 |
| 41.2 | 457 | 11.1 | 0.498 | 0.625 |
| 13.7 | 152 | 11.1 | 0.480 | 0.674 |
| 13.7 | 457 | 33.3 | 0.676 | 0.866 |

[Table 22]

| Cell line: | SK-BR-3 | | | |
|---|---|---|---|---|
| Combined use ratio | 5-FU (nM) | Example 11 (nM) | Fa | CI |
| 810000 | 111111 | 0.137 | 0.502 | 0.772 |
| 270000 | 37037 | 0.137 | 0.390 | 0.571 |
| 90000 | 12346 | 0.137 | 0.242 | 0.662 |
| 90000 | 37037 | 0.412 | 0.352 | 0.801 |
| 30000 | 12346 | 0.412 | 0.311 | 0.438 |
| 30000 | 37037 | 1.23 | 0.412 | 0.612 |
| 10000 | 37037 | 3.70 | 0.417 | 0.856 |
| 3333 | 4115 | 1.23 | 0.250 | 0.740 |
| 1111 | 1372 | 1.23 | 0.236 | 0.704 |
| 1111 | 37037 | 33.3 | 0.627 | 0.842 |
| 1111 | 111111 | 100 | 0.764 | 0.754 |

(continued)

| Cell line: | SK-BR-3 | | | |
|---|---|---|---|---|
| Combined use ratio | 5-FU (nM) | Example 11 (nM) | Fa | CI |
| 370 | 12346 | 33.3 | 0.642 | 0.685 |
| 370 | 37037 | 100 | 0.775 | 0.610 |
| 123 | 4115 | 33.3 | 0.641 | 0.667 |
| 123 | 12346 | 100 | 0.757 | 0.714 |
| 41.2 | 1372 | 33.3 | 0.617 | 0.796 |
| 41.2 | 4115 | 100 | 0.750 | 0.752 |
| 13.7 | 457 | 33.3 | 0.608 | 0.854 |
| 13.7 | 1372 | 100 | 0.759 | 0.684 |

Combined use of compound of Example 11 and trifluridine (FTD)

[Table 23]

| Cell line: | BT-474 | | | |
|---|---|---|---|---|
| Combined use ratio | FTD (nM) | Example 11 (nM) | Fa | CI |
| 7290000 | 1000000 | 0.137 | 0.363 | 0.107 |
| 2430000 | 1000000 | 0.412 | 0.384 | 0.113 |
| 810000 | 1000000 | 1.23 | 0.398 | 0.176 |
| 270000 | 1000000 | 3.70 | 0.477 | 0.256 |
| 90000 | 333333 | 3.70 | 0.275 | 0.777 |
| 90000 | 1000000 | 11.1 | 0.642 | 0.302 |
| 30000 | 333333 | 11.1 | 0.530 | 0.534 |
| 30000 | 1000000 | 33.3 | 0.760 | 0.440 |
| 10000 | 111111 | 11.1 | 0.432 | 0.870 |
| 10000 | 333333 | 33.3 | 0.707 | 0.617 |
| 370 | 12346 | 33.3 | 0.661 | 0.805 |
| 13.7 | 152 | 11.1 | 0.425 | 0.898 |
| 13.7 | 457 | 33.3 | 0.642 | 0.893 |

[Table 24]

| Cell line: | SK-BR-3 | | | |
|---|---|---|---|---|
| Combined use ratio | FTD (nM) | Example 11 (nM) | Fa | CI |
| 2430000 | 333333 | 0.137 | 0.510 | 0.712 |
| 810000 | 111111 | 0.137 | 0.456 | 0.332 |
| 810000 | 333333 | 0.412 | 0.555 | 0.545 |
| 270000 | 37037 | 0.137 | 0.225 | 0.581 |
| 270000 | 111111 | 0.412 | 0.440 | 0.378 |

(continued)

| Cell line: | SK-BR-3 | | | |
|---|---|---|---|---|
| Combined use ratio | FTD (nM) | Example 11 (nM) | Fa | CI |
| 270000 | 333333 | 1.23 | 0.504 | 0.767 |
| 90000 | 111111 | 1.23 | 0.364 | 0.679 |
| 90000 | 333333 | 3.70 | 0.540 | 0.664 |
| 30000 | 37037 | 1.23 | 0.269 | 0.602 |
| 30000 | 111111 | 3.70 | 0.381 | 0.775 |
| 30000 | 333333 | 11.1 | 0.553 | 0.747 |
| 30000 | 1000000 | 33.3 | 0.703 | 0.785 |
| 10000 | 111111 | 11.1 | 0.459 | 0.736 |
| 10000 | 333333 | 33.3 | 0.660 | 0.530 |
| 3333 | 37037 | 11.1 | 0.405 | 0.781 |
| 3333 | 111111 | 33.3 | 0.639 | 0.411 |
| 3333 | 333333 | 100 | 0.796 | 0.296 |
| 1111 | 37037 | 33.3 | 0.624 | 0.383 |
| 1111 | 111111 | 100 | 0.769 | 0.313 |
| 370 | 12346 | 33.3 | 0.526 | 0.765 |
| 370 | 37037 | 100 | 0.739 | 0.387 |
| 123 | 4115 | 33.3 | 0.573 | 0.523 |
| 123 | 12346 | 100 | 0.726 | 0.427 |
| 41.2 | 1372 | 33.3 | 0.539 | 0.673 |
| 41.2 | 4115 | 100 | 0.701 | 0.537 |
| 13.7 | 457 | 33.3 | 0.533 | 0.707 |
| 13.7 | 1372 | 100 | 0.715 | 0.470 |
| 4.57 | 152 | 33.3 | 0.592 | 0.444 |
| 4.57 | 457 | 100 | 0.688 | 0.599 |
| 1.52 | 152 | 100 | 0.702 | 0.527 |

[Table 25]

| Cell line: | HCC827 | | | |
|---|---|---|---|---|
| Combined use ratio | FTD (nM) | Example 11 (nM) | Fa | CI |
| 7290000 | 1000000 | 0.137 | 0.478 | 0.789 |
| 2430000 | 333333 | 0.137 | 0.306 | 0.610 |
| 2430000 | 1000000 | 0.412 | 0.466 | 0.845 |
| 810000 | 1000000 | 1.23 | 0.469 | 0.864 |
| 30000 | 111111 | 3.70 | 0.233 | 0.717 |
| 30000 | 1000000 | 33.3 | 0.703 | 0.747 |
| 10000 | 37037 | 3.70 | 0.231 | 0.520 |

(continued)

| Cell line: | HCC827 | | | |
|---|---|---|---|---|
| Combined use ratio | FTD (nM) | Example 11 (nM) | Fa | CI |
| 3333 | 37037 | 11.1 | 0.335 | 0.805 |
| 1111 | 12346 | 11.1 | 0.323 | 0.809 |
| 1111 | 37037 | 33.3 | 0.578 | 0.844 |
| 123 | 4115 | 33.3 | 0.569 | 0.859 |

Combined use of compound of Example 11 and buparlisib (BKM120)

[Table 26]

| Cell line: | BT-474 | | | |
|---|---|---|---|---|
| Combined use ratio | BKM120 (nM) | Example 11 (nM) | Fa | CI |
| 24300 | 3333 | 0.137 | 0.712 | 0.478 |
| 24300 | 10000 | 0.412 | 0.789 | 0.861 |
| 8100 | 1111 | 0.137 | 0.498 | 0.497 |
| 8100 | 3333 | 0.412 | 0.741 | 0.409 |
| 2700 | 1111 | 0.412 | 0.538 | 0.428 |
| 2700 | 3333 | 1.23 | 0.760 | 0.387 |
| 900 | 370 | 0.412 | 0.238 | 0.760 |
| 900 | 1111 | 1.23 | 0.601 | 0.361 |
| 300 | 370 | 1.23 | 0.334 | 0.557 |
| 300 | 1111 | 3.70 | 0.718 | 0.287 |
| 100 | 123 | 1.23 | 0.226 | 0.530 |
| 100 | 370 | 3.70 | 0.537 | 0.400 |
| 33.3 | 123 | 3.70 | 0.428 | 0.464 |
| 33.3 | 370 | 11.1 | 0.749 | 0.398 |
| 11.1 | 41.2 | 3.70 | 0.420 | 0.424 |
| 11.1 | 123 | 11.1 | 0.696 | 0.465 |
| 3.70 | 13.7 | 3.70 | 0.426 | 0.398 |
| 3.70 | 41.2 | 11.1 | 0.697 | 0.451 |
| 1.23 | 4.57 | 3.70 | 0.380 | 0.463 |
| 1.23 | 13.7 | 11.1 | 0.666 | 0.505 |
| 0.412 | 4.57 | 11.1 | 0.687 | 0.464 |

[Table 27]

| Cell line: | SK-BR-3 | | | |
|---|---|---|---|---|
| Combined use ratio | BKM120 (nM) | Example 11 (nM) | Fa | CI |
| 8100 | 1111 | 0.137 | 0.744 | 0.318 |

(continued)

| Cell line: | SK-BR-3 | | | |
|---|---|---|---|---|
| Combined use ratio | BKM120 (nM) | Example 11 (nM) | Fa | CI |
| 2700 | 370 | 0.137 | 0.473 | 0.389 |
| 2700 | 1111 | 0.412 | 0.737 | 0.341 |
| 900 | 370 | 0.412 | 0.368 | 0.671 |
| 900 | 1111 | 1.23 | 0.753 | 0.337 |
| 300 | 370 | 1.23 | 0.505 | 0.449 |
| 300 | 1111 | 3.70 | 0.770 | 0.380 |
| 100 | 370 | 3.70 | 0.580 | 0.506 |
| 33.3 | 41.2 | 1.23 | 0.271 | 0.454 |
| 33.3 | 123 | 3.70 | 0.358 | 0.897 |
| 33.3 | 370 | 11.1 | 0.790 | 0.373 |
| 11.1 | 41.2 | 3.70 | 0.437 | 0.539 |
| 11.1 | 123 | 11.1 | 0.720 | 0.472 |
| 3.70 | 13.7 | 3.70 | 0.396 | 0.602 |
| 3.70 | 41.2 | 11.1 | 0.629 | 0.685 |
| 1.23 | 4.57 | 3.70 | 0.317 | 0.838 |
| 1.23 | 13.7 | 11.1 | 0.627 | 0.676 |
| 0.412 | 4.57 | 11.1 | 0.637 | 0.642 |
| 0.412 | 13.7 | 33.3 | 0.799 | 0.837 |
| 0.137 | 4.57 | 33.3 | 0.798 | 0.838 |

[Table 28]

| Cell line: | HCC827 | | | |
|---|---|---|---|---|
| Combined use ratio | BKM120 (nM) | Example 11 (nM) | Fa | CI |
| 2700 | 30000 | 11.1 | 0.769 | 0.081 |
| 300 | 10000 | 33.3 | 0.751 | 0.077 |
| 100 | 13.7 | 0.137 | 0.538 | 0.089 |
| 100 | 1111 | 11.1 | 0.672 | 0.125 |
| 100 | 3333 | 33.3 | 0.748 | 0.046 |
| 33.3 | 13.7 | 0.412 | 0.471 | 0.663 |
| 33.3 | 1111 | 33.3 | 0.775 | 0.021 |
| 11.1 | 41.2 | 3.70 | 0.536 | 0.307 |
| 11.1 | 123 | 11.1 | 0.643 | 0.058 |
| 11.1 | 370 | 33.3 | 0.768 | 0.021 |
| 3.70 | 4.57 | 1.23 | 0.489 | 0.140 |
| 3.70 | 41.2 | 11.1 | 0.637 | 0.043 |
| 3.70 | 123 | 33.3 | 0.768 | 0.021 |

(continued)

| Cell line: | HCC827 | | | |
|---|---|---|---|---|
| Combined use ratio | BKM120 (nM) | Example 11 (nM) | Fa | CI |
| 1.23 | 4.57 | 3.70 | 0.544 | 0.049 |
| 1.23 | 13.7 | 11.1 | 0.604 | 0.053 |
| 1.23 | 41.2 | 33.3 | 0.734 | 0.032 |
| 0.412 | 4.57 | 11.1 | 0.621 | 0.038 |
| 0.412 | 13.7 | 33.3 | 0.754 | 0.025 |
| 0.137 | 4.57 | 33.3 | 0.730 | 0.033 |
| 0.0457 | 4.57 | 100 | 0.791 | 0.045 |

Combined use of compound of Example 11 and taselisib (GDC-0032)

[Table 29]

| Cell line: | BT-474 | | | |
|---|---|---|---|---|
| Combined use ratio | GDC-0032 (nM) | Example 11 (nM) | Fa | CI |
| 81 | 11.1 | 0.137 | 0.239 | 0.875 |
| 81 | 33.3 | 0.412 | 0.444 | 0.893 |
| 27 | 11.1 | 0.412 | 0.257 | 0.821 |
| 27 | 33.3 | 1.23 | 0.485 | 0.776 |
| 9 | 11.1 | 1.23 | 0.361 | 0.530 |
| 9 | 33.3 | 3.70 | 0.550 | 0.659 |
| 3 | 11.1 | 3.70 | 0.415 | 0.566 |
| 3 | 33.3 | 11.1 | 0.650 | 0.579 |
| 1 | 3.70 | 3.70 | 0.233 | 0.864 |
| 1 | 11.1 | 11.1 | 0.573 | 0.513 |
| 1 | 33.3 | 33.3 | 0.774 | 0.555 |
| 0.333 | 3.70 | 11.1 | 0.484 | 0.599 |
| 0.333 | 11.1 | 33.3 | 0.713 | 0.633 |
| 0.111 | 1.23 | 11.1 | 0.452 | 0.624 |
| 0.111 | 3.70 | 33.3 | 0.662 | 0.747 |
| 0.0370 | 0.412 | 11.1 | 0.435 | 0.649 |
| 0.0370 | 1.23 | 33.3 | 0.643 | 0.793 |
| 0.0123 | 0.137 | 11.1 | 0.390 | 0.779 |
| 0.0123 | 0.412 | 33.3 | 0.625 | 0.852 |
| 0.00412 | 0.0457 | 11.1 | 0.409 | 0.714 |
| 0.00412 | 0.137 | 33.3 | 0.643 | 0.780 |
| 0.00137 | 0.0152 | 11.1 | 0.392 | 0.769 |
| 0.000457 | 0.0152 | 33.3 | 0.618 | 0.872 |

[Table 30]

| Cell line: | SK-BR-3 | | | |
|---|---|---|---|---|
| Combined use ratio | GDC-0032 (nM) | Example 11 (nM) | Fa | CI |
| 729 | 100 | 0.137 | 0.505 | 0.239 |
| 243 | 33.3 | 0.137 | 0.408 | 0.230 |
| 243 | 100 | 0.412 | 0.559 | 0.140 |
| 81 | 11.1 | 0.137 | 0.277 | 0.373 |
| 81 | 33.3 | 0.412 | 0.358 | 0.420 |
| 81 | 100 | 1.23 | 0.531 | 0.210 |
| 27 | 11.1 | 0.412 | 0.376 | 0.135 |
| 27 | 33.3 | 1.23 | 0.519 | 0.101 |
| 27 | 100 | 3.70 | 0.566 | 0.195 |
| 9 | 3.70 | 0.412 | 0.291 | 0.155 |
| 9 | 11.1 | 1.23 | 0.317 | 0.349 |
| 9 | 33.33 | 3.70 | 0.460 | 0.277 |
| 9 | 100 | 11.1 | 0.680 | 0.130 |
| 3 | 11.1 | 3.70 | 0.396 | 0.318 |
| 3 | 33.3 | 11.1 | 0.574 | 0.245 |
| 3 | 100 | 33.3 | 0.759 | 0.159 |
| 1 | 11.1 | 11.1 | 0.501 | 0.370 |
| 1 | 33.3 | 33.3 | 0.664 | 0.340 |
| 1 | 100 | 100 | 0.800 | 0.304 |
| 0.333 | 3.70 | 11.1 | 0.440 | 0.535 |
| 0.333 | 11.1 | 33.3 | 0.560 | 0.699 |
| 0.333 | 33.3 | 100 | 0.752 | 0.481 |
| 0.111 | 1.23 | 11.1 | 0.392 | 0.729 |
| 0.111 | 3.70 | 33.3 | 0.632 | 0.415 |
| 0.111 | 11.1 | 100 | 0.746 | 0.501 |
| 0.0370 | 1.23 | 33.3 | 0.604 | 0.505 |
| 0.0370 | 3.70 | 100 | 0.688 | 0.813 |
| 0.0123 | 0.137 | 11.1 | 0.383 | 0.770 |
| 0.0123 | 0.412 | 33.3 | 0.652 | 0.357 |
| 0.0123 | 1.23 | 100 | 0.691 | 0.792 |
| 0.00412 | 0.137 | 33.3 | 0.555 | 0.709 |
| 0.00412 | 0.412 | 100 | 0.708 | 0.695 |
| 0.00137 | 0.046 | 33.3 | 0.578 | 0.607 |
| 0.00137 | 0.137 | 100 | 0.735 | 0.550 |
| 0.000457 | 0.0152 | 33.3 | 0.545 | 0.759 |
| 0.000457 | 0.0457 | 100 | 0.717 | 0.641 |

(continued)

| Cell line: | SK-BR-3 | | | |
|---|---|---|---|---|
| Combined use ratio | GDC-0032 (nM) | Example 11 (nM) | Fa | CI |
| 0.000152 | 0.0152 | 100 | 0.699 | 0.746 |

[Table 31]

| Cell line: | HCC827 | | | |
|---|---|---|---|---|
| Combined use ratio | GDC-0032 (nM) | Example 11 (nM) | Fa | CI |
| 243 | 33.3 | 0.137 | 0.448 | 0.733 |
| 81 | 11.1 | 0.137 | 0.281 | 0.867 |
| 27 | 3.70 | 0.137 | 0.294 | 0.265 |
| 27 | 11.1 | 0.412 | 0.328 | 0.607 |
| 27 | 33.3 | 1.235 | 0.456 | 0.726 |
| 9 | 100 | 11.1 | 0.719 | 0.404 |
| 3 | 11.1 | 3.70 | 0.414 | 0.451 |
| 1 | 11.1 | 11.1 | 0.383 | 0.871 |
| 1 | 33.3 | 33.3 | 0.678 | 0.533 |
| 0.333 | 3.70 | 11.1 | 0.445 | 0.453 |
| 0.333 | 11.1 | 33.3 | 0.606 | 0.631 |
| 0.111 | 1.23 | 11.1 | 0.314 | 0.748 |
| 0.111 | 3.70 | 33.3 | 0.545 | 0.761 |
| 0.111 | 11.1 | 100 | 0.761 | 0.774 |
| 0.0370 | 3.70 | 100 | 0.739 | 0.866 |
| 0.0123 | 0.412 | 33.3 | 0.528 | 0.778 |
| 0.00412 | 0.0457 | 11.1 | 0.262 | 0.894 |
| 0.000457 | 0.0152 | 33.3 | 0.593 | 0.582 |

Combined use of compound of Example 11 and dactolisib (BEZ235)

[Table 32]

| Cell line: | BT-474 | | | |
|---|---|---|---|---|
| Combined use ratio | BEZ235 (nM) | Example 11 (nM) | Fa | CI |
| 729 | 100 | 0.137 | 0.531 | 0.821 |
| 243 | 33.3 | 0.137 | 0.441 | 0.599 |
| 243 | 100 | 0.412 | 0.543 | 0.749 |
| 81 | 33.3 | 0.412 | 0.434 | 0.652 |
| 81 | 100 | 1.23 | 0.530 | 0.869 |
| 27 | 33.3 | 1.23 | 0.484 | 0.466 |
| 27 | 100 | 3.70 | 0.559 | 0.766 |

(continued)

| Cell line: | BT-474 | | | |
|---|---|---|---|---|
| Combined use ratio | BEZ235 (nM) | Example 11 (nM) | Fa | CI |
| 9 | 33.3 | 3.70 | 0.482 | 0.589 |
| 9 | 100 | 11.1 | 0.607 | 0.728 |
| 9 | 300 | 33.3 | 0.776 | 0.627 |
| 3 | 33.3 | 11.1 | 0.552 | 0.617 |
| 3 | 100 | 33.3 | 0.748 | 0.577 |
| 1 | 11.1 | 11.1 | 0.522 | 0.543 |
| 1 | 33.3 | 33.3 | 0.699 | 0.671 |
| 0.333 | 3.7 | 11.1 | 0.426 | 0.739 |
| 0.333 | 11.1 | 33.3 | 0.654 | 0.789 |
| 0.111 | 1.23 | 11.1 | 0.386 | 0.824 |
| 0.111 | 3.70 | 33.3 | 0.641 | 0.817 |
| 0.0370 | 0.412 | 11.1 | 0.393 | 0.774 |
| 0.0370 | 1.23 | 33.3 | 0.633 | 0.839 |
| 0.0123 | 0.137 | 11.1 | 0.374 | 0.836 |
| 0.0123 | 0.412 | 33.3 | 0.635 | 0.828 |
| 0.00412 | 0.0457 | 11.1 | 0.403 | 0.734 |
| 0.00412 | 0.137 | 33.3 | 0.632 | 0.839 |
| 0.00137 | 0.0457 | 33.3 | 0.626 | 0.860 |

[Table 33]

| Cell line: | SK-BR-3 | | | |
|---|---|---|---|---|
| Combined use ratio | BEZ235 (nM) | Example 11 (nM) | Fa | CI |
| 729 | 100 | 0.137 | 0.727 | 0.786 |
| 243 | 33.3 | 0.137 | 0.646 | 0.443 |
| 243 | 100 | 0.412 | 0.725 | 0.801 |
| 81 | 11.1 | 0.137 | 0.462 | 0.427 |
| 81 | 33.3 | 0.412 | 0.653 | 0.428 |
| 81 | 100 | 1.235 | 0.785 | 0.511 |
| 27 | 3.70 | 0.137 | 0.205 | 0.780 |
| 27 | 33.3 | 1.23 | 0.641 | 0.473 |
| 27 | 100 | 3.70 | 0.777 | 0.557 |
| 9 | 3.70 | 0.412 | 0.216 | 0.756 |
| 9 | 11.1 | 1.23 | 0.352 | 0.883 |
| 9 | 33.3 | 3.70 | 0.644 | 0.497 |
| 9 | 100 | 11.1 | 0.790 | 0.540 |
| 3 | 3.70 | 1.23 | 0.289 | 0.528 |

(continued)

| Cell line: | SK-BR-3 | | | |
|---|---|---|---|---|
| Combined use ratio | BEZ235 (nM) | Example 11 (nM) | Fa | CI |
| 3 | 11.1 | 3.70 | 0.391 | 0.838 |
| 3 | 33.3 | 11.1 | 0.675 | 0.493 |
| 1 | 11.1 | 11.1 | 0.569 | 0.461 |
| 1 | 33.3 | 33.3 | 0.753 | 0.433 |
| 0.333 | 3.70 | 11.1 | 0.487 | 0.486 |
| 0.333 | 11.1 | 33.3 | 0.620 | 0.688 |
| 0.111 | 3.70 | 33.3 | 0.634 | 0.528 |
| 0.111 | 11.1 | 100 | 0.739 | 0.802 |
| 0.037 | 0.412 | 11.1 | 0.341 | 0.873 |
| 0.0370 | 1.23 | 33.3 | 0.578 | 0.683 |
| 0.0123 | 0.412 | 33.3 | 0.623 | 0.512 |
| 0.00412 | 0.412 | 100 | 0.710 | 0.889 |
| 0.000457 | 0.0457 | 100 | 0.708 | 0.895 |

Combined use of compound of Example 11 and everolimus (RAD001)

[Table 34]

| Cell line: | BT-474 | | | |
|---|---|---|---|---|
| Combined use ratio | RAD001 (nM) | Example 11 (nM) | Fa | CI |
| 2700 | 10000 | 3.70 | 0.658 | 0.289 |
| 900 | 123 | 0.137 | 0.422 | 0.768 |
| 900 | 3333 | 3.70 | 0.584 | 0.534 |
| 300 | 41.2 | 0.137 | 0.410 | 0.378 |
| 300 | 123 | 0.412 | 0.438 | 0.550 |
| 300 | 1111 | 3.70 | 0.586 | 0.366 |
| 300 | 3333 | 11.1 | 0.768 | 0.321 |
| 100 | 13.7 | 0.137 | 0.404 | 0.166 |
| 100 | 41.2 | 0.412 | 0.428 | 0.280 |
| 100 | 123 | 1.23 | 0.444 | 0.597 |
| 100 | 370 | 3.70 | 0.533 | 0.484 |
| 100 | 1111 | 11.1 | 0.736 | 0.394 |
| 33.3 | 4.57 | 0.137 | 0.402 | 0.074 |
| 33.3 | 13.7 | 0.412 | 0.419 | 0.163 |
| 33.3 | 41.2 | 1.23 | 0.447 | 0.314 |
| 33.3 | 123 | 3.70 | 0.544 | 0.381 |
| 33.3 | 370 | 11.1 | 0.717 | 0.440 |

(continued)

| Cell line: | BT-474 | | | |
|---|---|---|---|---|
| Combined use ratio | RAD001 (nM) | Example 11 (nM) | Fa | CI |
| 11.1 | 4.57 | 0.412 | 0.439 | 0.083 |
| 11.1 | 13.7 | 1.23 | 0.460 | 0.206 |
| 11.1 | 41.2 | 3.70 | 0.520 | 0.414 |
| 11.1 | 123 | 11.1 | 0.723 | 0.424 |
| 3.70 | 4.57 | 1.23 | 0.460 | 0.186 |
| 3.70 | 13.7 | 3.70 | 0.534 | 0.376 |
| 3.70 | 41.2 | 11.1 | 0.705 | 0.470 |
| 1.23 | 4.57 | 3.70 | 0.545 | 0.355 |
| 1.23 | 13.7 | 11.1 | 0.718 | 0.436 |
| 0.412 | 4.57 | 11.1 | 0.709 | 0.461 |
| 0.412 | 13.7 | 33.3 | 0.796 | 0.795 |

[Table 35]

| Cell line: | SK-BR-3 | | | |
|---|---|---|---|---|
| Combined use ratio | RAD001 (nM) | Example 11 (nM) | Fa | CI |
| 72900 | 10000 | 0.137 | 0.669 | 0.444 |
| 24300 | 30000 | 1.23 | 0.795 | 0.039 |
| 2700 | 370 | 0.137 | 0.546 | 0.655 |
| 2700 | 3333 | 1.23 | 0.623 | 0.689 |
| 2700 | 10000 | 3.70 | 0.730 | 0.173 |
| 900 | 123 | 0.137 | 0.500 | 0.815 |
| 900 | 3333 | 3.70 | 0.644 | 0.501 |
| 300 | 41.2 | 0.137 | 0.533 | 0.117 |
| 300 | 123 | 0.412 | 0.550 | 0.224 |
| 300 | 370 | 1.23 | 0.579 | 0.330 |
| 300 | 1111 | 3.70 | 0.665 | 0.219 |
| 300 | 3333 | 11.1 | 0.798 | 0.235 |
| 100 | 13.7 | 0.137 | 0.462 | 0.276 |
| 100 | 41.2 | 0.412 | 0.512 | 0.228 |
| 100 | 123 | 1.23 | 0.554 | 0.261 |
| 100 | 370 | 3.70 | 0.596 | 0.377 |
| 100 | 1111 | 11.1 | 0.798 | 0.235 |
| 33.3 | 4.57 | 0.137 | 0.484 | 0.060 |
| 33.3 | 13.7 | 0.412 | 0.516 | 0.092 |
| 33.3 | 41.2 | 1.23 | 0.527 | 0.224 |
| 33.3 | 123 | 3.70 | 0.579 | 0.323 |

(continued)

| Cell line: | SK-BR-3 | | | |
|---|---|---|---|---|
| Combined use ratio | RAD001 (nM) | Example 11 (nM) | Fa | CI |
| 33.3 | 370 | 11.1 | 0.778 | 0.266 |
| 11.1 | 4.57 | 0.412 | 0.509 | 0.059 |
| 11.1 | 13.7 | 1.23 | 0.476 | 0.303 |
| 11.1 | 41.2 | 3.70 | 0.619 | 0.210 |
| 3.70 | 4.57 | 1.23 | 0.529 | 0.113 |
| 3.70 | 13.7 | 3.70 | 0.604 | 0.220 |
| 3.70 | 41.2 | 11.1 | 0.745 | 0.323 |
| 1.23 | 4.57 | 3.70 | 0.588 | 0.234 |
| 1.23 | 13.7 | 11.1 | 0.764 | 0.290 |
| 0.412 | 4.57 | 11.1 | 0.771 | 0.278 |

[Table 36]

| Cell line: | HCC827 | | | |
|---|---|---|---|---|
| Combined use ratio | RAD001 (nM) | Example 11 (nM) | Fa | CI |
| 24300 | 3333 | 0.137 | 0.217 | 0.619 |
| 2700 | 370 | 0.137 | 0.258 | 0.049 |
| 900 | 30000 | 33.3 | 0.636 | 0.196 |
| 300 | 1111 | 3.70 | 0.303 | 0.232 |
| 300 | 3333 | 11.1 | 0.329 | 0.554 |
| 300 | 10000 | 33.3 | 0.599 | 0.194 |
| 300 | 30000 | 100 | 0.771 | 0.126 |
| 100 | 370 | 3.70 | 0.270 | 0.252 |
| 100 | 3333 | 33.3 | 0.607 | 0.159 |
| 100 | 10000 | 100 | 0.686 | 0.253 |
| 33.3 | 123 | 3.70 | 0.245 | 0.290 |
| 33.3 | 370 | 11.1 | 0.395 | 0.242 |
| 33.3 | 1111 | 33.3 | 0.522 | 0.284 |
| 33.3 | 3333 | 100 | 0.720 | 0.180 |
| 11.1 | 123 | 11.1 | 0.418 | 0.198 |
| 11.1 | 370 | 33.3 | 0.588 | 0.172 |
| 11.1 | 1111 | 100 | 0.669 | 0.274 |
| 3.70 | 4.57 | 1.23 | 0.200 | 0.147 |
| 3.70 | 41.2 | 11.1 | 0.238 | 0.888 |
| 3.70 | 123 | 33.3 | 0.528 | 0.265 |
| 3.70 | 370 | 100 | 0.691 | 0.227 |
| 1.23 | 13.7 | 11.1 | 0.292 | 0.533 |

(continued)

| Cell line: | HCC827 | | | |
|---|---|---|---|---|
| Combined use ratio | RAD001 (nM) | Example 11 (nM) | Fa | CI |
| 1.23 | 41.2 | 33.3 | 0.532 | 0.257 |
| 1.23 | 123 | 100 | 0.693 | 0.223 |
| 0.412 | 4.57 | 11.1 | 0.282 | 0.584 |
| 0.412 | 13.7 | 33.3 | 0.532 | 0.258 |
| 0.412 | 41.2 | 100 | 0.710 | 0.193 |
| 0.137 | 4.57 | 33.3 | 0.479 | 0.378 |
| 0.137 | 13.7 | 100 | 0.693 | 0.223 |
| 0.0457 | 4.57 | 100 | 0.706 | 0.199 |

Combined use of compound of Example 11 and AZD8055

[Table 37]

| Cell line: | BT-474 | | | |
|---|---|---|---|---|
| Combined use ratio | AZD8055 (nM) | Example 11 (nM) | Fa | CI |
| 2430 | 333 | 0.137 | 0.599 | 0.678 |
| 810 | 111 | 0.137 | 0.480 | 0.515 |
| 810 | 333 | 0.412 | 0.569 | 0.844 |
| 270 | 37.0 | 0.137 | 0.345 | 0.454 |
| 270 | 111 | 0.412 | 0.502 | 0.456 |
| 270 | 333 | 1.23 | 0.584 | 0.786 |
| 90 | 37.0 | 0.412 | 0.269 | 0.858 |
| 90 | 111 | 1.23 | 0.503 | 0.484 |
| 90 | 333 | 3.70 | 0.578 | 0.889 |
| 30 | 37.0 | 1.23 | 0.399 | 0.368 |
| 30 | 111 | 3.70 | 0.477 | 0.678 |
| 30 | 333 | 11.1 | 0.624 | 0.843 |
| 10 | 37.0 | 3.70 | 0.365 | 0.616 |
| 10 | 111 | 11.1 | 0.599 | 0.529 |
| 10 | 333 | 33.3 | 0.744 | 0.728 |
| 3.33 | 12.3 | 3.70 | 0.251 | 0.694 |
| 3.33 | 37.0 | 11.1 | 0.514 | 0.549 |
| 3.33 | 111 | 33.3 | 0.718 | 0.666 |
| 1.11 | 12.3 | 11.1 | 0.435 | 0.624 |
| 1.11 | 37.0 | 33.3 | 0.674 | 0.733 |
| 0.370 | 4.12 | 11.1 | 0.378 | 0.708 |
| 0.370 | 12.3 | 33.3 | 0.625 | 0.854 |

(continued)

| Cell line: | BT-474 | | | |
|---|---|---|---|---|
| Combined use ratio | AZD8055 (nM) | Example 11 (nM) | Fa | CI |
| 0.123 | 1.37 | 11.1 | 0.332 | 0.818 |
| 0.123 | 4.12 | 33.3 | 0.632 | 0.817 |
| 0.0412 | 0.457 | 11.1 | 0.365 | 0.710 |
| 0.0412 | 1.37 | 33.3 | 0.610 | 0.884 |
| 0.0137 | 0.152 | 11.1 | 0.429 | 0.558 |
| 0.0137 | 0.457 | 33.3 | 0.624 | 0.836 |
| 0.00457 | 0.152 | 33.3 | 0.614 | 0.868 |

[Table 38]

| Cell line: | SK-BR-3 | | | |
|---|---|---|---|---|
| Combined use ratio | AZD8055 (nM) | Example 11 (nM) | Fa | CI |
| 810 | 111 | 0.137 | 0.746 | 0.529 |
| 270 | 37.0 | 0.137 | 0.603 | 0.379 |
| 270 | 111 | 0.412 | 0.765 | 0.469 |
| 90 | 123 | 0.137 | 0.332 | 0.466 |
| 90 | 37.0 | 0.412 | 0.564 | 0.457 |
| 90 | 111 | 1.23 | 0.772 | 0.448 |
| 30 | 12.3 | 0.412 | 0.351 | 0.431 |
| 30 | 37.0 | 1.23 | 0.626 | 0.341 |
| 30 | 111 | 3.70 | 0.752 | 0.512 |
| 10 | 12.3 | 1.23 | 0.440 | 0.286 |
| 10 | 37.0 | 3.70 | 0.636 | 0.328 |
| 3.33 | 4.12 | 1.23 | 0.381 | 0.148 |
| 3.33 | 12.3 | 3.70 | 0.438 | 0.319 |
| 3.33 | 37.0 | 11.1 | 0.690 | 0.253 |
| 1.11 | 0.457 | 0.412 | 0.224 | 0.090 |
| 1.11 | 4.12 | 3.70 | 0.217 | 0.875 |
| 1.11 | 12.3 | 11.1 | 0.531 | 0.233 |
| 1.11 | 37.0 | 33.3 | 0.793 | 0.137 |
| 0.370 | 0.152 | 0.412 | 0.211 | 0.082 |
| 0.370 | 4.12 | 11.1 | 0.358 | 0.443 |
| 0.370 | 12.35 | 33.3 | 0.653 | 0.145 |
| 0.123 | 1.37 | 11.1 | 0.286 | 0.769 |
| 0.123 | 4.12 | 33.3 | 0.608 | 0.117 |
| 0.041 | 0.457 | 11.1 | 0.276 | 0.817 |
| 0.0412 | 1.37 | 33.3 | 0.500 | 0.245 |

(continued)

| Cell line: | SK-BR-3 | | | |
|---|---|---|---|---|
| Combined use ratio | AZD8055 (nM) | Example 11 (nM) | Fa | CI |
| 0.0412 | 4.12 | 100 | 0.746 | 0.067 |
| 0.0137 | 0.152 | 11.1 | 0.268 | 0.883 |
| 0.0137 | 0.457 | 33.3 | 0.485 | 0.265 |
| 0.0137 | 1.37 | 100 | 0.728 | 0.066 |
| 0.00457 | 0.152 | 33.3 | 0.500 | 0.225 |
| 0.00457 | 0.457 | 100 | 0.736 | 0.056 |
| 0.00152 | 0.152 | 100 | 0.720 | 0.066 |

[Table 39]

| Cell line: | HCC827 | | | |
|---|---|---|---|---|
| Combined use ratio | AZD8055 (nM) | Example 11 (nM) | Fa | CI |
| 2430 | 333 | 0.137 | 0.794 | 0.815 |
| 810 | 111 | 0.137 | 0.684 | 0.637 |
| 270 | 37.0 | 0.137 | 0.512 | 0.621 |
| 270 | 111 | 0.412 | 0.660 | 0.749 |
| 90 | 12.3 | 0.137 | 0.392 | 0.434 |
| 90 | 37.0 | 0.412 | 0.477 | 0.776 |
| 90 | 111 | 1.23 | 0.741 | 0.432 |
| 30 | 12.3 | 0.412 | 0.313 | 0.764 |
| 30 | 37.0 | 1.23 | 0.540 | 0.564 |
| 30 | 111 | 3.70 | 0.792 | 0.297 |
| 10 | 12.3 | 1.23 | 0.331 | 0.794 |
| 10 | 37.0 | 3.70 | 0.659 | 0.309 |
| 3.33 | 12.3 | 3.70 | 0.456 | 0.510 |
| 3.33 | 37.0 | 11.1 | 0.760 | 0.204 |
| 1.11 | 4.12 | 3.70 | 0.380 | 0.518 |
| 1.11 | 123 | 11.1 | 0.593 | 0.409 |
| 0.370 | 1.37 | 3.70 | 0.358 | 0.483 |
| 0.370 | 4.12 | 11.1 | 0.506 | 0.564 |
| 0.370 | 12.3 | 33.3 | 0.773 | 0.259 |
| 0.123 | 0.457 | 3.70 | 0.368 | 0.417 |
| 0.123 | 1.37 | 11.1 | 0.550 | 0.390 |
| 0.123 | 4.12 | 33.3 | 0.735 | 0.323 |
| 0.123 | 12.3 | 100 | 0.796 | 0.570 |
| 0.0412 | 0.152 | 3.70 | 0.380 | 0.373 |
| 0.0412 | 0.457 | 11.1 | 0.448 | 0.720 |

(continued)

| Cell line: | HCC827 | | | |
|---|---|---|---|---|
| Combined use ratio | AZD8055 (nM) | Example 11 (nM) | Fa | CI |
| 0.0412 | 1.37 | 33.3 | 0.675 | 0.491 |
| 0.0137 | 0.152 | 11.1 | 0.466 | 0.635 |
| 0.0137 | 0.457 | 33.3 | 0.642 | 0.614 |
| 0.00457 | 0.152 | 33.3 | 0.637 | 0.633 |

Combined use of compound of Example 11 and MK-2206

[Table 40]

| Cell line: | BT-474 | | | |
|---|---|---|---|---|
| Combined use ratio | MK-2206 (nM) | Example 11 (nM) | Fa | CI |
| 8100 | 1111 | 0.137 | 0.509 | 0.551 |
| 2700 | 370 | 0.137 | 0.443 | 0.346 |
| 2700 | 1111 | 0.412 | 0.489 | 0.679 |
| 900 | 123 | 0.137 | 0.321 | 0.395 |
| 900 | 370 | 0.412 | 0.389 | 0.597 |
| 900 | 1111 | 1.23 | 0.529 | 0.494 |
| 900 | 10000 | 11.1 | 0.739 | 0.590 |
| 300 | 123 | 0.412 | 0.290 | 0.588 |
| 300 | 370 | 1.23 | 0.430 | 0.449 |
| 300 | 1111 | 3.70 | 0.550 | 0.483 |
| 300 | 3333 | 11.1 | 0.684 | 0.459 |
| 100 | 123 | 1.23 | 0.301 | 0.608 |
| 100 | 370 | 3.70 | 0.473 | 0.419 |
| 100 | 1111 | 11.1 | 0.630 | 0.391 |
| 100 | 3333 | 33.3 | 0.776 | 0.372 |
| 33.3 | 41.2 | 1.23 | 0.228 | 0.596 |
| 33.3 | 123 | 3.70 | 0.411 | 0.375 |
| 33.3 | 370 | 11.1 | 0.592 | 0.352 |
| 33.3 | 1111 | 33.3 | 0.752 | 0.368 |
| 11.1 | 41.2 | 3.70 | 0.281 | 0.651 |
| 11.1 | 123 | 11.1 | 0.567 | 0.341 |
| 11.1 | 370 | 33.3 | 0.723 | 0.412 |
| 3.70 | 41.2 | 11.1 | 0.496 | 0.459 |
| 3.70 | 123 | 33.3 | 0.699 | 0.462 |
| 1.23 | 13.7 | 11.1 | 0.476 | 0.489 |
| 1.23 | 41.2 | 33.3 | 0.701 | 0.449 |

(continued)

| Cell line: | BT-474 | | | |
|---|---|---|---|---|
| Combined use ratio | MK-2206 (nM) | Example 11 (nM) | Fa | CI |
| 1.23 | 123 | 100 | 0.793 | 0.730 |
| 0.412 | 4.57 | 11.1 | 0.460 | 0.523 |
| 0.412 | 13.7 | 33.3 | 0.674 | 0.521 |
| 0.137 | 1.52 | 11.1 | 0.453 | 0.539 |
| 0.137 | 4.57 | 33.3 | 0.663 | 0.555 |
| 0.137 | 13.7 | 100 | 0.792 | 0.734 |
| 0.0457 | 1.52 | 33.3 | 0.641 | 0.623 |
| 0.0457 | 4.57 | 100 | 0.775 | 0.833 |
| 0.0152 | 1.52 | 100 | 0.770 | 0.861 |

[Table 41]

| Cell line: | SK-BR-3 | | | |
|---|---|---|---|---|
| Combined use ratio | MK-2206 (nM) | Example 11 (nM) | Fa | CI |
| 24300 | 3333 | 0.137 | 0.591 | 0.880 |
| 8100 | 1111 | 0.137 | 0.550 | 0.524 |
| 8100 | 3333 | 0.412 | 0.601 | 0.767 |
| 2700 | 370 | 0.137 | 0.522 | 0.261 |
| 2700 | 1111 | 0.412 | 0.577 | 0.362 |
| 900 | 123 | 0.137 | 0.380 | 0.656 |
| 900 | 370 | 0.412 | 0.459 | 0.639 |
| 900 | 1111 | 1.23 | 0.626 | 0.193 |
| 900 | 10000 | 11.1 | 0.697 | 0.632 |
| 300 | 370 | 1.23 | 0.507 | 0.355 |
| 300 | 1111 | 3.70 | 0.596 | 0.337 |
| 300 | 3333 | 11.1 | 0.663 | 0.437 |
| 300 | 10000 | 33.3 | 0.720 | 0.662 |
| 100 | 123 | 1.23 | 0.393 | 0.598 |
| 100 | 370 | 3.70 | 0.562 | 0.225 |
| 100 | 1111 | 11.1 | 0.732 | 0.128 |
| 100 | 3333 | 33.3 | 0.730 | 0.392 |
| 100 | 10000 | 100 | 0.782 | 0.743 |
| 33.3 | 123 | 3.70 | 0.462 | 0.327 |
| 33.3 | 370 | 11.1 | 0.676 | 0.159 |
| 33.3 | 1111 | 33.3 | 0.744 | 0.295 |
| 33.3 | 3333 | 100 | 0.786 | 0.650 |
| 11.1 | 41.2 | 3.70 | 0.365 | 0.473 |

(continued)

| Cell line: | SK-BR-3 | | | |
|---|---|---|---|---|
| Combined use ratio | MK-2206 (nM) | Example 11 (nM) | Fa | CI |
| 11.1 | 123 | 11.1 | 0.543 | 0.321 |
| 11.1 | 370 | 33.3 | 0.740 | 0.286 |
| 3.70 | 41.2 | 11.1 | 0.510 | 0.334 |
| 3.70 | 123 | 33.3 | 0.717 | 0.322 |
| 1.23 | 13.7 | 11.1 | 0.374 | 0.654 |
| 1.23 | 41.2 | 33.3 | 0.635 | 0.499 |
| 1.23 | 123 | 100 | 0.763 | 0.719 |
| 0.412 | 13.7 | 33.3 | 0.627 | 0.516 |
| 0.412 | 41.2 | 100 | 0.736 | 0.853 |
| 0.137 | 4.57 | 33.3 | 0.555 | 0.728 |
| 0.0457 | 1.52 | 33.3 | 0.544 | 0.765 |

[Table 42]

| Cell line: | HCC827 | | | |
|---|---|---|---|---|
| Combined use ratio | MK-2206 (nM) | Example 11 (nM) | Fa | CI |
| 8100 | 1111 | 0.137 | 0.299 | 0.710 |
| 8100 | 3333 | 0.412 | 0.389 | 0.847 |
| 2700 | 1111 | 0.412 | 0.311 | 0.639 |
| 900 | 123 | 0.137 | 0.265 | 0.131 |
| 900 | 3333 | 3.70 | 0.446 | 0.549 |
| 900 | 10000 | 11.1 | 0.593 | 0.420 |
| 300 | 370 | 1.23 | 0.298 | 0.334 |
| 300 | 1111 | 3.70 | 0.420 | 0.292 |
| 300 | 3333 | 11.1 | 0.561 | 0.236 |
| 100 | 370 | 3.70 | 0.338 | 0.346 |
| 100 | 1111 | 11.1 | 0.529 | 0.168 |
| 100 | 3333 | 33.3 | 0.769 | 0.040 |
| 33.3 | 370 | 11.1 | 0.573 | 0.078 |
| 33.3 | 1111 | 33.3 | 0.739 | 0.041 |
| 33.3 | 3333 | 100 | 0.782 | 0.072 |
| 11.1 | 123 | 11.1 | 0.507 | 0.124 |
| 11.1 | 370 | 33.3 | 0.700 | 0.055 |
| 11.1 | 1111 | 100 | 0.790 | 0.055 |
| 3.70 | 41.2 | 11.1 | 0.377 | 0.400 |
| 3.70 | 123 | 33.3 | 0.718 | 0.043 |
| 1.23 | 41.2 | 33.3 | 0.634 | 0.103 |

(continued)

| Cell line: | HCC827 | | | |
|---|---|---|---|---|
| Combined use ratio | MK-2206 (nM) | Example 11 (nM) | Fa | CI |
| 1.23 | 123 | 100 | 0.790 | 0.051 |
| 0.412 | 13.7 | 33.3 | 0.510 | 0.334 |
| 0.412 | 41.2 | 100 | 0.744 | 0.092 |
| 0.137 | 4.57 | 33.3 | 0.519 | 0.304 |
| 0.137 | 13.7 | 100 | 0.702 | 0.149 |
| 0.0457 | 1.52 | 33.3 | 0.427 | 0.717 |
| 0.0457 | 4.57 | 100 | 0.690 | 0.171 |
| 0.0152 | 1.52 | 100 | 0.611 | 0.381 |

Combined use of compound of Example 11 and palbociclib

[Table 43]

| Cell line: | BT-474 | | | |
|---|---|---|---|---|
| Combined use ratio | Palbociclib (nM) | Example 11 (nM) | Fa | CI |
| 145800 | 20000 | 0.137 | 0.298 | 0.016 |
| 48600 | 20000 | 0.412 | 0.231 | 0.076 |
| 16200 | 20000 | 1.235 | 0.283 | 0.158 |
| 5400 | 20000 | 3.704 | 0.272 | 0.510 |
| 1800 | 20000 | 11.1 | 0.433 | 0.568 |
| 600 | 20000 | 33.3 | 0.664 | 0.458 |
| 200 | 6667 | 33.3 | 0.593 | 0.698 |
| 66.7 | 2222 | 33.3 | 0.563 | 0.827 |
| 66.7 | 6667 | 100 | 0.785 | 0.589 |
| 22.2 | 741 | 33.3 | 0.602 | 0.664 |
| 22.2 | 2222 | 100 | 0.752 | 0.759 |
| 7.41 | 247 | 33.3 | 0.611 | 0.631 |
| 2.47 | 82.3 | 33.3 | 0.588 | 0.719 |
| 2.47 | 247 | 100 | 0.776 | 0.634 |
| 0.823 | 27.4 | 33.3 | 0.608 | 0.642 |
| 0.823 | 82.3 | 100 | 0.777 | 0.627 |
| 0.274 | 3.05 | 11.1 | 0.363 | 0.852 |
| 0.274 | 9.14 | 33.3 | 0.582 | 0.744 |
| 0.274 | 27.4 | 100 | 0.756 | 0.740 |
| 0.0914 | 3.05 | 33.3 | 0.618 | 0.605 |
| 0.0914 | 9.14 | 100 | 0.753 | 0.753 |
| 0.0305 | 3.05 | 100 | 0.783 | 0.599 |

[Table 44]

| Cell line: | SK-BR-3 | | | |
|---|---|---|---|---|
| Combined use ratio | Palbociclib (nM) | Example 11 (nM) | Fa | CI |
| 145800 | 20000 | 0.137 | 0.685 | 0.039 |
| 48600 | 20000 | 0.412 | 0.679 | 0.046 |
| 16200 | 20000 | 1.23 | 0.693 | 0.048 |
| 5400 | 20000 | 3.70 | 0.710 | 0.065 |
| 1800 | 20000 | 11.1 | 0.759 | 0.101 |
| 600 | 20000 | 33.3 | 0.790 | 0.227 |
| 66.7 | 6667 | 100 | 0.742 | 0.888 |
| 7.41 | 247 | 33.3 | 0.523 | 0.841 |
| 2.47 | 27.4 | 11.1 | 0.310 | 0.741 |
| 2.47 | 82.3 | 33.3 | 0.510 | 0.885 |
| 0.274 | 9.14 | 33.3 | 0.510 | 0.886 |
| 0.0914 | 3.05 | 33.3 | 0.558 | 0.718 |

[Table 45]

| Cell line: | HCC827 | | | |
|---|---|---|---|---|
| Combined use ratio | Palbociclib (nM) | Example 11 (nM) | Fa | CI |
| 16200 | 2222 | 0.137 | 0.214 | 0.820 |
| 600 | 741 | 1.23 | 0.217 | 0.393 |
| 200 | 247 | 1.23 | 0.243 | 0.187 |
| 66.7 | 82.3 | 1.23 | 0.239 | 0.139 |
| 66.7 | 741 | 11.1 | 0.382 | 0.604 |
| 22.2 | 247 | 11.1 | 0.433 | 0.411 |
| 22.2 | 741 | 33.3 | 0.615 | 0.550 |
| 7.41 | 82.3 | 11.1 | 0.470 | 0.328 |
| 7.41 | 247 | 33.3 | 0.673 | 0.388 |
| 7.41 | 741 | 100 | 0.732 | 0.850 |
| 2.47 | 27.4 | 11.1 | 0.341 | 0.584 |
| 2.47 | 82.3 | 33.3 | 0.516 | 0.787 |
| 0.823 | 9.14 | 11.1 | 0.259 | 0.899 |
| 0.274 | 3.05 | 11.1 | 0.350 | 0.557 |
| 0.274 | 9.14 | 33.3 | 0.516 | 0.778 |
| 0.0914 | 3.05 | 33.3 | 0.490 | 0.872 |

Combined use of compound of Example 11 and Fulvestrant

[Table 46]

| Cell line: | BT-474 | | | |
|---|---|---|---|---|
| Combined use ratio | Fulvestrant (nM) | Example 11 (nM) | Fa | CI |
| 729000 | 100000 | 0.137 | 0.283 | 0.654 |
| 243000 | 33333 | 0.137 | 0.206 | 0.773 |
| 81000 | 11111 | 0.137 | 0.205 | 0.281 |
| 81000 | 33333 | 0.412 | 0.231 | 0.549 |
| 81000 | 100000 | 1.23 | 0.293 | 0.699 |
| 27000 | 11111 | 0.412 | 0.227 | 0.241 |
| 27000 | 33333 | 1.23 | 0.232 | 0.677 |
| 27000 | 100000 | 3.70 | 0.326 | 0.741 |
| 9000 | 11111 | 1.23 | 0.268 | 0.261 |
| 9000 | 33333 | 3.70 | 0.317 | 0.540 |
| 9000 | 100000 | 11.1 | 0.496 | 0.618 |
| 3000 | 3704 | 1.23 | 0.227 | 0.273 |
| 3000 | 11111 | 3.70 | 0.273 | 0.587 |
| 3000 | 33333 | 11.1 | 0.540 | 0.491 |
| 3000 | 100000 | 33.3 | 0.690 | 0.765 |
| 1000 | 3704 | 3.70 | 0.275 | 0.527 |
| 1000 | 11111 | 11.1 | 0.531 | 0.503 |
| 1000 | 33333 | 33.3 | 0.761 | 0.531 |
| 333 | 3704 | 11.1 | 0.555 | 0.456 |
| 333 | 11111 | 33.3 | 0.787 | 0.459 |
| 111 | 1235 | 11.1 | 0.453 | 0.686 |
| 111 | 3704 | 33.3 | 0.769 | 0.508 |
| 37.0 | 412 | 11.1 | 0.458 | 0.673 |
| 37.0 | 1235 | 33.3 | 0.734 | 0.614 |
| 12.3 | 137 | 11.1 | 0.447 | 0.703 |
| 12.3 | 412 | 33.3 | 0.707 | 0.702 |
| 4.12 | 45.7 | 11.1 | 0.430 | 0.754 |
| 4.12 | 137 | 33.3 | 0.710 | 0.693 |
| 1.37 | 15.2 | 11.1 | 0.460 | 0.667 |
| 1.37 | 45.7 | 33.3 | 0.674 | 0.820 |
| 0.457 | 15.2 | 33.3 | 0.708 | 0.700 |

[Table 47]

| Cell line: | SK-BR-3 | | | |
|---|---|---|---|---|
| Combined use ratio | Fulvestrant (nM) | Example 11 (nM) | Fa | CI |
| 729000 | 100000 | 0.137 | 0.283 | 0.706 |

(continued)

| Cell line: | SK-BR-3 | | | |
|---|---|---|---|---|
| Combined use ratio | Fulvestrant (nM) | Example 11 (nM) | Fa | CI |
| 243000 | 33333 | 0.137 | 0.242 | 0.451 |
| 81000 | 33333 | 0.412 | 0.273 | 0.314 |
| 81000 | 100000 | 1.23 | 0.295 | 0.705 |
| 27000 | 11111 | 0.412 | 0.357 | 0.056 |
| 27000 | 100000 | 3.70 | 0.402 | 0.344 |
| 9000 | 11111 | 1.23 | 0.350 | 0.118 |
| 9000 | 33333 | 3.70 | 0.335 | 0.400 |
| 9000 | 100000 | 11.1 | 0.470 | 0.474 |
| 3000 | 11111 | 3.70 | 0.254 | 0.596 |
| 3000 | 33333 | 11.1 | 0.489 | 0.385 |
| 3000 | 100000 | 33.3 | 0.616 | 0.571 |
| 1000 | 11111 | 11.1 | 0.467 | 0.421 |
| 1000 | 33333 | 33.3 | 0.646 | 0.476 |
| 333 | 1235 | 3.70 | 0.210 | 0.689 |
| 333 | 3704 | 11.1 | 0.482 | 0.385 |
| 333 | 11111 | 33.3 | 0.690 | 0.364 |
| 333 | 33333 | 100 | 0.753 | 0.720 |
| 111 | 412 | 3.7 | 0.241 | 0.529 |
| 111 | 1235 | 11.1 | 0.406 | 0.574 |
| 111 | 3704 | 33.3 | 0.637 | 0.498 |
| 111 | 11111 | 100 | 0.746 | 0.756 |
| 37.0 | 412 | 11.1 | 0.342 | 0.823 |
| 37.0 | 1235 | 33.3 | 0.615 | 0.563 |
| 37.0 | 3704 | 100 | 0.753 | 0.723 |
| 12.3 | 137 | 11.1 | 0.343 | 0.817 |
| 12.3 | 412 | 33.3 | 0.604 | 0.599 |
| 12.3 | 1235 | 100 | 0.746 | 0.756 |
| 4.12 | 137 | 33.3 | 0.594 | 0.631 |
| 4.12 | 412 | 100 | 0.726 | 0.868 |
| 1.37 | 15.2 | 11.1 | 0.329 | 0.886 |
| 1.37 | 45.7 | 33.3 | 0.565 | 0.740 |
| 1.37 | 137 | 100 | 0.738 | 0.802 |
| 0.457 | 15.2 | 33.3 | 0.605 | 0.596 |
| 0.457 | 45.7 | 100 | 0.732 | 0.833 |
| 0.152 | 15.2 | 100 | 0.748 | 0.746 |

[Table 48]

| Cell line: | HCC827 | | | |
|---|---|---|---|---|
| Combined use ratio | Fulvestrant (nM) | Example 11 (nM) | Fa | CI |
| 243000 | 33333 | 0.137 | 0.248 | 0.754 |
| 81000 | 11111 | 0.137 | 0.301 | 0.128 |
| 27000 | 11111 | 0.412 | 0.299 | 0.162 |
| 9000 | 11111 | 1.23 | 0.245 | 0.475 |
| 9000 | 33333 | 3.7 | 0.460 | 0.181 |
| 9000 | 100000 | 11 | 0.474 | 0.484 |
| 3000 | 11111 | 3.70 | 0.398 | 0.236 |
| 3000 | 33333 | 11.1 | 0.420 | 0.600 |
| 3000 | 100000 | 33.3 | 0.693 | 0.252 |
| 1000 | 3704 | 3.70 | 0.240 | 0.774 |
| 1000 | 11111 | 11.1 | 0.386 | 0.696 |
| 1000 | 33333 | 33.3 | 0.682 | 0.269 |
| 1000 | 100000 | 100 | 0.797 | 0.299 |
| 333 | 3704 | 11.1 | 0.392 | 0.648 |
| 333 | 11111 | 33.3 | 0.635 | 0.376 |
| 333 | 33333 | 100 | 0.725 | 0.573 |
| 111 | 412 | 3.70 | 0.227 | 0.778 |
| 111 | 1235 | 11.1 | 0.354 | 0.835 |
| 111 | 3704 | 33.3 | 0.600 | 0.480 |
| 111 | 11111 | 100 | 0.736 | 0.520 |
| 37.0 | 412 | 11.1 | 0.394 | 0.627 |
| 37.0 | 1235 | 33.3 | 0.522 | 0.806 |
| 37.0 | 3704 | 100 | 0.715 | 0.616 |
| 12.3 | 412 | 33.3 | 0.627 | 0.396 |
| 123 | 1235 | 100 | 0.675 | 0.842 |
| 4.12 | 45.7 | 11.1 | 0.362 | 0.784 |
| 4.12 | 137 | 33.3 | 0.553 | 0.653 |
| 4.12 | 412 | 100 | 0.677 | 0.828 |
| 1.37 | 45.7 | 33.3 | 0.600 | 0.476 |
| 0.457 | 15.2 | 33.3 | 0.588 | 0.519 |
| 0.152 | 15.2 | 100 | 0.678 | 0.824 |

Combined use of compound of Example 11 and cisplatin (CDDP)

[Table 49]

| Cell line: | HCC827 | | | |
|---|---|---|---|---|
| Combined use ratio | CDDP (nM) | Example 11 (nM) | Fa | CI |
| 24300 | 3333 | 0.137 | 0.744 | 0.685 |
| 8100 | 1111 | 0.137 | 0.576 | 0.775 |
| 8100 | 3333 | 0.412 | 0.726 | 0.793 |
| 2700 | 1111 | 0.412 | 0.589 | 0.714 |
| 900 | 370 | 0.412 | 0.393 | 0.865 |
| 900 | 1111 | 1.23 | 0.626 | 0.560 |
| 900 | 3333 | 3.70 | 0.750 | 0.662 |
| 300 | 1111 | 3.70 | 0.659 | 0.459 |
| 300 | 3333 | 11.1 | 0.785 | 0.496 |
| 100 | 370 | 3.70 | 0.466 | 0.593 |
| 100 | 1111 | 11.1 | 0.673 | 0.448 |
| 33.3 | 1111 | 33.3 | 0.762 | 0.270 |
| 11.1 | 370 | 33.3 | 0.623 | 0.407 |
| 3.70 | 41.2 | 11.1 | 0.346 | 0.597 |
| 3.70 | 123 | 33.3 | 0.616 | 0.296 |
| 3.70 | 370 | 100 | 0.763 | 0.288 |
| 1.23 | 13.7 | 11.1 | 0.368 | 0.436 |
| 1.23 | 41.2 | 33.3 | 0.589 | 0.302 |
| 1.23 | 123 | 100 | 0.767 | 0.236 |
| 0.412 | 13.7 | 33.3 | 0.534 | 0.409 |
| 0.412 | 41.2 | 100 | 0.746 | 0.267 |
| 0.137 | 4.57 | 33.3 | 0.523 | 0.430 |
| 0.137 | 13.7 | 100 | 0.716 | 0.335 |
| 0.0457 | 1.52 | 33.3 | 0.497 | 0.507 |
| 0.0457 | 4.57 | 100 | 0.696 | 0.388 |
| 0.0152 | 1.52 | 100 | 0.672 | 0.465 |

Combined use of compound of Example 11 and SN-38

[Table 50]

| Cell line: | HCC827 | | | |
|---|---|---|---|---|
| Combined use ratio | SN-38 (nM) | Example 11 (nM) | Fa | CI |
| 81 | 11.1 | 0.137 | 0.562 | 0.441 |
| 27 | 3.70 | 0.137 | 0.302 | 0.401 |
| 27 | 11.1 | 0.412 | 0.501 | 0.556 |
| 9 | 3.70 | 0.412 | 0.299 | 0.422 |
| 9 | 11.1 | 1.23 | 0.496 | 0.582 |

(continued)

| Cell line: | HCC827 | | | |
|---|---|---|---|---|
| Combined use ratio | SN-38 (nM) | Example 11 (nM) | Fa | CI |
| 3 | 3.70 | 1.23 | 0.369 | 0.344 |
| 3 | 11.1 | 3.70 | 0.517 | 0.579 |
| 1 | 3.70 | 3.70 | 0.275 | 0.680 |
| 1 | 11.1 | 11.1 | 0.588 | 0.528 |
| 0.333 | 3.70 | 11.1 | 0.401 | 0.606 |
| 0.333 | 11.1 | 33.3 | 0.686 | 0.496 |
| 0.111 | 3.70 | 33.3 | 0.546 | 0.641 |
| 0.0370 | 1.23 | 33.3 | 0.491 | 0.704 |
| 0.0370 | 3.70 | 100 | 0.748 | 0.530 |
| 0.0123 | 0.412 | 33.3 | 0.497 | 0.644 |
| 0.0123 | 1.23 | 100 | 0.664 | 0.809 |
| 0.00412 | 0.137 | 33.3 | 0.550 | 0.482 |
| 0.00412 | 0.412 | 100 | 0.674 | 0.743 |
| 0.00137 | 0.137 | 100 | 0.665 | 0.773 |
| 0.000457 | 0.0152 | 33.3 | 0.455 | 0.773 |
| 0.000457 | 0.0457 | 100 | 0.663 | 0.782 |
| 0.000152 | 0.0152 | 100 | 0.651 | 0.834 |

Form these results, it was shown that combined use of the compound of Example 11 which is the pyrimidine compound of the present invention with the antimetabolite gemcitabine, 5-fluorouracil, or trifluridine, the platinum drug cisplatin, the alkaloid drug paclitaxel, the topoisomerase inhibitor SN-38, the estrogen receptor inhibitor fulvestrant, the PI3K/AKT/mTOR signaling pathway inhibitor AZD8055, everolimus, dactolisib, buparlisib, taselisib, or MK-2206, or the CDK4/6 inhibitor palbociclib synergistically potentiates an antitumor effect.

Test Example 15 Combination *(in vivo)* of pyrimidine compound and trastuzumab

Human stomach cancer-derived tumor cells 4-1ST were obtained as fragment tumor from the Central Institute for Experimental Animals. Tumor was removed approximately 1 month after subcutaneous transplantation and passage in a 6 week old male nude mouse (BALB/cAJcl-nu/nu, CLEA Japan, Inc.), and a fragment of approximately 2 mm square was prepared. A transplantation needle was filled with one fragment and subcutaneously inserted from around the right last rib of each nude mouse so as to enter the right back. The tumor was pushed out by pressing the inner syringe and thereby transplanted. For the measurement of the tumor size, electronic calipers were used. The tumor of each animal was measured by sandwiching the major axis and the minor axis between the measuring surfaces of the electronic calipers (Days 0, 5, 8, 12 and 15). A tumor volume (TV) was calculated from this major axis and minor axis. A relative tumor volume change percentage (T/C) was calculated from the calculated tumor volume. TV and T/C were calculated according to the following equations:

$$\text{Tumor Volume (TV) (mm}^3\text{)} = \text{(Major axis, mm)} \times \text{(Minor axis, mm)} \times \text{(Minor axis, mm)}/2$$

$$\text{Relative tumor volume change percentage (T/C) (\%)} = \text{(Mean TV of the administration group)}/\text{(Mean TV of a control group)} \times 100$$

For the measurement of the body weight, an animal electronic balance was used. A body weight change percentage on the $n^{th}$ day (BWCn) from the body weight on 0th day (BW0) and the body weight on the $n^{th}$ day (BWn) was calculated according to the following equation:

$$\text{Body weight change percentage BWCn (\%)} = (\text{BWn - BW0})/\text{BW0} \times 100$$

Nude mice having TV of 50 to 300 $mm^3$ were selected and assigned to groups each involving 6 animals by the equal number method [MiSTAT (ver. 2.1)] such that the means of TV were equal among the groups. Trastuzumab (Herceptin, Roche) was administered from the tail vein once a day on the 1st and 8th days (Days 1 and 8). The compound of Example 11 was orally administered once a day, every day, for 14 days (Days 1 - 14). Each dose was set to the dose shown in the tables given below. An untreated group was used as a control.

The presence or absence of a combinatorial effect and toxicity was judged from the tumor sizes and the body weights on the grouping day (Day 0) and the judgement day (Day 15). During the administration period, the body weight was measured every day for calculation of the amount of the dosing solution.

The results are shown in the tables given below and Figure 11.

[Table 51]

| Group | Dose (mg/kg/day) | Treatment | Number of Mice | Number of Deaths | TV ($mm^3$, mean ± SE) |
|---|---|---|---|---|---|
| Control (Non-treatment) | – | – | 6 | 0 | 2041.3 ± 94.7 |
| Example 11 | 6.25 | Days 1-14, po | 6 | 0 | 448.7 ± 79.0 |
| Example 11 | 12.5 | Days 1-14, po | 6 | 0 | 62.0 ± 6.9 |
| Trastuzumab | 20 | Days 1, 8, iv | 6 | 0 | 1621.4 ± 273.5 |
| Trastuzumab | 40 | Days 1, 8, iv | 6 | 0 | 1518.8 ± 204.2 |
| Example 11 + Trastuzumab | 6.25 + 20 | Days 1-14, po + Days 1, 8, iv | 6 | 0 | 149.5 ± 62.6 |
| Example 11 + Trastuzumab | 12.5 + 20 | Days 1-14, po + Days 1, 8, iv | 6 | 0 | 7.4 ± 3.5 |
| Example 11 + Trastuzumab | 6.25 + 40 | Days 1-14, po + Days 1, 8, iv | 6 | 0 | 39.6 ± 8.6 |
| Example 11 + Trastuzumab | 12.5 + 40 | Days 1-14, po + Days 1, 8, iv | 6 | 0 | 17.3 ± 3.6 |

| Group | Dunnett's test vs Control | Aspin-Welch's *t*-test vs Example 11,Tmab | T/C (%) | BWC (%, Mean ± SE) |
|---|---|---|---|---|
| Control (Non-treatment) | – | – | 100.0 | 8.43 ± 1.38 |
| Example 11 | *** | – | 22.0 | 3.69 ± 0.75 |
| Example 11 | *** | – | 3.0 | 3.79 ± 1.07 |
| Trastuzumab | ns | – | 79.4 | 10.82 ± 0.90 |
| Trastuzumab | * | – | 74.4 | 9.22 ± 1.00 |
| Example 11 + Trastuzumab | *** | *, ** | 7.3 | 6.43 ± 1.11 |
| Example 11 + Trastuzumab | *** | ***, ** | 0.4 | 3.03 ± 1.09 |
| Example 11 + Trastuzumab | *** | **, *** | 1.9 | 5.76 ± 0.50 |
| Example 11 + Trastuzumab | *** | ***, *** | 0.8 | 4.81 ± 0.99 |

Abbreviations: Tmab, trastuzumab; po, per os; iv, intravenous; SE, standard error; ns, not significant; *, $p < 0.05$; **, $p < 0.01$; ***, $p < 0.001$

As a result of analyzing TV on the 15th day (Day 15) of each group by the Dunnett's test (Dunnett's test vs Control), it was shown that the single administration groups of trastuzumab (except for the 20 mg/kg/day administration group) or Example 11 and the combined administration groups of the trastuzumab/Example 11 administration groups (Example 11 + Trastuzumab) had significantly lower TV than that of the control group. As a result of further conducting analysis by the Aspin-Welch's t-test, it was shown that the trastuzumab/Example 11 (Example 11 + Trastuzumab) combined administration groups had significantly lower TV than that of the single administration groups of the trastuzumab groups or Example 11. The mean body weight change percentages (BWC) of the combined administration groups on the judgement day were free from the enhancement of toxicity as compared with the single administration groups of the trastuzumab groups or the Example 11 groups.

Test Example 16 Combination (*in vivo*) of pyrimidine compound, trastuzumab, and pertuzumab

Human stomach cancer-derived tumor cells 4-1ST were obtained as fragment tumor from the Central Institute for Experimental Animals. Tumor was removed approximately 1 month after subcutaneous transplantation and passage in a 6 week old male nude mouse (BALB/cAJcl-nu/nu, CLEA Japan, Inc.), and a fragment of approximately 2 mm square was prepared. A transplantation needle was filled with one fragment and subcutaneously inserted from around the right last rib of each nude mouse so as to enter the right back. The tumor was pushed out by pressing the inner syringe and thereby transplanted. For the measurement of the tumor size, electronic calipers were used. The tumor of each animal was measured by sandwiching the major axis and the minor axis between the measuring surfaces of the electronic calipers (Days 0, 5, 8, 12 and 15). A tumor volume (TV) was calculated from this major axis and minor axis. A relative tumor volume change percentage (T/C) was calculated from the calculated tumor volume. TV and T/C were calculated according to the following equations:

$$\text{Tumor Volume (TV) (mm}^3\text{)} = \text{(Major axis, mm)} \times \text{(Minor axis, mm)} \times \text{(Minor axis, mm)/2}$$

$$\text{Relative tumor volume change percentage (T/C) (\%)} = \text{(Mean TV of the administration group)/(Mean TV of a control group)} \times 100$$

For the measurement of the body weight, an animal electronic balance was used. A body weight change percentage on the $n^{th}$ day (BWCn) from the body weight on 0th day (BW0) and the body weight on the $n^{th}$ day (BWn) was calculated according to the following equation:

$$\text{Body weight change percentage BWCn (\%)} = \text{(BWn - BW0)/BW0} \times 100$$

Nude mice having TV of 100 to 300 $mm^3$ were selected and assigned to groups each involving 6 animals by the equal number method [MiSTAT (ver. 2.0)] such that the means of TV were equal among the groups. Trastuzumab (T-mab, Herceptin, Roche) and pertuzumab (P-mab, Perjeta, Roche) were administered from the tail vein once a day on the 1st day (Day 1). The compound of Example 11 was orally administered once a day, every day, for 14 days (Days 1 - 14). Each dose was set to the dose shown in the tables given below. An untreated group was used as a control.

The presence or absence of a combinatorial effect and toxicity was judged from the tumor sizes and the body weights on the grouping day (Day 0) and the judgement day (Day 15). During the administration period, the body weight was measured every day for calculation of the amount of the dosing solution.

When an individual of TV = 0 was found, a complete response (hereinafter, CR) rate was also set as an evaluation item. For the judgement of CR, clinical CR (cCR) based on TV = 0 and pathological CR (pCR) based on pathological test were used. The significance of difference in CR rate of the trastuzumab/pertuzumab/Example 11 triple combined administration groups (Example 11 + T-mab/P-mab) from the corresponding Example 11 single administration groups and trastuzumab/pertuzumab combined administration group at the same doses thereas was analyzed by the Fisher's exact test.

$$\text{CR rate} = \text{(The number of CR individuals in a group found to have CR)/(Total number of individuals in the group found to have CR)} \times 100$$

The results are shown in the tables given below and Figure 12.

[Table 52]

| Group | Dose (mg/kg/day) | Treatment | Number of Animals | Number of Death | TV ($mm^3$, mean ± SE) | Dunnett's test vs control | Aspin-Welch's *t*-test vs Example 11, T-mab/P-mab |
|---|---|---|---|---|---|---|---|
| Control | — | — | 6 | 0 | 1714.1 ± 175.9 | — | — |
| Example 11 | 6.25 | Days 1-14, po | 6 | 0 | 295.9 ± 67.3 | *** | — |
| Example 11 | 12.5 | Days 1-14, po | 6 | 0 | 31.9 ± 10.7 | *** | — |
| T-mab/P-mab | 10/20 | Day 1, iv / Day 1,iv | 6 | 0 | 52.8 ± 13.0 | *** | — |
| Example 11 + T-mab/P-mab | 6.25 + 10/20 | Days 1-14, po + Day 1, iv/Day 1, iv | 6 | 0 | 0.0 ± 0.0 | *** | ** , ** |
| Example 11 + T-mab/P-mab | 12.5 + 10/20 | Days 1-14, po + Day 1, iv/Day 1, iv | 6 | 0 | 0.0 ± 0.0 | *** | * , ** |

| Group | T/C (%) | cCR (%) (number of cCR animals) | Fisher's exact test vs Example 11, T-mab/P-mab | pCR (%) (number of pCR animals) | Fisher's exact test vs Example 11, T-mab/P-mab | BWC (%, Mean ± SE) |
|---|---|---|---|---|---|---|
| Control | 100.0 | — | — | — | — | 9.71 ± 2.18 |
| Example 11 | 17.3 | 0 (0) | — | 0 (0) | — | 5.93 ± 0.25 |
| Example 11 | 1.9 | 33 (2) | — | 33 (2) | — | 4.77 ± 1.14 |
| T-mab/P-mab | 3.1 | 17 (1) | — | 17 (1) | — | 8.70 ± 1.50 |
| Example 11 + T-mab/P-mab | 0.0 | 100 (6) | ** , * | 33 (2) | ns , ns | 6.68 ± 2.11 |
| Example 11 + T-mab/P-mab | 0.0 | 100 (6) | ns , * | 100 (6) | ns , * | 5.20 ± 0.85 |

Abbreviations: T-mab, Trastuzumab; P-mab, Pertuzumab; po, per os; iv, intravenous; SE, standard error; cCR, clinical CR; pCR, pathological CR; ns, not significant; *, $p < 0.05$; **, $p < 0.01$; ***, $p < 0.001$

As a result of analyzing TV on the 15th day (Day 15) of each group by the Dunnett's test (Dunnett's test vs Control), it was shown that the single administration groups of Example 11, the trastuzumab/pertuzumab double combined administration group (T-mab/P-mab), and the trastuzumab/pertuzumab/Example 11 triple combined administration groups (Example 11 + T-mab/P-mab) had significantly lower TV than that of the control group. As a result of further conducting analysis by the Aspin-Welch's t-test, it was shown that the trastuzumab/pertuzumab/Example 11 triple combined administration groups had significantly lower TV than that of the single administration groups of Example 11, and the trastuzumab/pertuzumab double combined administration group. The mean body weight change percentages (BWC) of the trastuzumab/pertuzumab/Example 11 triple combined administration groups on the judgement day were free from the enhancement of toxicity as compared with the single administration groups of Example 11 group, and the trastuzumab/pertuzumab double combined administration group.

Test Example 17 Combination (*in vivo*) of pyrimidine compound and trastuzumab emtansine

The experiment was conducted in the same manner as in Test Example 15 except that trastuzumab emtansine (Kadcyla, Roche) was used instead of trastuzumab and administered from the tail vein once a day on the 1 st day (Day 1). Each dose was set to the dose shown in the tables given below. An untreated group was used as a control.

The results are shown in the tables given below and Figure 13.

[Table 53]

| Drug | Dose (mg/kg/day) | Treatment | Number of Animals | Number of Death | TV ($mm^3$, Mean ± SE) |
|---|---|---|---|---|---|
| Control (non-treatment) | – | – | 6 | 0 | 1609.5 ± 138.9 |
| Example 11 | 6.25 | Days 1-14, po | 6 | 0 | 233.6 ± 42.6 |
| Trastuzumab emtansine | 1.88 | Day 1, iv | 6 | 0 | 1183.2 ± 229.0 |
| Example 11 + Trastuzumab emtansine | 6.25 + 1.88 | Days 1-14, po + Day 1, iv | 6 | 0 | 115.9 ± 11.2 |

| Drug | *p*-value (Dunnetts' test) vs Control | *p*-value (Aspin-Welch's *t*-test) vs an Example 11 monotherapy | *p*-value (Aspin-Welch's *t*-test) vs a Trastuzumab emtansine monotherapy | T/C (%) | BWC (%, Mean ± SE) |
|---|---|---|---|---|---|
| Control (non-treatment) | – | – | – | 100.0 | 10.4 ± 2.2 |
| Example 11 | *** | – | – | 14.5 | 4.0 ± 0.7 |
| Trastuzumab emtansine | * | – | – | 73.5 | 8.2 ± 1.1 |
| Example 11 + Trastuzumab emtansine | *** | * | ** | 7.2 | 7.4 ± 1.1 |

Abbreviations: po, per os; iv, intravenous; SE, standard error; *, $p < 0.05$; **, $p < 0.01$; ***, $p < 0.001$.

As a result of analyzing TV on the 15th day (Day 15) of each group by the Dunnett's test (Dunnett's test vs Control), it was shown that the single administration group of the trastuzumab emtansine group or the Example 11 group and the combined administration group of the trastuzumab emtansine/Example 11 group (Trastuzumab emtansine + Example 11) had significantly lower TV than that of the control group. As a result of further conducting analysis by the Aspin-Welch's t-test, it was shown that the combined administration group of the trastuzumab emtansine/Example 11 group had significantly lower TV than that of the single administration group of the trastuzumab emtansine group or the Example 11 group. The mean body weight change percentage (BWC) of the combined administration group on the judgement day was free from the enhancement of toxicity as compared with the single administration group of the trastuzumab emtansine group or the Example 11 group.

Test Example 18 Combination (*in vivo*) of pyrimidine compound and capecitabine

A human stomach cancer cell line NCI-N87 was obtained from American Type Culture Collection (ATCC). The cell line was cultured in RPMI-1640 (containing 4.5 g/L glucose, 10 mM HEPES and 1 mM sodium pyruvate) (FUJIFILM Wako Pure Chemical Corporation) medium containing 10% fetal bovine serum (FBS) in a 5% $CO_2$ incubator at 37°C.

The NCI-N87 cells were resuspended at a concentration of $8 \times 10^7$ cells/mL in PBS. The cell suspension was subcutaneously transplanted at $8 \times 10^6$ cells/0.1 mL to the right chest of each 6 week old nude mouse (BALB/cAJcl-nu/nu, CLEA Japan, Inc.) using a 1 mL syringe for tuberculin and a 25 G injection needle.

For the measurement of the tumor size, electronic calipers were used. The major axis and minor axis of the tumor were measured, and TV and T/C were calculated according to the equations mentioned above.

For the measurement of the body weight, an animal electronic balance was used. A body weight change percentage on the $n^{th}$ day (BWCn) from the body weight on 0th day (BW0) and the body weight on the $n^{th}$ day (BWn) was calculated according to the equation mentioned above.

Nude mice having TV of 100 to 300 $mm^3$ were selected and assigned to groups each involving 6 animals by the equal number method [MiSTAT (ver. 2.0)] such that the means of TV were equal among the groups. Capecitabine (Tokyo Chemical Industry Co., Ltd.) and the compound of Example 11 were orally administered once a day, every day, for 14 days (Days 1 - 14). Each dose was set to the dose shown in the tables given below. An aqueous solution containing 0.1 N HCl and 0.5% HPMC was used as a control.

The presence or absence of a combinatorial effect and toxicity was judged from the tumor sizes and the body weights on the grouping day (Day 0) and the judgement day (Day 15). During the administration period, the body weight was measured every day for calculation of the amount of the dosing solution.

The results are shown in the following tables and Figures 14 and 15.

[Table 54]

| Group | Dose (mg/kg/day) | Schedule (day) | Route | Number of Animals | Number of Death | Tumor volume ($mm^3$) day 15 Mean ± S.E. |
|---|---|---|---|---|---|---|
| Control | - | 1-14 | p.o. | 6 | 0 | 581.47 ± 44.61 |
| Example 11 | 6.25 | 1-14 | p.o. | 6 | 0 | 272.43 ± 22.22 |
| Capecitabine | 359 | 1-14 | p.o. | 6 | 0 | 250.52 ± 18.71 |
| Capecitabine | 809 | 1-14 | p.o. | 6 | 2 | 224.76 ± 27.37 |
| Example 11 + Capecitabine | 6.25+359 | 1-14 | p.o. | 6 | 0 | 121.93 ± 13.68 |
| Example 11 + Capecitabine | 6.25+809 | 1-14 | p.o. | 6 | 0 | 101.34 ± 6.74 |

| Group | P-value | | | | T/C | BWC(%) |
|---|---|---|---|---|---|---|
| | Dunnett's | Aspin-Welch's test | | | | |
| | v.s. Control | v.s. Example 11 6.25 mg/kg/day | v.s. Cape 359 mg/kg/day | v.s. Cape 809 mg/kg/day | (%) | Mean ± S.E. |
| Control | - | - | - | - | 100 | 6.7 ± 1.8 |
| Example 11 | *** | - | ns | ns | 47 | 6.3 ± 0.9 |
| Capecitabine | *** | ns | - | ns | 43 | 5.7 ± 1.3 |
| Capecitabine | *** | ns | ns | - | 39 | 3.5 ± 4.5 |
| Example 11 + Capecitabine | *** | *** | *** | * | 21 | 3.6 ± 1.3 |
| Example 11 + Capecitabine | *** | *** | *** | * | 17 | -5.4 ± 4.9 |

*: p< 0.05 **: p< 0.01 ***: p< 0.001, ns:not significant (p>0.05)

As a result of analyzing TV on the 15th day (Day 15) of each group by the Dunnett's test (Dunnett's test vs Control), it was shown that the single administration groups of the capecitabine groups or the Example 11 group and the combined administration groups of the Example 11/capecitabine groups (Example 11 + Capecitabine) had significantly lower TV than that of the control group. It was further shown that the combined administration groups of the Example 11/capecitabine groups had significantly lower TV than that of the single administration groups of the capecitabine groups or the Example 11 group. The mean body weight change percentages (BWC) of the combined administration groups on the judgement day were free from the enhancement of toxicity as compared with the single administration groups of the capecitabine groups or the Example 11 group.

It is to be noted that all documents and publications cited in the present description are incorporated herein by reference in their entirety, regardless of the purpose thereof. Moreover, the present description includes the contents disclosed in the claims, specification and drawings of Japanese Patent Application No. 2020-121733 (filed on July 15, 2020), from which the present application claims priority.

Several embodiments of the present invention are described above. However, these embodiments are provided for illustrative purpose only, and thus, are not intended to limit the scope of the present invention. These novel embodiments can be carried out in various other forms, and various abbreviations, substitutions and alternations can be carried out, unless they are deviated from the spirit of the invention. These embodiments and the modifications thereof are included in the scope or spirit of the invention, and are also included in the invention according to the claims and the

scope equivalent thereto.

## Claims

1. An antitumor agent for combined administration with other antitumor agent, the antitumor agent comprising a pyrimidine compound represented by the following formula (I), or a salt thereof:

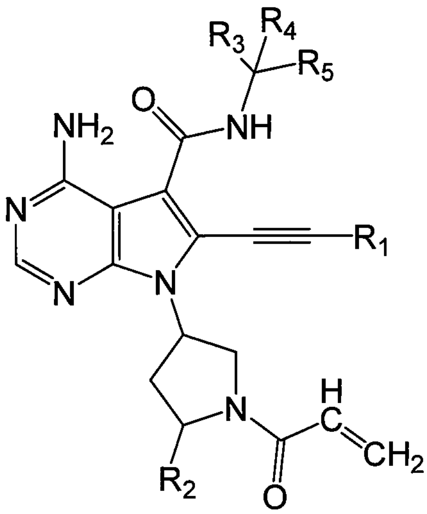

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

2. The antitumor agent according to claim 1, wherein the pyrimidine compound is a compound represented by the following formula (II):

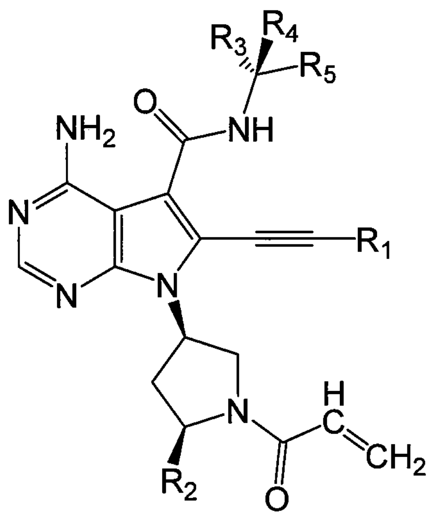

(II)

wherein $R_1$ represents a C1-C4 alkyl group optionally having a C1-C4 alkoxy group as a substituent, or a C3-C4

cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**3.** The antitumor agent according to claim 1 or 2, wherein the pyrimidine compound is represented by the formula (I) or (II) wherein

$R_1$ is a methyl group, an ethyl group, an isopropyl group, a tert-butyl group, a 1-methyl-1-methoxyethyl group, or a cyclopropyl group;
$R_2$ is a methyl group, an ethyl group, a methoxymethyl group, or an ethoxymethyl group; $R_3$ is a methyl group, an ethyl group, or a trifluoromethyl group;
$R_4$ is a hydrogen atom or a methyl group; and
$R_5$ is a phenyl group, a 2-fluorophenyl group, a 3-chlorophenyl group, a 2,3-difluorophenyl group, a 2,4-difluorophenyl group, or a 3,5-difluorophenyl group.

**4.** The antitumor agent according to any one of claims 1 to 3, wherein the pyrimidine compound is represented by the formula (I) or (II) wherein

$R_1$ is a methyl group, a tert-butyl group, or a cyclopropyl group;
$R_2$ is a methyl group, an ethyl group, or a methoxymethyl group;
$R_3$ is a methyl group;
$R_4$ is a hydrogen atom; and
$R_5$ is a phenyl group.

**5.** The antitumor agent according to any one of claims 1 to 4, wherein the pyrimidine compound is a compound selected from the following (1) to (3):

(1) 7-((3R,5S)-1-acryloyl-5-methylpyrrolidin-3-yl)-4-amino-N-((R)-1-phenylethyl)-6-(prop-1-yn-1-yl)-7H-pyrrolo[2,3-d]pyrimidine-5-carboxamide,
(2) 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide, and
(3) 7-((3R,5S)-1-acryloyl-5-methylpyrrolidin-3-yl)-4-amino-6-(3,3-dimethylbut-1-yn-1-yl)-N-((R)-1 -phenylethyl)-7H-pyrrolo [2,3 -d]pyrimidine-5 -carboxamide.

**6.** The antitumor agent according to any one of claims 1 to 5, wherein the other antitumor agent is at least one agent selected from antimetabolites, molecular targeting drugs, platinum drugs and alkaloid drugs.

**7.** The antitumor agent according to any one of claims 1 to 6, wherein the other antitumor agent is at least one agent selected from 5-fluorouracil (5-FU), trifluridine, gemcitabine, capecitabine, trastuzumab, pertuzumab, trastuzumab emtansine, AZD8055, everolimus, dactolisib, buparlisib, taselisib, palbociclib, fulvestrant, cisplatin and paclitaxel.

**8.** The antitumor agent according to any one of claims 1 to 7 for the treatment of tumor.

**9.** The antitumor agent according to any one of claims 1 to 8, wherein the pyrimidine compound or the salt thereof is administered to a tumor patient given the other antitumor agent or a tumor patient to be given the other antitumor agent.

**10.** The antitumor agent according to any one of claims 1 to 9 for oral administration.

**11.** A pharmaceutical combination of a pyrimidine compound or a salt thereof and other antitumor agent, wherein

the pyrimidine compound is a compound represented by the following formula (I):

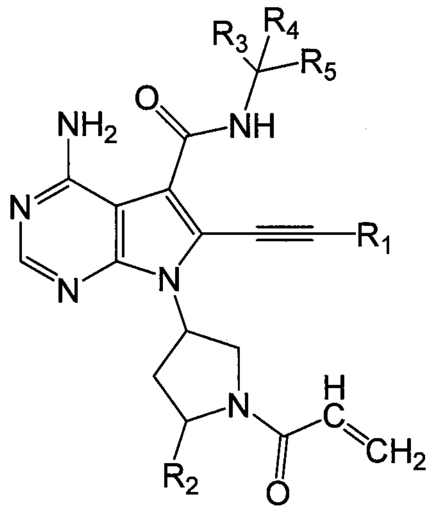

(I)

wherein $R_1$ represents a C1-C4 alkyl group optionally having a C1-C4 alkoxy group as a substituent, or a C3-C4 cycloalkyl group;
$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**12.** The pharmaceutical combination according to claim 11 for use in the treatment of tumor, wherein the pyrimidine compound or the salt thereof and the other antitumor agent are administered concurrently, sequentially, or at an interval.

**13.** A kit preparation comprising an antitumor agent according to any one of claims 1 to 10 or a pharmaceutical combination according to claim 11 or 12, and an instruction stating that the pyrimidine compound or the salt thereof and the other antitumor agent are combined-administered.

**14.** Use of a pyrimidine compound or a salt thereof for the production of a medicament that is used in combination with other antitumor agent in the treatment of tumor, wherein

the pyrimidine compound is a compound represented by the following formula (I):

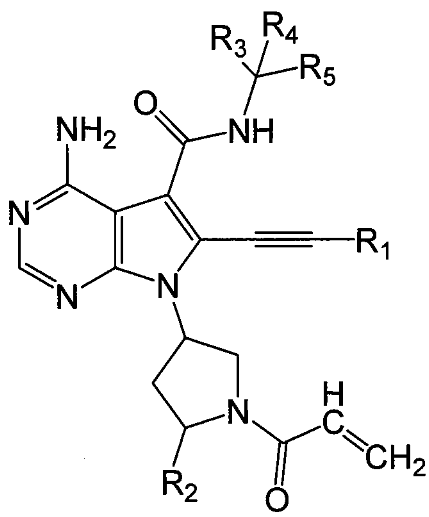

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**15.** An antitumor effect potentiator for potentiating the antitumor effect of other antitumor agent, the antitumor effect potentiator comprising a pyrimidine compound represented by the following formula (I), or a salt thereof:

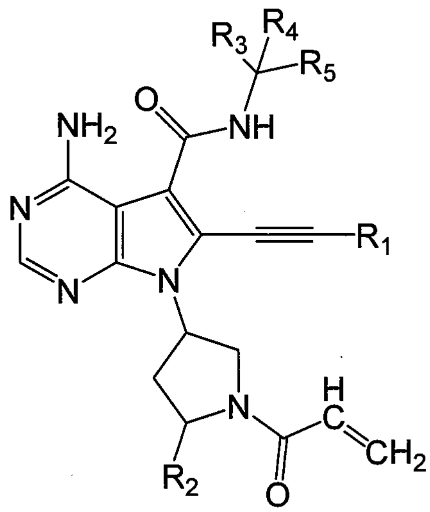

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**16.** A method for treating tumor, comprising administering an effective amount of an antitumor agent according to any one of claims 1 to 10 and other antitumor agent or a pharmaceutical combination according to claim 11 or 12 to a patient in need thereof.

**17.** A method for treating tumor by combined use with other antitumor agent, comprising administering an effective amount of a pyrimidine compound or a salt thereof to a patient in need thereof, wherein

the pyrimidine compound is a compound represented by the following formula (I):

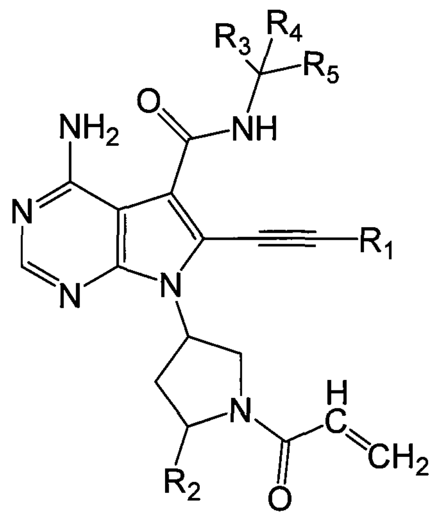

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**18.** A pyrimidine compound or a salt thereof for use in a method for treating tumor by combined use with other antitumor agent, wherein the pyrimidine compound is a compound represented by the following formula (I):

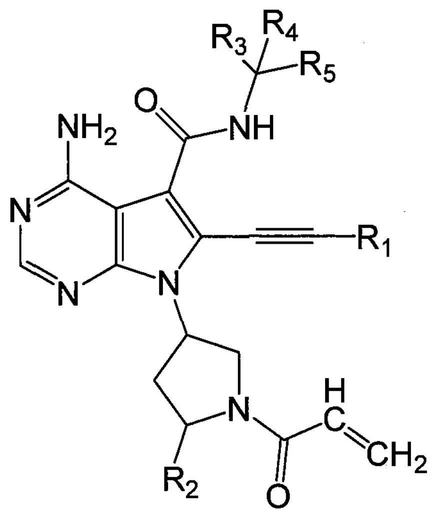

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**19.** Use of a pyrimidine compound or a salt thereof for treating tumor by combined use with other antitumor agent, wherein the pyrimidine compound is a compound represented by the following formula (I):

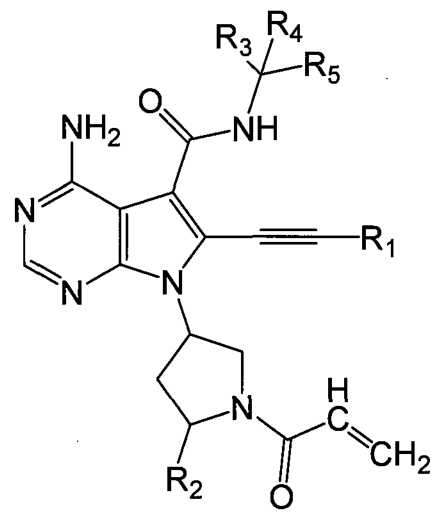

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

[Figure 1]

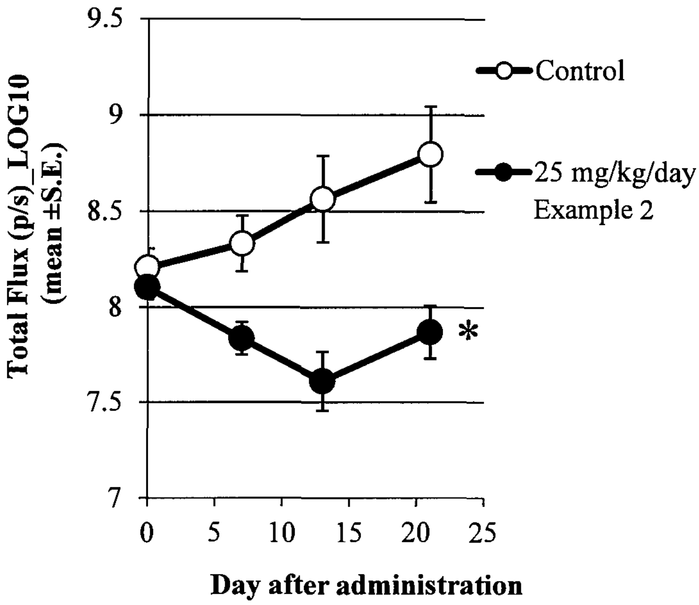


* : P=0.0077

[Figure 2]

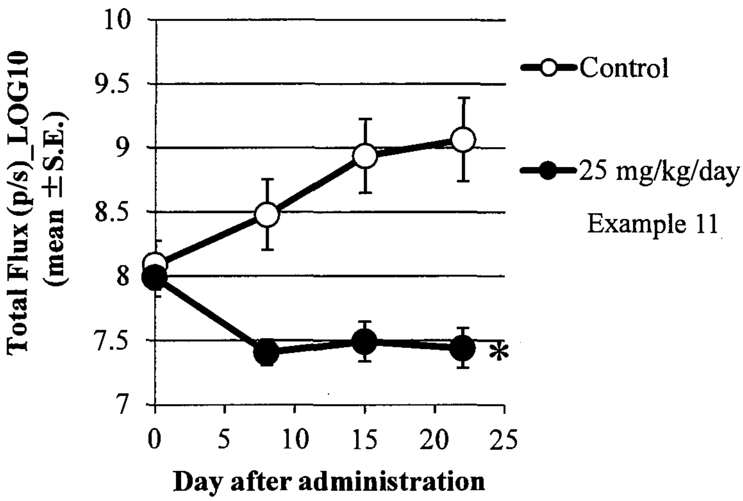


* : P=0.0007

[Figure 3]

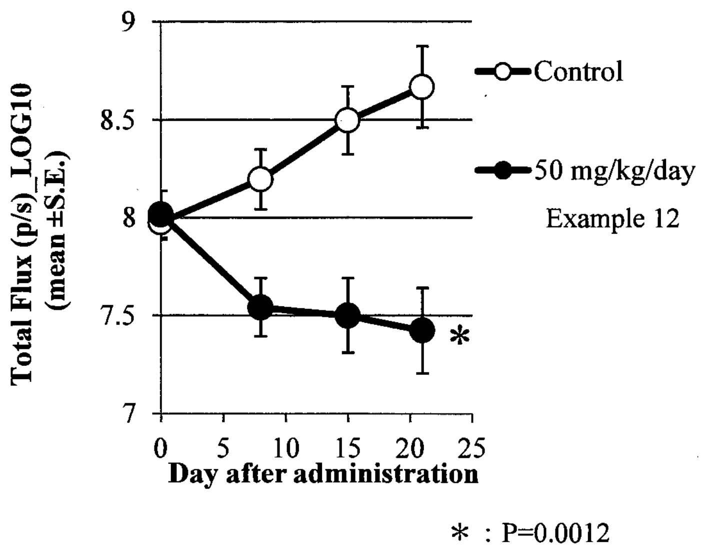


[Figure 4]

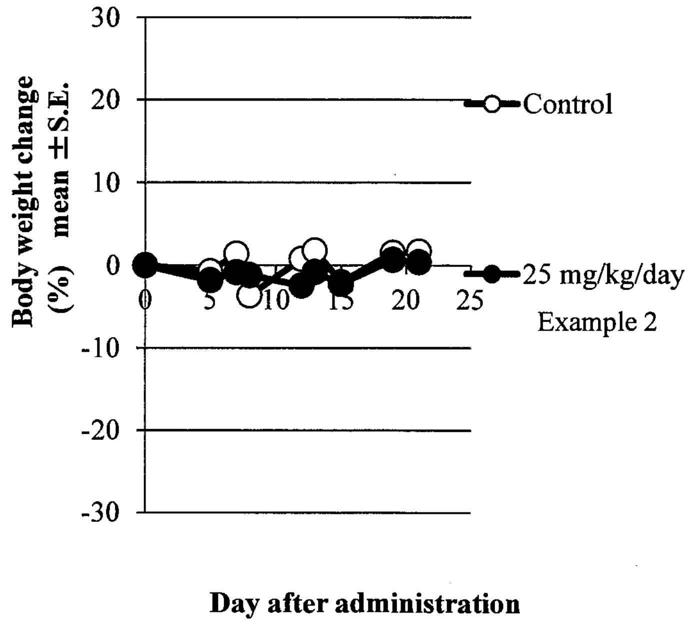

[Figure 5]

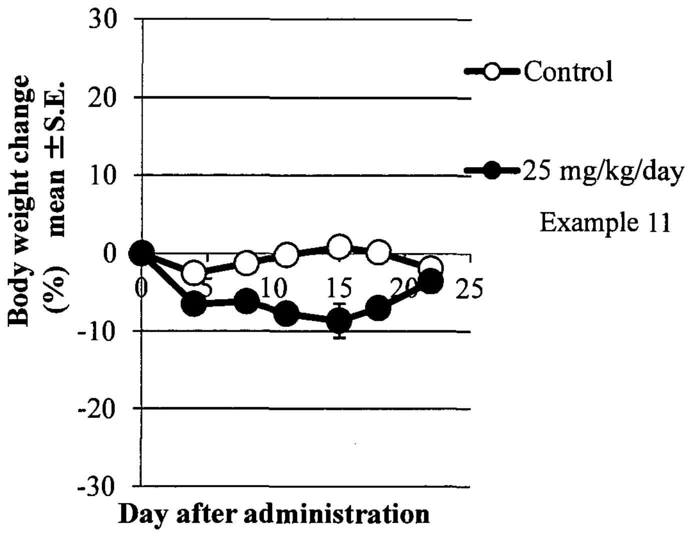


[Figure 6]

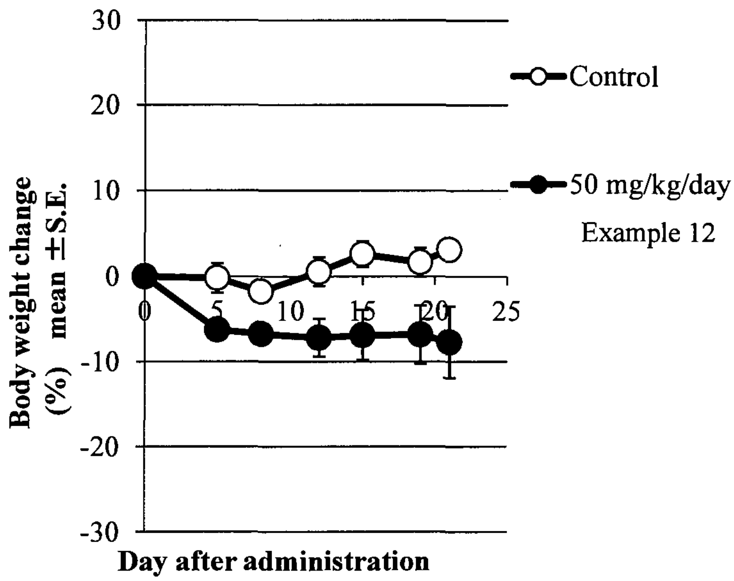

[Figure 7]

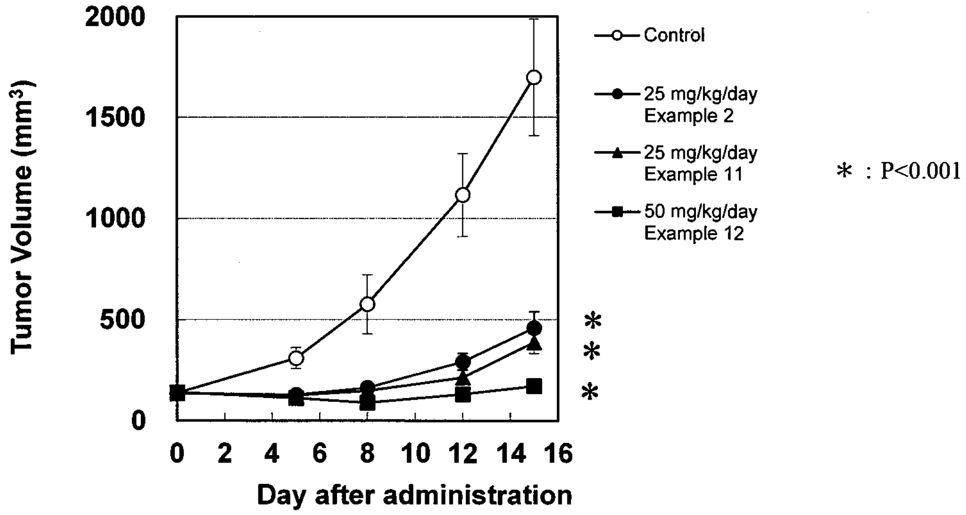


[Figure 8]

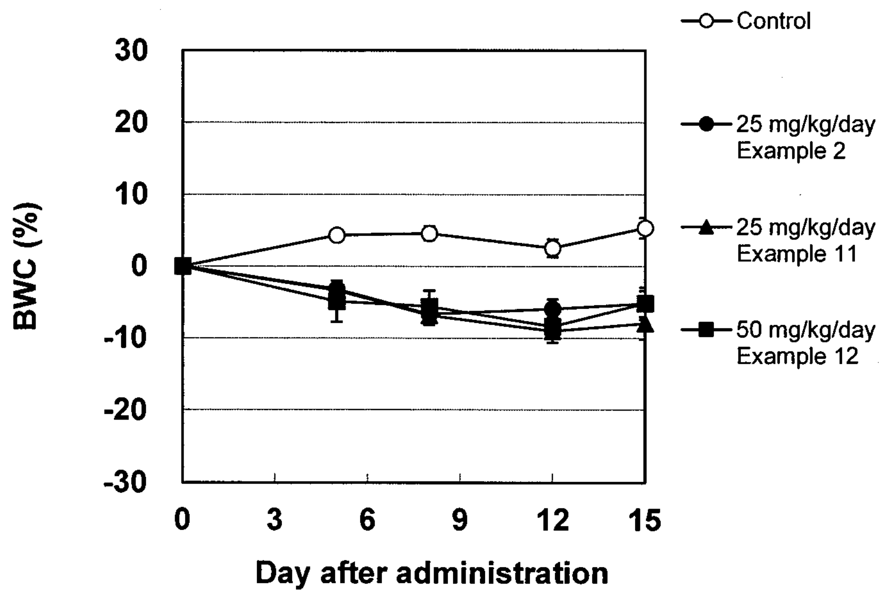

[Figure 9]

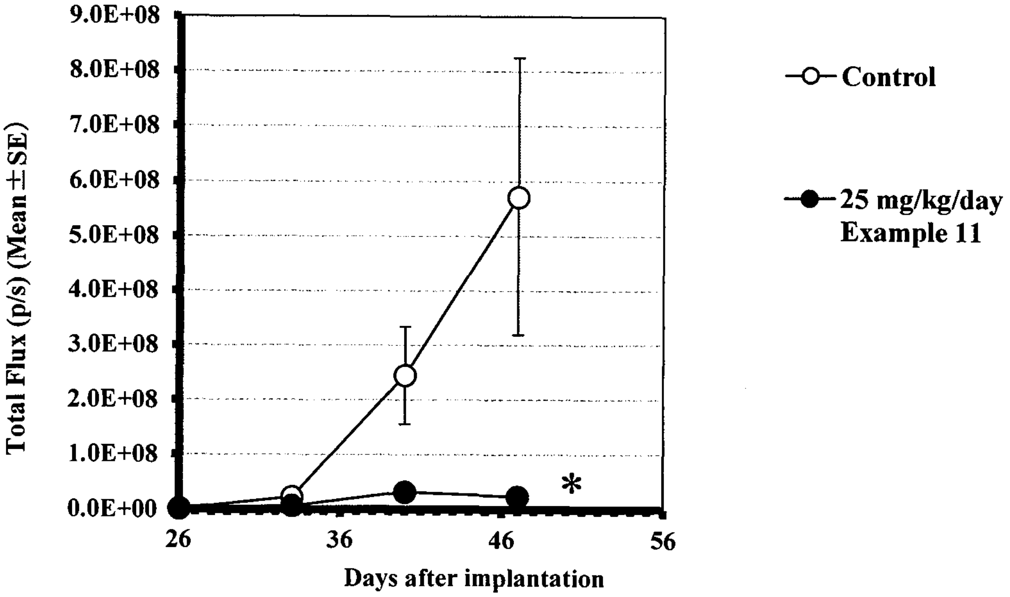


[Figure 10]

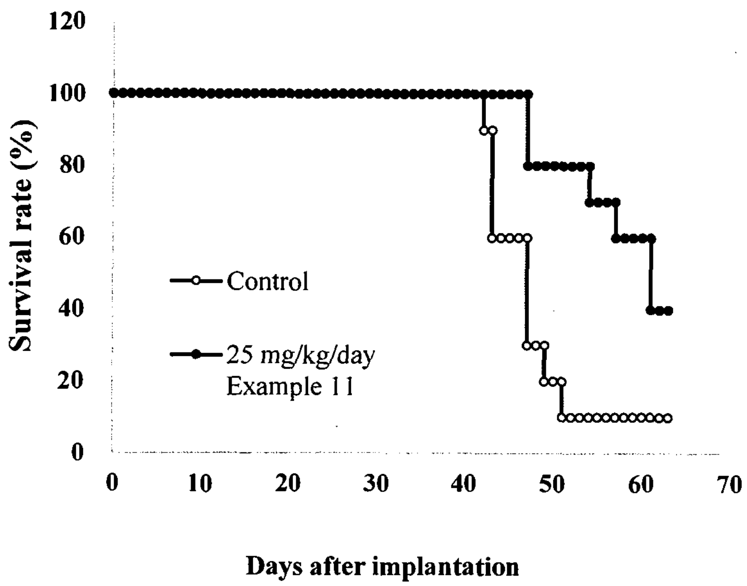

[Figure 11]

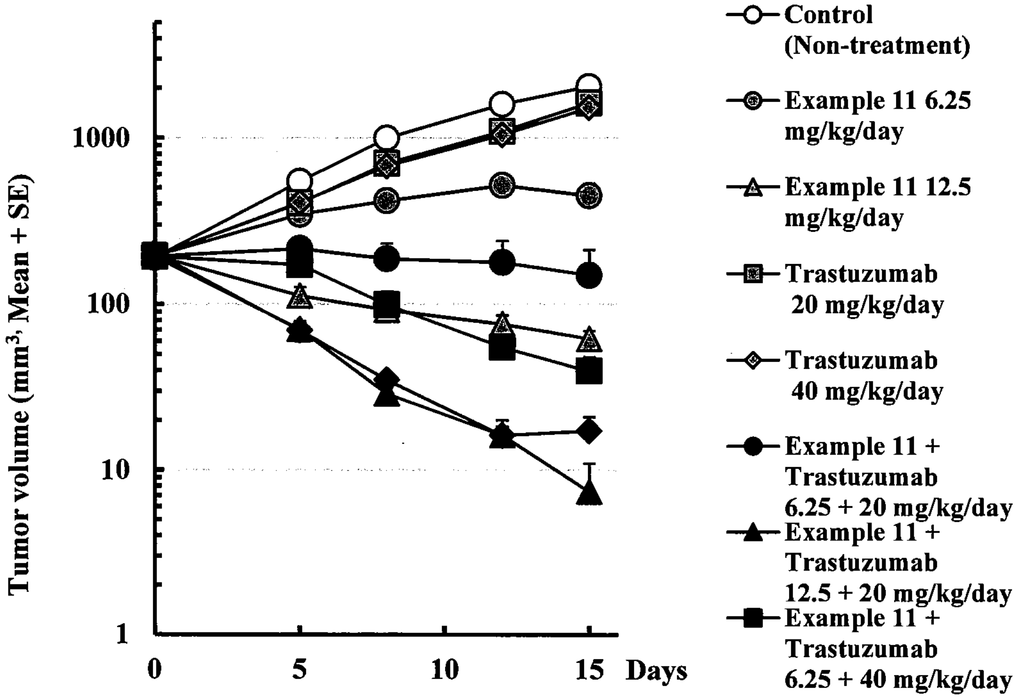


[Figure 12]

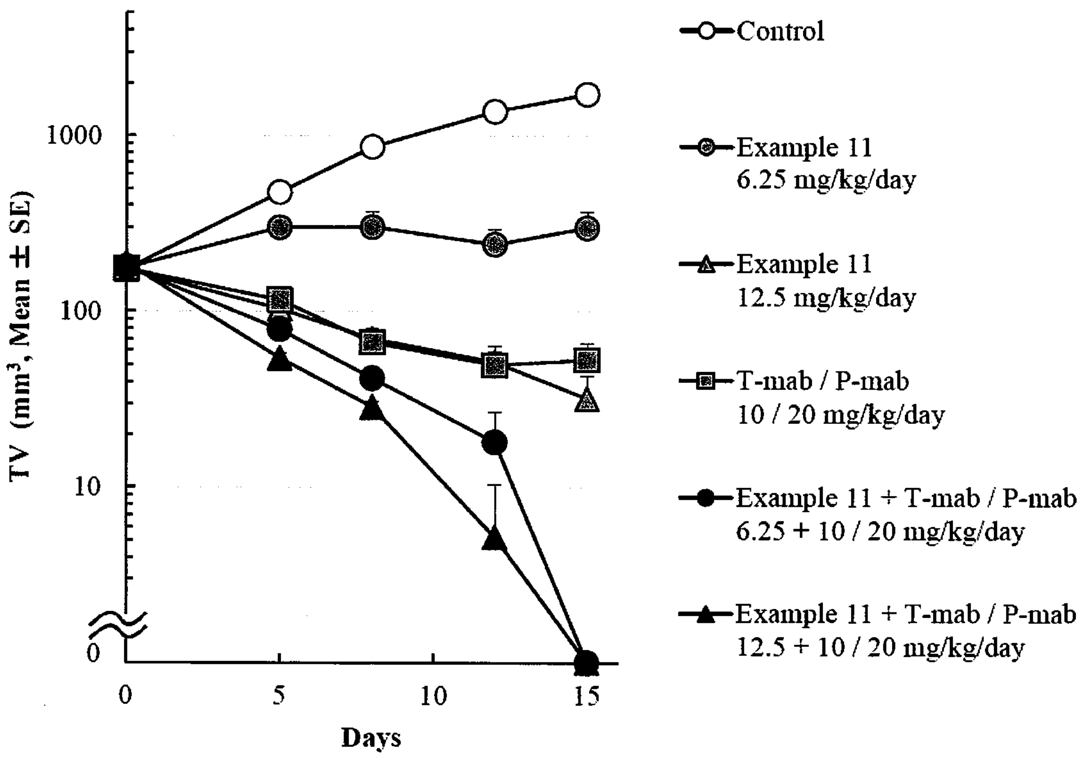

[Figure 13]

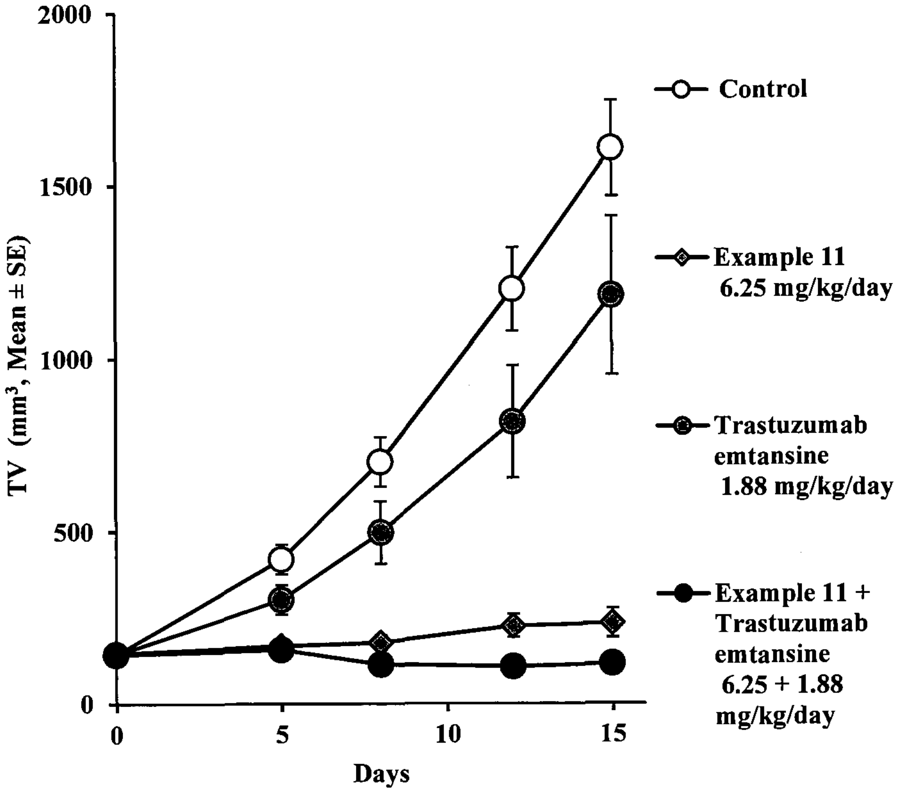


[Figure 14]

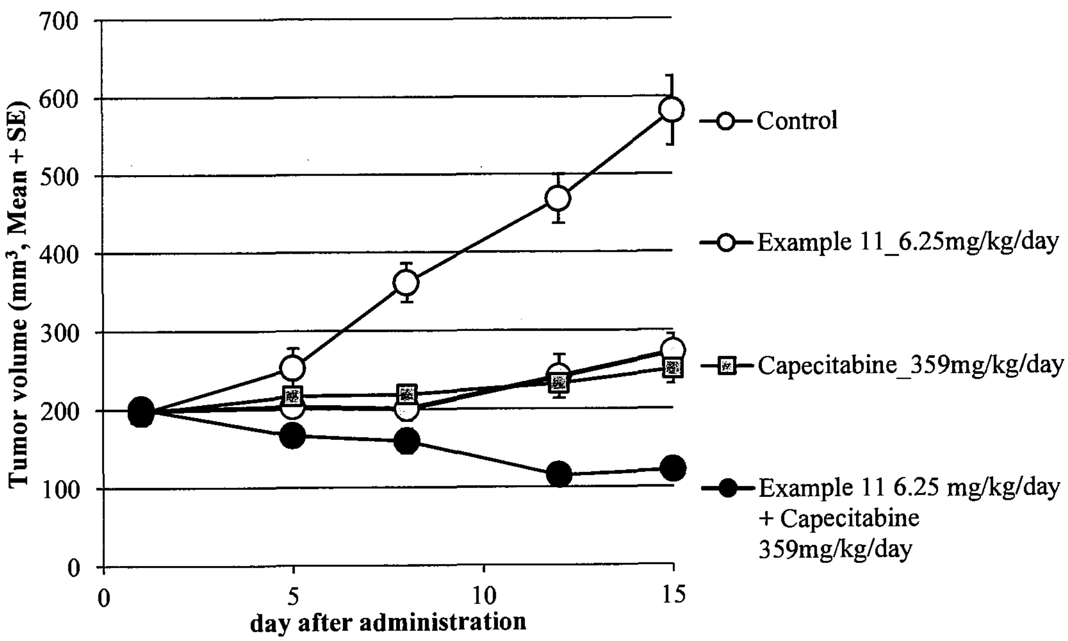

[Figure 15]

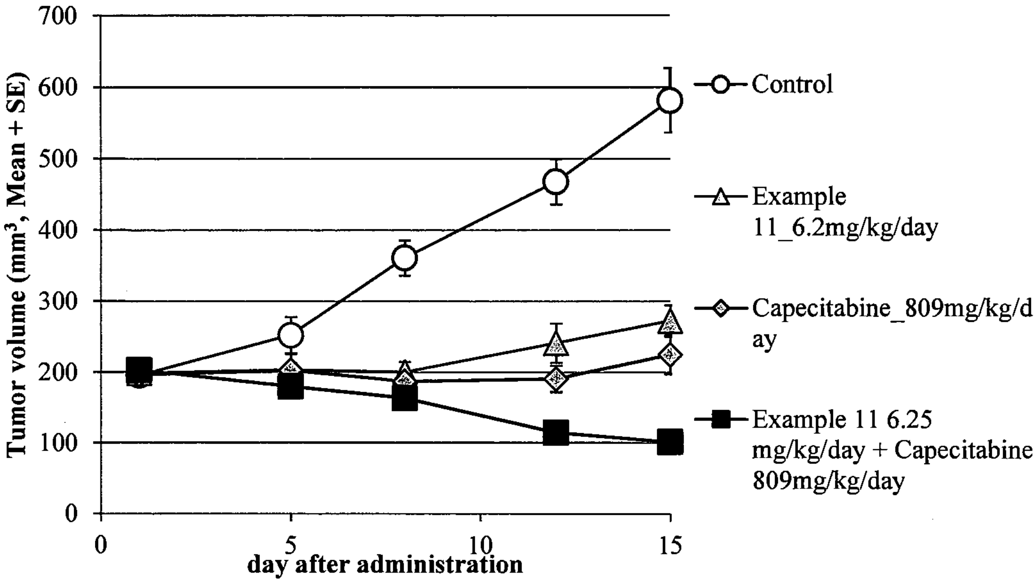
700
600
500
400
300
200
100
0
Tumor volume (mm³, Mean + SE)
0
5
10
15
day after administration
Control
Example 11_6.2mg/kg/day
Capecitabine_809mg/kg/day
Example 11 6.25 mg/kg/day + Capecitabine 809mg/kg/day

## INTERNATIONAL SEARCH REPORT

International application No. PCT/JP2021/026462

A. CLASSIFICATION OF SUBJECT MATTER
Int. Cl. A61K31/519(2006.01)i, A61P35/00(2006.01)i, A61P43/00(2006.01)i, C12N15/113(2010.01)i
FI: A61K31/519 ZNA, A61P43/00 121, A61P35/00, C12N15/113 130Z
According to International Patent Classification (IPC) or to both national classification and IPC

B. FIELDS SEARCHED
Minimum documentation searched (classification system followed by classification symbols)
Int. Cl. A61K31/519, A61P35/00, A61P43/00, C12N15/113

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| A | WO 2017/038838 A1 (TAIHO PHARMACEUTICAL CO., LTD.) 09 March 2017 (2017-03-09), entire text, all drawings | 1-19 |
| A | WO 2010/065898 A2 (PRINCIPIA BIOPHARMA INC.) 10 June 2010 (2010-06-10), entire text, all drawings | 1-19 |
| A | JP 2019-514869 A (THE REGENTS OF THE UNIVERSITY OF CALIFORNIA) 06 June 2019 (2019-06-06), entire text, all drawings | 1-19 |
| A | JP 2009-518434 A (ABBOTT LABORATORIES) 07 May 2009 (2009-05-07), entire text, all drawings | 1-19 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11.08.2021 | 24.08.2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2021/026462

C (Continuation). DOCUMENTS CONSIDERED TO BE RELEVANT

| Category* | Citation of document, with indication, where appropriate, of the relevant passages | Relevant to claim No. |
|---|---|---|
| A | WO 2013/108809 A1 (TAIHO PHARMACEUTICAL CO., LTD.) 25 July 2013 (2013-07-25), entire text, all drawings | 1-19 |
| A | WO 2015/022926 A1 (TAIHO PHARMACEUTICAL CO., LTD.) 19 February 2015 (2015-02-19), entire text, all drawings | 1-19 |
| P, A | WO 2020/145374 A1 (TAIHO PHARMACEUTICAL CO., LTD.) 16 July 2020 (2020-07-16), entire text, all drawings | 1-19 |

Form PCT/ISA/210 (continuation of second sheet) (January 2015)

**INTERNATIONAL SEARCH REPORT**
Information on patent family members

International application No.
PCT/JP2021/026462

| Patent Documents referred to in the Report | Publication Date | Patent Family | Publication Date |
|---|---|---|---|
| WO 2017/038838 A1 | 09.03.2017 | US 2017/0217970 A1<br>entire document<br>EP 3345907 A1<br>KR 10-2018-0041757 A<br>CN 108349981 A | |
| WO 2010/065898 A2 | 10.06.2010 | US 2010/0144705 A1<br>entire document | |
| JP 2019-514869 A | 06.06.2019 | US 2019/0119284 A1<br>entire document<br>EP 3445768 A1<br>CN 109952306 A | |
| JP 2009-518434 A | 07.05.2009 | US 2007/0135387 A1<br>entire document<br>EP 1968979 A1<br>CN 101336244 A | |
| WO 2013/108809 A1 | 25.07.2013 | US 2014/0343035 A1<br>entire document<br>EP 2657233 A1<br>KR 10-2014-0080551 A<br>CN 103958512 A | |
| WO 2015/022926 A1 | 19.02.2015 | US 2016/0115168 A1<br>entire document<br>EP 2947086 A1<br>CN 105452257 A<br>KR 10-2016-0038891 A | |
| WO 2020/145374 A1 | 16.07.2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017146116 A
- WO 2017038838 A
- WO 2015035223 A
- US 20030162712 A
- US 6413932 B
- US 6727225 B
- US 20020042368 A
- US 5981245 A
- US 5728813 A
- US 5969110 A
- US 6596852 B
- US 6232447 B
- US 6057124 A
- WO 9633172 A
- WO 9627583 A
- EP 1004578 A
- WO 9807697 A
- WO 9803516 A
- WO 9834918 A
- WO 9834915 A
- WO 9833768 A
- WO 9830566 A
- EP 0606046 A
- EP 0931788 A
- WO 9005719 A
- WO 9952910 A
- WO 9952889 A
- WO 9929667 A
- WO 1999007675 A
- EP 1786785 A
- EP 1181017 A
- US 20090012085 A
- US 5863949 A
- US 5861510 A
- EP 0780386 A
- WO 06044453 A
- WO 06122806 A
- WO 09036082 A
- WO 09055730 A
- WO 05011700 A
- US 6656963 B
- WO 2012137870 A
- WO 9409010 A
- WO 9802441 A
- WO 0114387 A
- WO 05005434 A
- US 5258389 A
- WO 94090101 A
- WO 9205179 A
- US 5118677 A
- US 5118678 A
- US 5100883 A
- US 5151413 A
- US 5120842 A
- WO 93111130 A
- WO 9402136 A
- WO 9402485 A
- WO 9514023 A
- WO 9516691 A
- WO 9641807 A
- US 5256790 A
- WO 05016252 A
- WO 2019167000 A
- WO 2020022323 A
- WO 2021033153 A
- JP 2020121733 A


### Non-patent literature cited in the description


- *Cancer Treatment Reviews,* 2014, vol. 40, 770-780
- *Current Oncology,* 2018, vol. 25, S103-S114
- *Breast Cancer Research,* 2016, vol. 18 (1), 1-9
- *Journal of Clinical Oncology,* 2010, vol. 28, 3271-3277
- *Journal of Medicinal Chemistry,* 2016, vol. 59, 10030-10066
- *Journal of Medicinal Chemistry,* 2008, vol. 26, 2999-3005
- *Journal of Clinical Oncology,* 2008, vol. 26, 1993-1999
- *Journal of Clinical Oncology,* 2010, vol. 28, 1301-1307
- *Journal of Clinical Oncology,* 2016, vol. 34, 945-952
- *Proc Natl Acad Sci USA.,* 2009, vol. 106, 474-479
- *N. Engl. J. Med.,* 2001, vol. 344, 783-792
- *J. Natl. Cancer Inst.,* 2004, vol. 96, 739-749
- *Biologics.,* 2008, vol. 2, 61-65
- *Oncol. Rep.,* 2012, vol. 27, 1639-1645
- *Mol. Cancer Ther.,* 2010, vol. 9, 1198-1207
- *Nat. Rev. Cancer.,* 2006, vol. 6, 803-812

- *Nature Medicine.,* 2013, vol. 19, 1389-1400
- *Nat. Rev. Cancer.,* 2007, vol. 7, 169-181
- *Signal Transduct Target Ther.,* 2019, vol. 4 (5), 1-10
- *Lancet Oncol.,* 2015, vol. 16, 830-838
- *Neoplasia.,* 2015, vol. 17, 190-200
- *Clin Cancer Res.,* 2007, vol. 13, 4628-4631
- **T. W. GREENE ; P. G. M. WUTS.** Protective Groups in Organic Synthesis. John Wiley & Sons, 1999
- **T. W. GREENE ; P. G. M. WUTS.** Protective Groups in Organic Synthesis. John Wiley & Sons, 1981
- **NOBUO IZUMIYA et al.** Peptide Gosei no Kiso to Jikken (Principle of Peptide Synthesis and Experiments). Maruzen Co., Ltd, 1983
- **HIROKAWA SHOTEN.** Bunshi Sekkei (Molecular Designing). *Iyakuhin no Kaihatsu (Development of Pharmaceutical Products),* 1990, vol. 7, 163-198
- **MODJTAHEDI, H. et al.** *Br. J. Cancer,* 1993, vol. 67, 247-253
- **TERAMOTO, T. et al.** *Cancer,* 1996, vol. 77, 639-645
- **GOLDSTEIN et al.** *Clin. Cancer Res.,* 1995, vol. 1, 1311-1318
- **HUANG, S. M. et al.** *Cancer Res.,* 1999, vol. 59 (8), 1935-40
- **YANG, X. et al.** *Cancer Res.,* 1999, vol. 59, 1236-1243
- **BARNETT et al.** *Biochem. J.,* 2005, vol. 385 (2), 399-408
- **JIN et al.** *Br. J. Cancer,* 2004, vol. 91, 1808-12
- **SARKAR ; LI.** *J Nutr.,* 2004, vol. 134 (12), 3493S-3498S
- **DASMAHAPATRA et al.** *Clin. Cancer Res.,* 2004, vol. 10 (15), 5242-52
- **GILLS ; DENNIS.** *Expert. Opin. Investig. Drugs,* 2004, vol. 13, 787-97
- **YANG et al.** *Cancer Res.,* 2004, vol. 64, 4394-9
- *Nature medicine,* 2018, vol. 24, 638-646
- **XIE H et al.** *PLoS One,* 2011, vol. 6 (7), e21487
- *Adv. Enzyme Regul.,* 1984, vol. 22, 27-55
- *Pharmacol Rev.,* 2006, vol. 58 (3), 621-81